(19) Europäisches Patentamt / European Patent Office / Office européen des brevets

(11) EP 3 402 797 B1

(12) EUROPÄISCHE PATENTSCHRIFT

(45) Veröffentlichungstag und Bekanntmachung des Hinweises auf die Patenterteilung:
**19.02.2020 Patentblatt 2020/08**

(21) Anmeldenummer: **17700114.6**

(22) Anmeldetag: **05.01.2017**

(51) Int Cl.:
***C07D 487/04*** $^{(2006.01)}$ ***A01N 43/90*** $^{(2006.01)}$

(86) Internationale Anmeldenummer:
**PCT/EP2017/050181**

(87) Internationale Veröffentlichungsnummer:
**WO 2017/121674 (20.07.2017 Gazette 2017/29)**

# (54) HETEROCYCLEN-DERIVATE ALS SCHÄDLINGSBEKÄMPFUNGSMITTEL

HETEROCYCLE DERIVATIVES AS PESTICIDES

DERIVE D'HETEROCYCLES COMME PRODUIT DE LUTTE CONTRE LES PARASITES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **11.01.2016 EP 16150757**

(43) Veröffentlichungstag der Anmeldung:
**21.11.2018 Patentblatt 2018/47**

(73) Patentinhaber: **Bayer CropScience AG**
**40789 Monheim am Rhein (DE)**

(72) Erfinder:
- **WILCKE, David**
  **40219 Düsseldorf (DE)**
- **FISCHER, Rüdiger**
  **50259 Pulheim (DE)**
- **HAGER, Dominik**
  **40789 Monheim (DE)**
- **HOFFMEISTER, Laura**
  **40593 Düsseldorf (DE)**
- **KAUSCH-BUSIES, Nina**
  **51467 Bergisch Gladbach (DE)**
- **ILG, Kerstin**
  **50670 Köln (DE)**
- **WILLOT, Matthieu**
  **40215 Düsseldorf (DE)**
- **MOSRIN, Marc**
  **50935 Köln (DE)**
- **GÖRGENS, Ulrich**
  **40882 Ratingen (DE)**
- **PORTZ, Daniela**
  **52391 Vettweiß (DE)**
- **EILMUS, Sascha**
  **42799 Leichlingen (DE)**
- **TURBERG, Andreas**
  **42781 Haan (DE)**

(74) Vertreter: **BIP Patents**
**c/o Bayer Intellectual Property GmbH**
**Alfred-Nobel-Straße 10**
**40789 Monheim am Rhein (DE)**

(56) Entgegenhaltungen:
**WO-A1-2015/133603 US-A1- 2012 328 569**

- **YOSHIO IMAI ET AL: "Preparation and properties of poly(imide-oxoisoindolobenzothiadiazine dioxides)", JOURNAL OF POLYMER SCIENCE, POLYMER CHEMISTRY EDITION., Bd. 11, Nr. 10, 1. Oktober 1973 (1973-10-01), Seiten 2623-2632, XP055276706, US ISSN: 0360-6376, DOI: 10.1002/pol.1973.170111016**

EP 3 402 797 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft neue Heterocyclen-Derivate der Formel (I), deren Anwendung als Akarizide und/oder Insektizide zur Bekämpfung tierischer Schädlinge, vor allem von Arthropoden und insbesondere von Insekten und Spinnentieren und Verfahren und Zwischenprodukte zu ihrer Herstellung.

Heterocyclen-Derivate mit insektiziden Eigenschaften sind in der Literatur bereits beschrieben, z.B. in WO 2010/125985, WO 2012/074135, WO 2012/086848, WO 2013/018928, WO 2013/191113, WO 2014/142292, WO 2014/148451, WO 2015/000715, WO 2015/002211, WO 2015/121136, WO 2016/124563, WO 2016/124557, PCT/EP2016/068599, EP 15187145.6, WO 2015/133603, WO 2015/198859, WO 2015/002211, WO 2015/071180, WO 2015/091945, WO 2016/005263, WO 2015/198817, WO 2016/041819, WO 2016/039441, WO 2016/026848, WO 2016/023954, WO 2016/020286, WO 2016/046071.

Die gemäß den oben genannten Schriften bereits bekannten Wirkstoffe weisen aber in ihrer Anwendung teils Nachteile auf, sei es, dass sie nur eine geringe Anwendungsbreite aufweisen, sei es, dass sie keine zufriedenstellende insektizide oder akarizide Wirkung aufweisen.

Es wurden nun neue Heterocyclen-Derivate gefunden, welche gegenüber den bereits bekannten Verbindungen Vorteile aufweisen, z.B. seien bessere biologische oder ökologische Eigenschaften, breitere Anwendungsmethoden, eine bessere insektizide, akarizide Wirkung, sowie eine gute Verträglichkeit gegenüber Nutzpflanzen beispielhaft genannt. Die Heterocyclen-Derivate können in Kombination mit weiteren Mitteln zur Verbesserung der Wirksamkeit insbesondere gegen schwierig zu bekämpfende Insekten eingesetzt werden.

Gegenstand der vorliegenden Erfindung sind daher neue Verbindungen der Formel (I)

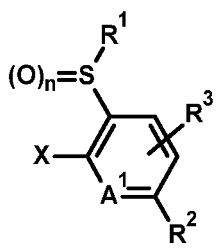

(I)

in welcher (Ausgestaltung 1)

$A^1$ für Stickstoff, $=N^+(O^-)-$ oder $=C(R^4)-$ steht,

$R^1$ für $(C_1-C_6)$Alkyl, $(C_1-C_6)$Halogenalkyl, $(C_1-C_6)$Cyanoalkyl, $(C_1-C_6)$Hydroxyalkyl, $(C_1-C_6)$Alkoxy-$(C_1-C_6)$alkyl, $(C_1-C_6)$Halogenalkoxy-$(C_1-C_6)$alkyl, $(C_2-C_6)$Alkenyl, $(C_2-C_6)$Alkenyloxy-$(C_1-C_6)$alkyl, $(C_2-C_6)$Halogenalkenyloxy-$(C_1-C_6)$alkyl, $(C_2-C_6)$Halogenalkenyl, $(C_2-C_6)$Cyanoalkenyl, $(C_2-C_6)$Alkinyl, $(C_2-C_6)$Alkinyloxy-$(C_1-C_6)$alkyl, $(C_2-C_6)$Halogenalkinyloxy-$(C_1-C_6)$alkyl, $(C_2-C_6)$Halogenalkinyl, $(C_2-C_6)$Cyanoalkinyl, $(C_3-C_8)$Cycloalkyl, $(C_3-C_8)$Cycloalkyl-$(C_3-C_8)$Cycloalkyl, $(C_1-C_6)$Alkyl-$(C_3-C_8)$Cycloalkyl, Halogen$(C_3-C_8)$cycloalkyl, Amino, $(C_1-C_6)$Alkylamino, Di-$(C_1-C_6)$alkyl-amino, $(C_3-C_8)$Cycloalkylamino, $(C_1-C_6)$Alkylcarbonyl-amino, $(C_1-C_6)$Alkylthio-$(C_1-C_6)$alkyl, $(C_1-C_6)$Halogenalkylthio-$(C_1-C_6)$alkyl, $(C_1-C_6)$Alkylsulfinyl-$(C_1-C_6)$alkyl, $(C_1-C_6)$Halogenalkylsulfinyl-$(C_1-C_6)$alkyl, $(C_1-C_6)$Alkylsulfonyl-$(C_1-C_6)$alkyl, $(C_1-C_6)$Halogenalkylsulfonyl-$(C_1-C_6)$alkyl, $(C_1-C_6)$Alkoxy-$(C_1-C_6)$alkylthio-$(C_1-C_6)$alkyl, $(C_1-C_6)$Alkoxy-$(C_1-C_6)$alkylsulfinyl-$(C_1-C_6)$alkyl, $(C_1-C_6)$Alkoxy-$(C_1-C_6)$alkylsulfonyl-$(C_1-C_6)$alkyl, $(C_1-C_6)$Alkylcarbonyl-$(C_1-C_6)$alkyl, $(C_1-C_6)$Halogenalkylcarbonyl-$(C_1-C_6)$alkyl, $(C_1-C_6)$Alkoxycarbonyl-$(C_1-C_6)$alkyl, $(C_1-C_6)$Halogenalkoxycarbonyl-$(C_1-C_6)$alkyl, $(C_1-C_6)$Alkylsulfonylamino, Aminosulfonyl-$(C_1-C_6)$alkyl, $(C_1-C_6)$Alkylaminosulfonyl-$(C_1-C_6)$alkyl, Di-$(C_1-C_6)$alkyl-aminosulfonyl-$(C_1-C_6)$alkyl, oder für jeweils gegebenenfalls einfach oder mehrfach, gleich oder verschieden durch Aryl, Hetaryl oder Heterocyclyl substituiertes $(C_1-C_6)$Alkyl, $(C_1-C_6)$Alkoxy, $(C_2-C_6)$Alkenyl, $(C_2-C_6)$Alkinyl, $(C_3-C_8)$Cycloalkyl steht, wobei Aryl, Hetaryl oder Heterocyclyl jeweils gegebenenfalls einfach oder mehrfach, gleich oder verschieden durch Halogen, Cyano, Nitro, Hydroxy, Amino, Carboxy, Carbamoyl, Aminosulfonyl, $(C_1-C_6)$Alkyl, $(C_3-C_6)$Cycloalkyl, $(C_1-C_6)$Alkoxy, $(C_1-C_6)$Halogenalkyl, $(C_1-C_6)$Halogenalkoxy, $(C_1-C_6)$Alkylthio, $(C_1-C_6)$Alkylsulfinyl, $(C_1-C_6)$Alkylsulfonyl, $(C_1-C_6)$Alkylsulfimino, $(C_1-C_6)$Alkylsulfimino-$(C_1-C_6)$alkyl, $(C_1-C_6)$Alkylsulfimino-$(C_2-C_6)$alkylcarbonyl, $(C_1-C_6)$Alkylsulfoximino, $(C_1-C_6)$Alkylsulfoximino-$(C_1-C_6)$alkyl, $(C_1-C_6)$Alkylsulfoximino-$(C_2-C_6)$alkylcarbonyl, $(C_1-C_6)$Alkoxycarbonyl, $(C_1-C_6)$Alkylcarbonyl, $(C_3-C_6)$Trialkylsilyl oder Benzyl substituiert sein können, oder

$R^1$ für jeweils gegebenenfalls einfach oder mehrfach, gleich oder verschieden durch Halogen, Cyano, Nitro, Hydroxy, Amino, Carboxy, Carbamoyl, $(C_1-C_6)$Alkyl, $(C_3-C_8)$Cycloalkyl, $(C_1-C_6)$-Alkoxy, $(C_1-C_6)$Halogenalkyl, $(C_1-C_6)$Ha-

logenalkoxy, ($C_1$-$C_6$)Alkylthio, ($C_1$-$C_6$)Alkyl-sulfinyl, ($C_1$-$C_6$)Alkylsulfonyl, ($C_1$-$C_6$)Alkylsulfimino, ($C_1$-$C_6$)Alkylsulfimino-($C_1$-$C_6$)alkyl, ($C_1$-$C_6$)Alkylsulfimino-($C_2$-$C_6$)alkylcarbonyl, ($C_1$-$C_6$)Alkylsulfoximino, ($C_1$-$C_6$)Alkylsulfoximino-($C_1$-$C_6$)alkyl, ($C_1$-$C_6$)Alkylsulfoximino-($C_2$-$C_6$)alkylcarbonyl, ($C_1$-$C_6$)Alkoxycarbonyl, ($C_1$-$C_6$)Alkylcarbonyl, ($C_3$-$C_6$)Trialkylsilyl, (=O) (nur im Fall von Heterocyclyl) oder $(=O)_2$ (nur im Fall von Heterocyclyl) substituiertes Aryl, Hetaryl oder Heterocyclyl steht,

$R^2$ für ein teilweise gesättigtes oder gesättigtes heterozyklisches oder heteroaromatisches 8-, 9-, 10-, 11- oder 12-gliedriges annelliertes bicyclisches Ringsystem steht, wobei gegebenenfalls mindestens eine Carbonylgruppe enthalten sein kann und/oder wobei das Ringsystem gegebenenfalls einfach oder mehrfach, gleich oder verschieden substituiert sein kann, und wobei die Substituenten unabhängig voneinander ausgewählt sein können aus Cyano, Carboxyl, Halogen, Nitro, Acetyl, Hydroxy, Amino, SCN, Tri-($C_1$-$C_6$)alkylsilyl, ($C_1$-$C_6$)Alkyl, ($C_1$-$C_6$)Halogenalkyl, ($C_1$-$C_6$)Cyanoalkyl, ($C_1$-$C_6$)Hydroxyalkyl, Hydroxycarbonyl-($C_1$-$C_6$)-alkoxy, ($C_1$-$C_6$)Alkoxycarbonyl-($C_1$-$C_6$)alkyl, ($C_1$-$C_6$)Alkoxy-($C_1$-$C_6$)alkyl, ($C_2$-$C_6$)Alkenyl, ($C_2$-$C_6$)Halogenalkenyl, ($C_2$-$C_6$)Cyanoalkenyl, ($C_2$-$C_6$)Alkinyl, ($C_2$-$C_6$)Halogenalkinyl, ($C_2$-$C_6$)Cyanoalkinyl, ($C_3$-$C_6$)Cycloalkyl, ($C_3$-$C_6$)Cycloalkyl-($C_3$-$C_6$)cycloalkyl, ($C_1$-$C_4$)Alkyl-($C_3$-$C_6$)cycloalkyl ($C_1$-$C_6$)Alkoxy, ($C_1$-$C_6$)Halogenalkoxy, ($C_1$-$C_6$)Cyanoalkoxy, ($C_1$-$C_6$)Alkoxycarbonyl-($C_1$-$C_6$)alkoxy, ($C_1$-$C_6$)Alkoxy-($C_1$-$C_6$)alkoxy, ($C_1$-$C_6$)Alkoxyimino,-N=C(H)-O($C_1$-$C_6$)Alkyl, -C(H)=N-O($C_1$-$C_6$)Alkyl, ($C_1$-$C_6$)Halogenalkyl-($C_1$-$C_6$)alkoxyimino, ($C_1$-$C_6$)Alkylthio, ($C_1$-$C_6$)Halogenalkylthio, ($C_1$-$C_6$)Alkoxy-($C_1$-$C_6$)alkylthio, ($C_1$-$C_6$)Alkylthio-($C_1$-$C_6$)alkyl, ($C_1$-$C_6$)Alkylsulfinyl, ($C_1$-$C_6$)Halogenalkylsulfinyl, ($C_1$-$C_6$)Alkoxy-($C_1$-$C_6$)alkylsulfinyl, ($C_1$-$C_6$)Alkylsulfinyl-($C_1$-$C_6$)alkyl, ($C_1$-$C_6$)Alkylsulfonyl, ($C_1$-$C_6$)Halogenalkylsulfonyl, ($C_1$-$C_6$)Alkoxy-($C_1$-$C_6$)alkylsulfonyl, ($C_1$-$C_6$)Alkylsulfonyl-($C_1$-$C_6$)alkyl, ($C_1$-$C_6$)Alkylsulfonyloxy, ($C_1$-$C_6$)Alkylcarbonyl, ($C_1$-$C_6$)Halogenalkylcarbonyl, ($C_1$-$C_6$)Alkylcarbonyloxy, ($C_1$-$C_6$)Alkoxycarbonyl, ($C_1$-$C_6$)Halogenalkoxycarbonyl, Aminocarbonyl, ($C_1$-$C_6$)Alkylaminocarbonyl, Di-($C_1$-$C_6$)alkyl-aminocarbonyl, ($C_2$-$C_6$)Alkenylaminocarbonyl, Di-($C_2$-$C_6$)-alkenylaminocarbonyl, ($C_3$-$C_8$)Cycloalkylaminocarbonyl, ($C_1$-$C_6$)Alkylsulfonylamino, ($C_1$-$C_6$)Alkylamino, Di-($C_1$-$C_6$)Alkylamino, Aminosulfonyl, ($C_1$-$C_6$)Alkylaminosulfonyl, Di-($C_1$-$C_6$)alkylaminosulfonyl, ($C_1$-$C_6$)Alkylsulfoximino, Aminothiocarbonyl, ($C_1$-$C_6$)Alkylaminothiocarbonyl, Di-($C_1$-$C_6$)alkylaminothiocarbonyl, ($C_3$-$C_8$)Cycloalkylamino, ($C_1$-$C_6$)Alkylcarbonylamino,

$R^3$ für Wasserstoff, Cyano, Halogen, Nitro, Acetyl, Hydroxy, Amino, SCN, Tri-($C_1$-$C_6$)alkylsilyl, ($C_3$-$C_8$)Cycloalkyl, ($C_3$-$C_8$)Cycloalkyl-($C_3$-$C_8$)Cycloalkyl, ($C_1$-$C_6$)Alkyl-($C_3$-$C_8$)cycloalkyl, Halogen($C_3$-$C_8$)cycloalkyl, ($C_1$-$C_6$)Alkyl, ($C_1$-$C_6$)Halogenalkyl, ($C_1$-$C_6$)Cyanoalkyl, ($C_1$-$C_6$)Hydroxyalkyl, Hydroxycarbonyl-($C_1$-$C_6$)-alkoxy, ($C_1$-$C_6$)Alkoxycarbonyl-($C_1$-$C_6$)alkyl, ($C_1$-$C_6$)Alkoxy-($C_1$-$C_6$)alkyl, ($C_2$-$C_6$)Alkenyl, ($C_2$-$C_6$)Halogenalkenyl, ($C_2$-$C_6$)Cyanoalkenyl, ($C_2$-$C_6$)Alkinyl, ($C_2$-$C_6$)Halogenalkinyl, ($C_2$-$C_6$)Cyanoalkinyl, ($C_1$-$C_6$)Alkoxy, ($C_1$-$C_6$)Halogenalkoxy, ($C_1$-$C_6$)Cyanoalkoxy, ($C_1$-$C_6$)Alkoxycarbonyl-($C_1$-$C_6$)alkoxy, ($C_1$-$C_6$)Alkoxy-($C_1$-$C_6$)alkoxy, ($C_1$-$C_6$)Alkylhydroxyimino, ($C_1$-$C_6$)Alkoxyimino, ($C_1$-$C_6$)Alkyl-($C_1$-$C_6$)alkoxyimino, ($C_1$-$C_6$)Halogenalkyl-($C_1$-$C_6$)alkoxyimino, ($C_1$-$C_6$)Alkylthio, ($C_1$-$C_6$)Halogenalkylthio, ($C_1$-$C_6$)Alkoxy-($C_1$-$C_6$)alkylthio, ($C_1$-$C_6$)Alkylthio-($C_1$-$C_6$)alkyl, ($C_1$-$C_6$)Alkylsulfinyl, ($C_1$-$C_6$)Halogenalkylsulfinyl, ($C_1$-$C_6$)Alkoxy-($C_1$-$C_6$)alkylsulfinyl, ($C_1$-$C_6$)Alkylsulfinyl-($C_1$-$C_6$)alkyl, ($C_1$-$C_6$)Alkylsulfonyl, ($C_1$-$C_6$)Halogenalkylsulfonyl, ($C_1$-$C_6$)Alkoxy-($C_1$-$C_6$)alkylsulfonyl, ($C_1$-$C_6$)Alkylsulfonyl-($C_1$-$C_6$)alkyl, ($C_1$-$C_6$)Alkylsulfonyloxy, ($C_1$-$C_6$)Alkylcarbonyl, ($C_1$-$C_6$)Alkylthiocarbonyl, ($C_1$-$C_6$)Halogenalkylcarbonyl, ($C_1$-$C_6$)Alkylcarbonyloxy, ($C_1$-$C_6$)Alkoxycarbonyl, ($C_1$-$C_6$)Halogenalkoxycarbonyl, Aminocarbonyl, ($C_1$-$C_6$)Alkylaminocarbonyl, ($C_1$-$C_6$)Alkylaminothiocarbonyl, Di-($C_1$-$C_6$)alkyl-aminocarbonyl, Di-($C_1$-$C_6$)alkyl-aminothiocarbonyl, ($C_2$-$C_6$)Alkenylaminocarbonyl, Di-($C_2$-$C_6$)-alkenylaminocarbonyl, ($C_3$-$C_8$)Cycloalkylaminocarbonyl, ($C_1$-$C_6$)Alkylsulfonylamino, ($C_1$-$C_6$)Alkylamino, Di-($C_1$-$C_6$)Alkylamino, Aminosulfonyl, ($C_1$-$C_6$)Alkylaminosulfonyl, Di-($C_1$-$C_6$)alkyl-aminosulfonyl, ($C_1$-$C_6$)Alkylsulfoximino, Aminothiocarbonyl, ($C_1$-$C_6$)Alkylaminothiocarbonyl, Di-($C_1$-$C_6$)alkyl-aminothiocarbonyl, ($C_3$-$C_8$)Cycloalkylamino, NHCO-($C_1$-$C_6$)alkyl(($C_1$-$C_6$)Alkylcarbonylamino),
für jeweils gegebenenfalls einfach oder mehrfach, gleich oder verschieden substituiertes Aryl oder Hetaryl steht, wobei (im Fall von Hetaryl) gegebenenfalls mindestens eine Carbonylgruppe enthalten sein kann und/oder wobei als Substituenten jeweils in Frage kommen: Cyano, Carboxyl, Halogen, Nitro, Acetyl, Hydroxy, Amino, SCN, Tri-($C_1$-$C_6$)alkylsilyl, ($C_3$-$C_8$)Cycloalkyl, ($C_3$-$C_8$)Cycloalkyl-($C_3$-$C_8$)cycloalkyl, ($C_1$-$C_6$)Alkyl-($C_3$-$C_8$)cycloalkyl, Halogen($C_3$-$C_8$)cycloalkyl, ($C_1$-$C_6$)Alkyl, ($C_1$-$C_6$)Halogenalkyl, ($C_1$-$C_6$)Cyanoalkyl, ($C_1$-$C_6$)Hydroxyalkyl, Hydroxycarbonyl-($C_1$-$C_6$)-alkoxy, ($C_1$-$C_6$)Alkoxycarbonyl-($C_1$-$C_6$)alkyl, ($C_1$-$C_6$)Alkoxy-($C_1$-$C_6$)alkyl, ($C_2$-$C_6$)Alkenyl, ($C_2$-$C_6$)Halogenalkenyl, ($C_2$-$C_6$)Cyanoalkenyl, ($C_2$-$C_6$)Alkinyl, ($C_2$-$C_6$)Halogenalkinyl, ($C_2$-$C_6$)Cyanoalkinyl, ($C_1$-$C_6$)Alkoxy, ($C_1$-$C_6$)Halogenalkoxy, ($C_1$-$C_6$)Cyanoalkoxy, ($C_1$-$C_6$)Alkoxycarbonyl-($C_1$-$C_6$)alkoxy, ($C_1$-$C_6$)Alkoxy-($C_1$-$C_6$)alkoxy, ($C_1$-$C_6$)Alkylhydroxyimino, ($C_1$-$C_6$)Alkoxyimino, ($C_1$-$C_6$)Alkyl-($C_1$-$C_6$)alkoxyimino, ($C_1$-$C_6$)Halogenalkyl-($C_1$-$C_6$)alkoxyimino, ($C_1$-$C_6$)Alkylthio, ($C_1$-$C_6$)Halogenalkylthio, ($C_1$-$C_6$)Alkoxy-($C_1$-$C_6$)alkylthio, ($C_1$-$C_6$)Alkylthio-($C_1$-$C_6$)alkyl, ($C_1$-$C_6$)Alkylsulfinyl, ($C_1$-$C_6$)Halogenalkylsulfinyl, ($C_1$-$C_6$)Alkoxy-($C_1$-$C_6$)alkylsulfinyl, ($C_1$-$C_6$)Alkylsulfinyl-($C_1$-$C_6$)alkyl, ($C_1$-$C_6$)Alkylsulfonyl, ($C_1$-$C_6$)Halogenalkylsulfonyl, ($C_1$-$C_6$)Alkoxy-($C_1$-$C_6$)alkylsulfonyl, ($C_1$-$C_6$)Alkylsulfonyl-($C_1$-$C_6$)alkyl, ($C_1$-$C_6$)Alkylsulfonyloxy, ($C_1$-$C_6$)Alkylcarbonyl, ($C_1$-$C_6$)Halogenalkylcarbonyl, ($C_1$-$C_6$)Alkylcarbonyloxy, ($C_1$-$C_6$)Alkoxycarbonyl, ($C_1$-$C_6$)Halogenalkoxycarbonyl, Aminocarbonyl,

$(C_1-C_6)$Alkylaminocarbonyl, Di-$(C_1-C_6)$alkyl-aminocarbonyl, $(C_2-C_6)$Alkenylaminocarbonyl, Di-$(C_2-C_6)$-alkenylaminocarbonyl, $(C_3-C_8)$Cycloalkylaminocarbonyl, $(C_1-C_6)$Alkylsulfonylamino, $(C_1-C_6)$Alkylamino, Di-$(C_1-C_6)$Alkylamino, Aminosulfonyl, $(C_1-C_6)$Alkylaminosulfonyl, Di-$(C_1-C_6)$alkylaminosulfonyl, $(C_1-C_6)$Alkylsulfoximino, Aminothiocarbonyl, $(C_1-C_6)$Alkylaminothiocarbonyl, Di-$(C_1-C_6)$alkylaminothiocarbonyl, $(C_3-C_8)$Cycloalkylamino, $(C_1-C_6)$Alkylcarbonylamino,

$R^4$ für Wasserstoff, Cyano, Halogen, Nitro, Acetyl, Hydroxy, Amino, SCN, Tri-$(C_1-C_6)$alkylsilyl, $(C_3-C_8)$Cycloalkyl, $(C_3-C_8)$Cycloalkyl-$(C_3-C_8)$Cycloalkyl, $(C_1-C_6)$Alkyl-$(C_3-C_8)$cycloalkyl, Halogen$(C_3-C_8)$cycloalkyl, $(C_1-C_6)$Alkyl, $(C_1-C_6)$Halogenalkyl, $(C_1-C_6)$Cyanoalkyl, $(C_1-C_6)$Hydroxyalkyl, Hydroxycarbonyl-$(C_1-C_6)$-alkoxy, $(C_1-C_6)$Alkoxycarbonyl-$(C_1-C_6)$alkyl, $(C_1-C_6)$Alkoxy-$(C_1-C_6)$alkyl, $(C_2-C_6)$Alkenyl, $(C_2-C_6)$Halogenalkenyl, $(C_2-C_6)$Cyanoalkenyl, $(C_2-C_6)$Alkinyl, $(C_2-C_6)$Halogenalkinyl, $(C_2-C_6)$Cyanoalkinyl, $(C_1-C_6)$Alkoxy, $(C_1-C_6)$Halogenalkoxy, $(C_1-C_6)$Cyanoalkoxy, $(C_1-C_6)$Alkoxycarbonyl-$(C_1-C_6)$alkoxy, $(C_1-C_6)$Alkoxy-$(C_1-C_6)$alkoxy, $(C_1-C_6)$Alkylhydroxyimino, $(C_1-C_6)$Alkoxyimino, $(C_1-C_6)$Alkyl-$(C_1-C_6)$alkoxyimino, $(C_1-C_6)$Halogenalkyl-$(C_1-C_6)$alkoxyimino, $(C_1-C_6)$Alkylthio, $(C_1-C_6)$Halogenalkylthio, $(C_1-C_6)$Alkoxy-$(C_1-C_6)$alkylthio, $(C_1-C_6)$Alkylthio-$(C_1-C_6)$alkyl, $(C_1-C_6)$Alkylsulfinyl, $(C_1-C_6)$Halogenalkylsulfinyl, $(C_1-C_6)$Alkoxy-$(C_1-C_6)$alkylsulfinyl, $(C_1-C_6)$Alkylsulfinyl-$(C_1-C_6)$alkyl, $(C_1-C_6)$Alkylsulfonyl, $(C_1-C_6)$Halogenalkylsulfonyl, $(C_1-C_6)$Alkoxy-$(C_1-C_6)$alkylsulfonyl, $(C_1-C_6)$Alkylsulfonyl-$(C_1-C_6)$alkyl, $(C_1-C_6)$Alkylsulfonyloxy, $(C_1-C_6)$Alkylcarbonyl, $(C_1-C_6)$Alkylthiocarbonyl, $(C_1-C_6)$Halogenalkylcarbonyl, $(C_1-C_6)$Alkylcarbonyloxy, $(C_1-C_6)$Alkoxycarbonyl, $(C_1-C_6)$Halogenalkoxycarbonyl, Aminocarbonyl, $(C_1-C_6)$Alkylaminocarbonyl, $(C_1-C_6)$Alkylaminothiocarbonyl, Di-$(C_1-C_6)$alkyl-aminocarbonyl, Di-$(C_1-C_6)$alkyl-aminothiocarbonyl, $(C_2-C_6)$Alkenylaminocarbonyl, Di-$(C_2-C_6)$-alkenylaminocarbonyl, $(C_3-C_8)$Cycloalkylaminocarbonyl, $(C_1-C_6)$Alkylsulfonylamino, $(C_1-C_6)$Alkylamino, Di-$(C_1-C_6)$Alkylamino, Aminosulfonyl, $(C_1-C_6)$Alkylaminosulfonyl, Di-$(C_1-C_6)$alkyl-aminosulfonyl, $(C_1-C_6)$Alkylsulfoximino, Aminothiocarbonyl, $(C_1-C_6)$Alkylaminothiocarbonyl, Di-$(C_1-C_6)$alkyl-aminothiocarbonyl, $(C_3-C_8)$Cycloalkylamino oder -NHCO-$(C_1-C_6)$alkyl ($(C_1-C_6)$Alkylcarbonylamino) steht,

X für ein teilweise gesättigtes oder gesättigtes heterozyklisches oder heteroaromatisches 8-, 9-, 10-, 11- oder 12-gliedriges annelliertes bicyclisches oder tricyclisches Ringsystem steht, wobei gegebenenfalls mindestens eine Carbonylgruppe enthalten sein kann und/oder wobei das Ringsystem gegebenenfalls einfach oder mehrfach, gleich oder verschieden substituiert ist, und wobei die Substituenten unabhängig voneinander ausgewählt sein können aus Cyano, Halogen, Nitro, Acetyl, Hydroxy, Amino, SCN, Tri-$(C_1-C_6)$alkylsilyl, $(C_3-C_8)$Cycloalkyl, $(C_3-C_8)$Cycloalkyl-$(C_3-C_8)$Cycloalkyl, $(C_1-C_6)$Alkyl-$(C_3-C_8)$cycloalkyl, Halogen$(C_3-C_8)$cycloalkyl, $(C_1-C_6)$Alkyl, $(C_1-C_6)$Halogenalkyl, $(C_1-C_6)$Cyanoalkyl, $(C_1-C_6)$Hydroxyalkyl, Hydroxycarbonyl-$(C_1-C_6)$-alkoxy, $(C_1-C_6)$Alkoxycarbonyl-$(C_1-C_6)$alkyl, $(C_1-C_6)$Alkoxy-$(C_1-C_6)$alkyl, $(C_2-C_6)$Alkenyl, $(C_2-C_6)$Halogenalkenyl, $(C_2-C_6)$Cyanoalkenyl, $(C_2-C_6)$Alkinyl, $(C_2-C_6)$Alkinyloxy-$(C_1-C_4)$alkyl, $(C_2-C_6)$Halogenalkinyl, $(C_2-C_6)$Cyanoalkinyl, $(C_1-C_6)$Alkoxy, $(C_1-C_6)$Halogenalkoxy, $(C_1-C_6)$Halogenalkoxy-$(C_1-C_6)$alkyl, $(C_2-C_6)$Alkenyloxy-$(C_1-C_6)$alkyl, $(C_2-C_6)$Halogenalkenyloxy-$(C_1-C_6)$alkyl, $(C_1-C_6)$Cyanoalkoxy, $(C_1-C_6)$Alkoxycarbonyl-$(C_1-C_6)$alkoxy, $(C_1-C_6)$Alkoxy-$(C_1-C_6)$alkoxy, $(C_1-C_6)$Alkylhydroxyimino, $(C_1-C_6)$Alkoxyimino, $(C_1-C_6)$Alkyl-$(C_1-C_6)$alkoxyimino, $(C_1-C_6)$Halogenalkyl-$(C_1-C_6)$alkoxyimino, $(C_1-C_6)$Alkylthio, $(C_1-C_6)$Halogenalkylthio, $(C_1-C_6)$Alkoxy-$(C_1-C_6)$alkylthio, $(C_1-C_6)$Alkylthio-$(C_1-C_6)$alkyl, $(C_1-C_6)$Alkylsulfinyl, $(C_1-C_6)$Halogenalkylsulfinyl, $(C_1-C_6)$Alkoxy-$(C_1-C_6)$alkylsulfinyl, $(C_1-C_6)$Alkylsulfinyl-$(C_1-C_6)$alkyl, $(C_1-C_6)$Alkylsulfonyl, $(C_1-C_6)$Halogenalkylsulfonyl, $(C_1-C_6)$Alkoxy-$(C_1-C_6)$alkylsulfonyl, $(C_1-C_6)$Alkylsulfonyl-$(C_1-C_6)$alkyl, $(C_1-C_6)$Alkylsulfonyloxy, $(C_1-C_6)$Alkylcarbonyl, $(C_1-C_6)$Alkylcarbonyl-$(C_1-C_6)$alkyl, $(C_1-C_6)$Alkylthiocarbonyl, $(C_1-C_6)$Halogenalkylcarbonyl, $(C_1-C_6)$Alkylcarbonyloxy, $(C_1-C_6)$Alkoxycarbonyl, $(C_1-C_6)$Halogenalkoxycarbonyl, Aminocarbonyl, $(C_1-C_6)$Alkylaminocarbonyl, $(C_1-C_6)$Alkylaminothiocarbonyl, Di-$(C_1-C_6)$alkyl-aminocarbonyl, Di-$(C_1-C_6)$alkyl-aminothiocarbonyl, $(C_2-C_6)$Alkenylaminocarbonyl, Di-$(C_2-C_6)$-alkenylaminocarbonyl, $(C_3-C_8)$Cycloalkylaminocarbonyl, $(C_1-C_6)$Alkylsulfonylamino, $(C_1-C_6)$Alkylamino, Di-$(C_1-C_6)$Alkylamino, Aminosulfonyl, $(C_1-C_6)$Alkylaminosulfonyl, Di-$(C_1-C_6)$alkyl-aminosulfonyl, $(C_1-C_6)$Alkylsulfoximino, Aminothiocarbonyl, $(C_1-C_6)$Alkylaminothiocarbonyl, Di-$(C_1-C_6)$alkyl-aminothiocarbonyl, $(C_3-C_8)$Cycloalkylamino, NHCO-$(C_1-C_6)$alkyl ($(C_1-C_6)$Alkylcarbonylamino),
oder wobei die Substituenten unabhängig voneinander ausgewählt sein können aus Phenyl oder einem 5- oder 6-gliedrigen heteroaromatischen Ring, wobei Phenyl oder der Ring gegebenenfalls einfach oder mehrfach, gleich oder verschieden durch $C_1$-$C_6$-Alkyl, $C_2$-$C_6$-Alkenyl, $C_2$-$C_6$-Alkinyl, $C_3$-$C_6$-Cycloalkyl, $C_1$-$C_6$-Haloalkyl, $C_2$-$C_6$-Haloalkenyl, $C_2$-$C_6$-Haloalkinyl, $C_3$-$C_6$-Halocycloalkyl, Halogen, CN, $NO_2$, $C_1$-$C_4$-Alkoxy, $C_1$-$C_4$-Haloalkoxy substituiert sein können,

n für 0, 1 oder 2 steht, ausgenommen

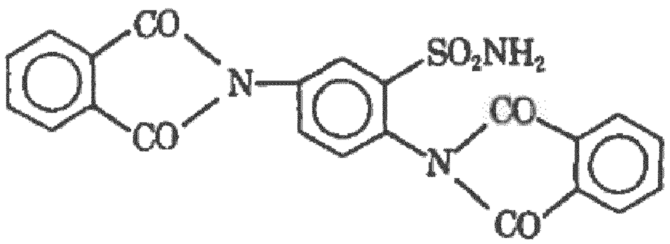

.

Weiterhin wurde gefunden, dass die Verbindungen der Formel (I) eine sehr gute Wirksamkeit als Schädlingsbekämpfungsmittel, vorzugsweise als Insektizide und/oder Akarizide aufweisen, darüber hinaus in der Regel insbesondere gegenüber Kulturpflanzen sehr gut pflanzenverträglich sind.

Die erfindungsgemäßen Verbindungen sind durch die Formel (I) allgemein definiert. Bevorzugte Substituenten bzw. Bereiche der in der oben und nachstehend erwähnten Formeln aufgeführten Reste werden im Folgenden erläutert:

Ausgestaltung 2

$A^1$ steht bevorzugt für Stickstoff, $=N^+(O^-)-$ oder $=C(R^4)-$,

$R^1$ steht bevorzugt für $(C_1-C_4)$Alkyl, $(C_1-C_4)$Hydroxyalkyl, $(C_1-C_4)$Halogenalkyl, $(C_1-C_4)$Cyanoalkyl, $(C_1-C_4)$Alkoxy-$(C_1-C_4)$alkyl, $(C_1-C_4)$Halogenalkoxy-$(C_1-C_4)$alkyl, $(C_2-C_4)$Alkenyl, $(C_2-C_4)$Alkenyloxy-$(C_1-C_4)$alkyl, $(C_2-C_4)$Halogenalkenyloxy-$(C_1-C_4)$alkyl, $(C_2-C_4)$Halogenalkenyl, $(C_2-C_4)$Cyanoalkenyl, $(C_2-C_4)$Alkinyl, $(C_2-C_4)$Alkinyloxy-$(C_1-C_4)$alkyl, $(C_2-C_4)$Halogenalkinyloxy-$(C_1-C_4)$alkyl, $(C_2-C_4)$Halogenalkinyl, $(C_2-C_4)$Cyanoalkinyl, $(C_3-C_6)$Cycloalkyl, $(C_3-C_6)$Cycloalkyl$(C_3-C_6)$cycloalkyl, $(C_1-C_4)$Alkyl-$(C_3-C_6)$cycloalkyl, Halogen$(C_3-C_6)$cycloalkyl, $(C_1-C_4)$Alkylamino, Di-$(C_1-C_4)$alkyl-amino, $(C_3-C_6)$Cycloalkylamino, $(C_1-C_4)$Alkylcarbonyl-amino, $(C_1-C_4)$Alkylthio-$(C_1-C_4)$alkyl, $(C_1-C_4)$Halogenalkylthio-$(C_1-C_4)$alkyl, $(C_1-C_4)$Alkylsulfinyl-$(C_1-C_4)$alkyl, $(C_1-C_4)$Halogenalkylsulfinyl-$(C_1-C_4)$alkyl, $(C_1-C_4)$Alkylsulfonyl-$(C_1-C_4)$alkyl, $(C_1-C_4)$Alkylcarbonyl-$(C_1-C_4)$alkyl, $(C_1-C_4)$Halogenalkylcarbonyl-$(C_1-C_4)$alkyl, $(C_1-C_4)$ Alkylsulfonylamino,
oder für jeweils gegebenenfalls einfach oder zweifach, gleich oder verschieden durch Aryl, Hetaryl oder Heterocyclyl substituiertes $(C_1-C_4)$Alkyl, $(C_1-C_4)$Alkoxy, $(C_2-C_4)$Alkenyl, $(C_2-C_4)$Alkinyl, $(C_3-C_6)$Cycloalkyl, wobei Aryl, Hetaryl oder Heterocyclyl jeweils gegebenenfalls einfach oder zweifach gleich oder verschieden durch Halogen, Cyano, Carbamoyl, Aminosulfonyl, $(C_1-C_4)$Alkyl, $(C_3-C_4)$Cycloalkyl, $(C_1-C_4)$Alkoxy, $(C_1-C_4)$Halogenalkyl, $(C_1-C_4)$Halogenalkoxy, $(C_1-C_4)$Alkylthio, $(C_1-C_4)$Alkylsulfinyl, $(C_1-C_4)$Alkylsulfonyl, $(C_1-C_4)$Alkylsulfimino substituiert sein können, oder

$R^1$ steht bevorzugt für jeweils gegebenenfalls einfach oder zweifach, gleich oder verschieden durch Halogen, Cyano, Carbamoyl, $(C_1-C_4)$Alkyl, $(C_3-C_6)$Cycloalkyl, $(C_1-C_4)$-Alkoxy, $(C_1-C_4)$Halogenalkyl, $(C_1-C_4)$Halogenalkoxy, $(C_1-C_4)$Alkylthio, $(C_1-C_4)$Alkylsulfinyl, $(C_1-C_4)$Alkylsulfonyl, $(C_1-C_4)$Alkylsulfimino, $(C_1-C_4)$Alkylsulfoximino, $(C_1-C_4)$Alkylcarbonyl, $(C_3-C_4)$Trialkylsilyl, (=O) (nur im Fall von Heterocyclyl) oder $(=O)_2$ (nur im Fall von Heterocyclyl) substituiertes Aryl, Hetaryl oder Heterocyclyl,

$R^2$ steht bevorzugt für ein teilweise gesättigtes oder gesättigtes heterozyklisches oder heteroaromatisches 8-, 9- oder 10-gliedriges annelliertes bicyclisches Ringsystem, wobei gegebenenfalls mindestens eine Carbonylgruppe enthalten sein kann, und wobei das Ringsystem gegebenenfalls einfach oder mehrfach, gleich oder verschieden substituiert sein kann wobei die Substituenten unabhängig voneinander ausgewählt sein können aus Cyano, Halogen, Nitro, Acetyl, Amino, $(C_1-C_4)$Alkyl, $(C_1-C_4)$Halogenalkyl, $(C_1-C_4)$Cyanoalkyl, $(C_1-C_4)$Hydroxyalkyl, $(C_1-C_4)$Alkoxy-$(C_1-C_4)$alkyl, $(C_2-C_4)$Alkenyl, $(C_2-C_4)$Halogenalkenyl, $(C_2-C_4)$Cyanoalkenyl, $(C_2-C_4)$Alkinyl, $(C_2-C_4)$Halogenalkinyl, $(C_2-C_4)$Cyanoalkinyl, $(C_3-C_6)$Cycloalkyl, $(C_3-C_6)$Cycloalkyl-$(C_3-C_6)$cycloalkyl, $(C_1-C_4)$Alkyl-$(C_3-C_6)$cycloalkyl, $(C_1-C_4)$Alkoxy, $(C_1-C_4)$Halogenalkoxy, $(C_1-C_4)$Cyanoalkoxy, $(C_1-C_4)$Alkoxy-$(C_1-C_4)$alkoxy, $(C_1-C_4)$Alkoxyimino, $-N=C(H)-O(C_1-C_4)$Alkyl, $-C(H)=N-O(C_1-C_4)$Alkyl, $(C_1-C_4)$Halogenalkyl-$(C_1-C_4)$alkoxyimino, $(C_1-C_4)$Alkylthio, $(C_1-C_4)$Halogenalkylthio, $(C_1-C_4)$Alkylthio-$(C_1-C_4)$alkyl, $(C_1-C_4)$Alkylsulfinyl, $(C_1-C_4)$Halogenalkylsulfinyl, $(C_1-C_4)$Alkylsulfinyl-$(C_1-C_4)$alkyl, $(C_1-C_4)$Alkylsulfonyl, $(C_1-C_4)$Halogenalkylsulfonyl, $(C_1-C_4)$Alkylsulfonyl-$(C_1-C_4)$alkyl, $(C_1-C_4)$Alkylsulfonyloxy, $(C_1-C_4)$Alkylcarbonyl, $(C_1-C_4)$Halogenalkylcarbonyl, Aminocarbonyl, $(C_1-C_4)$Alkylaminocarbonyl, Di-$(C_1-C_4)$alkyl-aminocarbonyl, $(C_1-C_4)$Alkylsulfonylamino, $(C_1-C_4)$Alkylamino, Di-$(C_1-C_4)$Alkylamino, Aminosulfonyl, $(C_1-C_4)$Alkylaminosulfonyl, Di-$(C_1-C_4)$alkylaminosulfonyl, $(C_1-C_4)$Alkylcar-

bonylamino

$R^3$ steht bevorzugt für Wasserstoff, Cyano, Halogen, Nitro, Acetyl, Hydroxy, Amino, SCN, Tri-($C_1$-$C_4$)alkylsilyl, ($C_3$-$C_6$)Cycloalkyl, ($C_3$-$C_6$)Cycloalkyl-($C_3$-$C_6$)cycloalkyl, ($C_1$-$C_4$)Alkyl-($C_3$-$C_6$)cycloalkyl, Halogen($C_3$-$C_6$)cycloalkyl, ($C_1$-$C_4$)Alkyl, ($C_1$-$C_4$)Halogenalkyl, ($C_1$-$C_4$)Cyanoalkyl, ($C_1$-$C_4$)Hydroxyalkyl, ($C_1$-$C_4$)Alkoxy-($C_1$-$C_4$)alkyl, ($C_2$-$C_4$)Alkenyl, ($C_2$-$C_4$)Halogenalkenyl, ($C_2$-$C_4$)Cyanoalkenyl, ($C_2$-$C_4$)Alkinyl, ($C_2$-$C_4$)Halogenalkinyl, ($C_2$-$C_4$)Cyanoalkinyl, ($C_1$-$C_4$)Alkoxy, ($C_1$-$C_4$)Halogenalkoxy, ($C_1$-$C_4$)Cyanoalkoxy, ($C_1$-$C_4$)Alkoxy-($C_1$-$C_4$)alkoxy, ($C_1$-$C_4$)Alkylhydroxyimino, ($C_1$-$C_4$)Alkoxyimino, ($C_1$-$C_4$)Alkyl-($C_1$-$C_4$)alkoxyimino, ($C_1$-$C_4$)Halogenalkyl-($C_1$-$C_4$)alkoxyimino, ($C_1$-$C_4$)Alkylthio, ($C_1$-$C_4$)Halogenalkylthio, ($C_1$-$C_4$)Alkylthio-($C_1$-$C_4$)alkyl, ($C_1$-$C_4$)Alkylsulfinyl, ($C_1$-$C_4$)Halogenalkylsulfinyl, ($C_1$-$C_4$)Alkylsulfinyl-($C_1$-$C_4$)alkyl, ($C_1$-$C_4$)Alkylsulfonyl, ($C_1$-$C_4$)Halogenalkylsulfonyl, ($C_1$-$C_4$)Alkylsulfonyl-($C_1$-$C_4$)alkyl, ($C_1$-$C_4$)Alkylsulfonyloxy, ($C_1$-$C_4$)Alkylcarbonyl, ($C_1$-$C_4$)Halogenalkylcarbonyl, Aminocarbonyl, Aminothiocarbonyl, ($C_1$-$C_4$)Alkylaminocarbonyl, Di-($C_1$-$C_4$)alkyl-aminocarbonyl, ($C_1$-$C_4$)Alkylsulfonylamino, ($C_1$-$C_4$)Alkylamino, Di-($C_1$-$C_4$)Alkylamino, Aminosulfonyl, ($C_1$-$C_4$)Alkylaminosulfonyl, Di-($C_1$-$C_4$)alkyl-aminosulfonyl, Aminothiocarbonyl, NHCO-($C_1$-$C_4$)alkyl (($C_1$-$C_4$)Alkylcarbonylamino),
weiterhin bevorzugt für jeweils gegebenenfalls einfach oder zweifach, gleich oder verschieden substituiertes Phenyl oder Hetaryl, wobei (im Fall von Hetaryl) gegebenenfalls mindestens eine Carbonylgruppe enthalten sein kann und/oder wobei als Substituenten jeweils in Frage kommen: Cyano, Halogen, Nitro, Acetyl, Amino, ($C_3$-$C_6$)Cycloalkyl, ($C_3$-$C_6$)Cycloalkyl-($C_3$-$C_6$)cycloalkyl, ($C_1$-$C_4$)Alkyl-($C_3$-$C_6$)cycloalkyl, Halogen($C_3$-$C_6$)cycloalkyl, ($C_1$-$C_4$)Alkyl, ($C_1$-$C_4$)Halogenalkyl, ($C_1$-$C_4$)Cyanoalkyl, ($C_1$-$C_4$)Hydroxyalkyl, ($C_1$-$C_4$)Alkoxy-($C_1$-$C_4$)alkyl, ($C_2$-$C_4$)Alkenyl, ($C_2$-$C_4$)Halogenalkenyl, ($C_2$-$C_4$)Cyanoalkenyl, ($C_2$-$C_4$)Alkinyl, ($C_2$-$C_4$)Halogenalkinyl, ($C_2$-$C_4$)Cyanoalkinyl, ($C_1$-$C_4$)Alkoxy, ($C_1$-$C_4$)Halogenalkoxy, ($C_1$-$C_4$)Cyanoalkoxy, ($C_1$-$C_4$)Alkoxy-($C_1$-$C_4$)alkoxy, ($C_1$-$C_4$)Alkylhydroxyimino, ($C_1$-$C_4$)Alkoxyimino, ($C_1$-$C_4$)Alkyl-($C_1$-$C_4$)alkoxyimino, ($C_1$-$C_4$)Halogenalkyl-($C_1$-$C_4$)alkoxyimino, ($C_1$-$C_4$)Alkylthio, ($C_1$-$C_4$)Halogenalkylthio, ($C_1$-$C_4$)Alkylthio-($C_1$-$C_4$)alkyl, ($C_1$-$C_4$)Alkylsulfinyl, ($C_1$-$C_4$)Halogenalkylsulfinyl, ($C_1$-$C_4$)Alkylsulfinyl-($C_1$-$C_4$)alkyl, ($C_1$-$C_4$)Alkylsulfonyl, ($C_1$-$C_4$)Halogenalkylsulfonyl, ($C_1$-$C_4$)Alkylsulfonyl-($C_1$-$C_4$)alkyl, ($C_1$-$C_4$)Alkylsulfonyloxy, ($C_1$-$C_4$)Alkylcarbonyl, ($C_1$-$C_4$)Halogenalkylcarbonyl, Aminocarbonyl, ($C_1$-$C_4$)Alkylaminocarbonyl, Di-($C_1$-$C_4$)alkyl-aminocarbonyl, ($C_1$-$C_4$)Alkylsulfonylamino, ($C_1$-$C_4$)Alkylamino, Di-($C_1$-$C_4$)Alkylamino, Aminosulfonyl, ($C_1$-$C_4$)Alkylaminosulfonyl, Di-($C_1$-$C_4$)alkylaminosulfonyl, NHCO-($C_1$-$C_4$)alkyl (($C_1$-$C_4$)Alkylcarbonylamino),

$R^4$ steht bevorzugt für Wasserstoff, Cyano, Halogen, Nitro, Acetyl, Hydroxy, Amino, SCN, Tri-($C_1$-$C_4$)alkylsilyl, ($C_3$-$C_6$)Cycloalkyl, ($C_3$-$C_6$)Cycloalkyl-($C_3$-$C_6$)cycloalkyl, ($C_1$-$C_4$)Alkyl-($C_3$-$C_6$)cycloalkyl, Halogen($C_3$-$C_6$)cycloalkyl, ($C_1$-$C_4$)Alkyl, ($C_1$-$C_4$)Halogenalkyl, ($C_1$-$C_4$)Cyanoalkyl, ($C_1$-$C_4$)Hydroxyalkyl, ($C_1$-$C_4$)Alkoxy-($C_1$-$C_4$)alkyl, ($C_2$-$C_4$)Alkenyl, ($C_2$-$C_4$)Halogenalkenyl, ($C_2$-$C_4$)Cyanoalkenyl, ($C_2$-$C_4$)Alkinyl, ($C_2$-$C_4$)Halogenalkinyl, ($C_2$-$C_4$)Cyanoalkinyl, ($C_1$-$C_4$)Alkoxy, ($C_1$-$C_4$)Halogenalkoxy, ($C_1$-$C_4$)Cyanoalkoxy, ($C_1$-$C_4$)Alkoxy-($C_1$-$C_4$)alkoxy, ($C_1$-$C_4$)Alkylhydroxyimino, ($C_1$-$C_4$)Alkoxyimino, ($C_1$-$C_4$)Alkyl-($C_1$-$C_4$)alkoxyimino, ($C_1$-$C_4$)Halogenalkyl-($C_1$-$C_4$)alkoxyimino, ($C_1$-$C_4$)Alkylthio, ($C_1$-$C_4$)Halogenalkylthio, ($C_1$-$C_4$)Alkylthio-($C_1$-$C_4$)alkyl, ($C_1$-$C_4$)Alkylsulfinyl, ($C_1$-$C_4$)Halogenalkylsulfinyl, ($C_1$-$C_4$)Alkylsulfinyl-($C_1$-$C_4$)alkyl, ($C_1$-$C_4$)Alkylsulfonyl, ($C_1$-$C_4$)Halogenalkylsulfonyl, ($C_1$-$C_4$)Alkylsulfonyl-($C_1$-$C_4$)alkyl, ($C_1$-$C_4$)Alkylsulfonyloxy, ($C_1$-$C_4$)Alkylcarbonyl, ($C_1$-$C_4$)Halogenalkylcarbonyl, Aminocarbonyl, Aminothiocarbonyl, ($C_1$-$C_4$)Alkylaminocarbonyl, Di-($C_1$-$C_4$)alkyl-aminocarbonyl, ($C_1$-$C_4$)Alkylsulfonylamino, ($C_1$-$C_4$)Alkylamino, Di-($C_1$-$C_4$)Alkylamino, Aminosulfonyl, ($C_1$-$C_4$)Alkylaminosulfonyl, Di-($C_1$-$C_4$)alkyl-aminosulfonyl, Aminothiocarbonyl oder NHCO-($C_1$-$C_4$)alkyl (($C_1$-$C_4$)Alkylcarbonylamino),

X steht bevorzugt für ein heteroaromatisches 8-, 9-, 10-, 11- oder 12-gliedriges annelliertes bicyclisches oder tricyclisches Ringsystem, wobei das Ringsystem gegebenenfalls einfach oder mehrfach, gleich oder verschieden substituiert ist, und wobei die Substituenten unabhängig voneinander ausgewählt sein können aus Cyano, Halogen, Nitro, Acetyl, Hydroxy, Amino, SCN, Tri-($C_1$-$C_6$)alkylsilyl, ($C_3$-$C_8$)Cycloalkyl, ($C_3$-$C_8$)Cycloalkyl-($C_3$-$C_8$)Cycloalkyl, ($C_1$-$C_6$)Alkyl-($C_3$-$C_8$)cycloalkyl, Halogen($C_3$-$C_8$)cycloalkyl, ($C_1$-$C_6$)Alkyl, ($C_1$-$C_6$)Halogenalkyl, ($C_1$-$C_6$)Cyanoalkyl, ($C_1$-$C_6$)Hydroxyalkyl, ($C_1$-$C_6$)Alkoxy-($C_1$-$C_6$)alkyl, ($C_2$-$C_6$)Alkenyl, ($C_2$-$C_6$)Halogenalkenyl, ($C_2$-$C_6$)Cyanoalkenyl, ($C_2$-$C_6$)Alkinyl, ($C_2$-$C_6$)Alkinyloxy-($C_1$-$C_4$)alkyl, ($C_2$-$C_6$)Halogenalkinyl, ($C_1$-$C_6$)Alkoxy, ($C_1$-$C_6$)Halogenalkoxy, ($C_1$-$C_6$)Halogenalkoxy-($C_1$-$C_6$)alkyl, ($C_2$-$C_6$)Alkenyloxy-($C_1$-$C_6$)alkyl, ($C_2$-$C_6$)Halogenalkenyloxy-($C_1$-$C_6$)alkyl, ($C_1$-$C_6$)Cyanoalkoxy, ($C_1$-$C_6$)Alkoxy-($C_1$-$C_6$)alkoxy, ($C_1$-$C_6$)Alkylhydroxyimino, ($C_1$-$C_6$)Alkoxyimino, ($C_1$-$C_6$)Alkyl-($C_1$-$C_6$)alkoxyimino, ($C_1$-$C_6$)Alkylthio, ($C_1$-$C_6$)Halogenalkylthio, ($C_1$-$C_6$)Alkoxy-($C_1$-$C_6$)alkylthio, ($C_1$-$C_6$)Alkylthio-($C_1$-$C_6$)alkyl, ($C_1$-$C_6$)Alkylsulfinyl, ($C_1$-$C_6$)Halogenalkylsulfinyl, ($C_1$-$C_6$)Alkoxy-($C_1$-$C_6$)alkylsulfinyl, ($C_1$-$C_6$)Alkylsulfinyl-($C_1$-$C_6$)alkyl, ($C_1$-$C_6$)Alkylsulfonyl, ($C_1$-$C_6$)Halogenalkylsulfonyl, ($C_1$-$C_6$)Alkoxy-($C_1$-$C_6$)alkylsulfonyl, ($C_1$-$C_6$)Alkylsulfonyl-($C_1$-$C_6$)alkyl, ($C_1$-$C_6$)Alkylsulfonyloxy, ($C_1$-$C_6$)Alkylcarbonyl, ($C_1$-$C_6$)Alkylcarbonyl-($C_1$-$C_6$)alkyl, ($C_1$-$C_6$)Alkylthiocarbonyl, ($C_1$-$C_6$)Halogenalkylcarbonyl, ($C_1$-$C_6$)Alkylcarbonyloxy, ($C_1$-$C_6$)Alkoxycarbonyl, ($C_1$-$C_6$)Halogenalkoxycarbonyl, Aminocarbonyl, ($C_1$-$C_6$)Alkylaminocarbonyl, ($C_1$-$C_6$)Alkylaminothiocarbonyl, Di-($C_1$-$C_6$)alkyl-aminocarbonyl, Di-($C_1$-$C_6$)alkyl-aminothiocarbonyl, ($C_3$-$C_8$)Cycloalkylaminocarbonyl,

($C_1$-$C_6$)Alkylsulfonylamino, ($C_1$-$C_6$)Alkylamino, Di-($C_1$-$C_6$)Alkylamino, Aminosulfonyl, ($C_1$-$C_6$)Alkylaminosulfonyl, Di-($C_1$-$C_6$)alkyl-aminosulfonyl, ($C_1$-$C_6$)Alkylsulfoximino, Aminothiocarbonyl, ($C_1$-$C_6$)Alkylaminothiocarbonyl, Di-($C_1$-$C_6$)alkyl-aminothiocarbonyl, ($C_3$-$C_8$)Cycloalkylamino, NHCO-($C_1$-$C_6$)alkyl (($C_1$-$C_6$)Alkylcarbonylamino), oder wobei die Substituenten unabhängig voneinander ausgewählt sein können aus Phenyl oder einem 5- oder 6-gliedrigen heteroaromatischen Ring, wobei Phenyl oder der Ring gegebenenfalls einfach oder mehrfach, gleich oder verschieden durch $C_1$-$C_6$-Alkyl, $C_2$-$C_6$-Alkenyl, $C_2$-$C_6$-Alkinyl, $C_3$-$C_6$-Cycloalkyl, $C_1$-$C_6$-Haloalkyl, $C_2$-$C_6$-Haloalkenyl, $C_2$-$C_6$-Haloalkinyl, $C_3$-$C_6$-Halocycloalkyl, Halogen, CN, $C_1$-$C_4$-Alkoxy, $C_1$-$C_4$-Haloalkoxy substituiert sein können,

n steht bevorzugt für 0, 1 oder 2.

Ausgestaltung 3-1

$A^1$ steht besonders bevorzugt für Stickstoff oder =C($R^4$)-,

$R^1$ steht besonders bevorzugt für ($C_1$-$C_4$)Alkyl, ($C_1$-$C_4$)Hydroxyalkyl, ($C_1$-$C_4$)Halogenalkyl, ($C_2$-$C_4$)Alkenyl, ($C_2$-$C_4$)Halogenalkenyl, ($C_2$-$C_4$)Alkinyl, ($C_2$-$C_4$)Halogenalkinyl, ($C_3$-$C_6$)Cycloalkyl, ($C_1$-$C_4$)Alkylthio-($C_1$-$C_4$)alkyl, ($C_1$-$C_4$)Alkylsulfinyl-($C_1$-$C_4$)alkyl oder ($C_1$-$C_4$)Alkylsulfonyl-($C_1$-$C_4$)alkyl,

$R^2$ steht besonders bevorzugt für ein teilweise gesättigtes oder heteroaromatisches annelliertes bicyclisches Ringsystem aus der Reihe Q1 bis Q151, wobei das Ringsystem gegebenenfalls einfach oder mehrfach, gleich oder verschieden substituiert sein kann, wobei die Substituenten unabhängig voneinander ausgewählt sein können aus: Nitro, Hydroxy, Amino, Cyano, Halogen, ($C_1$-$C_4$)Alkyl, ($C_1$-$C_4$)Cycloalkyl, ($C_1$-$C_4$)Halogenalkyl, ($C_1$-$C_4$)Alkoxy, ($C_1$-$C_4$)Halogenalkoxy oder ($C_1$-$C_4$)Halogenalkylthio

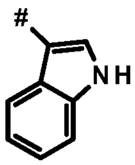
Q1

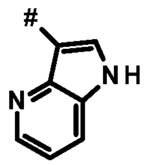
Q2

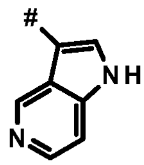
Q3

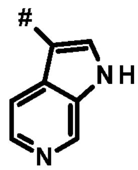
Q4

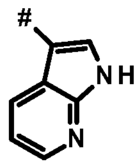
Q5

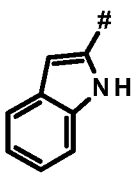
Q6

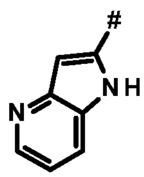
Q7

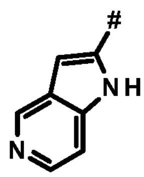
Q8

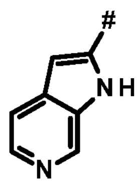
Q9

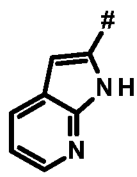
Q10

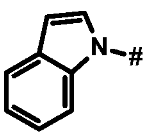
Q11

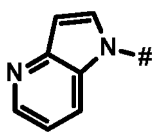
Q12

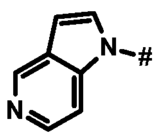
Q13

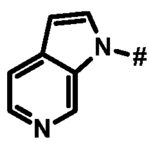
Q14

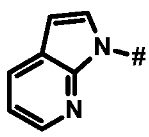
Q15

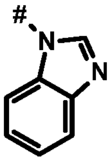
Q16

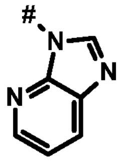
Q17

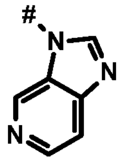
Q18

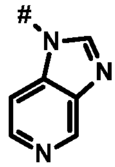
Q19

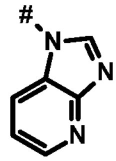
Q20

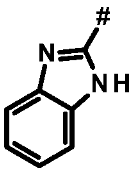
Q21

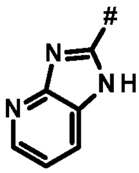
Q22

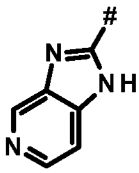
Q23

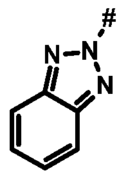
Q24

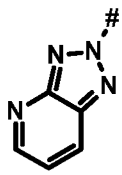
Q25

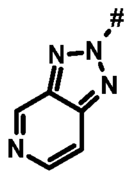
Q26

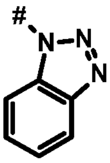
Q27

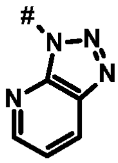
Q28

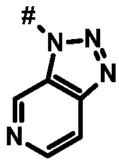
Q29

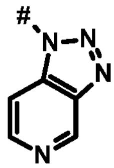
Q30

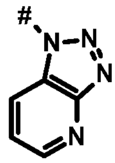
Q31

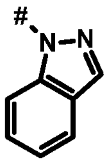
Q32

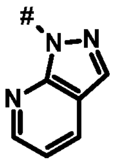
Q33

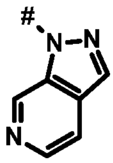
Q34

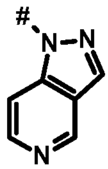
Q35

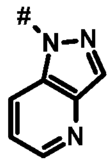
Q36

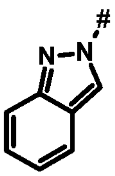
Q37

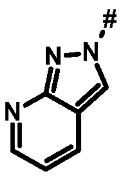
Q38

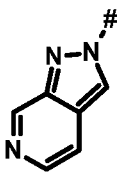
Q39

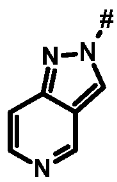
Q40

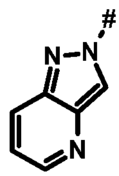
Q41

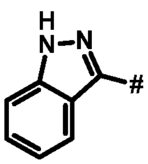
Q42

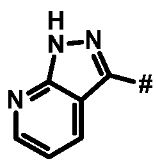
Q43

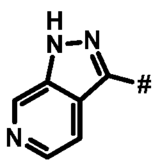
Q44

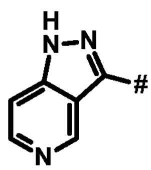
Q45

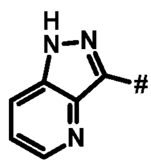
Q46

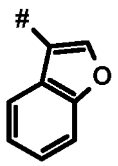
Q47

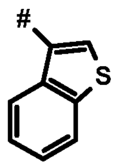
Q48

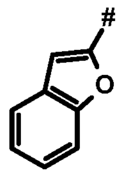
Q49

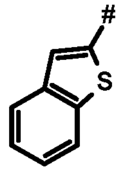
Q50

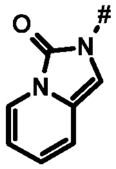
Q51

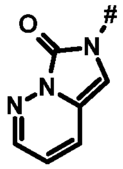
Q52

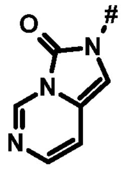
Q53

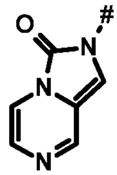
Q54

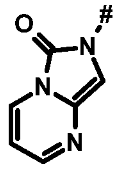
Q55

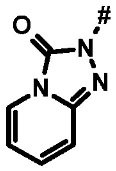
Q56

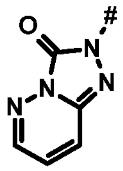
Q57

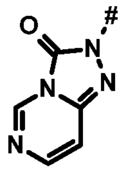
Q58

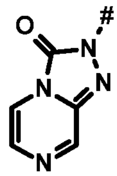
Q59

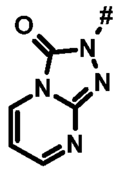
Q60

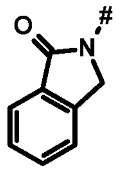
Q61

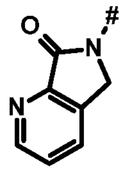
Q62

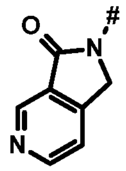
Q63

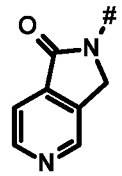
Q64

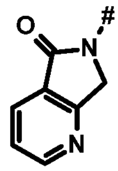
Q65

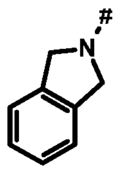
Q66

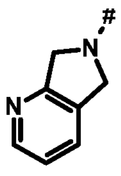
Q67

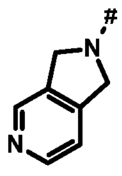
Q68

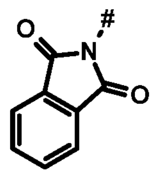
Q69

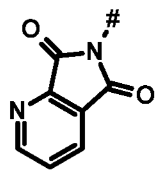
Q70

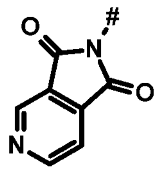
Q71

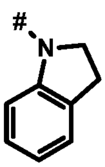
Q72

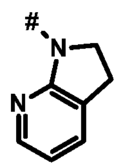
Q73

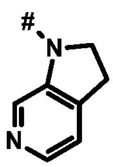
Q74

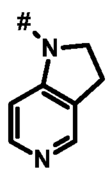
Q75

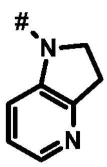
Q76

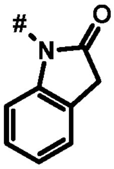
Q77
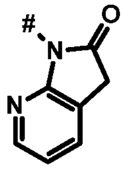
Q78
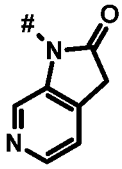
Q79
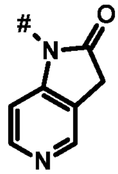
Q80
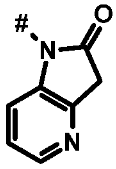
Q81

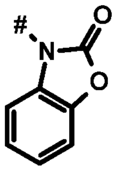
Q82
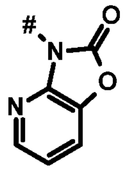
Q83
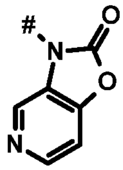
Q84
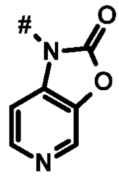
Q85
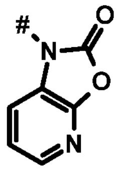
Q86

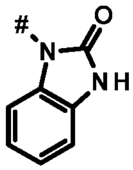
Q87
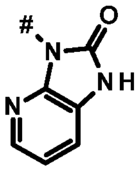
Q88
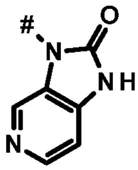
Q89
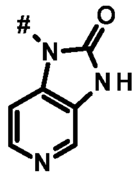
Q90
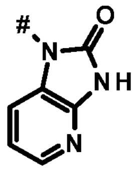
Q91

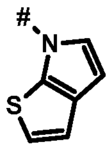
Q92
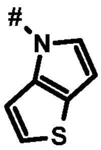
Q93
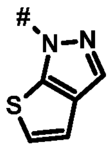
Q94
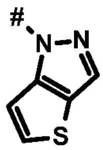
Q95
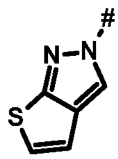
Q96
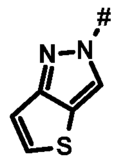
Q97

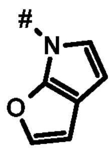
Q98
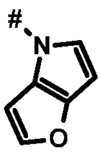
Q99
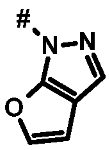
Q100
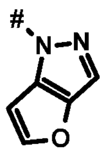
Q101
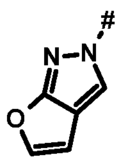
Q102
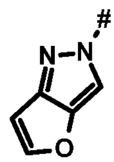
Q103

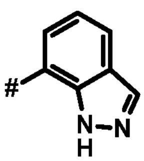
Q104
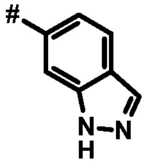
Q105
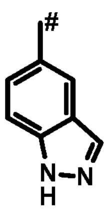
Q106
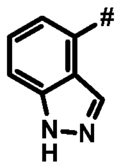
Q107
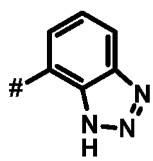
Q108
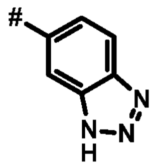
Q109

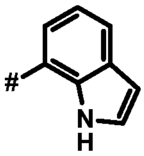
Q110

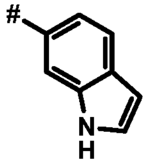
Q111

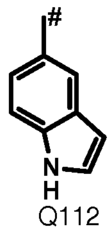
Q112

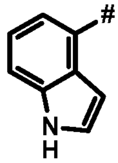
Q113

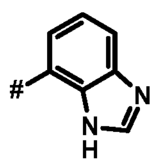
Q114

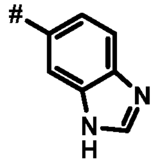
Q115

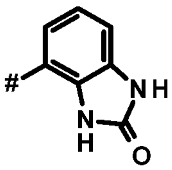
Q116

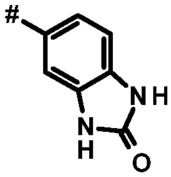
Q117

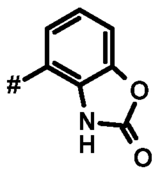
Q118

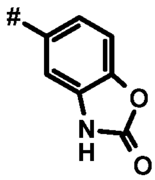
Q119

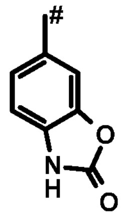
Q120

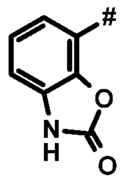
Q121

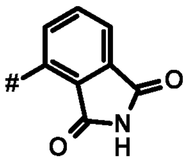
Q122

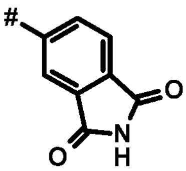
Q123

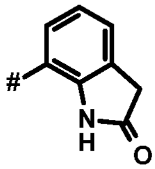
Q124

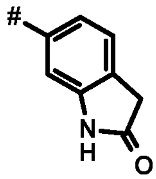
Q125

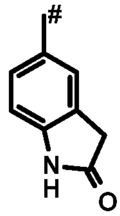
Q126

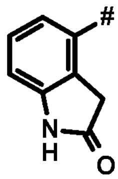
Q127

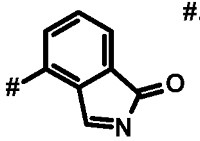
Q128

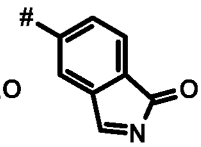
Q129

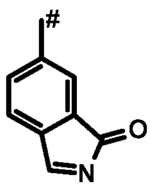
Q130

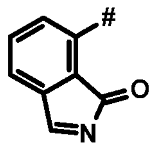
Q131

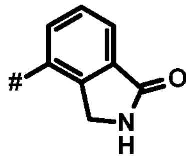
Q132

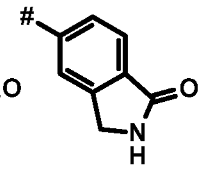
Q133

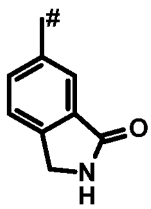
Q134

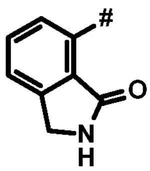
Q135

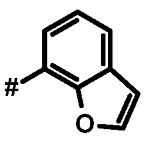
Q136

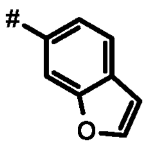
Q137

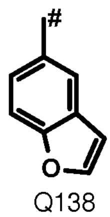
Q138

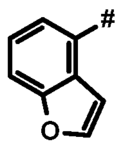
Q139

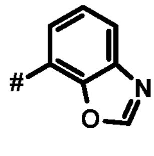
Q140

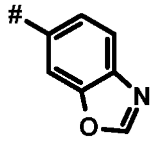
Q141

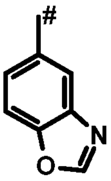
Q142

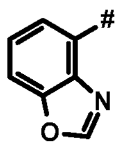
Q143

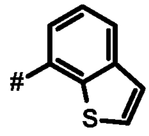
Q144

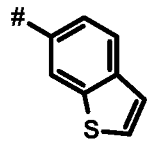
Q145

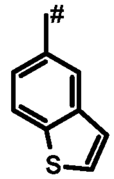
Q146

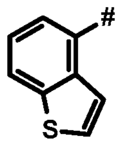
Q147

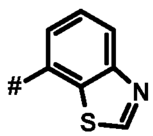
Q148

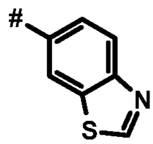
Q149

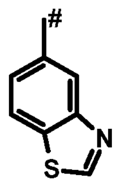
Q150

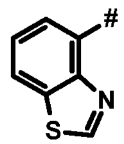
Q151

wobei die Bindung zum Restmolekül mit einem Sternchen # gekennzeichnet ist,

$R^3$ steht besonders bevorzugt für Wasserstoff, Cyano, Halogen, Nitro, Hydroxy, Amino, SCN, Tri-($C_1$-$C_4$)alkylsilyl, ($C_3$-$C_6$)Cycloalkyl, ($C_3$-$C_6$)Cycloalkyl-($C_3$-$C_6$)cycloalkyl, ($C_1$-$C_4$)Alkyl-($C_3$-$C_6$)cycloalkyl, Halogen($C_3$-$C_6$)cycloalkyl, ($C_1$-$C_4$)Alkyl, ($C_1$-$C_4$)Halogenalkyl, ($C_1$-$C_4$)Cyanoalkyl, ($C_1$-$C_4$)Alkoxy-($C_1$-$C_4$)alkyl, ($C_2$-$C_4$)Alkenyl, ($C_2$-$C_4$)Halogenalkenyl, ($C_2$-$C_4$)Cyanoalkenyl, ($C_2$-$C_4$)Alkinyl, ($C_2$-$C_4$)Halogenalkinyl, ($C_2$-$C_4$)Cyanoalkinyl, ($C_1$-$C_4$)Alkoxy, ($C_1$-$C_4$)Halogenalkoxy, ($C_1$-$C_4$)Cyanoalkoxy, ($C_1$-$C_4$)Alkylhydroxyimino, ($C_1$-$C_4$)Alkoxyimino, ($C_1$-$C_4$)Alkyl-($C_1$-$C_4$)alkoxyimino, ($C_1$-$C_4$)Alkylthio, ($C_1$-$C_4$)Halogenalkylthio, ($C_1$-$C_4$)Alkylsulfinyl, ($C_1$-$C_4$)Halogenalkylsulfinyl, ($C_1$-$C_4$)Alkylsulfonyl, ($C_1$-$C_4$)Halogenalkylsulfonyl, ($C_1$-$C_4$)Alkylsulfonyloxy, ($C_1$-$C_4$)Alkylcarbonyl, ($C_1$-$C_4$)Halogenalkylcarbonyl, Aminocarbonyl, ($C_1$-$C_4$)Alkylaminocarbonyl, Di-($C_1$-$C_4$)alkyl-aminocarbonyl, ($C_1$-$C_4$)Alkylsulfonylamino, ($C_1$-$C_4$)Alkylamino, Di-($C_1$-$C_4$)Alkylamino, Aminosulfonyl, ($C_1$-$C_4$)Alkylaminosulfonyl, Di-($C_1$-$C_4$)alkyl-aminosulfonyl oder NHCO-($C_1$-$C_4$)alkyl (($C_1$-$C_4$)Alkylcarbonylamino),

$R^4$ steht besonders bevorzugt für Wasserstoff, Halogen, Cyano oder ($C_1$-$C_4$)Alkyl,

X steht besonders bevorzugt für ein heteroaromatisches 9-gliedriges oder 12-gliedriges annelliertes bicyclisches oder tricyclisches Ringsystem aus der Reihe H1 bis H20,

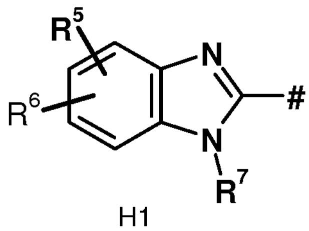
H1

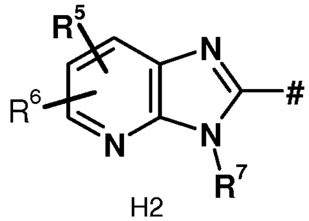
H2

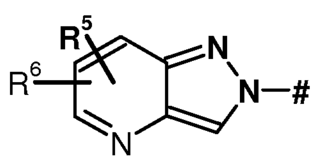
H3

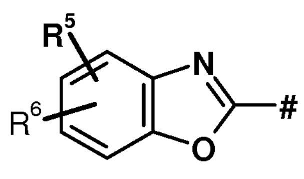
H4

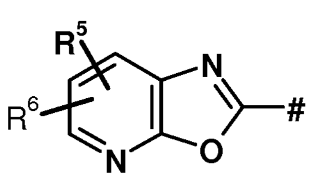
H5

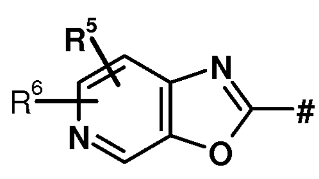
H6

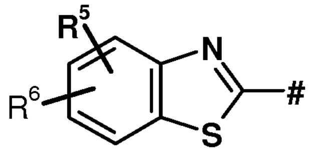
H7

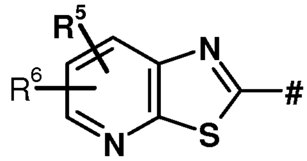
H8

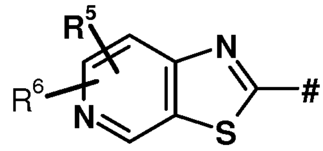
H9

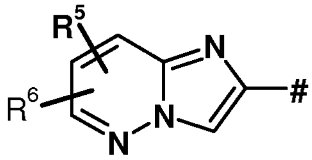
H10

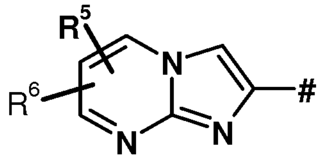
H11

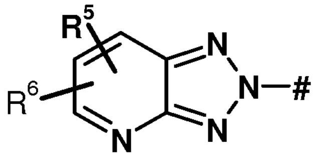
H12

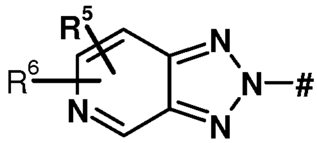
H13

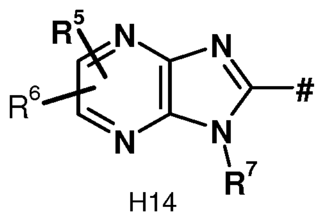
H14

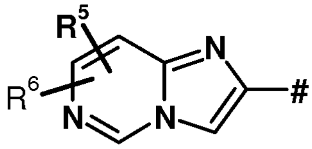
H15

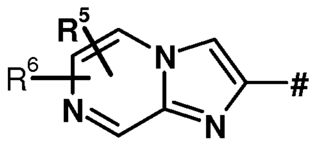
H16

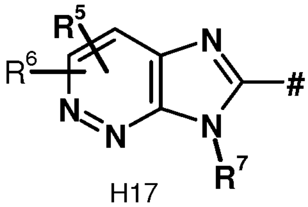
H17

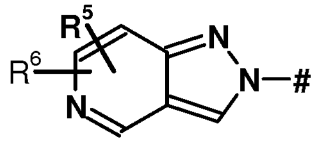
H18

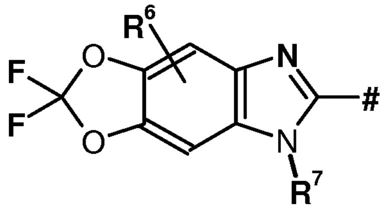
H19

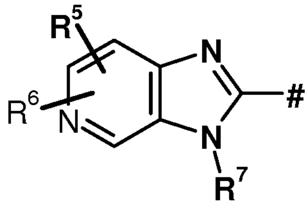
H20

$R^5$, $R^6$ stehen unabhängig voneinander besonders bevorzugt für Wasserstoff, Cyano, Halogen, $(C_1-C_4)$Alkyl, $(C_1-C_4)$Halogenalkyl, $(C_2-C_4)$Alkenyl, $(C_2-C_4)$Halogenalkenyl, $(C_2-C_4)$Alkinyl, $(C_2-C_4)$Halogenalkinyl, $(C_3-C_6)$Cycloalkyl, $(C_3-C_6)$Cycloalkyl-$(C_3-C_6)$cycloalkyl, $(C_1-C_4)$Alkyl-$(C_3-C_6)$cycloalkyl, $(C_1-C_4)$Alkoxy, $(C_1-C_4)$Halogenalkoxy, $(C_1-C_4)$Alkoxyimino, $(C_1-C_4)$Alkylthio, $(C_1-C_4)$Halogenalkylthio, $(C_1-C_4)$Alkylsulfinyl, $(C_1-C_4)$Halogenalkylsulfinyl, $(C_1-C_4)$Alkylsulfonyl, $(C_1-C_4)$Halogenalkylsulfonyl, $(C_1-C_4)$Alkylsulfonyloxy, $(C_1-C_4)$Alkylcarbonyl, $(C_1-C_4)$Halogenalkylcarbonyl, Aminocarbonyl, $(C_1-C_4)$Alkylaminocarbonyl, Di-$(C_1-C_4)$alkyl-aminocarbonyl, $(C_1-C_4)$Alkylsulfonylamino, $(C_1-C_4)$Alkylamino, Di-$(C_1-C_4)$Alkylamino, Aminosulfonyl, $(C_1-C_4)$Alkylaniiinosulfonyl oder Di-$(C_1-C_4)$alkylaminosulfonyl,

$R^7$ steht besonders bevorzugt für Wasserstoff, $(C_1-C_4)$Alkyl, $(C_1-C_4)$Halogenalkyl, $(C_1-C_4)$Cyanoalkyl, $(C_1-C_4)$Hydroxyalkyl, $(C_1-C_4)$Alkoxy-$(C_1-C_4)$alkyl, $(C_1-C_4)$Halogenalkoxy-$(C_1-C_4)$alkyl, $(C_2-C_4)$Alkenyl, $(C_2-C_4)$Alkenyloxy-$(C_1-C_4)$alkyl, $(C_2-C_4)$Halogenalkenyloxy-$(C_1-C_4)$alkyl, $(C_2-C_4)$Halogenalkenyl, $(C_2-C_4)$Cyanoalkenyl, $(C_2-C_4)$Alkinyl, $(C_2-C_4)$Alkinyloxy-$(C_1-C_4)$alkyl, $(C_2-C_4)$Halogenalkinyl, $(C_3-C_6)$Cycloalkyl, $(C_3-C_6)$Cycloalkyl-$(C_3-C_6)$cycloalkyl, $(C_1-C_4)$Alkyl-$(C_3-C_6)$cycloalkyl, Halogen$(C_3-C_6)$cycloalkyl, $(C_1-C_4)$Alkylthio-$(C_1-C_4)$al-

kyl, $(C_1-C_4)$Alkylsulfinyl-$(C_1-C_4)$alkyl, $(C_1-C_4)$Alkylsulfonyl-$(C_1-C_4)$alkyl oder $(C_1-C_4)$Alkylcarbonyl-$(C_1-C_4)$alkyl,

n steht besonders bevorzugt für 0, 1 oder 2.

Die Substitution an den Ringen Q1 bis Q151 kann durch Substitution von Wasserstoff am C-Atom und/oder am N-Atom erfolgen. Die isomeren Formen der Ringe Q1 bis Q151 sind umfasst und Teil der Erfindung.

Ausgestaltung 3-2

$A^1$ steht besonders bevorzugt für Stickstoff oder =C($R^4$)-,

$R^1$ steht besonders bevorzugt für $(C_1-C_4)$Alkyl, $(C_1-C_4)$Hydroxyalkyl, $(C_1-C_4)$Halogenalkyl, $(C_2-C_4)$Alkenyl, $(C_2-C_4)$Halogenalkenyl, $(C_2-C_4)$Alkinyl, $(C_2-C_4)$Halogenalkinyl, $(C_3-C_6)$Cycloalkyl, $(C_1-C_4)$Alkylthio-$(C_1-C_4)$alkyl, $(C_1-C_4)$Alkylsulfinyl-$(C_1-C_4)$alkyl oder $(C_1-C_4)$Alkylsulfonyl-$(C_1-C_4)$alkyl,

$R^2$ steht besonders bevorzugt für ein teilweise gesättigtes oder heteroaromatisches annelliertes bicyclisches Ringsystem aus der Reihe Q1 bis Q152, wobei das Ringsystem gegebenenfalls einfach oder mehrfach, gleich oder verschieden substituiert sein kann, wobei die Substituenten unabhängig voneinander ausgewählt sein können aus: Nitro, Hydroxy, Amino, Cyano, Halogen, $(C_1-C_4)$Alkyl, $(C_1-C_4)$Cycloalkyl, $(C_1-C_4)$Halogenalkyl, $(C_1-C_4)$Alkoxy, $(C_1-C_4)$Halogenalkoxy oder $(C_1-C_4)$Halogenalkylthio

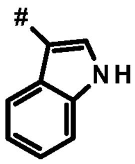
Q1

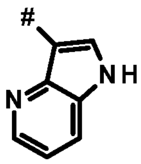
Q2

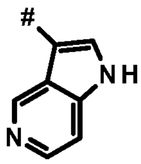
Q3

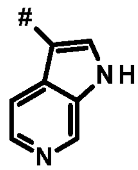
Q4

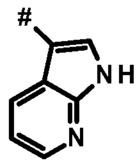
Q5

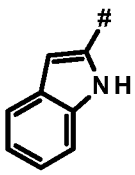
Q6

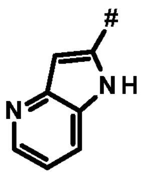
Q7

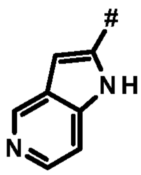
Q8

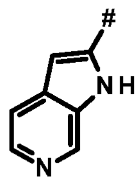
Q9

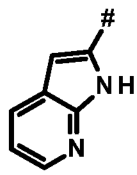
Q10

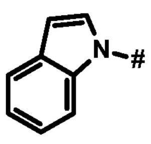
Q11

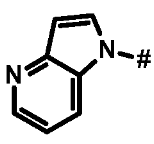
Q12

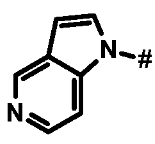
Q13

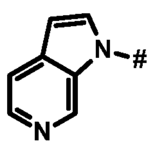
Q14

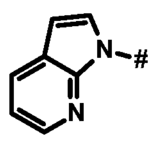
Q15

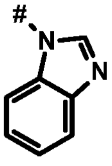
Q16

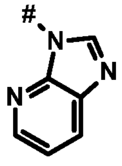
Q17

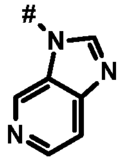
Q18

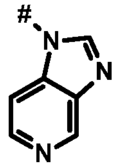
Q19

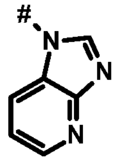
Q20

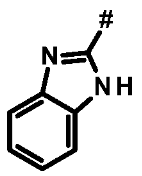
Q21
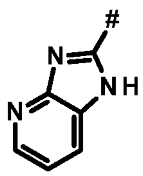
Q22
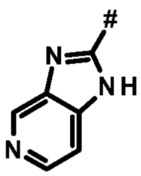
Q23
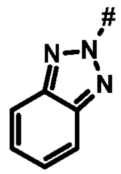
Q24
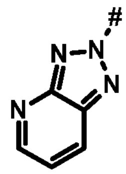
Q25
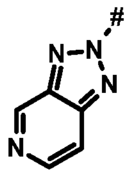
Q26

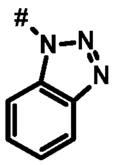
Q27
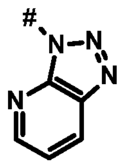
Q28
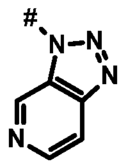
Q29
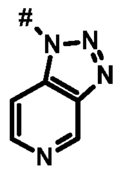
Q30
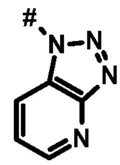
Q31

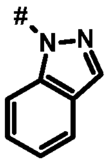
Q32
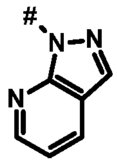
Q33
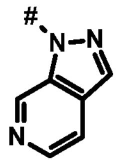
Q34
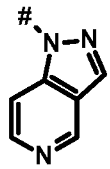
Q35
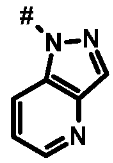
Q36

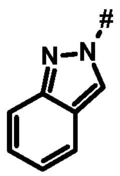
Q37
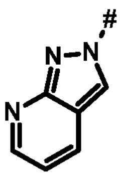
Q38
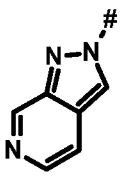
Q39
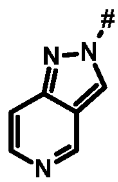
Q40
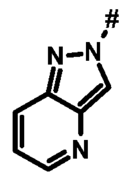
Q41

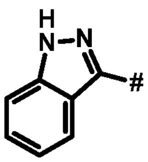
Q42
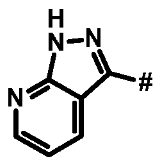
Q43
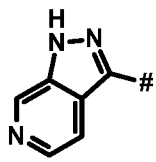
Q44
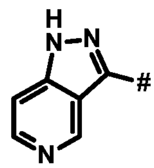
Q45
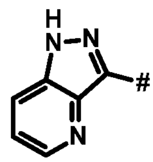
Q46

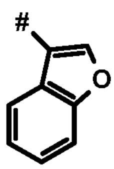
Q47
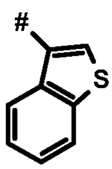
Q48
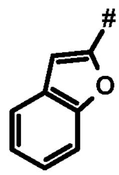
Q49
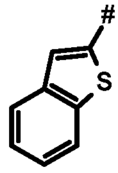
Q50

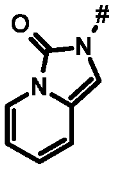
Q51
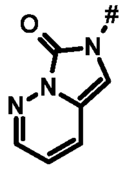
Q52
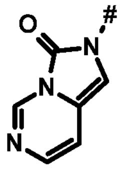
Q53
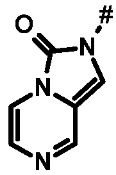
Q54
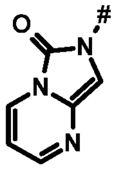
Q55

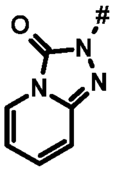
Q56
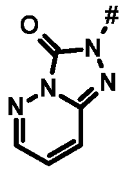
Q57
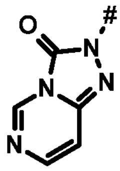
Q58
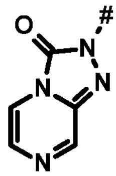
Q59
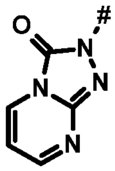
Q60

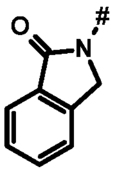
Q61
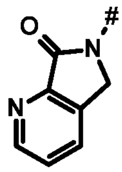
Q62
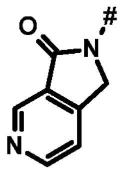
Q63
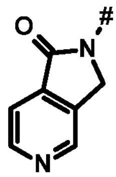
Q64
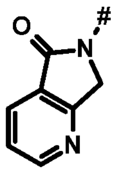
Q65

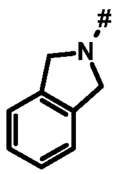
Q66
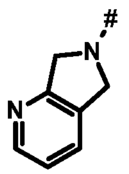
Q67
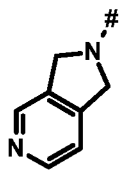
Q68
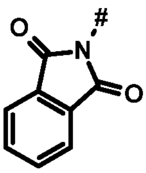
Q69
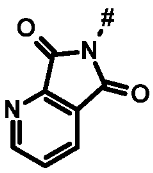
Q70
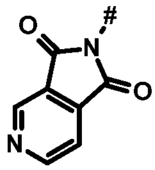
Q71

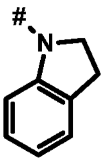
Q72
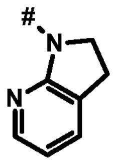
Q73
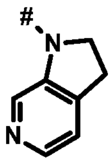
Q74
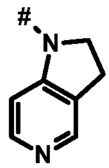
Q75
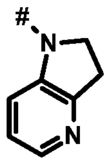
Q76

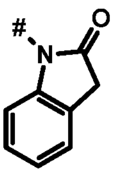
Q77
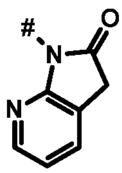
Q78
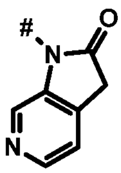
Q79
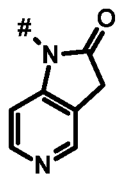
Q80
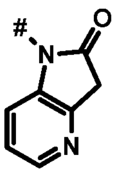
Q81

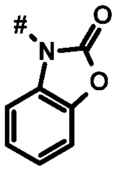 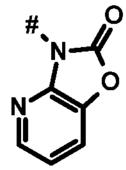 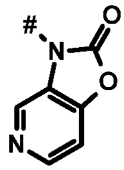 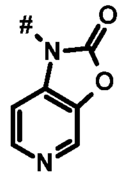 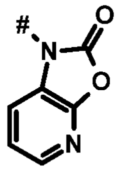

Q82 Q83 Q84 Q85 Q86

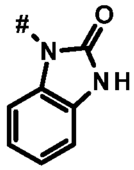 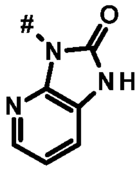 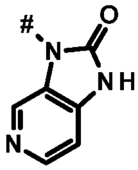 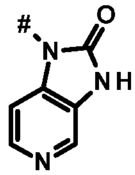 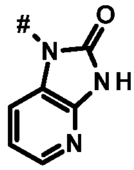

Q87 Q88 Q89 Q90 Q91

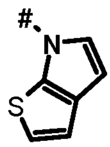 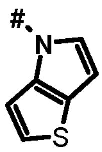 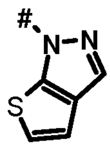 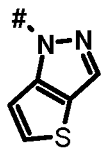 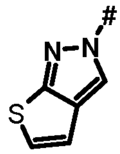 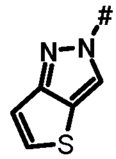

Q92 Q93 Q94 Q95 Q96 Q97

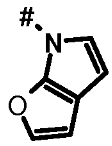 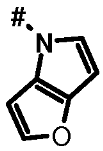 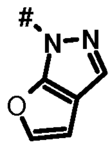 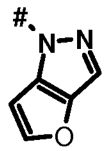 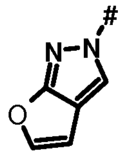 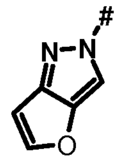

Q98 Q99 Q100 Q101 Q102 Q103

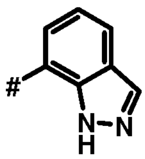 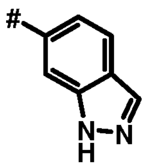 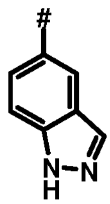 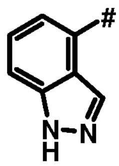 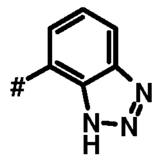 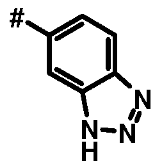

Q104 Q105 Q106 Q107 Q108 Q109

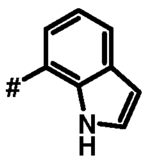 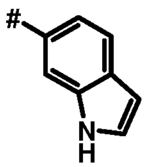 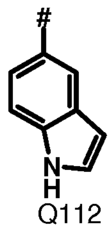 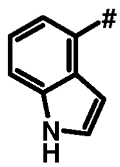 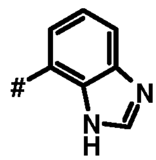 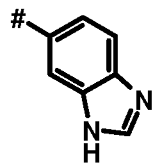

Q110 Q111 Q112 Q113 Q114 Q115

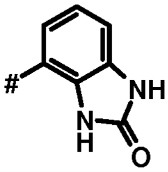
Q116
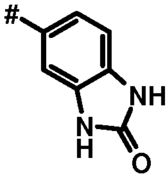
Q117
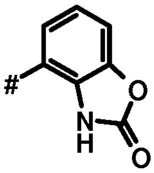
Q118
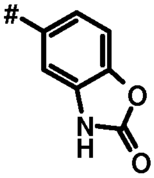
Q119
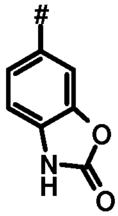
Q120
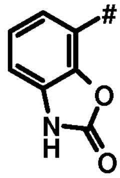
Q121

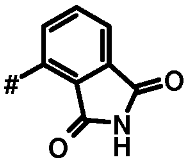
Q122
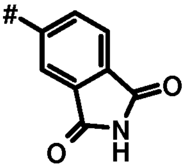
Q123
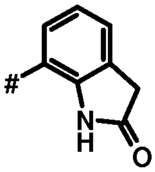
Q124
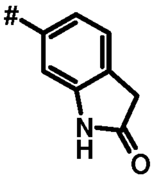
Q125
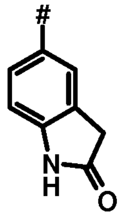
Q126
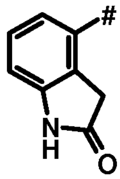
Q127

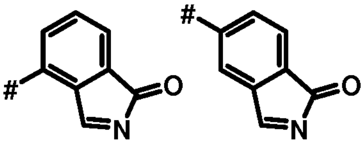
Q128 Q129
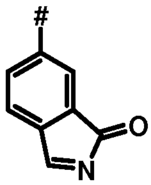
Q130
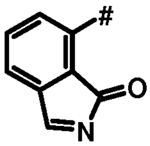
Q131
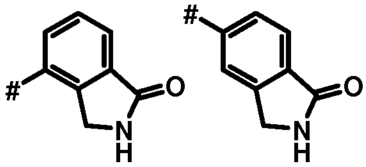
Q132 Q133

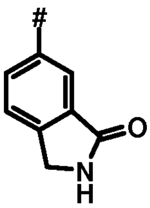
Q134
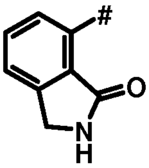
Q135

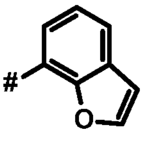
Q136
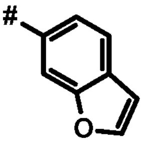
Q137
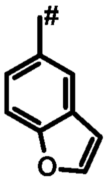
Q138
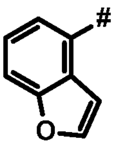
Q139
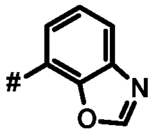
Q140
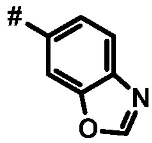
Q141

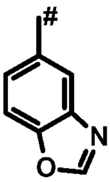
Q142
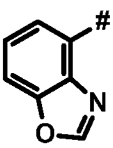
Q143
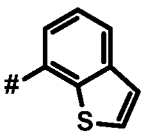
Q144
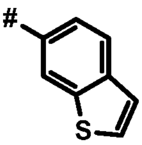
Q145
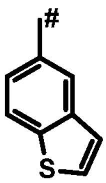
Q146
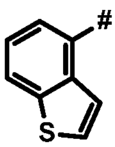
Q147

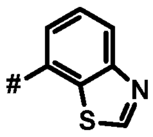

Q148

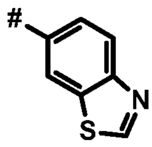

Q149

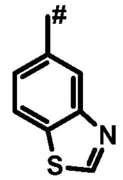

Q150

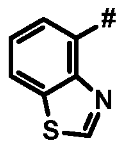

Q151

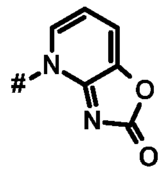

Q152

wobei die Bindung zum Restmolekül mit einem Sternchen # gekennzeichnet ist,

$R^3$ steht besonders bevorzugt für Wasserstoff, Cyano, Halogen, Nitro, Hydroxy, Amino, SCN, Tri-($C_1$-$C_4$)alkylsilyl, ($C_3$-$C_6$)Cycloalkyl, ($C_3$-$C_6$)Cycloalkyl-($C_3$-$C_6$)cycloalkyl, ($C_1$-$C_4$)Alkyl-($C_3$-$C_6$)cycloalkyl, Halogen($C_3$-$C_6$)cycloalkyl, ($C_1$-$C_4$)Alkyl, ($C_1$-$C_4$)Halogenalkyl, ($C_1$-$C_4$)Cyanoalkyl, ($C_1$-$C_4$)Alkoxy-($C_1$-$C_4$)alkyl, ($C_2$-$C_4$)Alkenyl, ($C_2$-$C_4$)Halogenalkenyl, ($C_2$-$C_4$)Cyanoalkenyl, ($C_2$-$C_4$)Alkinyl, ($C_2$-$C_4$)Halogenalkinyl, ($C_2$-$C_4$)Cyanoalkinyl, ($C_1$-$C_4$)Alkoxy, ($C_1$-$C_4$)Halogenalkoxy, ($C_1$-$C_4$)Cyanoalkoxy, ($C_1$-$C_4$)Alkylhydroxyimino, ($C_1$-$C_4$)Alkoxyimino, ($C_1$-$C_4$)Alkyl-($C_1$-$C_4$)alkoxyimino, ($C_1$-$C_4$)Alkylthio, ($C_1$-$C_4$)Halogenalkylthio, ($C_1$-$C_4$)Alkylsulfinyl, ($C_1$-$C_4$)Halogenalkylsulfinyl, ($C_1$-$C_4$)Alkylsulfonyl, ($C_1$-$C_4$)Halogenalkylsulfonyl, ($C_1$-$C_4$)Alkylsulfonyloxy, ($C_1$-$C_4$)Alkylcarbonyl, ($C_1$-$C_4$)Halogenalkylcarbonyl, Aminocarbonyl, ($C_1$-$C_4$)Alkylaminocarbonyl, Di-($C_1$-$C_4$)alkyl-aminocarbonyl, ($C_1$-$C_4$)Alkylsulfonylamino, ($C_1$-$C_4$)Alkylamino, Di-($C_1$-$C_4$)Alkylamino, Aminosulfonyl, ($C_1$-$C_4$)Alkylaminosulfonyl, Di-($C_1$-$C_4$)alkyl-aminosulfonyl oder NHCO-($C_1$-$C_4$)alkyl (($C_1$-$C_4$)Alkylcarbonylamino),

$R^4$ steht besonders bevorzugt für Wasserstoff, Halogen, Cyano oder ($C_1$-$C_4$)Alkyl,

X steht besonders bevorzugt für ein heteroaromatisches 9-gliedriges oder 12-gliedriges annelliertes bicyclisches oder tricyclisches Ringsystem aus der Reihe H1 bis H20,

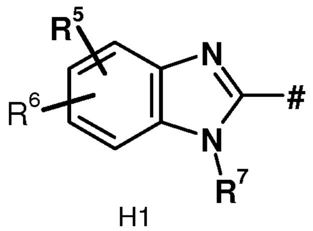

H1

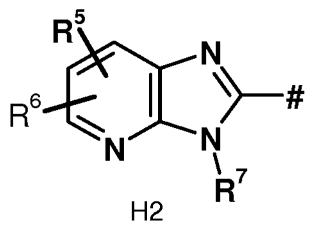

H2

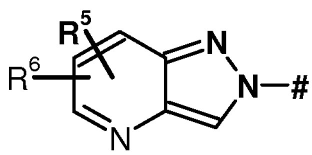

H3

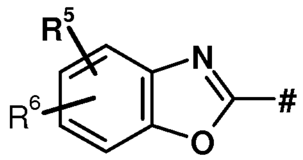

H4

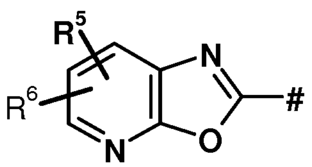

H5

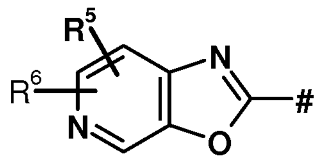

H6

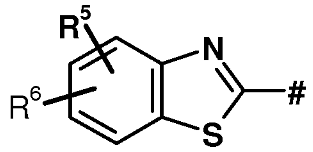

H7

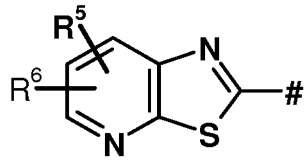

H8

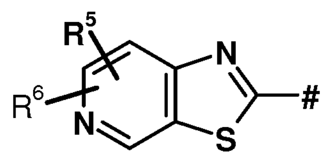

H9

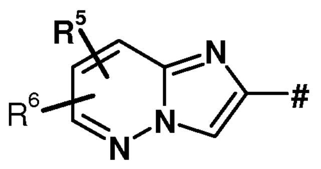

H10

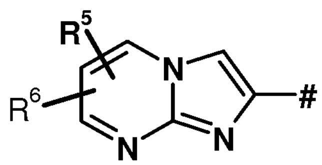

H11

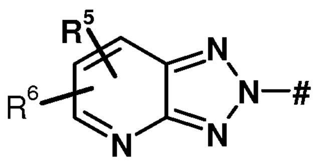

H12

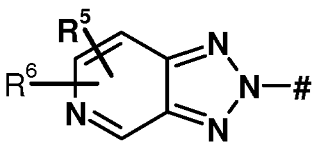

H13

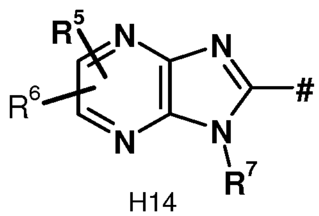

H14

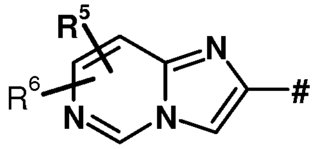

H15

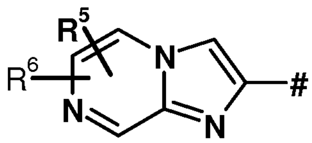

H16

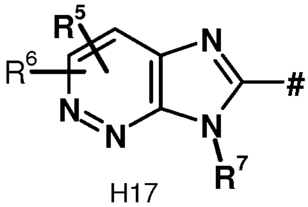

H17

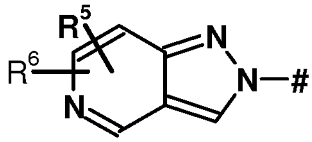

H18

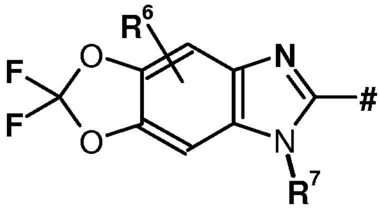

H19

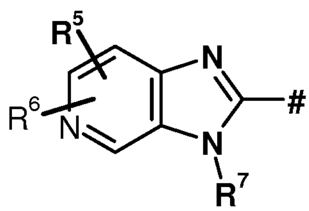

H20

$R^5$, $R^6$ stehen unabhängig voneinander besonders bevorzugt für Wasserstoff, Cyano, Halogen, ($C_1$-$C_4$)Alkyl, ($C_1$-$C_4$)Halogenalkyl, ($C_2$-$C_4$)Alkenyl, ($C_2$-$C_4$)Halogenalkenyl, ($C_2$-$C_4$)Alkinyl, ($C_2$-$C_4$)Halogenalkinyl, ($C_3$-$C_6$)Cycloalkyl, ($C_3$-$C_6$)Cycloalkyl-($C_3$-$C_6$)cycloalkyl, ($C_1$-$C_4$)Alkyl-($C_3$-$C_6$)cycloalkyl, ($C_1$-$C_4$)Alkoxy, ($C_1$-$C_4$)Halogenalkoxy, ($C_1$-$C_4$)Alkoxyimino, ($C_1$-$C_4$)Alkylthio, ($C_1$-$C_4$)Halogenalkylthio, ($C_1$-$C_4$)Alkylsulfinyl, ($C_1$-$C_4$)Halogenalkylsulfinyl, ($C_1$-$C_4$)Alkylsulfonyl, ($C_1$-$C_4$)Halogenalkylsulfonyl, ($C_1$-$C_4$)Alkylsulfonyloxy, ($C_1$-$C_4$)Alkylcarbonyl, ($C_1$-$C_4$)Halogenalkylcarbonyl, Aminocarbonyl, ($C_1$-$C_4$)Alkylaminocarbonyl, Di-($C_1$-$C_4$)alkyl-aminocarbonyl, ($C_1$-$C_4$)Alkylsulfonylamino, ($C_1$-$C_4$)Alkylamino, Di-($C_1$-$C_4$)Alkylamino, Aminosulfonyl, ($C_1$-$C_4$)Alkylaminosulfonyl oder Di-($C_1$-$C_4$)alkylaminosulfonyl,

$R^7$ steht besonders bevorzugt für Wasserstoff, ($C_1$-$C_4$)Alkyl, ($C_1$-$C_4$)Halogenalkyl, ($C_1$-$C_4$)Cyanoalkyl, ($C_1$-$C_4$)Hydroxyalkyl, ($C_1$-$C_4$)Alkoxy-($C_1$-$C_4$)alkyl, ($C_1$-$C_4$)Halogenalkoxy-($C_1$-$C_4$)alkyl, ($C_2$-$C_4$)Alkenyl, ($C_2$-$C_4$)Alkenyloxy-($C_1$-$C_4$)alkyl, ($C_2$-$C_4$)Halogenalkenyloxy-($C_1$-$C_4$)alkyl, ($C_2$-$C_4$)Halogenalkenyl, ($C_2$-$C_4$)Cyanoalkenyl, ($C_2$-$C_4$)Alkinyl, ($C_2$-$C_4$)Alkinyloxy-($C_1$-$C_4$)alkyl, ($C_2$-$C_4$)Halogenalkinyl, ($C_3$-$C_6$)Cycloalkyl, ($C_3$-$C_6$)Cycloalkyl-($C_3$-$C_6$)cycloalkyl, ($C_1$-$C_4$)Alkyl-($C_3$-$C_6$)cycloalkyl, Halogen($C_3$-$C_6$)cycloalkyl, ($C_1$-$C_4$)Alkylthio-($C_1$-$C_4$)alkyl, ($C_1$-$C_4$)Alkylsulfinyl-($C_1$-$C_4$)alkyl, ($C_1$-$C_4$)Alkylsulfonyl-($C_1$-$C_4$)alkyl oder ($C_1$-$C_4$)Alkylcarbonyl-($C_i$-$C_4$)alkyl,

n steht besonders bevorzugt für 0, 1 oder 2.

Die Substitution an den Ringen Q1 bis Q152 kann durch Substitution von Wasserstoff am C-Atom und/oder am N-Atom erfolgen. Die isomeren Formen der Ringe Q1 bis Q152 sind umfasst und Teil der Erfindung.

Isomere Formen der Ringe bedeutet zum Beispiel:

Q105:

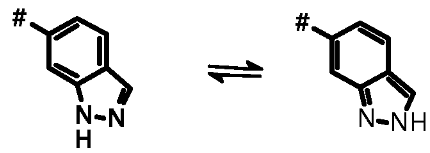

Q106:

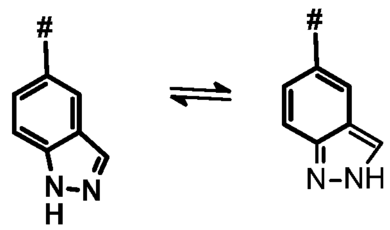

Q107:

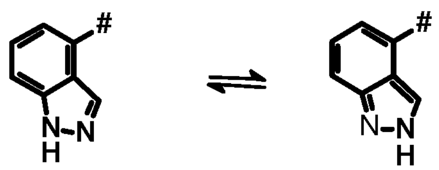

Die Verbindungen können sowohl als Gemische als auch in Form ihrer reinen Isomeren vorliegen. Gemische lassen sich gegebenenfalls durch physikalische Methoden trennen, beispielsweise durch chromatographische Methoden.

Aus Gründen der besseren Übersichtlichkeit wird im Folgenden jeweils nur eines der möglichen Isomeren aufgeführt. Das schließt nicht aus, dass die Verbindungen gegebenenfalls in Form der Isomerengemische oder in der jeweils anderen isomeren Form vorliegen können.

Ausgestaltung 4-1

$A^1$ steht ganz besonders bevorzugt für Stickstoff oder $=C(R^4)-$,

$R^1$ steht ganz besonders bevorzugt für Methyl, Ethyl, n-Propyl, i-Propyl, cyclo-Propyl, n-Butyl, i-Butyl, tert.-Butyl, cyclo-Butyl, Fluormethyl, Difluormethyl, Trifluormethyl, Fluorethyl, Difluorethyl, Trifluorethyl, Tetrafluorethyl oder Pentafluorethyl,

$R^2$ steht ganz besonders bevorzugt für ein gegebenenfalls einfach oder zweifach, gleich oder verschieden substituiertes, annelliertes bicyclisches Ringsystem aus der Reihe Q1, Q2, Q3, Q4, Q5, Q6, Q7, Q8, Q9, Q10, Q11, Q12, Q13, Q14, Q15, Q16, Q17, Q18, Q19, Q20, Q21, Q22, Q23, Q27, Q28, Q29, Q30, Q31, Q32, Q33, Q34, Q35, Q36, Q37, Q38, Q39, Q40, Q41, Q42, Q43, Q44, Q45, Q46, Q51, Q52, Q53, Q54, Q55, Q56, Q57, Q58, Q59, Q60, Q61, Q62, Q63, Q64, Q65, Q66, Q67, Q68, Q77, Q78, Q79, Q80, Q81, Q82, Q83, Q84, Q85, Q86, Q87, Q88, Q89, Q90, Q91, Q92, Q93, Q94, Q95, Q96, Q97, Q104, Q105, Q106, Q107, Q108, Q109, Q114, Q115, Q116, Q117, Q118, Q119, Q120, Q121, Q124, Q125, Q126, Q127, Q128, Q129, Q130, Q131, Q132, Q133, Q134, Q135, Q136, Q137, Q138, Q139, Q140, Q141, Q142, Q143, Q144, Q145, Q146, Q147, Q148, Q149, Q150, Q151 und wobei als Substituenten jeweils in Frage kommen: Cyano, Fluor, Chlor, Brom, Methyl, Ethyl, n-Propyl, i-Propyl, Cyclopropyl, Trifluormethyl, Pentafluoroethyl, Methoxy, Ethoxy, Trifluormethoxy, Trifluorethoxy, Pentafluorethoxy oder Trifluormethylthio,

$R^3$ steht ganz besonders bevorzugt für Wasserstoff, Cyano, Halogen, $(C_1-C_4)$Alkyl, $(C_1-C_4)$Halogenalkyl, $(C_1-C_4)$Halogenalkoxy, $(C_1-C_4)$Alkylthio, $(C_1-C_4)$Alkylsulfinyl, $(C_1-C_4)$Alkylsulfonyl, $(C_1-C_4)$Halogenalkylthio, $(C_1-C_4)$Halogenalkylsulfinyl, $(C_1-C_4)$Halogenalkylsulfonyl oder NHCO-$(C_1-C_4)$alkyl ($(C_1-C_4)$Alkylcarbonylamino),

$R^4$ steht ganz besonders bevorzugt für Wasserstoff, Fluor, Chlor, Brom oder Cyano,

X steht ganz besonders bevorzugt für ein heteroaromatisches 9-gliedriges oder 12-gliedriges annelliertes bicyclisches oder tricyclisches Ringsystem aus der Reihe H1, H2, H3, H4, H5, H6, H7, H8, H9, H10, H11, H12, H13, H14, H15, H16, H17, H18, H19 oder H20,

$R^5$ steht ganz besonders bevorzugt für Fluor, Chlor, Fluormethyl, Difluormethyl, Trifluormethyl, Fluorethyl, Difluorethyl, Trifluorethyl, Tetrafluorethyl, Pentafluorethyl, Trifluormethoxy, Difluorchlormethoxy, Dichlorfluormethoxy, Trifluormethylthio, Trifluormethylsulfonyl oder Trifluormethylsulfinyl,

$R^6$ steht ganz besonders bevorzugt für Wasserstoff,

$R^7$ steht ganz besonders bevorzugt für Wasserstoff, Methyl, Ethyl, n-Propyl, i-Propyl, n-Butyl, i-Butyl, tert.-Butyl, Methoxymethyl oder Methoxyethyl,

n steht ganz besonders bevorzugt für 0, 1 oder 2.

Ausgestaltung 4-2

$A^1$ steht ganz besonders bevorzugt für Stickstoff,

$R^1$ steht ganz besonders bevorzugt für Methyl, Ethyl, n-Propyl, i-Propyl, cyclo-Propyl, n-Butyl, i-Butyl, tert.-Butyl, cyclo-Butyl, Fluormethyl, Difluormethyl, Trifluormethyl, Fluorethyl, Difluorethyl, Trifluorethyl, Tetrafluorethyl oder Pentafluorethyl,

$R^2$ steht ganz besonders bevorzugt für ein gegebenenfalls einfach oder zweifach, gleich oder verschieden substituiertes, annelliertes bicyclisches Ringsystem aus der Reihe Q1, Q2, Q3, Q4, Q5, Q6, Q7, Q8, Q9, Q10, Q11, Q12, Q13, Q14, Q15, Q16, Q17, Q18, Q19, Q20, Q21, Q22, Q23, Q27, Q28, Q29, Q30, Q31, Q32, Q33, Q34, Q35, Q36, Q37, Q38, Q39, Q40, Q41, Q42, Q43, Q44, Q45, Q46, Q51, Q52, Q53, Q54, Q55, Q56, Q57, Q58, Q59, Q60, Q61, Q62, Q63, Q64, Q65, Q66, Q67, Q68, Q69, Q70, Q71, Q77, Q78, Q79, Q80, Q81, Q82, Q83, Q84, Q85, Q86, Q87, Q88, Q89, Q90, Q91, Q92, Q93, Q94, Q95, Q96, Q97, Q104, Q105, Q106, Q107, Q108, Q109, Q114, Q115, Q116, Q117, Q118, Q119, Q120, Q121, Q124, Q125, Q126, Q127, Q128, Q129, Q130, Q131, Q132, Q133, Q134, Q135, Q136, Q137, Q138, Q139, Q140, Q141, Q142, Q143, Q144, Q145, Q146, Q147, Q148, Q149, Q150, Q151, Q152 und wobei als Substituenten jeweils in Frage kommen: Cyano, Fluor, Chlor, Brom, Methyl, Ethyl, n-Propyl, i-Propyl, Cyclopropyl, Trifluormethyl, Pentafluoroethyl, Methoxy, Ethoxy, Trifluormethoxy, Trifluorethoxy, Pentafluorethoxy oder Trifluormethylthio,

$R^3$ steht ganz besonders bevorzugt für Wasserstoff, Cyano, Halogen, $(C_1-C_4)$Alkyl, $(C_1-C_4)$Halogenalkyl, $(C_1-C_4)$Halogenalkoxy, $(C_1-C_4)$Alkylthio, $(C_1-C_4)$Alkylsulfinyl, $(C_1-C_4)$Alkylsulfonyl, $(C_1-C_4)$Halogenalkylthio, $(C_1-C_4)$Halogenalkylsulfinyl, $(C_1-C_4)$Halogenalkylsulfonyl oder NHCO-$(C_1-C_4)$alkyl ($(C_1-C_4)$Alkylcarbonylamino),

X steht ganz besonders bevorzugt für ein heteroaromatisches 9-gliedriges oder 12-gliedriges annelliertes bicyclisches oder tricyclisches Ringsystem aus der Reihe H1, H2, H3, H4, H5, H6, H7, H8, H9, H10, H11, H12, H13, H14, H15, H16, H17, H18, H19 oder H20,

$R^5$ steht ganz besonders bevorzugt für Fluor, Chlor, Fluormethyl, Difluormethyl, Trifluormethyl, Fluorethyl, Difluorethyl, Trifluorethyl, Tetrafluorethyl, Pentafluorethyl, Trifluormethoxy, Difluorchlormethoxy, Dichlorfluormethoxy, Trifluormethylthio, Trifluormethylsulfonyl oder Trifluormethylsulfinyl,

$R^6$ steht ganz besonders bevorzugt für Wasserstoff,

$R^7$ steht ganz besonders bevorzugt für Wasserstoff, Methyl, Ethyl, n-Propyl, i-Propyl, n-Butyl, i-Butyl, tert.-Butyl,

Methoxymethyl oder Methoxyethyl,

n steht ganz besonders bevorzugt für 0, 1 oder 2.

Ausgestaltung 4-3

$A^1$ steht ganz besonders bevorzugt für Stickstoff,

$R^1$ steht ganz besonders bevorzugt für Methyl, Ethyl, n-Propyl, i-Propyl, cyclo-Propyl, n-Butyl, i-Butyl, tert.-Butyl, cyclo-Butyl, Fluormethyl, Difluormethyl, Trifluormethyl, Fluorethyl, Difluorethyl, Trifluorethyl, Tetrafluorethyl oder Pentafluorethyl,

$R^2$ steht ganz besonders bevorzugt für ein gegebenenfalls einfach oder zweifach, gleich oder verschieden substituiertes, annelliertes bicyclisches Ringsystem aus der Reihe Ql, Q6, Q11, Q12, Q15, Q16, Q17, Q20, Q27, Q28, Q32, Q33, Q34, Q35, Q37, Q38, Q51, Q56, Q57, Q61, Q62, Q66, Q67, Q69, Q82, Q87, Q105, Q106, Q107 oder Q152 und wobei als Substituenten jeweils in Frage kommen: Cyano, Fluor, Chlor, Brom, Methyl, Ethyl, n-Propyl, i-Propyl, Cyclopropyl, Trifluormethyl, Pentafluoroethyl, Methoxy, Ethoxy, Trifluormethoxy, Trifluorethoxy, Pentafluorethoxy oder Trifluormethylthio,

$R^3$ steht ganz besonders bevorzugt für Wasserstoff, Cyano, Halogen, $(C_1-C_4)$Alkyl, $(C_1-C_4)$Halogenalkyl, $(C_1-C_4)$Halogenalkoxy, $(C_1-C_4)$Alkylthio, $(C_1-C_4)$Alkylsulfinyl, $(C_1-C_4)$Alkylsulfonyl, $(C_1-C_4)$Halogenalkylthio, $(C_1-C_4)$Halogenalkylsulfinyl, $(C_1-C_4)$Halogenalkylsulfonyl oder NHCO-$(C_1-C_4)$alkyl ($(C_1-C_4)$Alkylcarbonylamino),

X steht ganz besonders bevorzugt für ein heteroaromatisches 9-gliedriges oder 12-gliedriges annelliertes bicyclisches oder tricyclisches Ringsystem aus der Reihe H1, H2, H3, H4, H5, H6, H7, H8, H9, H10, H11, H12, H13, H14, H15, H16, H17, H18, H19 oder H20,

$R^5$ steht ganz besonders bevorzugt für Fluor, Chlor, Fluormethyl, Difluormethyl, Trifluormethyl, Fluorethyl, Difluorethyl, Trifluorethyl, Tetrafluorethyl, Pentafluorethyl, Trifluormethoxy, Difluorchlormethoxy, Dichlorfluormethoxy, Trifluormethylthio, Trifluormethylsulfonyl oder Trifluormethylsulfinyl,

$R^6$ steht ganz besonders bevorzugt für Wasserstoff,

$R^7$ steht ganz besonders bevorzugt für Wasserstoff, Methyl, Ethyl, n-Propyl, i-Propyl, n-Butyl, i-Butyl, tert.-Butyl, Methoxymethyl oder Methoxyethyl,

n steht ganz besonders bevorzugt für 0, 1 oder 2.

Ausgestaltung 5-1

$A^1$ steht hervorgehoben für Stickstoff,

$R^1$ steht hervorgehoben für Ethyl,

$R^2$ steht hervorgehoben für einen einfach oder zweifach, gleich oder verschieden durch Chlor, Cyano, Methyl oder Trifluormethyl substituierten Ring aus der Reihe Ql, Q6, Q32, Q33, Q34, Q35, Q37 oder Q51,

$R^3$ steht hervorgehoben für Wasserstoff,

X steht hervorgehoben für ein heteroaromatisches Ringsystem aus der Reihe H20,

$R^5$ steht hervorgehoben für Trifluormethyl,

$R^6$ steht hervorgehoben für Wasserstoff,

$R^7$ steht hervorgehoben für Methyl,

n steht hervorgehoben für 2.

Ausgestaltung 5-2

$A^1$ steht hervorgehoben für Stickstoff,

$R^1$ steht hervorgehoben für Ethyl,

$R^2$ steht hervorgehoben für ein einfach oder zweifach, gleich oder verschieden durch Chlor, Brom, Cyano, Methyl, Methoxy oder Trifluormethyl substituiertes, annelliertes bicyclisches Ringsystem aus der Reihe Q1, Q6, Q11, Q12, Q15, Q16, Q17, Q20, Q27, Q28, Q32, Q33, Q34, Q35, Q37, Q38,Q51, Q56, Q57, Q61, Q62, Q66, Q67, Q69, Q82, Q87, Q105, Q106, Q107 oder Q152,

$R^3$ steht hervorgehoben für Wasserstoff,

X steht hervorgehoben für ein heteroaromatisches Ringsystem aus der Reihe H20,

$R^5$ steht hervorgehoben für Trifluormethyl,

$R^6$ steht hervorgehoben für Wasserstoff,

$R^7$ steht hervorgehoben für Methyl,

n steht hervorgehoben für 1 oder 2.

In einer bevorzugten Ausführungsform betrifft die Erfindung Verbindungen der Formel (I), wobei X für H1 steht und $A^1$, $R^1$, $R^2$, $R^3$, $R^4$, $R^5$, $R^6$, $R^7$ und n die in Ausgestaltung (3-1) oder Ausgestaltung (3-2) oder Ausgestaltung (4-1) oder Ausgestaltung (4-2) oder Ausgestaltung (4-3) oder Ausgestaltung (5-1) oder Ausgestaltung (5-2) beschriebenen Bedeutungen haben.

In einer bevorzugten Ausführungsform betrifft die Erfindung Verbindungen der Formel (I), wobei X für H2 steht und $A^1$, $R^1$, $R^2$, $R^3$, $R^4$, $R^5$, $R^6$, $R^7$ und n die in Ausgestaltung (3-1) oder Ausgestaltung (3-2) oder Ausgestaltung (4-1) oder Ausgestaltung (4-2) oder Ausgestaltung (4-3) oder Ausgestaltung (5-1) oder Ausgestaltung (5-2) beschriebenen Bedeutungen haben.

In einer bevorzugten Ausführungsform betrifft die Erfindung Verbindungen der Formel (I), wobei X für H3 steht und $A^1$, $R^1$, $R^2$, $R^3$, $R^4$, $R^5$, $R^6$ und n die in Ausgestaltung (3-1) oder Ausgestaltung (3-2) oder Ausgestaltung (4-1) oder Ausgestaltung (4-2) oder Ausgestaltung (4-3) oder Ausgestaltung (5-1) oder Ausgestaltung (5-2) beschriebenen Bedeutungen haben.

In einer bevorzugten Ausführungsform betrifft die Erfindung Verbindungen der Formel (I), wobei X für H4 steht und $A^1$, $R^1$, $R^2$, $R^3$, $R^4$, $R^5$, $R^6$ und n die in Ausgestaltung (3-1) oder Ausgestaltung (3-2) oder Ausgestaltung (4-1) oder Ausgestaltung (4-2) oder Ausgestaltung (4-3) oder Ausgestaltung (5-1) oder Ausgestaltung (5-2) beschriebenen Bedeutungen haben.

In einer bevorzugten Ausführungsform betrifft die Erfindung Verbindungen der Formel (I), wobei X für H5 steht und $A^1$, $R^1$, $R^2$, $R^3$, $R^4$, $R^5$, $R^6$ und n die in Ausgestaltung (3-1) oder Ausgestaltung (3-2) oder Ausgestaltung (4-1) oder Ausgestaltung (4-2) oder Ausgestaltung (4-3) oder Ausgestaltung (5-1) oder Ausgestaltung (5-2) beschriebenen Bedeutungen haben.

In einer bevorzugten Ausführungsform betrifft die Erfindung Verbindungen der Formel (I), wobei X für H6 steht und $A^1$, $R^1$, $R^2$, $R^3$, $R^4$, $R^5$, $R^6$ und n die in Ausgestaltung (3-1) oder Ausgestaltung (3-2) oder Ausgestaltung (4-1) oder Ausgestaltung (4-2) oder Ausgestaltung (4-3) oder Ausgestaltung (5-1) oder Ausgestaltung (5-2) beschriebenen Bedeutungen haben.

In einer bevorzugten Ausführungsform betrifft die Erfindung Verbindungen der Formel (I), wobei X für H7 steht und $A^1$, $R^1$, $R^2$, $R^3$, $R^4$, $R^5$, $R^6$ und n die in Ausgestaltung (3-1) oder Ausgestaltung (3-2) oder Ausgestaltung (4-1) oder Ausgestaltung (4-2) oder Ausgestaltung (4-3) oder Ausgestaltung (5-1) oder Ausgestaltung (5-2) beschriebenen Bedeutungen haben.

In einer bevorzugten Ausführungsform betrifft die Erfindung Verbindungen der Formel (I), wobei X für H8 steht

und $A^1$, $R^1$, $R^2$, $R^3$, $R^4$, $R^5$, $R^6$ und n die in Ausgestaltung (3-1) oder Ausgestaltung (3-2) oder Ausgestaltung (4-1) oder Ausgestaltung (4-2) oder Ausgestaltung (4-3) oder Ausgestaltung (5-1) oder Ausgestaltung (5-2) beschriebenen Bedeutungen haben.

In einer bevorzugten Ausführungsform betrifft die Erfindung Verbindungen der Formel (I), wobei X für H9 steht und $A^1$, $R^1$, $R^2$, $R^3$, $R^4$, $R^5$, $R^6$ und n die in Ausgestaltung (3-1) oder Ausgestaltung (3-2) oder Ausgestaltung (4-1) oder Ausgestaltung (4-2) oder Ausgestaltung (4-3) oder Ausgestaltung (5-1) oder Ausgestaltung (5-2) beschriebenen Bedeutungen haben.

In einer bevorzugten Ausführungsform betrifft die Erfindung Verbindungen der Formel (I), wobei X für H10 steht und $A^1$, $R^1$, $R^2$, $R^3$, $R^4$, $R^5$, $R^6$ und n die in Ausgestaltung (3-1) oder Ausgestaltung (3-2) oder Ausgestaltung (4-1) oder Ausgestaltung (4-2) oder Ausgestaltung (4-3) oder Ausgestaltung (5-1) oder Ausgestaltung (5-2) beschriebenen Bedeutungen haben.

In einer bevorzugten Ausführungsform betrifft die Erfindung Verbindungen der Formel (I), wobei X für H11 steht und $A^1$, $R^1$, $R^2$, $R^3$, $R^4$, $R^5$, $R^6$ und n die in Ausgestaltung (3-1) oder Ausgestaltung (3-2) oder Ausgestaltung (4-1) oder Ausgestaltung (4-2) oder Ausgestaltung (4-3) oder Ausgestaltung (5-1) oder Ausgestaltung (5-2) beschriebenen Bedeutungen haben.

In einer bevorzugten Ausführungsform betrifft die Erfindung Verbindungen der Formel (I), wobei X für H12 steht und $A^1$, $R^1$, $R^2$, $R^3$, $R^4$, $R^5$, $R^6$ und n die in Ausgestaltung (3-1) oder Ausgestaltung (3-2) oder Ausgestaltung (4-1) oder Ausgestaltung (4-2) oder Ausgestaltung (4-3) oder Ausgestaltung (5-1) oder Ausgestaltung (5-2) beschriebenen Bedeutungen haben.

In einer bevorzugten Ausführungsform betrifft die Erfindung Verbindungen der Formel (I), wobei X für H13 steht und $A^1$, $R^1$, $R^2$, $R^3$, $R^4$, $R^5$, $R^6$ und n die in Ausgestaltung (3-1) oder Ausgestaltung (3-2) oder Ausgestaltung (4-1) oder Ausgestaltung (4-2) oder Ausgestaltung (4-3) oder Ausgestaltung (5-1) oder Ausgestaltung (5-2) beschriebenen Bedeutungen haben.

In einer bevorzugten Ausführungsform betrifft die Erfindung Verbindungen der Formel (I), wobei X für H14 steht und $A^1$, $R^1$, $R^2$, $R^3$, $R^4$, $R^5$, $R^6$, $R^7$ und n die in Ausgestaltung (3-1) oder Ausgestaltung (3-2) oder Ausgestaltung (4-1) oder Ausgestaltung (4-2) oder Ausgestaltung (4-3) oder Ausgestaltung (5-1) oder Ausgestaltung (5-2) beschriebenen Bedeutungen haben.

In einer bevorzugten Ausführungsform betrifft die Erfindung Verbindungen der Formel (I), wobei X für H15 steht und $A^1$, $R^1$, $R^2$, $R^3$, $R^4$, $R^5$, $R^6$ und n die in Ausgestaltung (3-1) oder Ausgestaltung (3-2) oder Ausgestaltung (4-1) oder Ausgestaltung (4-2) oder Ausgestaltung (4-3) oder Ausgestaltung (5-1) oder Ausgestaltung (5-2) beschriebenen Bedeutungen haben.

In einer bevorzugten Ausführungsform betrifft die Erfindung Verbindungen der Formel (I), wobei X für H16 steht und $A^1$, $R^1$, $R^2$, $R^3$, $R^4$, $R^5$, $R^6$ und n die in Ausgestaltung (3-1) oder Ausgestaltung (3-2) oder Ausgestaltung (4-1) oder Ausgestaltung (4-2) oder Ausgestaltung (4-3) oder Ausgestaltung (5-1) oder Ausgestaltung (5-2) beschriebenen Bedeutungen haben.

In einer bevorzugten Ausführungsform betrifft die Erfindung Verbindungen der Formel (I), wobei X für H17 steht und $A^1$, $R^1$, $R^2$, $R^3$, $R^4$, $R^5$, $R^6$, $R^7$ und n die in Ausgestaltung (3-1) oder Ausgestaltung (3-2) oder Ausgestaltung (4-1) oder Ausgestaltung (4-2) oder Ausgestaltung (4-3) oder Ausgestaltung (5-1) oder Ausgestaltung (5-2) beschriebenen Bedeutungen haben.

In einer bevorzugten Ausführungsform betrifft die Erfindung Verbindungen der Formel (I), wobei X für H18 steht und $A^1$, $R^1$, $R^2$, $R^3$, $R^4$, $R^5$, $R^6$ und n die in Ausgestaltung (3-1) oder Ausgestaltung (3-2) oder Ausgestaltung (4-1) oder Ausgestaltung (4-2) oder Ausgestaltung (4-3) oder Ausgestaltung (5-1) oder Ausgestaltung (5-2) beschriebenen Bedeutungen haben.

In einer bevorzugten Ausführungsform betrifft die Erfindung Verbindungen der Formel (I), wobei X für H19 steht und $A^1$, $R^1$, $R^2$, $R^3$, $R^4$, $R^6$, $R^7$ und n die in Ausgestaltung (3-1) oder Ausgestaltung (3-2) oder Ausgestaltung (4-1) oder Ausgestaltung (4-2) oder Ausgestaltung (4-3) oder Ausgestaltung (5-1) oder Ausgestaltung (5-2) beschriebenen Bedeutungen haben.

In einer bevorzugten Ausführungsform betrifft die Erfindung Verbindungen der Formel (I), wobei X für H20 steht und $A^1$, $R^1$, $R^2$, $R^3$, $R^4$, $R^5$, $R^6$, $R^7$ und n die in Ausgestaltung (3-1) oder Ausgestaltung (3-2) oder Ausgestaltung (4-1) oder Ausgestaltung (4-2) oder Ausgestaltung (4-3) oder Ausgestaltung (5-1) oder Ausgestaltung (5-2) beschriebenen Bedeutungen haben.

In einer bevorzugten Ausführungsform betrifft die Erfindung Verbindungen der Formel (I), wobei X für

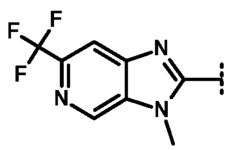

steht
und $A^1$, $R^1$, $R^2$, $R^3$, $R^4$ und n die in Ausgestaltung (3-1) oder Ausgestaltung (3-2) oder Ausgestaltung (4-1) oder Ausgestaltung (4-2) oder Ausgestaltung (4-3) oder Ausgestaltung (5-1) oder Ausgestaltung (5-2) beschriebenen Bedeutungen haben.

In einer bevorzugten Ausführungsform betrifft die Erfindung Verbindungen der Formel (I), wobei $A^1$ für Stickstoff steht und X, $R^1$, $R^2$, $R^3$, $R^4$, $R^5$, $R^6$, $R^7$ und n die in Ausgestaltung (3-1) oder Ausgestaltung (3-2) oder Ausgestaltung (4-1) oder Ausgestaltung (4-2) oder Ausgestaltung (4-3) oder Ausgestaltung (5-1) oder Ausgestaltung (5-2) beschriebenen Bedeutungen haben.

In den bevorzugten Definitionen ist, sofern nichts anderes angegeben ist,

Halogen ausgewählt aus der Reihe Fluor, Chlor, Brom und Iod, bevorzugt wiederum aus der Reihe Fluor, Chlor und Brom.

In den besonders bevorzugten Definitionen ist, sofern nichts anderes angegeben ist,

Halogen ausgewählt aus der Reihe Fluor, Chlor, Brom und Iod, bevorzugt wiederum aus der Reihe Fluor, Chlor und Brom,

Sofern nicht an anderer Stelle anders definiert, wird unter dem Begriff "Alkyl", entweder in Alleinstellung oder aber in Kombination mit weiteren Begriffen, wie beispielsweise Halogenalkyl, im Rahmen der vorliegenden Erfindung ein Rest einer gesättigten, aliphatischen Kohlenwasserstoffgruppe mit 1 bis 12 Kohlenstoffatomen verstanden, die verzweigt oder unverzweigt sein kann. Beispiele für $C_1$-$C_{12}$-Alkylreste sind Methyl, Ethyl, n-Propyl, iso-Propyl, n-Butyl, iso-Butyl, sek.-Butyl, tert.-Butyl, n-Pentyl, iso-Pentyl, Neopentyl, tert.-Pentyl, 1-Methylbutyl, 2-Methylbutyl, 1-Ethylpropyl, 1,2-Dimethylpropyl, Hexyl n-Heptyl, n-Octyl, n-Nonyl, n-Decyl, n-Undecyl und n-Dodecyl. Von diesen Alkylresten sind $C_1$-$C_6$-Alkylreste besonders bevorzugt. Insbesondere bevorzugt sind $C_1$-$C_4$-Alkylreste.

Sofern nicht an anderer Stelle anders definiert, wird unter dem Begriff "Alkenyl", entweder in Alleinstellung oder aber in Kombination mit weiteren Begriffen, erfindungsgemäß ein linearer oder verzweigter $C_2$-$C_{12}$-Alkenylrest, welcher mindestens eine Doppelbindung aufweist, beispielsweise Vinyl, Allyl, 1-Propenyl, Isopropenyl, 1-Butenyl, 2-Butenyl, 3-Butenyl, 1,3-Butadienyl, 1-Pentenyl, 2-Pentenyl, 3-Pentenyl, 4-Pentenyl, 1,3-Pentadienyl, 1-Hexenyl, 2-Hexenyl, 3-Hexenyl, 4-Hexenyl, 5-Hexenyl und 1,4-Hexadienyl, verstanden. Bevorzugt hiervon sind $C_2$-$C_6$-Alkenylreste und besonders bevorzugt sind $C_2$-$C_4$-Alkenylreste.

Sofern nicht an anderer Stelle anders definiert, wird unter dem Begriff "Alkinyl", entweder in Alleinstellung oder aber in Kombination mit weiteren Begriffen, erfindungsgemäß ein linearer oder verzweigter $C_2$-$C_{12}$-Alkinylrest, welcher mindestens eine Dreifachbindung aufweist, beispielsweise Ethinyl, 1-Propinyl und Propargyl, verstanden. Bevorzugt hiervon sind $C_3$-$C_6$-Alkinylreste und besonders bevorzugt sind $C_3$-$C_4$-Alkinylreste. Der Alkinylrest kann dabei auch mindestens eine Doppelbindung aufweisen.

Sofern nicht an anderer Stelle anders definiert, wird unter dem Begriff "Cycloalkyl", entweder in Alleinstellung oder aber in Kombination mit weiteren Begriffen, erfindungsgemäß ein $C_3$-$C_8$-Cycloalkylrest verstanden, beispielsweise Cyclopropyl, Cyclobutyl, Cyclopentyl, Cyclohexyl, Cycloheptyl und Cyclooctyl, verstanden. Bevorzugt hiervon sind $C_3$-$C_6$-Cycloalkylreste.

Unter dem Begriff "Alkoxy", entweder in Alleinstellung oder aber in Kombination mit weiteren Begriffen, wie beispielsweise Halogenalkoxy, wird vorliegend ein Rest O-Alkyl verstanden, wobei der Begriff "Alkyl" die oben stehende Bedeutung aufweist.

Durch Halogen substituierte Reste, z.B. Halogenalkyl (=Haloalkyl), sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder Iod, insbesondere für Fluor, Chlor oder Brom.

Gegebenenfalls substituierte Reste können, wenn nichts anderes erwähnt ist, einfach oder mehrfach substituiert sein, wobei bei Mehrfachsubstitutionen die Substituenten gleich oder verschieden sein können.

Die oben aufgeführten allgemeinen oder in Vorzugsbereichen aufgeführten Restedefinitionen bzw. Erläuterungen gelten für die Endprodukte und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt verwendet werden Verbindungen der Formel (I), in welchen eine Kombination der vorstehend als bevorzugt aufgeführten Bedeutungen vorliegt.

Erfindungsgemäß besonders bevorzugt verwendet werden Verbindungen der Formel (I), in welchen eine Kombination der vorstehend als besonders bevorzugt aufgeführten Bedeutungen vorliegt.

Erfindungsgemäß ganz besonders bevorzugt verwendet werden Verbindungen der Formel (I), in welchen eine Kombination der vorstehend als ganz besonders bevorzugt aufgeführten Bedeutungen vorliegt.

Erfindungsgemäß hervorgehoben verwendet werden Verbindungen der Formel (I), in welchen eine Kombination der vorstehend als hervorgehoben aufgeführten Bedeutungen vorliegt.

Die Verbindungen der Formel (I) können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische

Isomere. Die Erfindung umfasst somit reine Stereoisomere als auch beliebige Gemische dieser Isomere.

Die erfindungsgemäßen Verbindungen der Formel (I) können durch die in den folgenden Schemata dargestellten Verfahren erhalten werden:

Verfahren A

Die Verbindungen der Formel (I), bei denen X für H1, H2, H4, H5, H6, H7, H8, H9, H14, H19 oder H20 steht, können nach bekannten Methoden hergestellt werden, beispielsweise analog der in WO2009/131237, WO2010/125985, WO2011/043404, WO2011/040629, WO2012/086848, WO2013/018928, WO2015/000715 oder WO2015/133603 beschriebenen Verfahren.

Das allgemeine Verfahren für die Herstellung von Verbindungen der Formel (I), bei denen X für H1, H2, H4, H5, H6, H7, H8, H9, H14, H19 oder H20 steht, wird nachfolgend anhand von Beispiel H2, H5 bzw. H8 beschrieben.

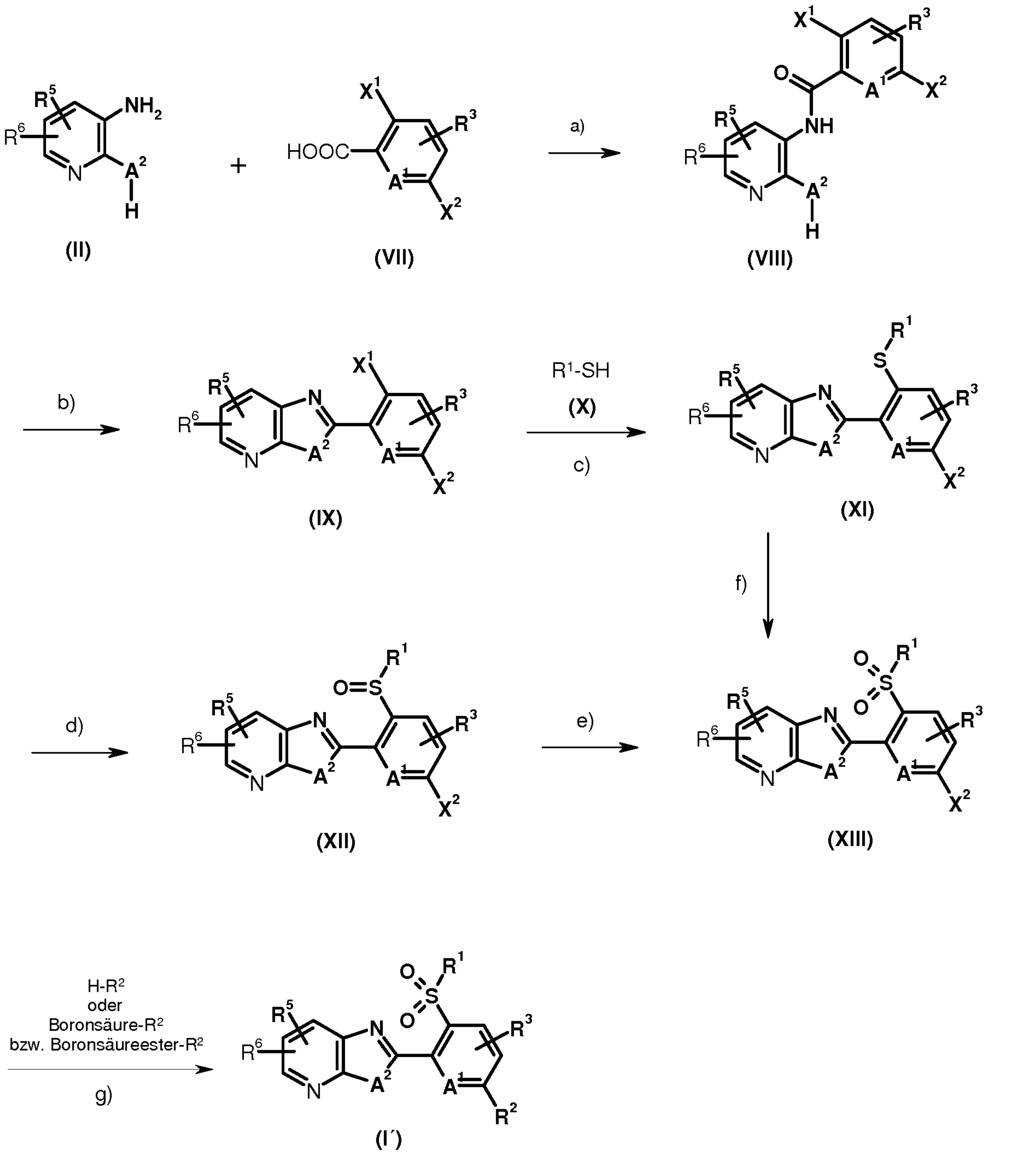


Die Reste $R^1$, $R^2$, $R^3$, $R^5$, $R^6$ und $A^1$ haben die oben beschriebenen Bedeutungen, $A^2$ steht für -N-$R^7$, O oder

S, wobei $R^7$ die oben beschriebene Bedeutung hat und $X^1$ bzw. $X^2$ stehen für Halogen.

**Schritt a)**

Die Verbindungen der Formel (VIII) können in Analogie zu dem in US5576335 beschriebenen Verfahren durch die Umsetzung von Verbindungen der Formel (II) mit einer Carbonsäure der Formel (VII) in Gegenwart eines Kondensationsmittels bzw. einer Base hergestellt werden.

Verbindungen der Formel (II) sind entweder kommerziell erhältlich oder können nach bekannten Methoden hergestellt werden, beispielsweise analog der in US2003/69257 oder WO2006/65703 beschriebenen Verfahren.

Carbonsäuren der Formel (VII) sind entweder kommerziell erhältlich oder können nach bekannten Methoden hergestellt werden, beispielsweise analog der in US2010/234604, WO2012/61926 oder Bioorganic and Medicinal Chemistry Letters, 18 (2008), 5023-5026 beschriebenen Verfahren.

Die Umsetzung der Verbindungen der Formel (II) mit Carbonsäuren der Formel (VII) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan; halogenierte Kohlenwasserstoffe wie beispielsweise Dichlormethan, Chloroform, Tetrachlorkohlenstoff, 1,2-Dichlorethan oder Chlorbenzol; Nitrile, wie beispielsweise Acetonitril oder Propionitril; aromatische Kohlenwasserstoffe wie beispielsweise Toluol, oder Xylol; aprotische polare Lösungsmittel wie beispielsweise N,N-Dimethylformamid oder N-Methylpyrrolidon oder Stickstoffhaltige Verbindungen wie beispielsweise Pyridin.

Geeignete Kondensationsmittel sind beispielsweise Carbodiimide wie 1-(3-Dimethylaminopropyl)-3-ethylcarbodiimid hydrochlorid (EDCI) oder 1,3-Dicyclohexylcarbodiimid.

Geeignete Basen sind anorganische Basen, die üblicherweise in solchen Reaktionen verwendet werden. Vorzugsweise werden Basen verwendet, die beispielhaft ausgewählt sind aus der Gruppe bestehend aus Acetaten, Phosphaten, Carbonaten und Hydrogencarbonaten von Alkali- oder Erdalkalimetallen. Besonders bevorzugt sind dabei Natriumacetat, Natriumphosphat, Kaliumphosphat, Caesiumcarbonat, Natriumcarbonat, Kaliumcarbonat, Natriumhydrogencarbonat, Kaliumhydrogencarbonat.

Die Reaktion kann im Vakuum, bei Normaldruck oder unter Überdruck und bei Temperaturen von 0 °C bis 180 °C durchgeführt werden, vorzugsweise erfolgt die Reaktion bei Normaldruck und Temperaturen von 20 bis 140 °C.

**Schritt** b)

Die Verbindungen der Formel (IX) lassen sich herstellen durch Kondensation der Verbindungen der Formel (VIII) z.B. analog der in WO2012/86848 beschriebenen Verfahren.

Die Umsetzung zu Verbindungen der Formel (IX) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan, tert.-Butylmethylether; halogenierte Kohlenwasserstoffe wie beispielsweise Dichlormethan, Chloroform, Tetrachlorkohlenstoff, 1,2-Dichlorethan oder Chlorbenzol; Nitrile, wie beispielsweise Acetonitril oder Propionitril; aromatische Kohlenwasserstoffe wie beispielsweise Toluol oder Xylol; aprotische polare Lösungsmittel wie beispielsweise N,N-Dimethylformamid oder N-Methylpyrrolidon oder stickstoffhaltige Verbindungen wie beispielsweise Pyridin.

Die Reaktion lässt sich durchführen in Gegenwart eines Kondensationsmittels, einer Säure, einer Base oder eines Chlorierungsmittels.

Beispiele für geeignete Kondensationsmittel sind Carbodiimide wie 1-(3-Dimethylaminopropyl)-3-ethylcarbodiimid hydrochlorid (EDCI) oder 1,3-Dicyclohexylcarbodiimid; Anhydride wie Essigsäureanhydrid, Trifluoressigsäureanhydrid; eine Mischung aus Triphenylphosphin, einer Base und Tetrachlorkohlenstoff oder eine Mischung aus Triphenylphosphin und einem Azodiester wie z.B. Diethylazodicarbonsäure.

Beispiele für geeignete Säuren, die in der beschriebenen Reaktion eingesetzt werden können, sind Sulfonsäuren wie para-Toluolsulfonsäure; Carbonsäuren wie Essigsäure oder Polyphosphorsäuren.

Beispiele für geeignete Basen sind stickstoffhaltige Heterocyclen wie Pyridin, Picolin, 2,6-Lutidin, 1,8-Diazabicyclo[5.4.0]-7-undecen (DBU); tertiäre Amine wie Triethylamin und N,N-Diisopropylethylamin; anorganische Basen wie Kaliumphosphat, Kaliumcarbonat und Natriumhydrid.

Ein Beispiel für ein geeignetes Chlorierungsmittel ist Phosphoroxychlorid.

Die Reaktion kann im Vakuum, bei Normaldruck oder unter Überdruck und bei Temperaturen von 0 °C bis 200 °C durchgeführt werden.

**Schritt c)**

Die Verbindungen der Formel (XI), lassen sich herstellen durch Umsetzung der Verbindungen der Formel (IX) mit den Verbindungen der Formel (X) in Gegenwart einer Base.

Mercaptanderivate der Formel (X) wie beispielsweise Methylmercaptan, Ethylmercaptan oder Isopropylmercaptan sind entweder kommerziell erhältlich oder können nach bekannten Methoden hergestellt werden, beispielsweise analog der in US2006/25633, US2006/111591, US2820062, Chemical Communications, 13 (2000), 1163-1164 oder Journal of the American Chemical Society, 44 (1922), p. 1329 beschriebenen Verfahren.

Die Umsetzung zu Verbindungen der Formel (XI) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan, tert.-Butylmethylether; Nitrile, wie beispielsweise Acetonitril oder Propionitril; aromatische Kohlenwasserstoffe wie beispielsweise Toluol oder Xylol; aprotische polare Lösungsmittel wie beispielsweise N,N-Dimethylformamid, N-Methylpyrrolidon oder Dimethylsulfoxid.

Beispiele für geeignete Basen sind anorganische Basen aus der Gruppe bestehend aus Acetaten, Phosphaten und Carbonaten von Alkali- oder Erdalkalimetallen. Bevorzugt sind dabei Caesiumcarbonat, Natriumcarbonat und Kaliumcarbonat. Weitere geeignete Basen sind Alkalimetallhydride wie z.B. Natriumhydrid.

Die Reaktion kann im Vakuum, bei Normaldruck oder unter Überdruck und bei Temperaturen von 0 °C bis 200 °C durchgeführt werden.

**Schritt d)**

Die Verbindungen der Formel (XII) lassen sich herstellen durch Oxidation der Verbindungen der Formel (XI). Die Oxidation wird generell in einem Lösungsmittel durchgeführt, welches ausgewählt ist aus üblichen, bei den vorherrschenden Reaktionsbedingungen inerten Lösungsmitteln. Bevorzugt werden halogenierte Kohlenwasserstoffe wie beispielsweise Dichlormethan, Chloroform, Tetrachlorkohlenstoff, 1,2-Dichlorethan oder Chlorbenzol; Alkohole wie Methanol oder Ethanol; Ameisensäure, Essigsäure, Propionsäure oder Wasser.

Beispiele für geeignete Oxidationsmittel sind Wasserstoffperoxid, meta-Chlorperbenzoesäure oder Natriumperiodat.

Die Reaktion kann im Vakuum, bei Normaldruck oder unter Überdruck und bei Temperaturen von - 20°C bis 120 °C durchgeführt werden.

**Schritt e)**

Die Verbindungen der Formel (XIII) lassen sich herstellen durch Oxidation der Verbindungen der Formel (XII). Die Oxidation wird generell in einem Lösungsmittel durchgeführt. Bevorzugt werden halogenierte Kohlenwasserstoffe wie beispielsweise Dichlormethan, Chloroform, Tetrachlorkohlenstoff, 1,2-Dichlorethan oder Chlorbenzol; Alkohole wie Methanol oder Ethanol; Ameisensäure, Essigsäure, Propionsäure oder Wasser.

Beispiele für geeignete Oxidationsmittel sind Wasserstoffperoxid und meta-Chlorperbenzoesäure.

Die Reaktion kann im Vakuum, bei Normaldruck oder unter Überdruck und bei Temperaturen von -20°C bis 120 °C durchgeführt werden.

**Schritt f)**

Die Verbindungen der Formel (XIII) lassen sich auch in einem einstufigen Prozess herstellen durch Oxidation der Verbindungen der Formel (XI). Die Oxidation wird generell in einem Lösungsmittel durchgeführt. Bevorzugt werden halogenierte Kohlenwasserstoffe wie beispielsweise Dichlormethan, Chloroform, Tetrachlorkohlenstoff, 1,2-Dichlorethan oder Chlorbenzol; Alkohole wie Methanol oder Ethanol; Ameisensäure, Essigsäure, Propionsäure oder Wasser.

Beispiele für geeignete Oxidationsmittel sind Wasserstoffperoxid und meta-Chlorperbenzoesäure.

Die Reaktion kann im Vakuum, bei Normaldruck oder unter Überdruck und bei Temperaturen von - 20°C bis 120 °C durchgeführt werden.

**Schritt g)**

Die Herstellung von Verbindungen der Formel (I') in denen $R^2$ für einen -zu dem Rest des Moleküls- N-verknüpften Bizyklus steht, kann beispielsweise aus Verbindungen der Formel (XIII), für die $X^2$ bevorzugt für Halogen aus der Reihe Chlor oder Brom steht, nach literaturbekannten Methoden (siehe z.B. Journal of Organic Chemistry (2010), 69, 5578) z.B. in Gegenwart von Kupfer(I)-iodid und basischen Reaktionshilfsmitteln, wie beispielsweise *trans*-N,N'-Dimethylcyclohexan-1,2-diamin und Kaliumcarbonat, in einem geeigneten Lösungs- oder Verdünnungsmittel erfolgen.

Die benötigten Verbindungen der Formel H-$R^2$ sind entweder kommerziell erhältlich oder können nach bekannten Methoden hergestellt werden.

Als Lösungs- oder Verdünnungsmittel kommen alle inerten organischen Lösungsmittel in Betracht, beispielsweise aliphatische oder aromatische Kohlenwasserstoffe. Bevorzugt wird dabei Toluol eingesetzt. Weiterhin kann die Kupplung aus Verbindungen der Formel (XIII), für die $X^2$ bevorzugt für Halogen aus der Reihe Fluor, Chlor oder Brom steht, ohne Metallkatalyse in Gegenwart einer geeigneten Base wie beispielsweise Kaliumcarbonat oder Cäsiumcarbonat in einem geeigneten Lösungs-oder Verdünnungsmittel erfolgen. Als Lösungs- oder Verdünnungsmittel kommen alle inerten organischen Lösungsmittel in Betracht, beispielsweise aliphatische oder aromatische Kohlenwasserstoffe. Bevorzugt wird dabei Acetonitril oder Dimethylformamid eingesetzt.

Verbindungen der Formel (I') für die $R^2$ für einen -zu dem Rest des Moleküls- C-verknüpften Bizyklus steht, können beispielsweise aus Verbindungen der Formel (XIII), für die $X^2$ bevorzugt für Halogen aus der Reihe Chlor oder Brom steht, nach allgemein bekannten Methoden (vgl. Chem. Rev. 1995, 95, 2457-2483; Tetrahedron 2002, 58, 9633-9695; Metal-Catalyzed Cross-Coupling Reactions (Eds.: A. de Meijere, F. Diederich), 2nd ed., Wiley-VCH, Weinheim, 2004) hergestellt werden.

Beispielsweise können Verbindungen der Formel (XIII), in denen $X^2$ bevorzugt für Chlor oder Brom steht, mit geeigneten Arylboronsäuren oder deren Estern nach bekannten Methoden (vgl. WO2010071819) in Gegenwart geeigneter Katalysatoren aus der Reihe der Übergangsmetallsalze zu Verbindungen der Formel (I'), umgesetzt werden. Als Kupplungskatalysatoren kommen beispielsweise bevorzugt Palladiumkatalysatoren wie [1,1'-Bis (diphenylphosphino)ferrocen]dichlorpalladium (II) oder Tetrakis(triphenylphosphin)palladium in Betracht. Als geeignete basische Reaktionshilfsmittel zur Durchführung der Verfahren finden bevorzugt Carbonate des Natriums oder Kaliums Verwendung.

Die benötigten (Hetero)arylboronsäuren oder (Hetero)arylboronsäureester sind teilweise bekannt und/oder kommerziell erhältlich bzw. können nach allgemein bekannten Methoden hergestellt werden (vgl. Boronic Acids (Eds.: D. G. Hall), 2nd ed., Wiley-VCH, Weinheim, 2011).

Die Umsetzung gemäß Schritt g) kann auch ausgehend von Verbindungen der Formeln (XI) oder (XII) erfolgen.

**Verfahren B**

Die Verbindungen der Formel (I), bei denen X für H10, H11, H15 oder H16 steht, können nach bekannten Methoden hergestellt werden, beispielsweise analog der in US2009/203705, US2012/258951, WO2013/3298 oder J. Med. Chem. 31, (1988) 1590-1595 beschriebenen Verfahren.

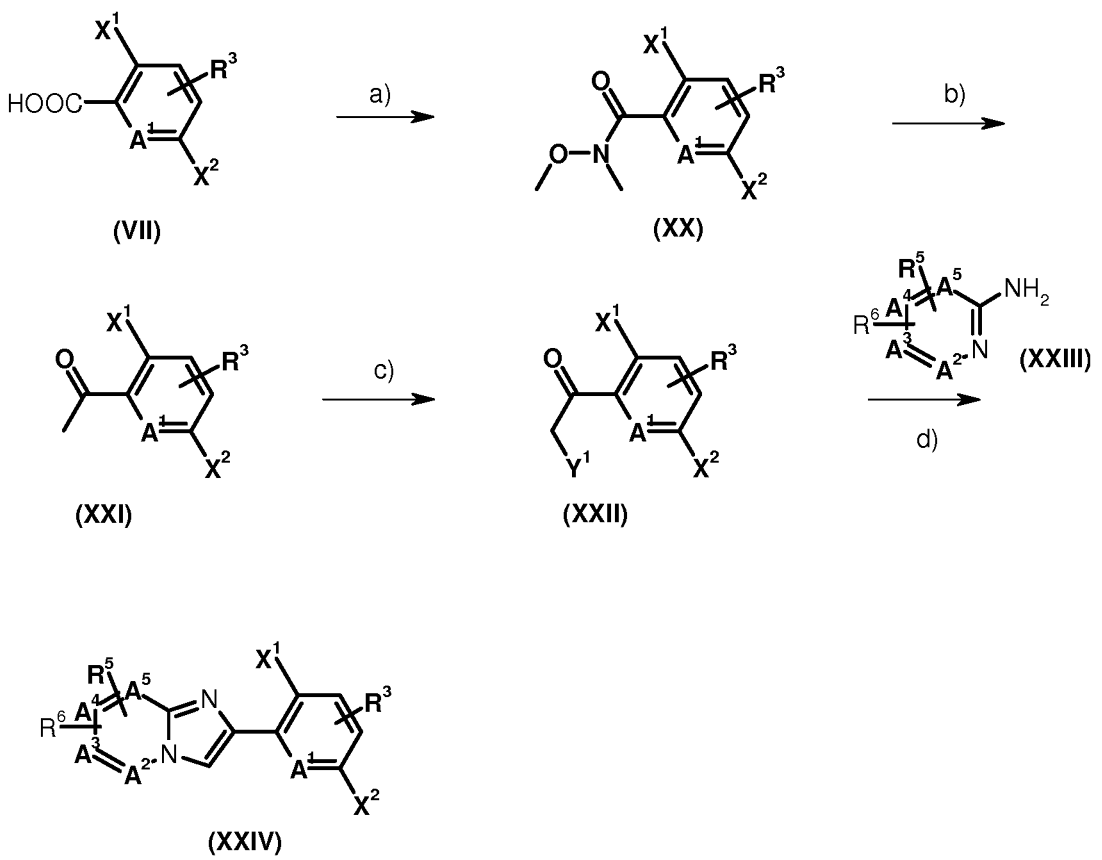


(VII) (XX) (XXI) (XXII) (XXIII) (XXIV)

Die Reste $A^1$, $R^3$, $R^5$ und $R^6$ haben die oben beschriebenen Bedeutungen. $X^1$, $X^2$ und $Y^1$ stehen für Halogen. $A^2$, $A^3$, $A^4$ und $A^5$ stehen unabhängig voneinander für CH oder N (wobei $A^2$, $A^3$, $A^4$ und $A^5$ nicht gleichzeitig für N stehen).

**Schritt a)**

Carbonsäuren der Formel (VII) werden in Analogie zu dem in WO2011/75643 oder EP-A-2671582 beschriebenen Verfahren in Gegenwart von O,N-Dimethyl-hydroxylamin Hydrochlorid in Weinreb-Amide der Formel (XX) überführt.

Carbonsäuren der Formel (VII) sind entweder kommerziell erhältlich oder können nach bekannten Methoden hergestellt werden, beispielsweise analog der in US2010/234604, WO2012/61926 oder Bioorganic and Medicinal Chemistry Letters, 18 (2008), 5023-5026 beschriebenen Verfahren.

**Schritt b, c)**

Verbindungen der Formel (XX) lassen sich anschließend nach bekannten Methoden, beispielsweise analog dem in WO2011/75643 beschriebenen Verfahren, mit einem Grignard Reagenz wie beispielsweise Methylmagnesiumbromid in Ketone der Formel (XXI) überführen. Durch anschließende Halogenierung analog der beispielsweise in US2012/302573 beschriebenen bekannten Methode sind Verbindungen der Formel (XXII) zugänglich.

**Schritt d)**

Die Verbindungen der Formel (XXIV) lassen sich herstellen durch Cyclisierung der Verbindungen der Formel (XXII) mit Aminen der Formel (XXIII). Die Cyclisierung erfolgt beispielsweise in Ethanol, Acetonitril oder N,N-Dimethylformamid nach bekannten Methoden analog der beispielsweise in WO2005/66177, WO2012/88411, WO2013/3298, US2009/203705, US2012/258951, WO2012/168733, WO2014/187762 oder J. Med. Chem. 31 (1988) 1590-1595 beschriebenen Verfahren.

Die Verbindungen der Formel (XXIII) sind kommerziell erhältlich.

Die weitere Umsetzung von Verbindungen der Formel (XXIV) zu Verbindungen der Formel (I) erfolgt analog zu Verfahren A.

**Verfahren C**

Die Verbindungen der Formel (I), bei denen X für H17 steht, können nach bekannten Methoden hergestellt werden, beispielsweise analog der in WO2014/142292 beschriebenen Verfahren.

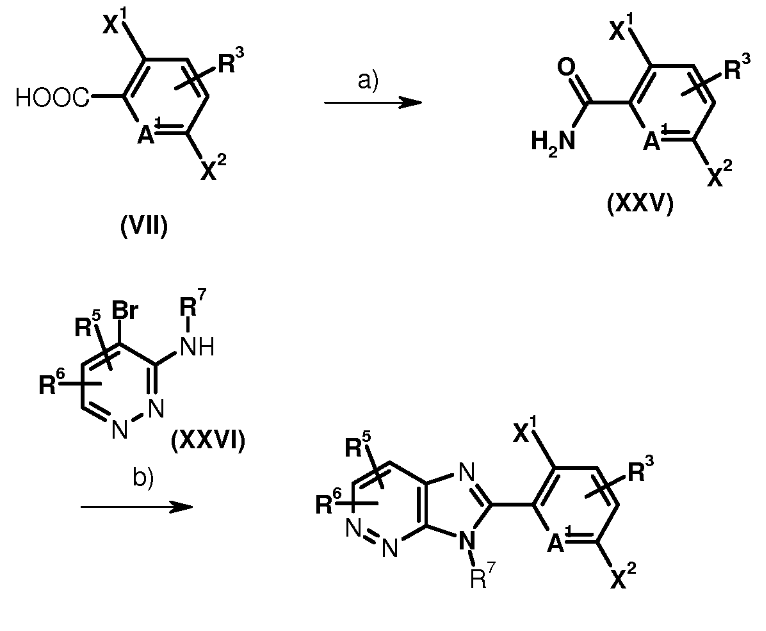


(VII) (XXV) (XXVI) (XXVII)

Die Reste $A^1$, $R^3$, $R^5$, $R^6$ und $R^7$ haben die oben beschriebenen Bedeutungen. $X^1$ und $X^2$ stehen für Halogen.

**Schritt a)**

Die Verbindungen der Formel (XXV) können in Analogie zu dem in US5374646 oder Bioorganic and Medicinal Chemistry Letters 2003, 13, 1093 - 1096 beschriebenen Verfahren durch die Umsetzung von Verbindungen der Formel (VII) mit einer Ammoniakquelle in Gegenwart eines Kondensationsmittels hergestellt werden.
Carbonsäuren der Formel (VII) sind entweder kommerziell erhältlich oder können nach bekannten Methoden hergestellt werden, beispielsweise analog der in US2010/234604, WO2012/61926 oder Bioorganic and Medicinal Chemistry Letters, 18 (2008), 5023-5026 beschriebenen Verfahren.
Die Umsetzung der Verbindungen der Formel (VII) mit der Ammoniakquelle wird vorzugsweise in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Dioxan oder Tetrahydrofuran.
Ein geeignetes Kondensationsmittel ist beispielsweise Carbonyldiimidazol.
Die Reaktion kann im Vakuum, bei Normaldruck oder unter Überdruck durchgeführt werden. Vorzugsweise erfolgt die Reaktion bei Normaldruck und Temperaturen von 20 bis 70 °C.

**Schritt b)**

Die Verbindungen der Formel (XXVII) können in Analogie zu dem in WO2014/142292 beschriebenen Verfahren durch die Umsetzung von Verbindungen der Formel (XXV) mit Verbindungen der Formel (XXVI) in Gegenwart eines Palladiumkatalysators im Basischen hergestellt werden.
Verbindungen der Formel (XXVI) können beispielsweise analog der in WO2014/142292 beschriebenen Verfahren hergestellt werden. Als Palladiumkatalysator kann beispielsweise [1,1'-Bis-(diphenylphosphino)ferrocen]dichlorpalladium(II) verwendet werden. Als Base finden häufig anorganische Basen wie Kaliumtertbutanolat Verwendung
Die Umsetzung erfolgt in einem Lösungsmittel. Häufig wird Toluol verwendet.
Die Reaktion kann im Vakuum, bei Normaldruck oder unter Überdruck durchgeführt werden. Vorzugsweise erfolgt die Reaktion bei Normaldruck und Temperaturen von 20 bis 110 °C.
Die weitere Umsetzung von Verbindungen der Formel (XXVII) zu Verbindungen der Formel (I) erfolgt analog zu Verfahren A.

Verfahren D

Die Verbindungen der Formel (I), bei denen X für H3, H12, H13 oder H18 steht, können nach bekannten Methoden hergestellt werden, beispielsweise analog der in WO2010/091310, WO 2012/66061 oder WO2013/099041 beschriebenen Verfahren.

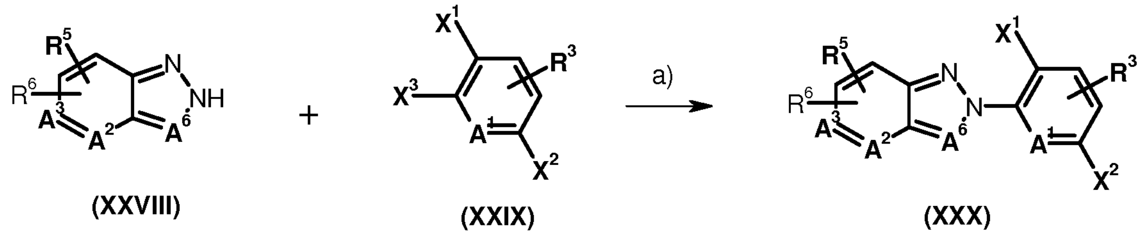


(XXVIII) (XXIX) (XXX)

Die Reste $A^1$, $R^3$, $R^5$ und $R^6$ haben die oben beschriebenen Bedeutungen. $A^2$, $A^3$ und $A^6$ stehen unabhängig voneinander für CH oder N (wobei $A^2$ und $A^3$ nicht gleichzeitig für N stehen können). $X^1$, $X^2$ und $X^3$ stehen für Halogen.

**Schritt a)**

Die Verbindungen der Formel (XXX) lassen sich herstellen durch Umsetzung von Verbindungen der Formel (XXVIII) mit Verbindungen der Formel (XXIX) mittels Palladium-katalysierter *N*-Arylierung, z.B. analog der in Angewandte Chemie Int. Ed. 2011, 50, 8944-8947 beschriebenen Verfahren.

Verbindungen der Formel (XXVIII) sind entweder kommerziell erhältlich oder können nach bekannten Methoden hergestellt werden, beispielsweise analog der in WO2005/100353, WO 2012/66061 oder in European Journal of Medicinal Chemistry 2010, 45, 2214 - 2222 beschriebenen Verfahren.

Verbindungen der Formel (XXIX) sind entweder kommerziell erhältlich oder können nach bekannten Methoden hergestellt werden, beispielsweise analog der in WO2013/43518, EP-A-2168965 oder in Journal of Medicinal Chemistry 2003, 46, 1449 - 1455 beschriebenen Verfahren.

Die weitere Umsetzung von Verbindungen der Formel (XXX) zu Verbindungen der Formel (I) erfolgt analog zu Verfahren A.

**Verfahren und Verwendungen**

Die Erfindung betrifft auch Verfahren zur Bekämpfung von tierischen Schädlingen, bei dem man Verbindungen der Formel (I) auf tierische Schädlinge und/oder ihren Lebensraum einwirken lässt. Bevorzugt wird die Bekämpfung der tierischen Schädlinge in der Land- und Forstwirtschaft und im Materialschutz durchgeführt. Hierunter vorzugsweise ausgeschlossen sind Verfahren zur chirurgischen oder therapeutischen Behandlung des menschlichen oder tierischen Körpers und Diagnostizierverfahren, die am menschlichen oder tierischen Körper vorgenommen werden.

Die Erfindung betrifft ferner die Verwendung der Verbindungen der Formel (I) als Schädlingsbekämpfungsmittel, insbesondere Pflanzenschutzmittel.

Im Rahmen der vorliegenden Anmeldung umfasst der Begriff Schädlingsbekämpfungsmittel jeweils immer auch den Begriff Pflanzenschutzmittel.

Die Verbindungen der Formel (I) eignen sich bei guter Pflanzenverträglichkeit, günstiger Warmblütertoxizität und guter Umweltverträglichkeit zum Schutz von Pflanzen und Pflanzenorganen vor biotischen und abiotischen Stressfaktoren, zur Steigerung der Ernteerträge, Verbesserung der Qualität des Erntegutes und zur Bekämpfung von tierischen Schädlingen, insbesondere Insekten, Spinnentieren, Helminthen, insbesondere Nematoden, und Mollusken, die in der Landwirtschaft, im Gartenbau, bei der Tierzucht, in Aquakulturen, in Forsten, in Gärten und Freizeiteinrichtungen, im Vorrats- und Materialschutz sowie auf dem Hygienesektor vorkommen.

Im Rahmen der vorliegenden Patentanmeldung ist der Begriff "Hygiene" so zu verstehen, dass damit jegliche und alle Maßnahmen, Vorschriften und Verfahrensweisen gemeint sind, deren Ziel es ist, Krankheiten, insbesondere Infektionskrankheiten, zu verhindern, und die dazu dienen, die Gesundheit von Menschen und Tieren zu schützen und/oder die Umwelt zu schützen, und/oder die Sauberkeit aufrechterhalten. Erfindungsgemäß schließt dies insbesondere Maßnahmen zur Reinigung, Desinfektion und Sterilisation beispielsweise von Textilien oder harten Oberflächen, insbesondere Oberflächen aus Glas, Holz, Zement, Porzellan, Keramik, Kunststoff oder auch Metall(en) ein, um sicherzustellen, dass diese frei von Hygieneschädlingen und/oder ihren Ausscheidungen sind. Vorzugsweise ausgeschlossen vom Schutzbereich der Erfindung sind in dieser Hinsicht chirurgische oder therapeutische, auf den menschlichen Körper oder die Körper von Tieren anzuwendende Behandlungsvorschriften und diagnostische Vorschriften, die am menschlichen Körper oder den Körpern von Tieren durchgeführt werden.

Der Begriff "Hygienesektor" deckt alle Gebiete, technischen Felder und industriellen Anwendungen ab, bei denen diese Hygienemaßnahmen, -vorschriften und -verfahrensweisen wichtig sind, zum Beispiel im Hinblick auf Hygiene in Küchen, Bäckereien, Flughäfen, Badezimmern, Schwimmbecken, Kaufhäusern, Hotels, Krankenhäusern, Ställen, Tierhaltungen usw.

Der Begriff "Hygieneschädling" ist daher so zu verstehen, dass damit ein oder mehrere Tierschädlinge gemeint sind, deren Gegenwart im Hygienesektor problematisch ist, insbesondere aus Gesundheitsgründen. Es ist daher ein Hauptziel, das Vorhandensein von Hygieneschädlingen und/oder das Ausgesetztsein ihnen gegenüber im Hygienesektor zu vermeiden oder auf ein Mindestmaß zu begrenzen. Dies lässt sich insbesondere durch die Anwendung eines Pestizids erreichen, das sich sowohl zum Verhindern eines Befalls als auch zum Verhindern eines bereits vorhandenen Befalls einsetzen lässt. Man kann auch Zubereitungen verwenden, die eine Exposition gegenüber Schädlingen verhindern oder reduzieren. Hygieneschädlinge schließen zum Beispiel die unten erwähnten Organismen ein.

Der Begriff "Hygieneschutz" deckt somit alle Handlungen ab, mit denen diese Hygienemaßnahmen, -vorschriften und -verfahrensweisen aufrechterhalten und/oder verbessert werden.

Die Verbindungen der Formel (I) können vorzugsweise als Schädlingsbekämpfungsmittel eingesetzt werden. Sie sind gegen normal sensible und resistente Arten sowie gegen alle oder einzelne Entwicklungsstadien wirksam. Zu den oben erwähnten Schädlingen gehören:

Schädlinge aus dem Stamm der Arthropoda, insbesondere aus der Klasse der Arachnida z. B. Acarus spp., z. B. Acarus siro, Aceria kuko, Aceria sheldoni, Aculops spp., Aculus spp., z. B. Aculus fockeui, Aculus schlechtendali, Amblyomma spp., Amphitetranychus viennensis, Argas spp., Boophilus spp., Brevipalpus spp., z. B. Brevipalpus phoenicis, Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., z. B. Eotetranychus hicoriae, Epitrimerus pyri, Eutetranychus spp., z. B. Eutetranychus banksi, Eriophyes spp., z. B. Eriophyes pyri, Glycyphagus domesticus, Halotydeus destructor, Hemitarsonemus spp., z. B. Hemitarsonemus latus (=Polyphagotarsonemus latus), Hyalomma spp., Ixodes spp., Latrodectus spp., Loxosceles spp., Neutrombicula autumnalis, Nuphersa spp., Oligonychus spp., z. B. Oligonychus coffeae, Oligonychus coniferarum, Oligonychus ilicis, Oligonychus indicus, Oligonychus mangiferus, Oligonychus pratensis, Oligonychus punicae, Oligonychus yothersi, Ornithodorus spp., Ornithonyssus spp., Panonychus spp., z. B. Panonychus citri (=Metatetranychus citri), Panonychus ulmi (=Metatetranychus ulmi), Phyllocoptruta oleivora, Platytetranychus multidigituli, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., z. B. Tarsonemus confusus, Tarsonemus pallidus, Tetranychus spp., z. B. Tetranychus canadensis, Tetranychus cinnabarinus, Tetranychus turkestani, Tetranychus urticae, Trombicula alfreddugesi, Vaejovis spp., Vasates lycopersici;

aus der Klasse der Chilopoda z. B. Geophilus spp., Scutigera spp.;

aus der Ordnung oder der Klasse der Collembola z. B. Onychiurus armatus; Sminthurus viridis;

aus der Klasse der Diplopoda z. B. Blaniulus guttulatus;

aus der Klasse der Insecta, z. B. aus der Ordnung der Blattodea z. B. Blatta orientalis, Blattella asahinai, Blattella germanica, Leucophaea maderae, Loboptera decipiens, Neostylopyga rhombifolia, Panchlora spp., Parcoblatta spp., Periplaneta spp., z. B. Periplaneta americana, Periplaneta australasiae, Pycnoscelus surinamensis, Supella longipalpa;

aus der Ordnung der Coleoptera z. B. Acalymma vittatum, Acanthoscelides obtectus, Adoretus spp., Aethina tumida, Agelastica alni, Agriotes spp., z. B. Agriotes linneatus, Agriotes mancus, Alphitobius diaperinus, Amphimallon solstitialis, Anobium punctatum, Anoplophora spp., Anthonomus spp., z. B. Anthonomus grandis, Anthrenus spp., Apion spp., Apogonia spp., Atomaria spp., z. B. Atomaria linearis, Attagenus spp., Baris caerulescens, Bruchidius obtectus, Bruchus spp., z. B. Bruchus pisorum, Bruchus rufimanus, Cassida spp., Cerotoma trifurcata, Ceutorrhynchus spp., z. B. Ceutorrhynchus assimilis, Ceutorrhynchus quadridens, Ceutorrhynchus rapae, Chaetocnema spp., z. B. Chaetocnema confinis, Chaetocnema denticulata, Chaetocnema ectypa, Cleonus mendicus, Conoderus spp., Cosmopolites spp., z. B. Cosmopolites sordidus, Costelytra zealandica, Ctenicera spp., Curculio spp., z. B. Curculio caryae, Curculio caryatrypes, Curculio obtusus, Curculio sayi, Cryptolestes ferrugineus, Cryptolestes pusillus, Cryptorhynchus lapathi, Cryptorhynchus mangiferae, Cylindrocopturus spp., Cylindrocopturus adspersus, Cylindrocopturus furnissi, Dermestes spp., Diabrotica spp., z. B. Diabrotica balteata, Diabrotica barberi, Diabrotica undecimpunctata howardi, Diabrotica undecimpunctata undecimpunctata, Diabrotica virgifera virgifera, Diabrotica virgifera

zeae, Dichocrocis spp., Dicladispa armigera, Diloboderus spp., Epicaerus spp., Epilachna spp., z. B. Epilachna borealis, Epilachna varivestis, Epitrix spp., z. B. Epitrix cucumeris, Epitrix fuscula, Epitrix hirtipennis, Epitrix subcrinita, Epitrix tuberis, Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., z. B. Hypothenemus hampei, Hypothenemus obscurus, Hypothenemus pubescens, Lachnosterna consanguinea, Lasioderma serricorne, Latheticus oryzae, Lathridius spp., Lema spp., Leptinotarsa decemlineata, Leucoptera spp., z. B. Leucoptera coffeella, Lissorhoptrus oryzophilus, Listronotus (=Hyperodes) spp., Lixus spp., Luperodes spp., Luperomorpha xanthodera, Lyctus spp., Megascelis spp., Melanotus spp., z. B. Melanotus longulus oregonensis, Meligethes aeneus, Melolontha spp., z. B. Melolontha melolontha, Migdolus spp., Monochamus spp., Naupactus xanthographus, Necrobia spp., Neogalerucella spp., Niptus hololeucus, Oryctes rhinoceros, Oryzaephilus surinamensis, Oryzaphagus oryzae, Otiorhynchus spp., z. B. Otiorhynchus cribricollis, Otiorhynchus ligustici, Otiorhynchus ovatus, Otiorhynchus rugosostriarus, Otiorhynchus sulcatus, Oulema spp., z. B. Oulema melanopus, Oulema oryzae, Oxycetonia jucunda, Phaedon cochleariae, Phyllophaga spp., Phyllophaga helleri, Phyllotreta spp., z. B. Phyllotreta armoraciae, Phyllotreta pusilla, Phyllotreta ramosa, Phyllotreta striolata, Popillia japonica, Premnotrypes spp., Prostephanus truncatus, Psylliodes spp., z. B. Psylliodes affinis, Psylliodes chrysocephala, Psylliodes punctulata, Ptinus spp., Rhizobius ventralis, Rhizopertha dominica, Rhynchophorus spp., Rhynchophorus ferrugineus, Rhynchophorus palmarum, Sinoxylon perforans, Sitophilus spp., z. B. Sitophilus granarius, Sitophilus linearis, Sitophilus oryzae, Sitophilus zeamais, Sphenophorus spp., Stegobium paniceum, Sternechus spp., z. B. Sternechus paludatus, Symphyletes spp., Tanymecus spp., z. B. Tanymecus dilaticollis, Tanymecus indicus, Tanymecus palliatus, Tenebrio molitor, Tenebrioides mauretanicus, Tribolium spp., z. B. Tribolium audax, Tribolium castaneum, Tribolium confusum, Trogoderma spp., Tychius spp., Xylotrechus spp., Zabrus spp., z. B. Zabrus tenebrioides;

aus der Ordnung der Dermaptera z. B. Anisolabis maritime, Forficula auricularia, Labidura riparia;

aus der Ordnung der Diptera z. B. Aedes spp., z. B. Aedes aegypti, Aedes albopictus, Aedes sticticus, Aedes vexans, Agromyza spp., z. B. Agromyza frontella, Agromyza parvicornis, Anastrepha spp., Anopheles spp., z. B. Anopheles quadrimaculatus, Anopheles gambiae, Asphondylia spp., Bactrocera spp., z. B. Bactrocera cucurbitae, Bactrocera dorsalis, Bactrocera oleae, Bibio hortulanus, Calliphora erythrocephala, Calliphora vicina, Ceratitis capitata, Chironomus spp., Chrysomya spp., Chrysops spp., Chrysozona pluvialis, Cochliomya spp., Contarinia spp., z. B. Contarinia johnsoni, Contarinia nasturtii, Contarinia pyrivora, Contarinia schulzi, Contarinia sorghicola, Contarinia tritici, Cordylobia anthropophaga, Cricotopus sylvestris, Culex spp., z. B. Culex pipiens, Culex quinquefasciatus, Culicoides spp., Culiseta spp., Cuterebra spp., Dacus oleae, Dasineura spp., z. B. Dasineura brassicae, Delia spp., z. B. Delia antiqua, Delia coarctata, Delia florilega, Delia platura, Delia radicum, Dermatobia hominis, Drosophila spp., z. B. Drosphila melanogaster, Drosophila suzukii, Echinocnemus spp., Euleia heraclei, Fannia spp., Gasterophilus spp., Glossina spp., Haematopota spp., Hydrellia spp., Hydrellia griseola, Hylemya spp., Hippobosca spp., Hypoderma spp., Liriomyza spp., z. B. Liriomyza brassicae, Liriomyza huidobrensis, Liriomyza sativae, Lucilia spp., z. B. Lucilia cuprina, Lutzomyia spp., Mansonia spp., Musca spp., z. B. Musca domestica, Musca domestica vicina, Oestrus spp., Oscinella frit, Paratanytarsus spp., Paralauterborniella subcincta, Pegomya oder Pegomyia spp., z. B. Pegomya betae, Pegomya hyoscyami, Pegomya rubivora, Phlebotomus spp., Phorbia spp., Phormia spp., Piophila casei, Platyparea poeciloptera, Prodiplosis spp., Psila rosae, Rhagoletis spp., z. B. Rhagoletis cingulata, Rhagoletis completa, Rhagoletis fausta, Rhagoletis indifferens, Rhagoletis mendax, Rhagoletis pomonella, Sarcophaga spp., Simulium spp., z. B. Simulium meridionale, Stomoxys spp., Tabanus spp., Tetanops spp., Tipula spp., z. B. Tipula paludosa, Tipula simplex, Toxotrypana curvicauda;

aus der Ordnung der Hemiptera z. B. Acizzia acaciaebaileyanae, Acizzia dodonaeae, Acizzia uncatoides, Acrida turrita, Acyrthosipon spp., z. B. Acyrthosiphon pisum, Acrogonia spp., Aeneolamia spp., Agonoscena spp., Aleurocanthus spp., Aleyrodes proletella, Aleurolobus barodensis, Aleurothrixus floccosus, Allocaridara malayensis, Amrasca spp., z. B. Amrasca bigutulla, Amrasca devastans, Anuraphis cardui, Aonidiella spp., z. B. Aonidiella aurantii, Aonidiella citrina, Aonidiella inornata, Aphanostigma piri, Aphis spp., z. B. Aphis citricola, Aphis craccivora, Aphis fabae, Aphis forbesi, Aphis glycines, Aphis gossypii, Aphis hederae, Aphis illinoisensis, Aphis middletoni, Aphis nasturtii, Aphis nerii, Aphis pomi, Aphis spiraecola, Aphis viburniphila, Arboridia apicalis, Arytainilla spp., Aspidiella spp., Aspidiotus spp., z. B. Aspidiotus nerii, Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., z. B. Cacopsylla pyricola, Calligypona marginata, Capulinia spp., Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus aonidum, Chrysomphalus ficus, Cicadulina mbila, Coccomytilus halli, Coccus spp., z. B. Coccus hesperidum, Coccus longulus, Coccus pseudomagnoliarum, Coccus viridis, Cryptomyzus ribis, Cryptoneossa spp., Ctenarytaina spp., Dalbulus spp., Dialeurodes chittendeni, Dialeurodes citri,

Diaphorina citri, Diaspis spp., Diuraphis spp., Doralis spp., Drosicha spp., Dysaphis spp., z. B. Dysaphis apiifolia, Dysaphis plantaginea, Dysaphis tulipae, Dysmicoccus spp., Empoasca spp., z. B. Empoasca abrupta, Empoasca fabae, Empoasca maligna, Empoasca solana, Empoasca stevensi, Eriosoma spp., z. B. Eriosoma americanum, Eriosoma lanigerum, Eriosoma pyricola, Erythroneura spp., Eucalyptolyma spp., Euphyllura spp., Euscelis bilobatus, Ferrisia spp., Fiorinia spp., Furcaspis oceanica, Geococcus coffeae, Glycaspis spp., Heteropsylla cubana, Heteropsylla spinulosa, Homalodisca coagulata, Hyalopterus arundinis, Hyalopterus pruni, Icerya spp., z. B. Icerya purchasi, Idiocerus spp., Idioscopus spp., Laodelphax striatellus, Lecanium spp., z. B. Lecanium corni (=Parthenolecanium corni), Lepidosaphes spp., z. B. Lepidosaphes ulmi, Lipaphis erysimi, Lopholeucaspis japonica, Lycorma delicatula, Macrosiphum spp., z. B. Macrosiphum euphorbiae, Macrosiphum lilii, Macrosiphum rosae, Macrosteles facifrons, Mahanarva spp., Melanaphis sacchari, Metcalfiella spp., Metcalfa pruinosa, Metopolophium dirhodum, Monellia costalis, Monelliopsis pecanis, Myzus spp., z. B. Myzus ascalonicus, Myzus cerasi, Myzus ligustri, Myzus ornatus, Myzus persicae, Myzus nicotianae, Nasonovia ribisnigri, Neomaskellia spp., Nephotettix spp., z. B. Nephotettix cincticeps, Nephotettix nigropictus, Nettigoniclla spectra, Nilaparvata lugens, Oncometopia spp., Orthezia praelonga, Oxya chinensis, Pachypsylla spp., Parabemisia myricae, Paratrioza spp., z. B. Paratrioza cockerelli, Parlatoria spp., Pemphigus spp., z. B. Pemphigus bursarius, Pemphigus populivenae, Peregrinus maidis, Perkinsiella spp., Phenacoccus spp., z. B. Phenacoccus madeirensis, Phloeomyzus passerinii, Phorodon humuli, Phylloxera spp., z. B. Phylloxera devastatrix, Phylloxera notabilis, Pinnaspis aspidistrae, Planococcus spp., z. B. Planococcus citri, Prosopidopsylla flava, Protopulvinaria pyriformis, Pseudaulacaspis pentagona, Pseudococcus spp., z. B. Pseudococcus calceolariae, Pseudococcus comstocki, Pseudococcus longispinus, Pseudococcus maritimus, Pseudococcus viburni, Psyllopsis spp., Psylla spp., z. B. Psylla buxi, Psylla mali, Psylla pyri, Pteromalus spp., Pulvinaria spp., Pyrilla spp., Quadraspidiotus spp., z. B. Quadraspidiotus juglansregiae, Quadraspidiotus ostreaeformis, Quadraspidiotus perniciosus, Quesada gigas, Rastrococcus spp., Rhopalosiphum spp., z. B. Rhopalosiphum maidis, Rhopalosiphum oxyacanthae, Rhopalosiphum padi, Rhopalosiphum rufiabdominale, Saissetia spp., z. B. Saissetia coffeae, Saissetia miranda, Saissetia neglecta, Saissetia oleae, Scaphoideus titanus, Schizaphis graminum, Selenaspidus articulatus, Sipha flava, Sitobion avenae, Sogata spp., Sogatella furcifera, Sogatodes spp., Stictocephala festina, Siphoninus phillyreae, Tenalaphara malayensis, Tetragonocephela spp., Tinocallis caryaefoliae, Tomaspis spp., Toxoptera spp., z. B. Toxoptera aurantii, Toxoptera citricidus, Trialeurodes vaporariorum, Trioza spp., z. B. Trioza diospyri, Typhlocyba spp., Unaspis spp., Viteus vitifolii, Zygina spp.;

aus der Unterordnung der Heteroptera z. B. Aelia spp., Anasa tristis, Antestiopsis spp., Boisea spp., Blissus spp., Calocoris spp., Campylomma livida, Cavelerius spp., Cimex spp., z. B. Cimex adjunctus, Cimex hemipterus, Cimex lectularius, Cimex pilosellus, Collaria spp., Creontiades dilutus, Dasynus piperis, Dichelops furcatus, Diconocoris hewetti, Dysdercus spp., Euschistus spp., z. B. Euschistus heros, Euschistus servus, Euschistus tristigmus, Euschistus variolarius, Eurydema spp., Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygocoris spp., z. B. Lygocoris pabulinus, Lygus spp., z. B. Lygus elisus, Lygus hesperus, Lygus lineolaris, Macropes excavatus, Megacopta cribraria, Miridae, Monalonion atratum, Nezara spp., z. B. Nezara viridula, Nysius spp., Oebalus spp., Pentomidae, Piesma quadrata, Piezodorus spp., z. B. Piezodorus guildinii, Psallus spp., Pseudacysta persea, Rhodnius spp., Sahlbergella singularis, Scaptocoris castanea, Scotinophora spp., Stephanitis nashi, Tibraca spp., Triatoma spp.;

aus der Ordnung der Hymenoptera z. B. Acromyrmex spp., Athalia spp., z. B. Athalia rosae, Atta spp., Camponotus spp., Dolichovespula spp., Diprion spp., z. B. Diprion similis, Hoplocampa spp., z. B. Hoplocampa cookei, Hoplocampa testudinea, Lasius spp., Linepithema (Iridiomyrmex) humile, Monomorium pharaonis, Paratrechina spp., Paravespula spp., Plagiolepis spp., Sirex spp., Solenopsis invicta, Tapinoma spp., Technomyrmex albipes, Urocerus spp., Vespa spp., z. B. Vespa crabro, Wasmannia auropunctata, Xeris spp.;

aus der Ordnung der Isopoda z. B. Armadillidium vulgare, Oniscus asellus, Porcellio scaber;

aus der Ordnung der Isoptera z. B. Coptotermes spp., z. B. Coptotermes formosanus, Cornitermes cumulans, Cryptotermes spp., Incisitermes spp., Kalotermes spp., Microtermes obesi, Nasutitermis spp., Odontotermes spp., Porotermes spp., Reticulitermes spp., z. B. Reticulitermes flavipes, Reticulitermes hesperus;

aus der Ordnung der Lepidoptera z. B. Achroia grisella, Acronicta major, Adoxophyes spp., z. B. Adoxophyes orana, Aedia leucomelas, Agrotis spp., z. B. Agrotis segetum, Agrotis ipsilon, Alabama spp., z. B. Alabama argillacea, Amyelois transitella, Anarsia spp., Anticarsia spp., z. B. Anticarsia gemmatalis, Argyroploce spp., Autographa spp., Barathra brassicae, Blastodacna atra, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., z. B. Chilo plejadellus, Chilo suppressalis, Choreutis pariana, Choristoneura spp., Chrysodeixis

chalcites, Clysia ambiguella, Cnaphalocerus spp., Cnaphalocrocis medinalis, Cnephasia spp., Conopomorpha spp., Conotrachelus spp., Copitarsia spp., Cydia spp., z. B. Cydia nigricana, Cydia pomonella, Dalaca noctuides, Diaphania spp., Diparopsis spp., Diatraea saccharalis, Earias spp., Ecdytolopha aurantium, Elasmopalpus lignosellus, Eldana saccharina, Ephestia spp., z. B. Ephestia elutella, Ephestia kuehniella, Epinotia spp., Epiphyas postvittana, Erannis spp., Erschoviella musculana, Etiella spp., Eudocima spp., Eulia spp., Eupoecilia ambiguella, Euproctis spp., z. B. Euproctis chrysorrhoea, Euxoa spp., Feltia spp., Galleria mellonella, Gracillaria spp., Grapholitha spp., z. B. Grapholita molesta, Grapholita prunivora, Hedylepta spp., Helicoverpa spp., z. B. Helicoverpa armigera, Helicoverpa zea, Heliothis spp., z. B. Heliothis virescens Hofmannophila pseudospretella, Homoeosoma spp., Homona spp., Hyponomeuta padella, Kakivoria flavofasciata, Lampides spp., Laphygma spp., Laspeyresia molesta, Leucinodes orbonalis, Leucoptera spp., z. B. Leucoptera coffeella, Lithocolletis spp., z. B. Lithocolletis blancardella, Lithophane antennata, Lobesia spp., z. B. Lobesia botrana, Loxagrotis albicosta, Lymantria spp., z. B. Lymantria dispar, Lyonetia spp., z. B. Lyonetia clerkella, Malacosoma neustria, Maruca testulalis, Mamestra brassicae, Melanitis leda, Mocis spp., Monopis obviella, Mythimna separata, Nemapogon cloacellus, Nymphula spp., Oiketicus spp., Omphisa spp., Operophtera spp., Oria spp., Orthaga spp.,

Ostrinia spp., z. B. Ostrinia nubilalis, Panolis flammea, Parnara spp., Pectinophora spp., z. B. Pectinophora gossypiella, Perileucoptera spp., Phthorimaea spp., z. B. Phthorimaea operculella, Phyllocnistis citrella, Phyllonorycter spp., z. B. Phyllonorycter blancardella, Phyllonorycter crataegella, Pieris spp., z. B. Pieris rapae, Platynota stultana, Plodia interpunctella, Plusia spp., Plutella xylostella (=Plutella maculipennis), Prays spp., Prodenia spp., Protoparce spp., Pseudaletia spp., z. B. Pseudaletia unipuncta, Pseudoplusia includens, Pyrausta nubilalis, Rachiplusia nu, Schoenobius spp., z. B. Schoenobius bipunctifer, Scirpophaga spp., z. B. Scirpophaga innotata, Scotia segetum, Sesamia spp., z. B. Sesamia inferens, Sparganothis spp., Spodoptera spp., z. B. Spodoptera eradiana, Spodoptera exigua, Spodoptera frugiperda, Spodoptera praefica, Stathmopoda spp., Stenoma spp., Stomopteryx subsecivella, Synanthedon spp., Tecia solanivora, Thaumetopoea spp., Thermesia gemmatalis, Tinea cloacella, Tinea pellionella, Tineola bisselliella, Tortrix spp., Trichophaga tapetzella, Trichoplusia spp., z. B. Trichoplusia ni, Tryporyza incertulas, Tuta absoluta, Virachola spp.;

aus der Ordnung der Orthoptera oder Saltatoria z. B. Acheta domesticus, Dichroplus spp., Gryllotalpa spp., z. B. Gryllotalpa gryllotalpa, Hieroglyphus spp., Locusta spp., z. B. Locusta migratoria, Melanoplus spp., z. B. Melanoplus devastator, Paratlanticus ussuriensis, Schistocerca gregaria;

aus der Ordnung der Phthiraptera z. B. Damalinia spp., Haematopinus spp., Linognathus spp., Pediculus spp., Phylloxera vastatrix, Phthirus pubis, Trichodectes spp.;

aus der Ordnung der Psocoptera z. B. Lepinotus spp., Liposcelis spp.;

aus der Ordnung der Siphonaptera z. B. Ceratophyllus spp., Ctenocephalides spp., z. B. Ctenocephalides canis, Ctenocephalides felis, Pulex irritans, Tunga penetrans, Xenopsylla cheopis;

aus der Ordnung der Thysanoptera z. B. Anaphothrips obscurus, Baliothrips biformis, Chaetanaphothrips leeuweni, Drepanothrips reuteri, Enneothrips flavens, Frankliniella spp., z. B. Frankliniella fusca, Frankliniella occidentalis, Frankliniella schultzei, Frankliniella tritici, Frankliniella vaccinii, Frankliniella williamsi, Haplothrips spp., Heliothrips spp., Hercinothrips femoralis, Kakothrips spp., Rhipiphorothrips cruentatus, Scirtothrips spp., Taeniothrips cardamomi, Thrips spp., z. B. Thrips palmi, Thrips tabaci;

aus der Ordnung der Zygentoma (= Thysanura), z. B. Ctenolepisma spp., Lepisma saccharina, Lepismodes inquilinus, Thermobia domestica;

aus der Klasse der Symphyla z. B. Scutigerella spp., z. B. Scutigerella immaculata;

Schädlinge aus dem Stamm der Mollusca, z. B. aus der Klasse der Bivalvia, z. B. Dreissena spp.;

sowie aus der Klasse der Gastropoda z. B. Arion spp., z. B. Arion ater rufus, Biomphalaria spp., Bulinus spp., Deroceras spp., z. B. Deroceras laeve, Galba spp., Lymnaea spp., Oncomelania spp., Pomacea spp., Succinea spp.;

Pflanzenschädlinge aus dem Stamm der Nematoda, d. h. pflanzenparasitäre Nematoden, insbesondere Aglenchus spp., z. B. Aglenchus agricola, Anguina spp., z. B. Anguina tritici, Aphelenchoides spp., z. B. Aphelenchoides arachidis, Aphelenchoides fragariae, Belonolaimus spp., z. B. Belonolaimus gracilis, Belonolaimus longicaudatus,

Belonolaimus nortoni, Bursaphelenchus spp., z. B. Bursaphelenchus cocophilus, Bursaphelenchus eremus, Bursaphelenchus xylophilus, Cacopaurus spp., z. B. Cacopaurus pestis, Criconemella spp., z. B. Criconemella curvata, Criconemella onoensis, Criconemella ornata, Criconemella rusium, Criconemella xenoplax (= Mesocriconema xenoplax), Criconemoides spp., z. B. Criconemoides ferniae, Criconemoides onoense, Criconemoides ornatum, Ditylenchus spp., z. B. Ditylenchus dipsaci, Dolichodorus spp., Globodera spp., z. B. Globodera pallida, Globodera rostochiensis, Helicotylenchus spp., z. B. Helicotylenchus dihystera, Hemicriconemoides spp., Hemicycliophora spp., Heterodera spp., z. B. Heterodera avenae, Heterodera glycines, Heterodera schachtii, Hirschmaniella spp., Hoplolaimus spp., Longidorus spp., z. B. Longidorus africanus, Meloidogyne spp., z. B. Meloidogyne chitwoodi, Meloidogyne fallax, Meloidogyne hapla, Meloidogyne incognita, Meloinema spp., Nacobbus spp., Neotylenchus spp., Paralongidorus spp., Paraphelenchus spp., Paratrichodorus spp., z. B. Paratrichodorus minor, Paratylenchus spp., Pratylenchus spp., z. B. Pratylenchus penetrans, Pseudohalenchus spp., Psilenchus spp., Punctodera spp., Quinisulcius spp., Radopholus spp., z. B. Radopholus citrophilus, Radopholus similis, Rotylenchulus spp., Rotylenchus spp., Scutellonema spp., Subanguina spp., Trichodorus spp., z. B. Trichodorus obtusus, Trichodorus primitivus, Tylenchorhynchus spp., z. B. Tylenchorhynchus annulatus, Tylenchulus spp., z. B. Tylenchulus semipenetrans, Xiphinema spp., z. B. Xiphinema index.

Die Verbindungen der Formel (I) können gegebenenfalls in bestimmten Konzentrationen bzw. Aufwandmengen auch als Herbizide, Safener, Wachstumsregulatoren oder Mittel zur Verbesserung der Pflanzeneigenschaften, als Mikrobizide oder Gametozide, beispielsweise als Fungizide, Antimykotika, Bakterizide, Virizide (einschließlich Mittel gegen Viroide) oder als Mittel gegen MLO (Mycoplasmalike-organism) und RLO (Rickettsia-like-organism) verwendet werden. Sie lassen sich gegebenenfalls auch als Zwischen- oder Vorprodukte für die Synthese weiterer Wirkstoffe einsetzen.

**Formulierungen**

Die vorliegende Erfindung betrifft weiterhin Formulierungen und daraus bereitete Anwendungsformen als Schädlingsbekämpfungsmittel wie z. B. Drench-, Drip- und Spritzbrühen, umfassend mindestens eine Verbindung der Formel (I). Gegebenenfalls enthalten die Anwendungsformen weitere Schädlingsbekämpfungsmittel und/oder die Wirkung verbessernde Adjuvantien wie Penetrationsförderer, z. B. pflanzliche Öle wie beispielsweise Rapsöl, Sonnenblumenöl, Mineralöle wie beispielsweise Paraffinöle, Alkylester pflanzlicher Fettsäuren wie beispielsweise Rapsöl- oder Sojaölmethylester oder Alkanol-alkoxylate und/oder Spreitmittel wie beispielsweise Alkylsiloxane und/oder Salze, z. B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropylguar-Polymere und/oder Humectants wie z. B. Glycerin und/oder Dünger wie beispielsweise Ammonium, Kalium oder Phosphor enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einer oder mehreren Verbindungen der Formel (I) weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z. B. durch Vermischen der Verbindungen der Formel (I) mit Hilfsstoffen wie beispielsweise Streckmitteln, Lösemitteln und/oder festen Trägerstoffen und/oder weiteren Hilfsstoffen wie beispielsweise oberflächenaktiven Stoffen. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung der Verbindungen der Formel (I) oder den aus diesen Formulierungen bereiteten Anwendungsformen (wie z. B. gebrauchsfähigen Schädlingsbekämpfungsmitteln wie Spritzbrühen oder Saatgutbeizen) besondere Eigenschaften, wie bestimmte physikalische, technische und/oder biologische Eigenschaften zu verleihen.

Als Streckmittel eignen sich z. B. Wasser, polare und unpolare organische chemische Flüssigkeiten z. B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z. B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe, wie Cyclohexan oder Paraffine, z. B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z. B. Xylol, Toluol oder Alkylnaphthaline, chlorierte aromatische oder chlorierte aliphatische Kohlenwasserstoffe wie z. B. Chlorbenzol, Chlorethylen, oder Methylenchlorid, aliphatische Kohlenwasserstoffe wie z. B. Cyclohexan, Paraffine, Erdölfraktionen, mineralische und pflanzliche Öle, Alkohole wie z. B. Methanol, Ethanol, iso-Propanol, Butanol oder Glykol sowie deren Ether und Ester, Ketone wie z. B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z. B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und natürliche oder synthetische Silikate, Harze, Wachse und/oder feste Düngemittel. Mischungen solcher Trägerstoffe können ebenfalls verwendet werden. Als Trägerstoffe für Granulate kommen infrage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z. B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulfonsäure, Salze von Phenolsulfonsäure oder Naphthalinsulfonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulfobernsteinsäureestern, Taurinderivate (vorzugsweise Alkyltaurate), Phosphorsäureester von polyethoxylierten Alkoholen oder Phenolen, Fettsäureester von Polyolen und Derivate der Verbindungen enthaltend Sulfate, Sulfonate und Phosphate, z. B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn eine der Verbindungen der Formel (I) und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z. B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und/oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und pflanzliche Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Feuchthaltemittel, Spreitmittel. Im Allgemeinen können die Verbindungen der Formel (I) mit jedem festen oder flüssigen Zusatzstoff, welcher für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar-Polymere.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern.

Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Beweglichkeit der Wirkstoffe in der Kutikula erhöhen können.

Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettaminalkoxylate wie beispielsweise Tallowamine-ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% der Verbindung der Formel (I), besonders bevorzugt zwischen 0,01 und 95 Gew.-% der Verbindung der Formel (I), ganz besonders bevorzugt zwischen 0,5 und 90 Gew.-% der Verbindung der Formel (I), bezogen auf das Gewicht der Formulierung.

Der Gehalt an der Verbindung der Formel (I) in den aus den Formulierungen bereiteten Anwendungsformen (insbesondere Schädlingsbekämpfungsmittel) kann in weiten Bereichen variieren. Die Konzentration der Verbindung der Formel (I) in den Anwendungsformen kann üblicherweise zwischen 0,00000001 und 95 Gew.-% der Verbindung der Formel (I), vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

**Mischungen**

Die Verbindungen der Formel (I) können auch in Mischung mit einem oder mehreren geeigneten Fungiziden, Bakteriziden, Akariziden, Molluskiziden, Nematiziden, Insektiziden, Mikrobiologika, Nützlingen, Herbiziden, Düngemitteln, Vogelrepellentien, Phytotonics, Sterilantien, Safenern, Semiochemicals und/oder Pflanzenwachstumsregulatoren verwendet werden, um so z. B. das Wirkungsspektrum zu verbreitern, die Wirkdauer zu verlängern, die Wirkgeschwindigkeit zu steigern, Repellenz zu verhindern oder Resistenzentwicklungen vorzubeugen. Des Weiteren können solche Wirkstoffkombinationen das Pflanzenwachstum und/oder die Toleranz gegenüber abiotischen Faktoren wie z. B. hohen oder niedrigen Temperaturen, gegen Trockenheit oder gegen erhöhten Wasser- bzw. Bodensalzgehalt verbessern. Auch lässt sich das Blüh- und Fruchtverhalten verbessern, die Keimfähigkeit und Bewurzelung optimieren, die Ernte erleichtern und Ernteertrag steigern, die Reife beeinflussen, die Qualität und/oder der Ernährungswert der Ernteprodukte steigern, die Lagerfähigkeit verlängern und/oder die Bearbeitbarkeit der Ernteprodukte verbessern.

Weiterhin können die Verbindungen der Formel (I) in Mischung mit weiteren Wirkstoffen oder Semiochemicals, wie Lockstoffen und/oder Vogelrepellentien und/oder Pflanzenaktivatoren und/oder Wachstumsregulatoren und/oder Düngemitteln vorliegen. Gleichfalls können die Verbindungen der Formel (I) zur Verbesserung der Pflanzeneigenschaften wie zum Beispiel Wuchs, Ertrag und Qualität des Erntegutes eingesetzt werden.

In einer besonderen erfindungsgemäßen Ausführungsform liegen die Verbindungen der Formel (I) in Formulierungen bzw. in den aus diesen Formulierungen bereiteten Anwendungsformen in Mischung mit weiteren Verbindungen vor, vorzugsweise solchen wie nachstehend beschrieben.

Wenn eine der im Folgenden genannten Verbindungen in verschiedenen tautomeren Formen vorkommen kann, sind auch diese Formen mit umfasst, auch wenn sie sie nicht in jedem Fall explizit genannt wurden. Alle genannten Mischungspartner können außerdem, wenn sie auf Grund ihrer funktionellen Gruppen dazu imstande sind, gegebenenfalls mit geeigneten Basen oder Säuren Salze bilden.

**Insektizide/Akarizide/Nematizide**

Die hier mit ihrem "Common Name" genannten Wirkstoffe sind bekannt und beispielsweise im Pestizidhandbuch ("The Pesticide Manual" 16th Ed., British Crop Protection Council 2012) beschrieben oder im Internet recherchierbar (z. B. http://www.alanwood.net/pesticides). Die Klassifizierung basiert auf dem zum Zeitpunkt der Einreichung dieser Patentanmeldung gültigen IRAC Mode of Action Classification Scheme.

(1) Acetylcholinesterase(AChE)-Inhibitoren, wie beispielsweise Carbamate, z. B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb oder Organophosphate, z. B. Acephat, Azamethiphos, Azinphos-ethyl, Azinphos-methyl, Cadusafos, Chlorethoxyfos, Chlorfenvinphos, Chlormephos, Chlorpyrifos-methyl, Coumaphos, Cyanophos, Demeton-S-methyl, Diazinon, Dichlorvos/DDVP, Dicrotophos, Dimethoat, Dimethylvinphos, Disulfoton, EPN, Ethion, Ethoprophos, Famphur, Fenamiphos, Fenitrothion, Fenthion, Fosthiazat, Heptenophos, Imicyafos, Isofenphos, Isopropyl-O-(methoxyaminothio-phosphoryl)saficylat, Isoxathion, Malathion, Mecarbam, Methamidophos, Methidathion, Mevinphos, Monocrotophos, Naled, Omethoate, Oxydemeton-methyl, Parathion-methyl, Phenthoat, Phorat, Phosalon, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.

(2) GABA-gesteuerte Chlorid-Kanal-Blocker, wie beispielsweise Cyclodien-organochlorine, z. B. Chlordan und Endosulfan oder Phenylpyrazole (Fiprole), z. B. Ethiprol und Fipronil.

(3) Natrium-Kanal-Modulatoren, wie beispielsweise Pyrethroide, z. B. Acrinathrin, Allethrin, d-cistrans-Allethrin, d-trans-Allethrin, Bifenthrin, Bioallethrin, Bioallethrin-S-cyclopentenyl-Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomer], Deltamethrin, Empenthrin [(EZ)-(1R)-Isomer], Esfenvalerat, Etofenprox, Fenpropathrin, Fenvalerat, Flucythrinat, Flumethrin, tau-Fluvalinat, Halfenprox, Imiprothrin, Kadethrin, Momfluorothrin, Permethrin, Phenothrin [(1R)-trans-Isomer], Prallethrin, Pyrethrine (pyrethrum), Resmethrin, Silafluofen, Tefluthrin, Tetramethrin, Tetramethrin [(1R)-Isomer], Tralomethrin und Transfluthrin oder DDT oder Methoxychlor.

(4) Kompetitive Modulatoren des nicotinischen Acetylcholin-Rezeptors (nAChR), wie beispielsweise Neonicotinoide, z. B. Acetamiprid, Clothianidin, Dinotefuran, Imidacloprid, Nitenpyram, Thiacloprid und Thiamethoxam oder Nicotin oder Sulfoxaflor oder Flupyradifurone.

(5) Allosterische Modulatoren des nicotinischen Acetylcholin-Rezeptors (nAChR), wie beispielsweise Spinosyne, z. B. Spinetoram und Spinosad.

(6) Allosterische Modulatoren des Glutamat-abhängigen Chloridkanals(GluCl), wie beispielsweise Avermectine/Milbemycine, z. B. Abamectin, Emamectin-benzoat, Lepimectin und Milbemectin.

(7) Juvenilhormon-Mimetika, wie beispielsweise Juvenilhormon-Analoge, z. B. Hydropren, Kinopren und Methopren oder Fenoxycarb oder Pyriproxyfen.

(8) Verschiedene nicht spezifische (multi-site) Inhibitoren, wie beispielsweise Alkylhalogenide, z. B. Methylbromid und andere Alkylhalogenide; oder Chloropicrin oder Sulfurylfluorid oder Borax oder Brechweinstein oder Methylisocyanaterzeuger, z. B. Diazomet und Metam.

(9) Modulatoren chordotonaler Organe, z. B. Pymetrozin oder Flonicamid.

(10) Milbenwachstumsinhibitoren, wie z. B. Clofentezin, Hexythiazox und Diflovidazin oder Etoxazol.

(11) Mikrobielle Disruptoren der Insektendarmmembran, wie z. B. *Bacillus thuringiensis* Subspezies *israelensis, Bacillus sphaericus, Bacillus thuringiensis* Subspezies *aizawai, Bacillus thuringiensis* Subspezies *kurstaki, Bacillus thuringiensis* Subspezies *tenebrionis* und B.t.-Pflanzenproteine: CrylAb, CrylAc, Cry1Fa, CrylA.105, Cry2Ab, VIP3A, mCry3A, Cry3Ab, Cry3Bb, Cry34Ab1/35Ab1.

(12) Inhibitoren der mitochondrialen ATP-Synthase, wie ATP-Disruptoren, wie beispielsweise Diafenthiuron oder Organozinnverbindungen, z. B. Azocyclotin, Cyhexatin und Fenbutatin-oxid oder Propargit oder Tetradifon.

(13) Entkoppler der oxidativen Phoshorylierung durch Störung des Protonengradienten, wie beispielsweise Chlorfenapyr, DNOC und Sulfluramid.

(14) Blocker des nicotinischen Acetylcholinrezeptorkanals, wie beispielsweise Bensultap, Cartaphydrochlorid, Thiocyclam und Thiosultap-Natrium.

(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.

(16) Inhibitoren der Chitinbiosynthese, Typ 1, wie beispielsweise Buprofezin.

(17) Häutungsdisruptor (insbesondere bei Dipteren, d. h. Zweiflüglern), wie beispielsweise Cyromazin.

(18) Ecdyson-Rezeptor-Agonisten, wie beispielsweise Chromafenozid, Halofenozid, Methoxyfenozid und Tebufenozid.

(19) Oktopamin-Rezeptor-Agonisten, wie beispielsweise Amitraz.

(20) Mitochondriale Komplex-III-Elektronentransportinhibitoren, wie beispielsweise Hydramethylnon oder Acequinocyl oder Fluacrypyrim.

(21) Mitochondriale Komplex-I-Elektronentransportinhibitoren, wie beispielsweise METI-Akarizide, z. B. Fenazaquin, Fenpyroximat, Pyrimidifen, Pyridaben, Tebufenpyrad und Tolfenpyrad oder Rotenon (Derris).

(22) Blocker des spannungsabhängigen Natriumkanals, wie z. B. Indoxacarb oder Metaflumizone.

(23) Inhibitoren der Acetyl-CoA-Carboxylase, wie beispielsweise Tetron- und Tetramsäurederivate, z. B. Spirodiclofen, Spiromesifen und Spirotetramat.

(24) Inhibitoren des mitochondrialen Komplex-IV-Elektronentransports, wie beispielsweise Phosphine, z. B. Aluminiumphosphid, Calciumphosphid, Phosphin und Zinkphosphid oder Cyanide, Calciumcyanid, Kaliumcyanid und Natriumcyanid.

(25) Inhibitoren des mitochondrialen Komplex-II-Elektronentransports, wie beispielsweise beta-Ketonitrilderivate, z. B. Cyenopyrafen und Cyflumetofen und Carboxanilide, wie beispielsweise Pyflubumid.

(28) Ryanodinrezeptor-Modulatoren, wie beispielsweise Diamide, z. B. Chlorantraniliprol, Cyantraniliprol und Flubendiamid,

weitere Wirkstoffe wie beispielsweise Afidopyropen, Afoxolaner, Azadirachtin, Benclothiaz, Benzoximat, Bifenazat, Broflanilid, Bromopropylat, Chinomethionat, Chloroprallethrin, Cryolit, Cyclaniliprol, Cycloxaprid, Cyhalodiamid, Dicloromezotiaz, Dicofol, epsilon-Metofluthrin, epsilon-Momfluthrin, Flometoquin, Fluazaindolizin, Fluensulfon, Flufenerim, Flufenoxystrobin, Flufiprol, Fluhexafon, Fluopyram, Fluralaner, Fluxametamid, Fufenozid, Guadipyr, Heptafluthrin, Imidaclothiz, Iprodione, kappa-Bifenthrin, kappa-Tefluthrin, Lotilaner, Meperfluthrin, Paichongding, Pyridalyl, Pyrifluquinazon, Pyriminostrobin, Spirobudiclofen, Tetramethylfluthrin, Tetraniliprol, Tetrachlorantraniliprol, Tioxazafen, Thiofluoximat, Triflumezopyrim und Iodmethan; des Weiteren Präparate auf Basis von *Bacillus firmus* (I-1582, BioNeem, Votivo), sowie folgende Verbindungen: 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635) (CAS 885026-50-6), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457) (CAS 637360-23-7), 2-Chlor-N-[2-{1-[(2E)-3-(4-chlorphenyl)prop-2-en-1-yl]piperidin-4-yl}-4-(trifluormethyl)phenyl]isonicotinamid (bekannt aus WO2006/003494) (CAS 872999-66-1), 3-(4-Chlor-2,6-dimethylphenyl)-4-hydroxy-8-methoxy-1,8-diazaspiro[4.5]dec-3-en-2-on (bekannt aus WO 2010052161) (CAS 1225292-17-0), 3-(4-Chlor-2, 6-dimethylphenyl)-8-methoxy-2-oxo-1,8-diazaspiro[4.5]dec-3-en-4-yl-ethylcarbonat (bekannt aus EP 2647626) (CAS-1440516-42-6), 4-(But-2-in-1-yloxy)-6-(3,5-dimethylpiperidin-1-yl)-5-fluorpyrimidin (bekannt aus WO2004/099160) (CAS 792914-58-0), PF1364 (bekannt aus JP2010/018586) (CAS-Reg.No. 1204776-60-2), N-[(2E)-1-[(6-Chlorpyridin-3-yl)methyl]pyridin-2(1H)-yliden]-2,2,2-trifluoracetamid (bekannt aus WO2012/029672) (CAS 1363400-41-2), (3E)-3-[1-[(6-Chlor-3-pyridyl)methyl]-2-pyridyliden]-1,1,1-trifluorprop*an-2-on (bekannt aus WO2013/144213) (CAS 1461743-15-6), N-[3-(Benzylcarbamoyl)-4-chlorphenyl]-1-methyl-3-(pentafluorethyl)-4-(trifluormethyl)-lH-pyrazol-5-carboxamid (bekannt aus WO2010/051926) (CAS 1226889-14-0), 5-Brom-4-chlor-N- [4-chlor-2-methyl-6-(methylcarbamoyl)phenyl] -2-(3 -chlor-2-pyridyl)pyrazol-3-carboxamid (bekannt aus CN103232431) (CAS 1449220-44-3), 4-[5-(3,5-Dichlorphenyl)-4,5-dihydro-5-(trifluormethyl)-3-isoxazolyl]-2-methyl-N-(cis-1-oxido-3-thietanyl)benzamid, 4-[5-(3,5-Dichlorphenyl)-4,5-dihydro-5-(trifluormethyl)-3-isoxazolyl]-2-methyl-N-(trans-1-oxido-3-thietanyl)benzamid und 4-[(5S)-5-(3,5-Dichlorphenyl)-4,5-dihydro-5-(trifluormethyl)-3-isoxazolyl]-2-methyl-N-(cis-1-oxido-3-thietanyl)benzamid (bekannt aus WO 2013/050317 A1) (CAS 1332628-83-7), N-[3-Chlor-1-(3-pyridinyl)-1H-pyrazol-4-yl]-N-ethyl-3-[(3,3,3-trifluorpropyl)sulfinyl]propanamid, (+)-N-[3-Chlor-1-(3-pyridinyl)-1H-pyrazol-4-yl]-N-ethyl-3-[(3,3,3-trifluorpropyl)sulfinyl]propanamid und (-)-N-[3-Chlor-1-(3-pyridinyl)-1H-pyrazol-4-yl]-N-ethyl-3-[(3,3,3-trifluorpropyl)sulfinyl]propanamid (bekannt aus WO 2013/162715 A2, WO 2013/162716 A2, US 2014/0213448 A1) (CAS 1477923-37-7), 5-[[(2E)-3-Chlor-2-propen-1-yl]amino]-1-[2,6-dichlor-4-(trifluormethyl)phenyl]-4-[(trifluormethyl)sulfinyl]-lH-pyrazol-3-carbonitrile (bekannt aus CN 101337937 A) (CAS 1105672-77-2), 3-Brom-N-[4-chlor-2-methyl-6-[(methylamino)thioxomethyl]phenyl]-1-(3-chlor-2-pyridinyl)-1H-pyrazol-5-carboxamid, (Liudaibenjiaxuanan, bekannt aus CN 103109816 A) (CAS 1232543-85-9); N-[4-Chlor-2-[[(1,1-dimethylethyl)amino]carbonyl] -6-methylphenyl]-1-(3-chlor-2-pyridinyl)-3-(fluormethoxy)-1H-pyrazol-5-carboxamid (bekannt aus WO 2012/034403 A1) (CAS 1268277-22-0), N-[2-(5-Amino-1,3,4-thiadiazol-2-yl)-4-chlor-6-methylphenyl]-3-brom-1-(3-chlor-2-pyridinyl)-1H-pyrazol-5-carboxamid (bekannt aus WO 2011/085575 A1) (CAS 1233882-22-8), 4-[3-[2,6-Dichlor-4-[(3,3-dichlor-2-propen-1-yl)oxy]phenoxy]propoxy]-2-methoxy-6-(trifluormethyl)pyrimidin (bekannt aus CN 101337940 A) (CAS 1108184-52-6); (2E)- und 2(Z)-2-[2-(4-Cyanophenyl)-1-[3-(trifluormethyl)phenyl]ethyliden]-N-[4-(difluormethoxy)phenyl]hydrazincarboxamid (bekannt aus CN 101715774 A) (CAS 1232543-85-9); Cyclopropancarbonsäure-3-(2,2-dichlorethenyl)-2,2-dimethyl-4-(1H-benzimidazol-2-yl)phenylester (bekannt aus CN 103524422 A) (CAS 1542271-46-4); (4aS)-7-Chlor-2,5-dihydro-2-[[(methoxycarbonyl)[4-[(trifluormethyl)thio]phenyl]amino]carbonyl]indeno[1,2-e][1,3,4]oxadiazin-4a(3H)-carbonsäuremethylester (bekannt aus CN 102391261 A) (CAS 1370358-69-2); 6-Desoxy-3-O-ethyl-2,4-di-O-methyl-1-[N-[4-[1-[4-(1,1,2,2,2-pentafluorethoxy)phenyl]-1H-1,2,4-triazol-3-yl]phenyl]carbamat]-α-L-mannopyranose (bekannt aus US 2014/0275503 A1) (CAS 1181213-14-8); 8-(2-Cyclopropylmethoxy-4-trifluormethylphenoxy)-3-(6-trifluormethylpyridazin-3-yl)-3-azabicyclo[3.2.1]octan (CAS 1253850-56-4), (8-anti)-8-(2-Cyclopropylmethoxy-4-trifluormethylphenoxy)-3-(6-trifluormethylpyridazin-3-yl)-3-azabicyclo[3.2.1]octan (CAS 933798-27-7), (8-syn)-8-(2-Cyclopropylmethoxy-4-trifluormethylphenoxy)-3-(6-trifluormethylpyridazin-3-yl)-3-azabicyclo[3.2.1]octan (bekannt aus WO 2007040280 A1, WO 2007040282 A1) (CAS 934001-66-8) und N-[3-Chlor-1-(3-pyridinyl)-1H-pyrazol-4-yl]-N-ethyl-3-[(3,3,3-trifluorpropyl)thio]-propanamid (bekannt aus WO 2015/058021 A1, WO 2015/058028 A1) (CAS 1477919-27-9).

**Fungizide**

Die hier mit ihrem "Common Name" spezifizierten Wirkstoffe sind bekannt und beispielsweise im "Pesticide Manual" (16. Aufl. British Crop Protection Council) oder im Internet recherchierbar (beispielsweise: http://www.alanwood.net/pesticides) beschrieben.

Alle genannten Mischungspartner der Klassen (1) bis (15) können, wenn sie auf Grund ihrer funktionellen Gruppen dazu imstande sind, gegebenenfalls mit geeigneten Basen oder Säuren Salze bilden. Alle genannten fungiziden Mischungspartner der Klassen (1) bis (15) können gegebenenfalls tautomere Formen einschließen.

1) Inhibitoren der Ergosterol-Biosynthese, beispielsweise (1.001) Cyproconazol, (1.002) Difenoconazol, (1.003) Epoxiconazol, (1.004) Fenhexamid, (1.005) Fenpropidin, (1.006) Fenpropimorph, (1.007) Fenpyrazamin, (1.008) Fluquinconazol, (1.009) Flutriafol, (1.010) Imazalil, (1.011) Imazalil Sulfat, (1.012) Ipconazol, (1.013) Metconazol, (1.014) Myclobutanil, (1.015) Paclobutrazol, (1.016) Prochloraz, (1.017) Propiconazol, (1.018) Prothioconazol, (1.019) Pyrisoxazol, (1.020) Spiroxamin, (1.021) Tebuconazol, (1.022) Tetraconazol, (1.023) Triadimenol, (1.024) Tridemorph, (1.025) Triticonazol, (1.026) (1R,2S,5S)-5-(4-Chlorbenzyl)-2-(chlormethyl)-2-methyl-1-(1H-1,2,4-triazol-1-ylmethyl)cyclopentanol, (1.027) (1S,2R,5R)-5-(4-Chlorbenzyl)-2-(chlormethyl)-2-methyl-1-(1H-1,2,4-triazol-l-ylmethyl)cyclopentanol, (1.028) (2R)-2-(1-Chlorcyclopropyl)-4-[(1R)-2,2-dichlorcyclopropyl]-1-(1H-1,2,4-triazol-1-yl)butan-2-ol (1.029) (2R)-2-(1-Chlorcyclopropyl)-4-[(1S)-2,2-dichlorcyclopropyl] -1-(1H-1,2,4-triazol-1-yl)butan-2-ol, (1.030) (2R)-2- [4-(4-Chlorphenoxy)-2-(trifluormethyl)phenyl]-1-(1H-1,2,4-triazol-1-yl)propan-2-ol, (1.031) (2S)-2-(1-Chlorcyclopropyl)-4-[(1R)-2,2-dichlorcyclopropyl]-1-(1H-1,2,4-triazol-1-yl)butan-2-ol, (1.032)(2S)-2-(1-Chlorcyclopropyl)-4-[(1S)-2,2-dichlorcyclopropyl]-1-(1H-1,2,4-triazol-1-yl)butan-2-ol, (1.033) (2S)-2-[4-(4-Chlorphenoxy)-2-(trifluormethyl)phenyl]-1-(1H-1,2,4-triazol-1-yl)propan-2-ol, (1.034) (R)-[3 -(4-Chlor-2-fluorphenyl)-5-(2,4-difluorphenyl)-1,2-oxazol-4-yl](pyridin-3-yl)methanol, (1.035) (S)-[3-(4-Chlor-2-fluorphenyl)-5-(2,4-difluorphenyl)-1,2-oxazol-4-yl](pyridin-3-yl)methanol, (1.036) [3-(4-Chlor-2-fluorphenyl)-5-(2,4-difluorphenyl)-1,2-oxazol-4-yl](pyridin-3-yl)methanol, (1.037) 1-({(2R,4S)-2-[2-Chlor-4-(4-chlorphenoxy)phenyl]-4-methyl-1,3-dioxolan-2-yl}methyl)-1H-1,2,4-triazol, (1.03 8) 1-({(2S,4S)-2-[2-Chlor-4-(4-chlorphenoxy)phenyl]-4-methyl-1,3-dioxolan-2-yl}methyl)-1H-1,2,4-triazol, (1.039) 1-{[3-(2-Chlorphenyl)-2-(2,4-difluorphenyl)oxiran-2-yl]methyl}-1H-1,2,4-triazol-5-yl-thiocyanat, (1.040) 1-1[rel(2R,3R)-3-(2-Chlorphenyl)-2-(2,4-difluorphenyl)oxiran-2-yl]methyl}-1H-1,2,4-triazol-5-yl-thiocyanat, (1.041) 1-{[rel(2R,3S)-3-(2-Chlorphenyl)-2-(2,4-difluorphenyl)oxiran-2-yl]methyl}-1H-1,2,4-triazol-5-yl-thiocyanat, (1.042) 2-[(2R,4R,5R)-1-(2,4-Dichlorphenyl)-5-hydroxy-2,6,6-trimethylheptan-4-yl]-2,4-dihydro-3H-1,2,4-triazol-3-thion, (1.043) 2-[(2R,4R,5S)-1-(2,4-Dichlorphenyl)-5-hydroxy-2,6,6-trimethylheptan-4-yl]-2,4-dihydro-3H-1,2,4-triazol-3-thion, (1.044) 2-[(2R,4S,5R)-1-(2,4-Dichlorophenyl)-5-hydroxy-2,6,6-trimethylheptan-4-yl]-2,4-dihydro-3H-1,2,4-triazol-3-thion, (1.045) 2-[(2R,4S,5S)-1-(2,4-Dichlorphenyl)-5-hydroxy-2,6,6-trimethylheptan-4-yl]-2,4-dihydro-3H-1,2,4-triazol-3-thion, (1.046) 2-[(2S,4R,5R)-1-(2,4-Dichlorphenyl)-5-hydroxy-2,6,6-trimethylheptan-4-yl]-2,4-dihydro-3H-1,2,4-triazol-3-thion, (1.047) 2-[(2S,4R,5S)-1-(2,4-Dichlorphenyl)-5-hydroxy-2,6,6-trimethylheptan-4-yl]-2,4-dihydro-3H-1,2,4-triazol-3-thion, (1.048) 2-[(2S,4S,5R)-l-(2,4-Dichlorphenyl)-5-hydroxy-2,6,6-trimethylheptan-4-yl]-2,4-dihydro-3H-1,2,4-triazol-3-thion, (1.049) 2-[(2S,4S,5S)-1-(2,4-Dichlorphenyl)-5-hydroxy-2,6,6-trimethylheptan-4-yl]-2,4-dihydro-3H-1,2,4-triazol-3-thion, (1.050) 2-[1-(2,4-Dichlorphenyl)-5-hydroxy-2,6,6-trimethylheptan-4-yl]-2,4-dihydro-3H-1,2,4-triazol-3-thion, (1.051) 2-[2-Chlor-4-(2,4-dichlorophenoxy)phenyl]-1-(1H-1,2,4-triazol-1-yl)propan-2-ol, (1.052) 2-[2-Chlor-4-(4-chlorphenoxy)phenyl]-1-(1H-1,2,4-triazol-1-yl)butan-2-ol, (1.053) 2-[4-(4-Chlorphenoxy)-2-(trifluormethyl)phenyl]-1-(1H-1,2,4-triazol-1-yl)butan-2-ol, (1.054) 2-[4-(4-Chlorphenoxy)-2-(trifluormethyl)phenyl]-1-(1H-1,2,4-triazol-1-yl)pentan-2-ol, (1.055) 2-[4-(4-Chlorphenoxy)-2-(trifluormethyl)phenyl]-1-(1H-1,2,4-triazol-1-yl)propan-2-ol, (1.056) 2-{[3-(2-Chlorphenyl)-2-(2,4-difluorphenyl)oxiran-2-yl]methyl}-2,4-dihydro-3H-1,2,4-triazol-3-thion, (1.057) 2-{[rel(2R,3R)-3-(2-Chlorphenyl)-2-(2,4-difluorphenyl)oxiran-2-yl]methyl}-2,4-dihydro-3H-1,2,4-triazol-3-thion, (1.058) 2-{[rel(2R,3S)-3-(2-Chlorphenyl)-2-(2,4-difluorphenyl)oxiran-2-yl]methyl}-2,4-dihydro-3H-1,2,4-triazol-3-thion, (1.059) 5-(4-Chlorbenzyl)-2-(chlormethyl)-2-methyl-1-(1H-1,2,4-triazol-1-ylmethyl)cyclopentanol, (1.060) 5-(Allylsulfanyl)-1-{[3-(2-chlorphenyl)-2-(2,4-difluorphenyl)oxiran-2-yl]methyl}-1H-1,2,4-triazol, (1.061) 5-(Allylsulfanyl)-1-{[rel(2R,3R)-3-(2-chlorphenyl)-2-(2,4-difluorphenyl)oxiran-2-yl]methyl}-1H-1,2,4-triazol, (1.062) 5-(Allylsulfanyl)-1-{[rel(2R,3S)-3-(2-chlorphenyl)-2-(2,4-difluorphenyl)oxiran-2-yl]methyl}-1H-1,2,4-triazol, (1.063) N'-(2,5-Dimethyl-4-{[3-(1,1,2,2-tetrafluorethoxy)phenyl]sulfanyl}phenyl)-N-ethyl-N-methylimidoformamid, (1.064) N'-(2,5-Dimethyl-4-{ [3-(2,2,2-trifluorethoxy)phenyl]sulfanyl}phenyl)-N-ethyl-N-methylimidoformamid, (1.065) N'-(2,5-Dimethyl-4-{ [3-(2,2,3,3-tetrafluorpropoxy)phenyl]sulfanyl}phenyl)-N-ethyl-N-methylimidoformamid, (1.066) N'-(2,5-Dimethyl-4-{[3-(pentafluorethoxy)phenyl]sulfanyl}phenyl)-N-ethyl-N-methylimidoformamid, (1.067) N'-(2,5-Dimethyl-4-{3-[(1,1,2,2-tetrafluorethyl)sulfanyl]phenoxy}phenyl)-N-ethyl-N-methylimidoformamid , (1.068) N'-(2,5-Dimethyl-4-{3-[(2,2,2-trifluorethyl)sulfanyl]phenoxy}phenyl)-N-ethyl-N-methylimidoformamid, (1.069) N'-(2,5-Dimethyl-4-{3-[(2,2,3,3-tetrafluorpropyl)sulfanyl]phenoxy}phenyl)-N-ethyl-N-methylimidoformamid, (1.070) N'-(2,5-Dimethyl-4-{3-[(pentafluorethyl)sulfanyl]phenoxy}phenyl)-N-ethyl-N-methylimidoformamid, (1.071) N'-(2,5-Dimethyl-4-phenoxyphenyl)-N-ethyl-N-methylimidoformamid, (1.072) N'-(4-{ [3-(Difluormethoxy)phenyl]sulfanyl}-2,5-dimethylphenyl)-N-ethyl-N-methylimidoformamid, (1.073) N'-(4-{3-[(Difluormethyl)sulfanyl]phenoxy}-2,5-dimethylphenyl) -N-ethyl-N-methylimidoformamid, (1.074) N'-[5-Brom-6-(2,3-dihydro-1H-inden-2-yloxy)-2-methylpyridin-3-yl]-N-ethyl-N-methylimidoformamid, (1.075) N'-{4-[(4,5-Dichlor-1,3-thiazol-2-yl)oxy]-2,5-dimethylphenyl}-N-ethyl-N-methylimidoformamid, (1.076) N'-{5-Brom-6-[(1R)-1-(3,5-difluorophenyl)ethoxy]-2-methylpyridin-3-yl}-N-ethyl-N-methylimidoformamid, (1.077) N'-{5-Brom-6-[(1S)-1-(3,5-difluorphenyl)ethoxy]-2-methylpyridin-3-yl}-N-ethyl-N-methylimidoformamid, (1.078) N'-{5-Brom-6-[(cis-4-isopropylcyclohexyl)oxy]-2-methylpyridin-3-yl}-N-ethyl-N-methylimidoformamid, (1.079) N'- {5-Brom-6-[(trans-4-isopropylcyclohexyl)oxy]-2-methylpyridin-3-yl}-N-ethyl-N-methylimidoformamid, (1.080) N'-{5-Bromo-6-[1-(3,5-difluorphenyl)ethoxy] -2-methylpyridin-3-yl} -N-ethyl-N-methylimidoformamid.

2) Inhibitoren der Atmungskette am Komplex I oder II beispielsweise (2.001) Benzovindiflupyr, (2.002) Bixafen, (2.003) Boscalid, (2.004) Carboxin, (2.005) Fluopyram, (2.006) Flutolanil, (2.007) Fluxapyroxad, (2.008) Furametpyr, (2.009) Isofetamid, (2.010) Isopyrazam (anti-epimeres Enantiomer 1R,4S,9S), (2.011) Isopyrazam (anti-epimeres Enantiomer 1S,4R,9R), (2.012) Isopyrazam (anti-epimeres Racemat 1RS,4SR,9SR), (2.013) Isopyrazam (Mischung des syn-epimeren Razemates 1RS,4SR,9RS und des anti-epimeren Razemates 1RS,4SR,9SR), (2.014) Isopyrazam (syn-epimeres Enantiomer 1R,4S,9R), (2.015) Isopyrazam (syn-epimeres Enantiomer 1S,4R,9S), (2.016) Isopyrazam (syn-epimeres Racemat 1RS,4SR,9RS), (2.017) Penflufen, (2.018) Penthiopyrad, (2.019) Pydiflumetofen, (2.020) Pyraziflumid, (2.021) Sedaxane, (2.022) 1,3-Dimethyl-N-(1,1,3-trimethyl-2,3-dihydro-1H-inden-4-yl)-1H-pyrazol-4-carboxamid, (2.023) 1,3-Dimethyl-N-[(3R)-1,1,3-trimethyl-2,3-dihydro-1H-inden-4-yl]-1H-pyrazol-4-carboxamid, (2.024) 1,3-Dimethyl-N-[(3S)-1,1,3-trimethyl-2,3-dihydro-1H-inden-4-yl]-1H-pyrazol-4-carboxamid, (2.025) 1-Methyl-3-(trifluormethyl)-N-[2'-(trifluormethyl)biphenyl-2-yl]-1H-pyrazol-4-carboxamid, (2.026) 2-Fluor-6-(trifluoromethyl)-N-(1,1,3-trimethyl-2,3-dihydro-1H-inden-4-yl)benzamid, (2.027) 3-(Difluormethyl)-1-methyl-N-(1,1,3-trimethyl-2,3-dihydro-1H-inden-4-yl)-1H-pyrazol-4-carboxamid, (2.028) 3-(Difluormethyl)-1-methyl-N-[(3R)-1,1,3-trimethyl-2,3-dihydro-1H-inden-4-yl]-1H-pyrazol-4-carboxamid, (2.029) 3-(Difluormethyl)-1-methyl-N-[(3S)-1,1,3-trimethyl-2,3-dihydro-1H-inden-4-yl]-1H-pyrazol-4-carboxamid, (2.030) 3-(Difluormethyl)-N-(7-fluor-1,1,3-trimethyl-2,3-dihydro-1H-inden-4-yl)-1-methyl-1H-pyrazol-4-carboxamid, (2.031) 3-(Difluormethyl)-N-[(3R)-7-fluor-1,1,3-trimethyl-2,3-dihydro-1H-inden-4-yl]-1-methyl-1H-pyrazol-4-carboxamid, (2.032) 3-(Difluoromethyl)-N-[(3S)-7-fluor-1,1,3-trimethyl-2,3-dihydro-1H-inden-4-yl]-1-methyl-1H-pyrazol-4-carboxamid, (2.033) 5,8-Difluor-N-[2-(2-fluor-4-{[4-(trifluormethyl)pyridin-2-yl]oxy}phenyl)ethyl]quinazolin-4-amin, (2.034) N-(2-Cyclopentyl-5-fluorbenzyl)-N-cyclopropyl-3-(difluormethyl)-5-fluor-1-methyl-1H-pyrazol-4-carboxamid, (2.035) N-(2-tert-Butyl-5-methylbenzyl)-N-cyclopropyl-3-(difluormethyl)-5-fluor-1-methyl-1H-pyrazol-4-carboxamid, (2.036) N-(2-tert-Butylbenzyl)-N-cyclopropyl-3-(difluormethyl)-5-fluor-1-methyl-1H-pyrazol-4-carboxamid, (2.037) N-(5-Chlor-2-ethylbenzyl)-N-cyclopropyl-3-(difluormethyl)-5-fluor-1-methyl-1H-pyrazol-4-carboxamid, (2.038) N-(5-Chlor-2-isopropylbenzyl)-N-cyclopropyl-3-(difluormethyl)-5-fluor-1-methyl-1H-pyrazol-4-carboxamid, (2.039) N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid, (2.040) N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid, (2.041) N-[1-(2,4-Dichlorphenyl)-1-methoxypropan-2-yl]-3-(difluormethyl)-1-methyl-1H-pyrazol-4-carboxamid, (2.042) N-[2-Chlor-6-(trifluormethyl)benzyl]-N-cyclopropyl-3-(difluormethyl)-5-fluor-1-methyl-1H-pyrazol-4-carboxamid, (2.043) N-[3-Chlor-2-fluor-6-(trifluormethyl)benzyl]-N-cyclopropyl-3-(difluormethyl)-5-fluor-1-methyl-1H-pyrazol-4-carboxamid, (2.044) N-[5-Chlor-2-(trifluormethyl)benzyl]-N-cyclopropyl-3-(difluormethyl)-5-fluor-1-methyl-1H-pyrazol-4-carboxamid, (2.045) N-Cyclopropyl-3-(difluormethyl)-5-fluor-1-methyl-N-[5-methyl-2-(trifluormethyl)benzyl]-1H-pyrazol-4-carboxamid, (2.046) N-Cyclopropyl-3-(difluormethyl)-5-fluor-N-(2-fluor-6-isopropylbenzyl)-1-methyl-1H-pyrazol-4-carboxamid, (2.047) N-Cyclopropyl-3-(difluormethyl)-5-fluor-N-(2-isopropyl-5-methylbenzyl)-1-methyl-1H-pyrazol-4-carboxamid, (2.048) N-Cyclopropyl-3-(difluormethyl)-5-fluor-N-(2-isopropylbenzyl)-1-methyl-1H-pyrazol-4-carbothioamid, (2.049) N-Cyclopropyl-3-(difluoromethyl)-5-fluor-N-(2-isopropylbenzyl)-1-methyl-1H-pyrazol-4-carboxamid, (2.050) N-Cyclopropyl-3-(difluormethyl)-5-fluor-N-(5-fluor-2-isopropylbenzyl)-1-methyl-1H-pyrazol-4-carboxamid, (2.051) N-Cyclopropyl-3-(difluormethyl)-N-(2-ethyl-4,5-dimethylbenzyl)-5-fluor-1-methyl-1H-pyrazol-4-carboxamid, (2.052) N-Cyclopropyl-3-(difluormethyl)-N-(2-ethyl-5-fluorbenzyl)-5-fluor-1-methyl-1H-pyrazol-4-carboxamid, (2.053) N-Cyclopropyl-3-(difluormethyl)-N-(2-ethyl-5-methylbenzyl)-5-fluor-1-methyl-1H-pyrazole-4-carboxamid, (2.054) N-Cyclopropyl-N-(2-cyclopropyl-5-fluorbenzyl)-3-(difluormethyl)-5-fluor-1-methyl-1H-pyrazole-4-carboxamid, (2.055) N-Cyclopropyl-N-(2-cyclopropyl-5-methylbenzyl)-3-(difluormethyl)-5-fluor-1-methyl-1H-pyrazole-4-carboxamid, (2.056) N-Cyclopropyl-N-(2-cyclopropylbenzyl)-3-(difluormethyl)-5-fluor-1-methyl-1H-pyrazole-4-carboxamid.

3) Inhibitoren der Atmungskette am Komplex III, beispielsweise (3.001) Ametoctradin, (3.002) Amisulbrom, (3.003) Azoxystrobin, (3.004) Coumethoxystrobin, (3.005) Coumoxystrobin, (3.006) Cyazofamid, (3.007) Dimoxystrobin, (3.008) Enoxastrobin, (3.009) Famoxadon, (3.010) Fenamidon, (3.011) Flufenoxystrobin, (3.012) Fluoxastrobin, (3.013) Kresoxim-Methyl, (3.014) Metominostrobin, (3.015) Orysastrobin, (3.016) Picoxystrobin, (3.017) Pyraclostrobin, (3.018) Pyrametostrobin, (3.019) Pyraoxystrobin, (3.020) Trifloxystrobin (3.021) (2E)-2-{2-[({[(1E)-1-(3-{[(E)-1-Fluor-2-phenylvinyl]oxy}phenyl)ethyliden]amino}oxy)methyl]phenyl}-2-(methoxyimino)-N-methylacetamid, (3.022) (2E,3Z)-5-1[1-(4-Chlorphenyl)-1H-pyrazol-3-yl]oxy}-2-(methoxyimino)-N,3-dimethylpent-3-enamid, (3.023) (2R)-2- {2- [(2,5 -Dimethylphenoxy)methyl]phenyl}-2-methoxy-N-methylacetamid, (3.024) (2S)-2-{2-[(2,5-Dimethylphenoxy)methyl]phenyl}-2-methoxy-N-methylacetamid, (3.025) (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl-2-methylpropanoat, (3.026) 2-{2-[(2,5-Dimethylphenoxy)methyl]phenyl}-2-methoxy-N-methylacetamid, (3.027) N-(3-Ethyl-3,5,5-trimethylcyclohexyl)-3-formamido-2-hydroxybenzamid, (3.028) (2E,3Z)-5-{[1-(4-Chlor-2-fluorphenyl)-1H-pyrazol-3-yl]oxy}-2-(methoxyimino)-N,3-dimethylpent-3-enamid.

4) Inhibitoren der Mitose und Zellteilung, beispielsweise (4.001) Carbendazim, (4.002) Diethofencarb, (4.003) Ethaboxam, (4.004) Fluopicolid, (4.005) Pencycuron, (4.006) Thiabendazol, (4.007) Thiophanat-Methyl, (4.008) Zoxamid, , (4.009) 3-Chlor-4-(2,6-difluorphenyl)-6-methyl-5-phenylpyridazin, (4.010) 3-Chlor-5-(4-chlorphenyl)-4-(2,6-difluorphenyl)-6-methylpyridazin, (4.011) 3-Chlor-5-(6-chlorpyridin-3-yl)-6-methyl-4-(2,4,6-trifluorphenyl)pyridazin, (4.012) 4-(2-Brom-4-fluorphenyl)-N-(2,6-difluorphenyl)-1,3-dimethyl-1H-pyrazol-5-amin, (4.013) 4-(2-Brom-4-fluorphenyl)-N-(2-brom-6-fluorphenyl)-l,3-dimethyl-1H-pyrazol-5-amin, (4.014) 4-(2-Brom-4-fluorphenyl)-N-(2-bromphenyl)-1,3-dimethyl-1H-pyrazol-5-amin, (4.015) 4-(2-Brom-4-fluorphenyl)-N-(2-chlor-6-fluorphenyl)-1,3-dimethyl-1H-pyrazol-5-amin, (4.016) 4-(2-Brom-4-fluorphenyl)-N-(2-chlorphenyl)-1,3-dimethyl-1H-pyrazol-5-amin, (4.017) 4-(2-Brom-4-fluorphenyl)-N-(2-fluorphenyl)-1,3-dimethyl-1H-pyrazol-5-amin, (4.018) 4-(2-Chlor-4-fluorphenyl)-N-(2,6-difluorphenyl)-1,3-dimethyl-1H-pyrazol-5 -amin, (4.019) 4-(2-Chlor-4-fluorphenyl)-N-(2-chlor-6-fluorphenyl)-1,3-dimethyl-1H-pyrazol-5-amin, (4.020) 4-(2-Chlor-4-fluorphenyl)-N-(2-chlorphenyl)-1,3-dimethyl-1H-pyrazol-5-amin, (4.021) 4-(2-Chlor-4-fluorphenyl)-N-(2-fluorphenyl)-1,3-dimethyl-1H-pyrazol-5 -amin, (4.022) 4-(4-Chlorphenyl)-5-(2,6-difluorphenyl)-3,6-dimethylpyridazin, (4.023) N-(2-Brom-6-fluorphenyl)-4-(2-chlor-4-fluorphenyl)-1,3-dimethyl-1H-pyrazol-5-amin, (4.024) N-(2-Bromphenyl)-4-(2-chlor-4-fluorphenyl)-1,3-dimethyl-1H-pyrazol-5-amin, (4.025) N-(4-Chlor-2,6-difluorphenyl)-4-(2-chlor-4-fluorphenyl)-1,3-dimethyl-1H-pyrazo1-5-amin.

5) Verbindungen mit Befähigung zu Multisite-Aktivität, beispielsweise (5.001) Bordeauxmischung, (5.002) Captafol, (5.003) Captan, (5.004) Chlorthalonil, (5.005) Kupferhydroxid, (5.006) Kupfernaphthenat, (5.007) Kupferoxid, (5.008) Kupferoxychlorid, (5.009) Kupfer(2+)-sulfat, (5.010) Dithianon, (5.011) Dodin, (5.012) Folpet, (5.013) Mancozeb, (5.014) Maneb, (5.015) Metiram, (5.016) Zinkmetiram, (5.017) Kupfer-Oxin, (5.018) Propineb, (5.019) Schwefel und Schwefelzubereitungen einschließlich Calciumpolysulfid, (5.020) Thiram, (5.021) Zineb, (5.022) Ziram.

6) Verbindungen, die zum Auslösen einer Wirtsabwehr befähigt sind, beispielsweise (6.001) Acibenzolar-S-Methyl, (6.002) Isotianil, (6.003) Probenazol, (6.004) Tiadinil.

7) Inhibitoren der Aminosäure- und/oder Protein-Biosynthese, beispielsweise (7.001) Cyprodinil, (7.002) Kasugamycin, (7.003) Kasugamycinhydrochlorid-hydrat, (7.004) Oxytetracyclin (7.005) Pyrimethanil, (7.006) 3-(5-Fluor-3,3,4,4-tetramethyl-3,4-dihydroisochinolin-1-yl)chinolin.

(8) Inhibitoren der ATP-Produktion, beispielsweise (8.001) Silthiofam.

9) Inhibitoren der Zellwandsynthese, beispielsweise (9.001) Benthiavalicarb, (9.002) Dimethomorph, (9.003) Flumorph, (9.004) Iprovalicarb, (9.005) Mandipropamid, (9.006) Pyrimorph, (9.007) Valifenalat, (9.008) (2E)-3-(4-tert.-Butylphenyl)-3-(2-chlorpyridin-4-yl)-1-(morpholin-4-yl)prop-2-en-1-on, (9.009) (2Z)-3-(4-tert.-Butylphenyl)-3-(2-chlorpyridin-4-yl)-1-(morpholin-4-yl)prop-2-en-1-on.

10) Inhibitoren der Lipid- und Membran-Synthese, beispielsweise (10.001) Propamocarb, (10.002) Propamocarbhydrochlorid, (10.003) Tolclofos-Methyl.

11) Inhibitoren der Melanin-Biosynthese, beispielsweise (11.001) Tricyclazol, (11.002) 2,2,2-Trifluorethyl-{3-methyl-l-[(4-methylbenzoyl)amino]butan-2-yl}carbamat.

12) Inhibitoren der Nukleinsäuresynthese, beispielsweise (12.001) Benalaxyl, (12.002) Benalaxyl-M (Kiralaxyl), (12.003) Metalaxyl, (12.004) Metalaxyl-M (Mefenoxam).

13) Inhibitoren der Signaltransduktion, beispielsweise (13.001) Fludioxonil, (13.002) Iprodion, (13.003) Procymidon, (13.004) Proquinazid, (13.005) Quinoxyfen, (13.006) Vinclozolin.

14) Verbindungen, die als Entkoppler wirken können, beispielsweise (14.001) Fluazinam, (14.002) Meptyldinocap.

15) Weitere Verbindungen, beispielsweise (15.001) Abscisinsäure, (15.002) Benthiazol, (15.003) Bethoxazin, (15.004) Capsimycin, (15.005) Carvon, (15.006) Chinomethionat, (15.007) Cufraneb, (15.008) Cyflufenamid, (15.009) Cymoxanil, (15.010) Cyprosulfamid, (15.011) Flutianil, (15.012) Fosetyl-Aluminium, (15.013) Fosetyl-Calcium, (15.014) Fosetyl-Natrium, (15.015) Methylisothiocyanat, (15.016) Metrafenon, (15.017) Mildiomycin, (15.018) Natamycin, (15.019) Nickel-Dimethyldithiocarbamat, (15.020) Nitrothal-Isopropyl, (15.021) Oxamocarb, (15.022) Oxathiapiprolin, (15.023) Oxyfenthiin, (15.024) Pentachlorphenol und Salze, (15.025) Phosphonsäure und deren Salze, (15.026) Propamocarb-fosetylat, (15.027) Pyriofenone (Chlazafenone) (15.028) Tebufloquin, (15.029) Tecloftalam, (15.030) Tolnifanide, (15.031) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.032) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.033) 2-(6-Benzylpyridin-2-yl)quinazolin, (15.034) 2,6-Dimethyl-1H,5H-[1,4]dithiino[2,3-c:5,6-c']dipyrrol-1,3,5,7(2H,6H)-tetron, (15.035) 2-[3,5-Bis(difluormethyl)-1H-pyrazol-1-yl]-1-[4-(4-{5-[2-(prop-2-in-1-yloxy)phenyll-4,5-dihydro-1,2-oxazol-3-yl}-1,3-thiazol-2-yl)piperidin-1-yl]ethanon, (15.036) 2-[3,5-Bis(difluormethyl)-1H-pyrazol-1-yl]-1-[4-(4-{5-[2-chlor-6-(prop-2-in-1-yloxy)phenyl]-4,5-dihydro-1,2-oxazol-3-yl}-1,3-thiazol-2-yl)piperidin-1-yl]ethanon, (15.037) 2-[3,5-Bis(difluormethyl)-1H-pyrazol-1-yl]-l-[4-(4-{5-[2-fluor-6-(prop-2-in-l-yloxy)phenyl]-4,5-dihydro-l,2-oxazol-3-yl}-l,3-thiazol-2-yl)piperidin-1-yl]ethanon, (15.038) 2-[6-(3-Fluor-4-methoxyphenyl)-5-methylpyridin-2-yl]quinazolin, (15.039) 2-{(5R)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenyl methanesulfonat, (15.040) 2-{(5S)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenyl methanesulfonat, (15.041) 2-{2-[(7,8-Difluor-2-methylquinolin-3-yl)oxy]-6-fluorphenyl}propan-2-ol, (15.042) 2-{2-Fluor-6-[(8-fluor-2-methylquinolin-3-yl)oxy]phenyl}propan-2-ol, (15.043) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenyl-methansulfonat, (15.044) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenyl methanesulfonat, (15.045) 2-Phenylphenol und deren Salze, (15.046) 3-(4,4,5-Trifluor-3,3-dimethyl-3,4-dihydroisoquinolin-1 -yl)quinolin, (15.047) 3-(4,4-Difluor-3,3 -dimethyl-3,4-dihydroisoquinolin-1-yl)quinolin, (15.048) 4-Amino-5-fluorpyrimidin-2-ol (Tautomere Form: 4-Amino-5-fluorpyrimidin-2(1H)-on), (15.049) 4-Oxo-4-[(2-phenylethyl)amino]buttersäure, (15.050) 5-Amino-1,3,4-thiadiazol-2-thiol, (15.051) 5-Chlor-N'-phenyl-N'-(prop-2-yn-1-yl)thiophen-2-sulfonohydrazid, (15.052) 5-Fluor-2-[(4-fluorbenzyl)oxy]pyrimidin-4-amin, (15.053) 5-Fluor-2-[(4-methylbenzyl)oxy]pyrimidin-4-amin, (15.054) 9-Fluor-2,2-dimethyl-5-(quinolin-3-yl)-2,3-dihydro-1,4-benzoxazepin, (15.055) But-3-yn-1-yl {6-[({[(Z)-(1-methyl-1H-tetrazol-5-yl)(phenyl)methylen]amino}oxy)methyl]pyridin-2-yl}carbamat, (15.056) Ethyl (2Z)-3-amino-2-cyano-3-phenylacrylat, (15.057) Phenazin-1-carbonsäure, (15.058) Propyl 3,4,5-trihydroxybenzoat, (15.059) Quinolin-8-ol, (15.060) Quinolin-8-ol sulfat (2:1), (15.061) tert-Butyl {6-[({[(1-methyl-1H-tetrazol-5-yl)(phenyl)methylene]amino}oxy)methyl]pyridin-2-yl}carbamat.

**Biologische Schädlingsbekämpfungsmittel als Mischungskomponenten**

Die Verbindungen der Formel (I) können mit biologischen Schädlingsbekämpfungsmitteln kombiniert werden.

Biologische Schädlingsbekämpfungsmittel umfassen insbesondere Bakterien, Pilze, Hefen, Pflanzenextrakte und solche Produkte, die von Mikroorganismen gebildet wurden inklusive Proteine und sekundäre Stoffwechselprodukte.

Biologische Schädlingsbekämpfungsmittel umfassen Bakterien wie sporenbildende Bakterien, wurzelbesiedelnde Bakterien und Bakterien, die als biologische Insektizide, Fungizide oder Nematizide wirken.

Beispiele für solche Bakterien, die als biologische Schädlingsbekämpfungsmittel eingesetzt werden bzw. verwendet werden können, sind:

*Bacillus amyloliquefaciens,* Stamm FZB42 (DSM 231179), oder *Bacillus cereus,* insbesondere *B. cereus* Stamm CNCM I-1562 oder *Bacillus firmus,* Stamm I-1582 (Accession number CNCM I-1582) oder *Bacillus pumilus,* insbesondere Stamm GB34 (Accession No. ATCC 700814) und Stamm QST2808 (Accession No. NRRL B-30087), oder *Bacillus subtilis,* insbesondere Stamm GB03 (Accession No. ATCC SD-1397), oder *Bacillus subtilis* Stamm QST713 (Accession No. NRRL B-21661) oder *Bacillus subtilis* Stamm OST 30002 (Accession No. NRRL B-50421), *Bacillus thuringiensis,* insbesondere *B. thuringiensis* Subspezies *israelensis* (Serotyp H-14), Stamm AM65-52 (Accession No. ATCC 1276), oder *B. thuringiensis subsp. aizawai,* insbesondere Stamm ABTS-1857 (SD-1372), oder *B. thuringiensis subsp. kurstaki* Stamm HD-1, oder *B. thuringiensis subsp. tenebrionis* Stamm NB 176 (SD-5428), *Pasteuria penetrans, Pasteuria spp.* (Rotylenchulus reniformis nematode)-PR3 (Accession Number ATCC SD-5834), *Streptomyces microflavus* Stamm AQ6121 (= QRD 31.013, NRRL B-50550), *Streptomyces galbus* Stamm AQ 6047 (Acession Number NRRL 30232).

Beispiele für Pilze und Hefen, die als biologische Schädlingsbekämpfungsmittel eingesetzt werden bzw. verwendet werden können, sind:

*Beauveria bassiana,* insbesondere Stamm ATCC 74040, *Coniothyrium minitans,* insbesondere Stamm CON/M/91-8 (Accession No. DSM-9660), *Lecanicillium spp.,* insbesondere Stamm HRO LEC 12, *Lecanicillium lecanii* (ehemals bekannt als *Verticillium lecanii*), insbesondere Stamm KV01, *Metarhizium anisopliae,* insbesondere Stamm F52 (DSM3884/ ATCC 90448), *Metschnikowia fructicola,* insbesondere Stamm NRRL Y-30752, *Paecilomyces fumosoroseus* (neu: *Isaria fumosorosea),* insbesondere Stamm IFPC 200613, oder Stamm Apopka 97 (Accesion No. ATCC 20874), *Paecilomyces lilacinus,* insbesondere *P. lilacinus* Stamm 251 (AGAL 89/030550), *Talaromyces flavus,* insbesondere Stamm V117b, *Trichoderma atroviride,* insbesondere Stamm SC1 (Accession Number CBS 122089), *Trichoderma harzianum,* insbesondere *T. harzianum rifai T39.* (Accession Number CNCM I-952).

Beispiele für Viren, die als biologische Schädlingsbekämpfungsmittel eingesetzt werden bzw. verwendet werden können, sind:

*Adoxophyes orana* (Apfelschalenwickler) Granulosevirus (GV), *Cydia pomonella* (Apfelwickler) Granulosevirus (GV), *Helicoverpa armigera* (Baumwollkapselwurm) Nuklear Polyhedrosis Virus (NPV), *Spodoptera exigua* (Zuckerrübeneule) mNPV, *Spodoptera frugiperda* (Heerwurm) mNPV, *Spodoptera littoralis* (Afrikanischer Baumwollwurm) NPV.

Es sind auch Bakterien und Pilze umfasst, die als 'Inokulant' Pflanzen oder Pflanzenteilen oder Pflanzenorganen beigegeben werden und durch ihre besonderen Eigenschaften das Pflanzenwachstum und die Pflanzengesundheit fördern. Als Beispiele sind genannt:

*Agrobacterium spp., Azorhizobium caulinodans, Azospirillum spp., Azotobacter spp., Bradyrhizobium spp., Burkholderia spp.,* insbesondere *Burkholderia cepacia* (ehemals bekannt als *Pseudomonas cepacia*), *Gigaspora spp.,* oder *Gigaspora monosporum, Glomus spp., Laccaria spp., Lactobacillus buchneri, Paraglomus spp., Pisolithus tinctorus, Pseudomonas spp., Rhizobium spp.,* insbesondere *Rhizobium trifolii, Rhizopogon spp., Scleroderma spp., Suillus spp., Streptomyces spp..*

Beispiele für Pflanzenextrakte und solche Produkte, die von Mikroorganismen gebildet wurden inklusive Proteine und sekundäre Stoffwechselprodukte, die als biologische Schädlingsbekämpfungsmittel eingesetzt werden bzw. verwendet werden können, sind:

Allium sativum, Artemisia absinthium, Azadirachtin, Biokeeper WP, Cassia nigricans, Celastrus angulatus, Chenopodium anthelminticum, Chitin, Armour-Zen, Dryopteris filix-mas, Equisetum arvense, Fortune Aza, Fungastop, Heads Up (Chenopodium quinoa-Saponinextrakt), Pyrethrum/Pyrethrine, Quassia amara, Quercus, Quillaja, Regalia, "Requiem ™ Insecticide", Rotenon, Ryania/Ryanodine, Symphytum officinale, Tanacetum vulgare, Thymol, Triact 70, TriCon, Tropaeulum majus, Urtica dioica, Veratrin, Viscum album, Brassicacaeen-Extrakt, insbesondere Raps- oder Senfpulver.

### Safener als Mischungskomponenten

Die Verbindungen der Formel (I) können mit Safenern kombiniert werden, wie zum Beispiel Benoxacor, Cloquintocet (-mexyl), Cyometrinil, Cyprosulfamide, Dichlormid, Fenchlorazole (-ethyl), Fenclorim, Flurazole, Fluxofenim, Furilazole, Isoxadifen (-ethyl), Mefenpyr (-diethyl), Naphthalic anhydride, Oxabetrinil, 2-Methoxy-N-({4-[(methylcarbamoyl)amino]phenyl}sulfonyl)benzamid (CAS 129531-12-0), 4-(Dichloracetyl)-1-oxa-4-azaspiro[4.5]decan (CAS 71526-07-3), 2,2,5-Trimethyl-3-(dichloracetyl)-1,3-oxazolidin (CAS 52836-31-4).

### Pflanzen und Pflanzenteile

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen), beispielsweise Getreide (Weizen, Reis, Triticale, Gerste, Roggen, Hafer), Mais, Soja, Kartoffel, Zuckerrüben, Zuckerrohr, Tomaten, Paprika, Gurke, Melone, Möhre, Wassermelone, Zwiebel, Salat, Spinat, Porree, Bohnen, *Brassica oleracea* (z. B. Kohl) und andere Gemüsesorten, Baumwolle, Tabak, Raps, sowie Obstpflanzen (mit den Früchten Äpfel, Birnen, Zitrusfrüchte und Weintrauben). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzen sollen alle Entwicklungsstadien wie Saatgut, Stecklinge, junge (unausgereifte) Pflanzen bis hin zu ausgereiften Pflanzen verstanden werden. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehören auch geerntete Pflanzen oder geerntete Pflanzenteile sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Verbindungen der Formel (I) erfolgt direkt oder durch Einwirkung der Verbindungen auf die Umgebung, den Lebensraum oder den Lagerraum nach den üblichen Behandlungsmethoden, z. B. durch Eintauchen, Spritzen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die durch konventionelle Züchtung, durch Mutagenese oder durch rekombinante DNA-Techniken erhalten worden sind. Dies können Sorten, Rassen, Bio- und Genotypen sein.

**Transgene Pflanze, Saatgutbehandlung und Integrationsereignisse**

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehrfähigkeit der Pflanzen gegen tierische und mikrobielle Schädlinge, wie Insekten, Spinnentiere, Nematoden, Milben, Schnecken, bewirkt z. B. durch in den Pflanzen entstehende Toxine, insbesondere solche, die durch das genetische Material aus Bacillus Thuringiensis (z. B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden, ferner eine erhöhte Abwehrfähigkeit der Pflanzen gegen pflanzenpathogene Pilze, Bakterien und/oder Viren, bewirkt z. B. durch Systemisch Akquirierte Resistenz (SAR), Systemin, Phytoalexine, Elicitoren sowie Resistenzgene und entsprechend exprimierte Proteine und Toxine, sowie eine erhöhte Toleranz der Pflanzen gegen bestimmte herbizide Wirkstoffe, beispielsweise Imidazolinone, Sulfonylharnstoffe, Glyphosat oder Phosphinotricin (z. B. "PAT"-Gen). Die jeweils die gewünschten Eigenschaften ("Traits") verleihenden Gene können auch in Kombinationen miteinander in den transgenen Pflanzen vorkommen. Als Beispiele transgener Pflanzen werden die wichtigen Kulturpflanzen, wie Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchte und Weintrauben) erwähnt, wobei Mais, Soja, Weizen, Reis, Kartoffel, Baumwolle, Zuckerrohr, Tabak und Raps besonders hervorgehoben werden. Als Eigenschaften ("Traits") werden besonders hervorgehoben die erhöhte Abwehrfähigkeit der Pflanzen gegen Insekten, Spinnentiere, Nematoden und Schnecken.

**Pflanzenschutz - Behandlungsarten**

Die Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z. B. durch Tauchen, Spritzen, Sprühen, Berieseln, Verdampfen, Zerstäuben, Vernebeln, Verstreuen, Verschäumen, Bestreichen, Verstreichen, Injizieren, Gießen (drenchen), Tröpfchenbewässerung und bei Vermehrungsmaterial, insbesondere bei

Saatgut, weiterhin durch Trockenbeizen, Nassbeizen, Schlämmbeizen, Inkrustieren, ein- oder mehrschichtiges Umhüllen, usw. Es ist ferner möglich, die Verbindungen der Formel (I) nach dem Ultra-Low-Volume-Verfahren auszubringen oder die Anwendungsform oder die Verbindung der Formel (I) selbst in den Boden zu injizieren.

Eine bevorzugte direkte Behandlung der Pflanzen ist die Blattapplikation, d. h. die Verbindungen der Formel (I) werden auf das Blattwerk aufgebracht, wobei die Behandlungsfrequenz und die Aufwandmenge auf den Befallsdruck des jeweiligen Schädlings abgestimmt sein sollte.

Bei systemisch wirksamen Wirkstoffen gelangen die Verbindungen der Formel (I) auch über das Wurzelwerk in die Pflanzen. Die Behandlung der Pflanzen erfolgt dann durch Einwirkung der Verbindungen der Formel (I) auf den Lebensraum der Pflanze. Das kann beispielsweise durch Drenchen, Einmischen in den Boden oder die Nährlösung sein, d. h. der Standort der Pflanze (z. B. Boden oder hydroponische Systeme) wird mit einer flüssigen Form der Verbindungen der Formel (I) getränkt, oder durch die Bodenapplikation, d. h. die erfindungsgemäßen Verbindungen der Formel (I) werden in fester Form (z. B. in Form eines Granulats) in den Standort der Pflanzen eingebracht. Bei Wasserreiskulturen kann das auch durch Zudosieren der Verbindung der Formel (I) in einer festen Anwendungsform (z. B. als Granulat) in ein überflutetes Reisfeld sein.

**Saatgutbehandlung**

Die Bekämpfung von tierischen Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Schädlingsbekämpfungsmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. -toleranter transgener Pflanzen einbeziehen, um einen optimalen Schutz des Saatguts und auch der keimenden Pflanze bei einem minimalen Aufwand an Schädlingsbekämpfungsmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einer der Verbindungen der Formel (I) behandelt wird. Das erfindungsgemäße Verfahren zum Schutz von Saatgut und keimenden Pflanzen vor dem Befall von Schädlingen umfasst ferner ein Verfahren, in dem das Saatgut gleichzeitig in einem Vorgang oder sequentiell mit einer Verbindung der Formel (I) und einer Mischungskomponente behandelt wird. Es umfasst ferner auch ein Verfahren, in dem das Saatgut zu unterschiedlichen Zeiten mit einer Verbindung der Formel (I) und einer Mischungskomponente behandelt wird.

Die Erfindung bezieht sich ebenfalls auf die Verwendung der Verbindungen der Formel (I) zur Behandlung von Saatgut zum Schutz des Saatguts und der daraus entstehenden Pflanze vor tierischen Schädlingen.

Weiterhin bezieht sich die Erfindung auf Saatgut, welches zum Schutz vor tierischen Schädlingen mit einer erfindungsgemäßen Verbindung der Formel (I) behandelt wurde. Die Erfindung bezieht sich auch auf Saatgut, welches zur gleichen Zeit mit einer Verbindung der Formel (I) und einer Mischungskomponente behandelt wurde. Die Erfindung bezieht sich weiterhin auf Saatgut, welches zu unterschiedlichen Zeiten mit einer Verbindung der Formel (I) und einer Mischungskomponente behandelt wurde. Bei Saatgut, welches zu unterschiedlichen Zeiten mit einer Verbindung der Formel (I) und einer Mischungskomponente behandelt wurde, können die einzelnen Substanzen in unterschiedlichen Schichten auf dem Saatgut vorhanden sein. Dabei können die Schichten, die eine Verbindung der Formel (I) und Mischungskomponenten enthalten, gegebenenfalls durch eine Zwischenschicht getrennt sein. Die Erfindung bezieht sich auch auf Saatgut, bei dem eine Verbindung der Formel (I) und eine Mischungskomponente als Bestandteil einer Umhüllung oder als weitere Schicht oder weitere Schichten zusätzlich zu einer Umhüllung aufgebracht sind.

Des Weiteren bezieht sich die Erfindung auf Saatgut, welches nach der Behandlung mit einer Verbindung der Formel (I) einem Filmcoating-Verfahren unterzogen wird, um Staubabrieb am Saatgut zu vermeiden.

Einer der auftretenden Vorteile, wenn eine Verbindung der Formel (I) systemisch wirkt, ist es, dass die Behandlung des Saatguts nicht nur das Saatgut selbst, sondern auch die daraus hervorgehenden Pflanzen nach dem Auflaufen vor tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit einer Verbindung der Formel (I) Keimung und Auflauf des behandelten Saatguts gefördert werden können.

Ebenso ist es als vorteilhaft anzusehen, dass Verbindungen der Formel (I) insbesondere auch bei transgenem Saatgut eingesetzt werden können.

Verbindungen der Formel (I) können ferner in Kombination mit Mitteln der Signaltechnologie eingesetzt werden,

wodurch eine bessere Besiedlung mit Symbionten, wie zum Beispiel Rhizobien, Mycorrhiza und/oder endophytischen Bakterien oder Pilzen, stattfindet und/oder es zu einer optimierten Stickstofffixierung kommt.

Die Verbindungen der Formel (I) eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baumwolle, Soja, Reis, Kartoffeln, Sonnenblume, Kaffee, Tabak, Canola, Raps, Rübe (z. B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps, Gemüse und Reis zu.

Wie vorstehend bereits erwähnt, kommt auch der Behandlung von transgenem Saatgut mit einer Verbindung der Formel (I) eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw. nematiziden Eigenschaften steuert. Die heterologen Gene in transgenem Saatgut können dabei aus Mikroorganismen wie Bacillus, Rhizobium, Pseudomonas, Serratia, Trichoderma, Clavibacter, Glomus oder Gliocladium stammen. Die vorliegende Erfindung eignet sich besonders für die Behandlung von transgenem Saatgut, das zumindest ein heterologes Gen enthält, das aus Bacillus sp. stammt. Besonders bevorzugt handelt es sich dabei um ein heterologes Gen, das aus Bacillus thuringiensis stammt.

Im Rahmen der vorliegenden Erfindung wird die Verbindung der Formel (I) auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hüllen, Wolle oder Fruchtfleisch befreit wurde. So kann zum Beispiel Saatgut verwendet werden, das geerntet, gereinigt und bis zu einem lagerfähigen Feuchtigkeitsgehalt getrocknet wurde. Alternativ kann auch Saatgut verwendet werden, das nach dem Trocknen z. B. mit Wasser behandelt und dann erneut getrocknet wurde, zum Beispiel Priming. Im Fall von Reis-Saatgut ist es auch möglich, Saatgut zu verwenden, das getränkt wurde, zum Beispiel in Wasser bis zu einem bestimmten Stadium des Reisembryos ("Pigeon Breast Stage"), wodurch die Keimung und ein einheitlicheres Auflaufen stimuliert wird.

Im Allgemeinen muss bei der Behandlung des Saatguts darauf geachtet werden, dass die Menge der auf das Saatgut aufgebrachten Verbindung der Formel (I) und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die Verbindungen der Formel (I) werden in der Regel in Form einer geeigneten Formulierung auf das Saatgut aufgebracht. Geeignete Formulierungen und Verfahren für die Saatgutbehandlung sind dem Fachmann bekannt.

Die Verbindungen der Formel (I) können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Verbindungen der Formel (I) mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalinsulfonate, wie Diisopropyl- oder Diisobutylnaphthalinsulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vor-zugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid-Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für

derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen, Soja und Rüben oder auch von Gemüsesaatgut der verschiedensten Natur. Die erfindungsgemäß verwendbaren Beizmittel-Formulierungen oder deren verdünnte Anwendungsformen können auch zum Beizen von Saatgut transgener Pflanzen eingesetzt werden.

Zur Behandlung von Saatgut mit den erfindungsgemäß verwendbaren Beizmittel-Formulierungen oder dem daraus durch Zugabe von Wasser hergestellten Anwendungsformen kommen alle üblicherweise für die Beizung einsetzbaren Mischgeräte in Betracht. Im Einzelnen geht man bei der Beizung so vor, dass man das Saatgut in einen Mischer im diskontinuierlichen oder kontinuierlichen Betrieb gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann inner-halb eines größeren Bereiches variiert werden. Sie richtet sich nach dem jeweiligen Gehalt der Verbindungen der Formel (I) in den Formulierungen und nach dem Saatgut. Die Aufwandmengen bei der Verbindung der Formel (I) liegen im Allgemeinen zwischen 0,001 und 50 g pro Kilogramm Saatgut, vorzugsweise zwischen 0,01 und 15 g pro Kilogramm Saatgut.

**Tiergesundheit**

Auf dem Gebiet der Tiergesundheit, d. h. dem Gebiet der Tiermedizin, sind die Verbindungen der Formel (I) gegen Tierparasiten, insbesondere Ektoparasiten oder Endoparasiten, wirksam. Der Begriff Endoparasit umfasst insbesondere Helminthen und Protozoen wie Kokzidien. Ektoparasiten sind typischerweise und bevorzugt Arthropoden, insbesondere Insekten oder Akariden.

Auf dem Gebiet der Tiermedizin eignen sich die Verbindungen der Formel (I), die eine günstige Toxizität gegenüber Warmblütern aufweisen, für die Bekämpfung von Parasiten, die in der Tierzucht und Tierhaltung bei Nutztieren, Zuchttieren, Zootieren, Laboratoriumstieren, Versuchstieren und Haustieren auftreten. Sie sind gegen alle oder einzelne Entwicklungsstadien der Parasiten wirksam.

Zu den landwirtschaftlichen Nutztieren zählen zum Beispiel Säugetiere wie Schafe, Ziegen, Pferde, Esel, Kamele, Büffel, Kaninchen, Rentiere, Damhirsche und insbesondere Rinder und Schweine; oder Geflügel wie Truthähne, Enten, Gänse und insbesondere Hühner; oder Fische oder Krustentiere, z. B. in der Aquakultur, oder gegebenenfalls Insekten wie Bienen.

Zu den Haustieren zählen zum Beispiel Säugetiere wie Hamster, Meerschweinchen, Ratten, Mäuse, Chinchillas, Frettchen und insbesondere Hunde, Katzen, Stubenvögel; Reptilien, Amphibien oder Aquariumfische.

Gemäß einer bestimmten Ausführungsform werden die Verbindungen der Formel (I) an Säugetiere verabreicht.

Gemäß einer weiteren bestimmten Ausführungsform werden die Verbindungen der Formel (I) an Vögel, nämlich Stubenvögel oder insbesondere Geflügel, verabreicht.

Durch Verwendung der Verbindungen der Formel (I) für die Bekämpfung von Tierparasiten sollen Krankheit, Todesfälle und Leistungsminderungen (bei Fleisch, Milch, Wolle, Häuten, Eiern, Honig und dergleichen) verringert bzw. vorgebeugt werden, so dass eine wirtschaftlichere und einfachere Tierhaltung ermöglicht wird und ein besseres Wohlbefinden der Tiere erzielbar ist.

In Bezug auf das Gebiet der Tiergesundheit bedeutet der Begriff "Bekämpfung" oder "bekämpfen" im vorliegenden Zusammenhang, dass durch die Verbindungen der Formel (I) wirksam das Auftreten des jeweiligen Parasiten in einem Tier, das mit solchen Parasiten in einem harmlosen Ausmaß infiziert ist, reduziert wird. Genauer gesagt bedeutet "bekämpfen" im vorliegenden Zusammenhang, dass die Verbindungen der Formel (I) den jeweiligen Parasiten abtöten,

sein Wachstum verhindern oder seine Vermehrung verhindern.

Zu den Arthropoden zählen beispielsweise, ohne hierauf beschränkt zu sein,
aus der Ordnung Anoplurida zum Beispiel Haematopinus spp., Linognathus spp., Pediculus spp., Phtirus spp., Solenopotes spp.;
aus der Ordnung Mallophagida und den Unterordnungen Amblycerina und Ischnocerina, zum Beispiel Bovicola spp., Damalina spp., Felicola spp.; Lepikentron spp., Menopon spp., Trichodectes spp., Trimenopon spp., Trinoton spp., Werneckiella spp;
aus der Ordnung Diptera und den Unterordnungen Nematocerina und Brachycerina, zum Beispiel Aedes spp., Anopheles spp., Atylotus spp., Braula spp., Calliphora spp., Chrysomyia spp., Chrysops spp., Culex spp., Culicoides spp., Eusimulium spp., Fannia spp., Gasterophilus spp., Glossina spp., Haematobia spp., Haematopota spp., Hippobosca spp., Hybomitra spp., Hydrotaea spp., Hypoderma spp., Lipoptena spp., Lucilia spp., Lutzomyia spp., Melophagus spp., Morellia spp., Musca spp., Odagmia spp., Oestrus spp., Philipomyia spp., Phlebotomus spp., Rhinoestrus spp., Sarcophaga spp., Simulium spp., Stomoxys spp., Tabanus spp., Tipula spp., Wilhelmia spp., Wohlfahrtia spp.;
aus der Ordnung Siphonapterida, zum Beispiel Ceratophyllus spp., Ctenocephalides spp., Pulex spp., Tunga spp., Xenopsylla spp.;
aus der Ordnung Heteropterida, zum Beispiel Cimex spp., Panstrongylus spp., Rhodnius spp., Triatoma spp.; sowie Lästlinge und Hygieneschädlinge aus der Ordnung Blattarida.

Weiterhin sind bei den Arthropoden beispielhaft, ohne hierauf beschränkt zu sein, die folgenden Akari zu nennen: Aus der Unterklasse Akari (Acarina) und der Ordnung Metastigmata, zum Beispiel aus der Familie Argasidae, wie Argas spp., Ornithodorus spp., Otobius spp., aus der Familie Ixodidae, wie Amblyomma spp., Dermacentor spp., Haemaphysalis spp., Hyalomma spp., Ixodes spp., Rhipicephalus (Boophilus) spp., Rhipicephalus spp. (die ursprüngliche Gattung der mehrwirtigen Zecken); aus der Ordnung Mesostigmata, wie Dermanyssus spp., Ornithonyssus spp., Pneumonyssus spp., Raillietia spp., Sternostoma spp., Tropilaelaps spp., Varroa spp.; aus der Ordnung Actinedida (Prostigmata), zum Beispiel Acarapis spp., Cheyletiella spp., Demodex spp., Listrophorus spp., Myobia spp., Neotrombicula spp., Ornithocheyletia spp., Psorergates spp., Trombicula spp.; und aus der Ordung der Acaridida (Astigmata), zum Beispiel Acarus spp., Caloglyphus spp., Chorioptes spp., Cytodites spp., Hypodectes spp., Knemidocoptes spp., Laminosioptes spp., Notoedres spp., Otodectes spp., Psoroptes spp., Pterolichus spp., Sarcoptes spp., Trixacarus spp., Tyrophagus spp.

Zu Beispielen für parasitäre Protozoen zählen, ohne hierauf beschränkt zu sein:
Mastigophora (Flagellata), wie:

Metamonada: aus der Ordnung Diplomonadida zum Beispiel Giardia spp., Spironucleus spp.

Parabasala: aus der Ordnung Trichomonadida zum Beispiel Histomonas spp., Pentatrichomonas spp., Tetratrichomonas spp., Trichomonas spp., Tritrichomonas spp.

Euglenozoa: aus der Ordnung Trypanosomatida zum Beispiel Leishmania spp., Trypanosoma spp.

Sarcomastigophora (Rhizopoda), wie Entamoebidae, zum Beispiel Entamoeba spp., Centramoebidae, zum Beispiel Acanthamoeba sp., Euamoebidae, z. B. Hartmanella sp.

Alveolata wie Apicomplexa (Sporozoa): z. B. Cryptosporidium spp.; aus der Ordnung Eimeriida zum Beispiel Besnoitia spp., Cystoisospora spp., Eimeria spp., Hammondia spp., Isospora spp., Neospora spp., Sarcocystis spp., Toxoplasma spp.; aus der Ordnung Adeleida z. B. Hepatozoon spp., Klossiella spp.; aus der Ordnung Haemosporida z. B. Leucocytozoon spp., Plasmodium spp.; aus der Ordnung Piroplasmida z. B. Babesia spp., Ciliophora spp., Echinozoon spp., Theileria spp.; aus der Ordnung Vesibuliferida z. B. Balantidium spp., Buxtonella spp.

Microspora wie Encephalitozoon spp., Enterocytozoon spp., Globidium spp., Nosema spp., und außerdem z. B. Myxozoa spp.

Zu den für Menschen oder Tiere pathogenen Helminthen zählen zum Beispiel Acanthocephala, Nematoden, Pentastoma und Platyhelminthen (z.B. Monogenea, Cestodes und Trematodes).

Zu beispielhaften Helminthen zählen, ohne hierauf beschränkt zu sein:

Monogenea: z. B.: Dactylogyrus spp., Gyrodactylus spp., Microbothrium spp., Polystoma spp., Troglecephalus spp.;

Cestodes: aus der Ordnung Pseudophyllidea zum Beispiel: Bothridium spp., Diphyllobothrium spp., Diplogonoporus spp. Ichthyobothrium spp., Ligula spp., Schistocephalus spp., Spirometra spp.

Aus der Ordnung Cyclophyllida zum Beispiel: Andyra spp., Anoplocephala spp., Avitellina spp., Bertiella spp., Cittotaenia spp., Davainea spp., Diorchis spp., Diplopylidium spp., Dipylidium spp., Echinococcus spp., Echinocotyle

spp., Echinolepis spp., Hydatigera spp., Hymenolepis spp., Joyeuxiella spp., Mesocestoides spp., Moniezia spp., Paranoplocephala spp., Raillietina spp., Stilesia spp., Taenia spp., Thysaniezia spp., Thysanosoma spp.

Trematodes: aus der Klasse Digenea zum Beispiel: Austrobilharzia spp., Brachylaima spp., Calicophoron spp., Catatropis spp., Clonorchis spp. Collyriclum spp., Cotylophoron spp., Cyclocoelum spp., Dicrocoelium spp., Diplostomum spp., Echinochasmus spp., Echinoparyphium spp., Echinostoma spp., Eurytrema spp., Fasciola spp., Fasciolides spp., Fasciolopsis spp., Fischoederius spp., Gastrothylacus spp., Gigantobilharzia spp., Gigantocotyle spp., Heterophyes spp., Hypoderaeum spp., Leucochloridium spp., Metagonimus spp., Metorchis spp., Nanophyetus spp., Notocotylus spp., Opisthorchis spp., Ornithobilharzia spp., Paragonimus spp., Paramphistomum spp., Plagiorchis spp., Posthodiplostomum spp., Prosthogonimus spp., Schistosoma spp., Trichobilharzia spp., Troglotrema spp., Typhlocoelum spp.

Nematoden: aus der Ordnung Trichinellida zum Beispiel: Capillaria spp., Trichinella spp., Trichomosoides spp., Trichuris spp.

Aus der Ordnung Tylenchida zum Beispiel: Micronema spp., Parastrangyloides spp., Strongyloides spp.

Aus der Ordnung Rhabditina zum Beispiel: Aelurostrongylus spp., Amidostomum spp., Ancylostoma spp., Angiostrongylus spp., Bronchonema spp., Bunostomum spp., Chabertia spp., Cooperia spp., Cooperioides spp., Crenosoma spp., Cyathostomum spp., Cyclococercus spp., Cyclodontostomum spp., Cylicocyclus spp., Cylicostephanus spp., Cylindropharynx spp., Cystocaulus spp., Dictyocaulus spp., Elaphostrongylus spp., Filaroides spp., Globocephalus spp., Graphidium spp., Gyalocephalus spp., Haemonchus spp., Heligmosomoides spp., Hyostrongylus spp., Marshallagia spp., Metastrongylus spp., Muellerius spp., Necator spp., Nematodirus spp., Neostrongylus spp., Nippostrongylus spp., Obeliscoides spp., Oesophagodontus spp., Oesophagostomum spp., Ollulanus spp.; Ornithostrongylus spp., Oslerus spp., Ostertagia spp., Paracooperia spp., Paracrenosoma spp., Parafilaroides spp., Parelaphostrongylus spp., Pneumocaulus spp., Pneumostrongylus spp., Poteriostomum spp., Protostrongylus spp., Spicocaulus spp., Stephanurus spp., Strongylus spp., Syngamus spp., Teladorsagia spp., Trichonema spp., Trichostrongylus spp., Triodontophorus spp., Troglostrongylus spp., Uncinaria spp.

Aus der Ordnung Spirurida zum Beispiel: Acanthocheilonema spp., Anisakis spp., Ascaridia spp.; Ascaris spp., Ascarops spp., Aspiculuris spp., Baylisascaris spp., Brugia spp., Cercopithifilaria spp., Crassicauda spp., Dipetalonema spp., Dirofilaria spp., Dracunculus spp.; Draschia spp., Enterobius spp., Filaria spp., Gnathostoma spp., Gongylonema spp., Habronema spp., Heterakis spp.; Litomosoides spp., Loa spp., Onchocerca spp., Oxyuris spp., Parabronema spp., Parafilaria spp., Parascaris spp., Passalurus spp., Physaloptera spp., Probstmayria spp., Pseudofilaria spp., Setaria spp., Skjrabinema spp., Spirocerca spp., Stephanofilaria spp., Strongyluris spp., Syphacia spp., Thelazia spp., Toxascaris spp., Toxocara spp., Wuchereria spp.

Acanthocephala: aus der Ordnung Oligacanthorhynchida z.B: Macracanthorhynchus spp., Prosthenorchis spp.; aus der Ordnung Moniliformida zum Beispiel: Moniliformis spp.,

Aus der Ordnung Polymorphida zum Beispiel: Filicollis spp.; aus der Ordnung Echinorhynchida zum Beispiel Acanthocephalus spp., Echinorhynchus spp., Leptorhynchoides spp.

Pentastoma: aus der Ordnung Porocephalida zum Beispiel Linguatula spp.

Auf dem Gebiet der Tiermedizin und der Tierhaltung erfolgt die Verabreichung der Verbindungen der Formel (I) nach allgemein fachbekannten Verfahren, wie enteral, parenteral, dermal oder nasal in Form von geeigneten Präparaten. Die Verabreichung kann prophylaktisch; metaphylaktisch oder therapeutisch erfolgen.

So bezieht sich eine Ausführungsform der vorliegenden Erfindung auf die Verbindungen der Formel (I) zur Verwendung als Arzneimittel.

Ein weiterer Aspekt bezieht sich auf die Verbindungen der Formel (I) zur Verwendung als Antiendoparasitikum.

Ein weiterer spezieller Aspekt der Erfindung betrifft die Verbindungen der Formel (I) zur Verwendung als Antihelminthikum, insbesondere zur Verwendung als Nematizid, Platymelminthizid, Acanthocephalizid oder Pentastomizid.

Ein weiterer spezieller Aspekt der Erfindung betrifft die Verbindungen der Formel (I) zur Verwendung als Antiprotozoikum.

Ein weiterer Aspekt betrifft die Verbindungen der Formel (I) zur Verwendung als Antiektoparasitikum, insbesondere ein Arthropodizid, ganz besonders ein Insektizid oder ein Akarizid.

Weitere Aspekte der Erfindung sind veterinärmedizinische Formulierungen, die eine wirksame Menge mindestens einer Verbindung der Formel (I) und mindestens einen der folgenden umfassen: einen pharmazeutisch unbedenklichen Exzipienten (z.B. feste oder flüssige Verdünnungsmittel), ein pharmazeutisch unbedenkliches Hilfsmittel (z.B. Tenside), insbesondere einen herkömmlicherweise in veterinärmedizinischen Formulierungen verwendeten pharmazeutisch unbedenklichen Exzipienten und/oder ein herkömmlicherweise in veterinärmedizinischen Formulierungen verwendetes pharmazeutisch unbedenkliches Hilfsmittel.

Ein verwandter Aspekt der Erfindung ist ein Verfahren zur Herstellung einer wie hier beschriebenen veterinärmedizinischen Formulierung, welches den Schritt des Mischens mindestens einer Verbindung der Formel (I) mit pharmazeutisch unbedenklichen Exzipienten und/oder Hilfsmitteln, insbesondere mit herkömmlicherweise in veterinärmedizinischen Formulierungen verwendeten pharmazeutisch unbedenklichen Exzipienten und/oder herkömmlicherweise in veterinärmedizinischen Formulierungen verwendeten Hilfsmitteln umfasst.

Ein anderer spezieller Aspekt der Erfindung sind veterinärmedizinische Formulierungen ausgewählt aus der Gruppe ektoparasitizider und endoparasitizider Formulierungen, insbesondere ausgewählt aus der Gruppe anthelmintischer, antiprotozolischer und arthropodizider Formulierungen, ganz besonders ausgewählt aus der Gruppe nematizider, platyhelminthizider, acanthocephalizider, pentastomizider, insektizider und akkarizider Formulierungen, gemäß den erwähnten Aspekten, sowie Verfahren zu ihrer Herstellung.

Ein anderer Aspekt bezieht sich auf ein Verfahren zur Behandlung einer parasitischen Infektion, insbesondere einer Infektion durch einen Parasiten ausgewählt aus der Gruppe der hier erwähnten Ektoparasiten und Endoparasiten, durch Anwendung einer wirksamen Menge einer Verbindung der Formel (I) bei einem Tier, insbesondere einem nichthumanen Tier, das dessen bedarf.

Ein anderer Aspekt bezieht sich auf ein Verfahren zur Behandlung einer parasitischen Infektion, insbesondere einer Infektion durch einen Parasiten ausgewählt aus der Gruppe der hier erwähnten Ektoparasiten und Endoparasiten, durch Anwendung einer wie hier definierten veterinärmedizinischen Formulierung bei einem Tier, insbesondere einem nichthumanen Tier, das dessen bedarf.

Ein anderer Aspekt bezieht sich auf die Verwendung der Verbindungen der Formel (I) bei der Behandlung einer Parasiteninfektion, insbesondere einer Infektion durch einen Parasiten ausgewählt aus der Gruppe der hier erwähnten Ektoparasiten und Endoparasiten, bei einem Tier, insbesondere einem nichthumanen Tier.

Im vorliegenden tiergesundheitlichen oder veterinärmedizinischen Zusammenhang schließt der Begriff "Behandlung" die prophylaktische, die metaphylaktische und die therapeutische Behandlung ein.

Bei einer bestimmten Ausführungsform werden hiermit Mischungen mindestens einer Verbindung der Formel (I) mit anderen Wirkstoffen, insbesondere mit Endo- und Ektoparasitiziden, für das veterinärmedizinische Gebiet bereitgestellt.

Auf dem Gebiet der Tiergesundheit bedeutet "Mischung" nicht nur, dass zwei (oder mehr) verschiedene Wirkstoffe in einer gemeinsamen Formulierung formuliert werden und entsprechend zusammen angewendet werden, sondern bezieht sich auch auf Produkte, die für jeden Wirkstoff getrennte Formulierungen umfassen. Dementsprechend können, wenn mehr als zwei Wirkstoffe angewendet werden sollen, alle Wirkstoffe in einer gemeinsamen Formulierung formuliert werden oder alle Wirkstoffe in getrennten Formulierungen formuliert werden; ebenfalls denkbar sind gemischte Formen, bei denen einige der Wirkstoffe gemeinsam formuliert und einige der Wirkstoffe getrennt formuliert sind. Getrennte Formulierungen erlauben die getrennte oder aufeinanderfolgende Anwendung der in Rede stehenden Wirkstoffe.

Die hier mit ihrem "Common Name" spezifizierten Wirkstoffe sind bekannt und beispielsweise im "Pesticide Manual" (siehe oben) beschrieben oder im Internet recherchierbar (z.B. http://www.alanwood.net/pesticides).

Beispielhafte Wirkstoffe aus der Gruppe der Ektoparasitizide als Mischungspartner schließen, ohne dass dies eine Einschränkung darstellen soll, die oben ausführlich aufgelisteten Insektizide und Akkarizide ein. Weitere verwendbare Wirkstoffe sind unten gemäß der oben erwähnten Klassifikation, die auf dem aktuellen IRAC Mode of Action Classification Scheme beruht, aufgeführt: (1) Acetylcholinesterase (AChE)-Inhibitoren; (2) GABA-gesteuerte Chlorid-Kanal-Blocker; (3) Natrium-Kanal-Modulatoren; (4) kompetitive Modulatoren des nicotinischen Acetylcholin-Rezeptors (nAChR); (5) allosterische Modulatoren des nicotinischen Acetylcholin-Rezeptors (nAChR); (6) allosterische Modulatoren des Glutamat-abhängigen Chloridkanals (GluCl); (7) Juvenilhormon-Mimetika; (8) verschiedene nichtspezifische (Multi-Site) Inhibitoren; (9) Modulatoren Chordotonaler Organe; (10) Milbenwachstumsinhibitoren; (12) Inhibitoren der mitochondrialen ATP-Synthase, wie ATP-Disruptoren; (13) Entkoppler der oxidativen Phosphorylierung durch Störung des Protonengradienten; (14) Blocker des nicotinischen Acetylcholinrezeptorkanals; (15) Inhibitoren der Chitinbiosynthese, Typ 0; (16) Inhibitoren der Chitinbiosynthese, Typ 1; (17) Häutungsdisruptor (insbesondere bei Dipteren, d.h. Zweiflüglern); (18) Ecdyson-Rezeptor-Agonisten; (19) Octopamin-Rezeptor-Agonisten; (21) mitochondriale Komplex-I-Elektronentransportinhibitoren; (25) mitochondriale Komplex-II-Elektronentransportinhibitoren; (20) mitochondriale Komplex-III-Elektronentransportinhibitoren; (22) Blocker des spannungsabhängigen Natriumkanals; (23) Inhibitoren der Acetyl-CoA-Carboxylase; (28) Ryanodinrezeptor-Modulatoren;

Wirkstoffe mit unbekannten oder nicht spezifischen Wirkmechanismen, z. B. Fentrifanil, Fenoxacrim, Cyclopren, Chlorobenzilat, Chlordimeform, Flubenzimin, Dicyclanil, Amidoflumet, Quinomethionat, Triarathen, Clothiazoben, Tetrasul, Kaliumoleat, Petroleum, Metoxadiazon, Gossyplur, Flutenzin, Brompropylat, Cryolit;

Verbindungen aus anderen Klassen, z.B. Butacarb, Dimetilan, Cloethocarb, Phosphocarb, Pirimiphos(-ethyl), Parathion(-ethyl), Methacrifos, Isopropyl-o-salicylat, Trichlorfon, Sulprofos, Propaphos, Sebufos, Pyridathion, Prothoat, Dichlofenthion, Demeton-S-methylsulfon, Isazofos, Cyanofenphos, Dialifos, Carbophenothion, Autathiofos, Aromfenvinfos(-methyl), Azinphos(-ethyl), Chlorpyrifos(-ethyl), Fosmethilan, Iodofenphos, Dioxabenzofos, Formothion, Fonofos, Flupyrazofos, Fensulfothion, Etrimfos;

Organochlorverbindungen, z. B. Camphechlor, Lindan, Heptachlor; oder Phenylpyrazole, z. B. Acetoprol, Pyrafluprol, Pyriprol, Vaniliprol, Sisapronil; oder Isoxazoline, z. B. Sarolaner, Afoxolaner, Lotilaner, Fluralaner;

Pyrethroide, z. B. (cis-, trans-)Metofluthrin, Profluthrin, Flufenprox, Flubrocythrinat, Fubfenprox, Fenfluthrin, Protrifenbut, Pyresmethrin, RU15525, Terallethrin, cis-Resmethrin, Heptafluthrin, Bioethanomethrin, Biopermethrin, Fenpyrithrin, cis-Cypermethrin, cis-Permethrin, Clocythrin, Cyhalothrin (lambda-), Chlovaporthrin, oder halogenierte Kohlenwasserstoffverbindungen (HCHs),
Neonicotinoide, z. B. Nithiazin
Dicloromezotiaz, Triflumezopyrim
makrocyclische Lactone, z. B. Nemadectin, Ivermectin, Latidectin, Moxidectin, Selamectin, Eprinomectin, Doramectin, Emamectinbenzoat; Milbemycinoxim
Tripren, Epofenonan, Diofenolan;
Biologicals, Hormone oder Pheromone, zum Beispiel natürliche Produkte, z.B. Thuringiensin, Codlemon oder Neem-Komponenten
Dinitrophenole, z. B. Dinocap, Dinobuton, Binapacryl;
Benzoylharnstoffe, z. B. Fluazuron, Penfluron,
Amidinderivate, z. B. Chlormebuform, Cymiazol, Demiditraz
Bienenstockvarroa-Akarizide, zum Beispiel organische Säuren, z.B. Ameisensäure, Oxalsäure.

Zu beispielhaften Wirkstoffen aus der Gruppe der Endoparasitizide, als Mischungspartner, zählen, ohne hierauf beschränkt zu sein, anthelmintische Wirkstoffe und antiprotozoische Wirkstoffe.

Zu den anthelmintischen Wirkstoffen zählen, ohne hierauf beschränkt zu sein, die folgenden nematiziden, trematiziden und/oder cestoziden Wirkstoffe:

aus der Klasse der makrocyclischen Lactone zum Beispiel: Eprinomectin, Abamectin, Nemadectin, Moxidectin, Doramectin, Selamectin, Lepimectin, Latidectin, Milbemectin, Ivermectin, Emamectin, Milbemycin;

aus der Klasse der Benzimidazole und Probenzimidazole zum Beispiel: Oxibendazol, Mebendazol, Triclabendazol, Thiophanat, Parbendazol, Oxfendazol, Netobimin, Fenbendazol, Febantel, Thiabendazol, Cyclobendazol, Cambendazol, Albendazol-sulfoxid, Albendazol, Flubendazol;

aus der Klasse der Depsipeptide, vorzugsweise cyclischen Depsipetide, insbesondere 24-gliedrigen cyclischen Depsipeptide, zum Beispiel: Emodepsid, PF1022A;

aus der Klasse der Tetrahydropyrimidine zum Beispiel: Morantel, Pyrantel, Oxantel;

aus der Klasse der Imidazothiazole zum Beispiel: Butamisol, Levamisol, Tetramisol;

aus der Klasse der Aminophenylamidine zum Beispiel: Amidantel, deacyliertes Amidantel (dAMD), Tribendimidin;

aus der Klasse der Aminoacetonitrile zum Beispiel: Monepantel;

aus der Klasse der Paraherquamide zum Beispiel: Paraherquamid, Derquantel;

aus der Klasse der Salicylanilide zum Beispiel: Tribromsalan, Bromoxanid, Brotianid, Clioxanid, Closantel, Niclosamid, Oxyclozanid, Rafoxanid;

aus der Klasse der substituierten Phenole zum Beispiel: Nitroxynil, Bithionol, Disophenol, Hexachlorophen, Niclofolan, Meniclopholan;

aus der Klasse der Organophosphate zum Beispiel: Trichlorfon, Naphthalofos, Dichlorvos/DDVP, Crufomat, Coumaphos, Haloxon;

aus der Klasse der Piperazinone/Chinoline zum Beispiel: Praziquantel, Epsiprantel;

aus der Klasse der Piperazine zum Beispiel: Piperazin, Hydroxyzin;

aus der Klasse der Tetracycline zum Beispiel: Tetracyclin, Chlorotetracyclin, Doxycyclin, Oxytetracyclin, Rolitetracyclin;

aus diversen anderen Klassen zum Beispiel: Bunamidin, Niridazol, Resorantel, Omphalotin, Oltipraz, Nitroscanat, Nitroxynil, Oxamniquin, Mirasan, Miracil, Lucanthon, Hycanthon, Hetolin, Emetin, Diethylcarbamazin, Dichlorophen, Diamfenetid, Clonazepam, Bephenium, Amoscanat, Clorsulon.

Antiprotozoische Wirkstoffe, darunter, ohne hierauf beschränkt zu sein, die folgenden Wirkstoffe:

aus der Klasse der Triazine zum Beispiel: Diclazuril, Ponazuril, Letrazuril, Toltrazuril;

aus der Klasse Polyletherionophor zum Beispiel: Monensin, Salinomycin, Maduramicin, Narasin;

aus der Klasse der makrocyclischen Lactone zum Beispiel: Milbemycin, Erythromycin;

aus der Klasse der Chinolone zum Beispiel: Enrofloxacin, Pradofloxacin;

aus der Klasse der Chinine zum Beispiel: Chloroquin;

aus der Klasse der Pyrimidine zum Beispiel: Pyrimethamin;

aus der Klasse der Sulfonamide zum Beispiel: Sulfachinoxalin, Trimethoprim, Sulfaclozin;

aus der Klasse der Thiamine zum Beispiel: Amprolium;

aus der Klasse der Lincosamide zum Beispiel: Clindamycin;

aus der Klasse der Carbanilide zum Beispiel: Imidocarb;

aus der Klasse der Nitrofurane zum Beispiel: Nifurtimox;

aus der Klasse der Chinazolinonalkaloide zum Beispiel: Halofuginon;

aus diversen anderen Klassen zum Beispiel: Oxamniquin, Paromomycin;

aus der Klasse der Vakzine oder Antigene aus Mikroorganismen zum Beispiel: Babesia canis rossi, Eimeria tenella, Eimeria praecox, Eimeria necatrix, Eimeria mitis, Eimeria maxima, Eimeria brunetti, Eimeria acervulina, Babesia canis vogeli, Leishmania infantum, Babesia canis canis, Dictyocaulus viviparus.

Alle genannten Mischungspartner können außerdem, wenn sie auf Grund ihrer funktionellen Gruppen dazu imstande sind, gegebenenfalls mit geeigneten Basen oder Säuren Salze bilden.

**Vektorbekämpfung**

Die Verbindungen der Formel (I) können auch in der Vektorbekämpfung eingesetzt werden. Ein Vektor im Sinne der vorliegenden Erfindung ist ein Arthropode, insbesondere ein Insekt oder Arachnide, der in der Lage ist, Krankheitserreger wie z. B. Viren, Würmer, Einzeller und Bakterien aus einem Reservoir (Pflanze, Tier, Mensch, etc.) auf einen Wirt zu übertragen. Die Krankheitserreger können entweder mechanisch (z. B. Trachoma durch nicht-stechende Fliegen) auf einem Wirt, oder nach Injektion (z. B. Malaria-Parasiten durch Mücken) in einen Wirt übertragen werden.
Beispiele für Vektoren und die von ihnen übertragenen Krankheiten bzw. Krankheitserreger sind:

1) Mücken

- Anopheles: Malaria, Filariose;
- Culex: Japanische Encephalitis, Filariasis, weitere virale Erkrankungen, Übertragung von anderen Würmern;
- Aedes: Gelbfieber, Dengue-Fieber, weitere virale Erkrankungen, Filariasis;
- Simulien: Übertragung von Würmern, insbesondere Onchocerca volvulus;
- Psychodidae: Übertragung von Leishmaniose

2) Läuse: Hautinfektionen, epidemisches Fleckfieber;

3) Flöhe: Pest, endemisches Fleckfieber, Bandwürmer;

4) Fliegen: Schlafkrankheit (Trypanosomiasis); Cholera, weitere bakterielle Erkrankungen;

5) Milben: Acariose, epidemisches Fleckfieber, Rickettsipocken, Tularämie, Saint-Louis-Enzephalitis, Frühsommer-Meningoenzephalitis (FSME), Krim-Kongo-Fieber, Borreliose;

6) Zecken: Borelliosen wie Borrelia bungdorferi sensu lato., Borrelia duttoni, Frühsommer-Meningoenzephalitis, Q-Fieber (Coxiella burnetii), Babesien (Babesia canis canis), Ehrlichiose.

Beispiele für Vektoren im Sinne der vorliegenden Erfindung sind Insekten, zum Beispiel Aphiden, Fliegen, Zikaden oder Thripse, die Pflanzenviren auf Pflanzen übertragen können. Weitere Vektoren, die Pflanzenviren übertragen können, sind Spinnmilben, Läuse, Käfer und Nematoden.

Weitere Beispiele für Vektoren im Sinne der vorliegenden Erfindung sind Insekten und Arachniden wie Mücken, insbesondere der Gattungen Aedes, Anopheles, z. B. A. gambiae, A. arabiensis, A. funestus, A. dirus (Malaria) und Culex, Psychodide wie Phlebotomus, Lutzomyia, Läuse, Flöhe, Fliegen, Milben und Zecken, die Krankheitserreger auf Tiere und/oder Menschen übertragen können.

Eine Vektorbekämpfung ist auch möglich, wenn die Verbindungen der Formel (I) Resistenz-brechend sind.

Verbindungen der Formel (I) sind zur Verwendung in der Prävention von Krankheiten und/oder Krankheitserregern, die durch Vektoren übertragen werden, geeignet. Somit ist ein weiterer Aspekt der vorliegenden Erfindung die Verwendung von Verbindungen der Formel (I) zur Vektorbekämpfung, z. B. in der Landwirtschaft, im Gartenbau, in Forsten, in Gärten und Freizeiteinrichtungen sowie im Vorrats- und Materialschutz.

**Schutz von technischen Materialen**

Die Verbindungen der Formel (I) eignen sich zum Schutz von technischen Materialien gegen Befall oder Zerstörung durch Insekten, z. B. aus den Ordnungen Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer weiteren Ausführungsform werden die Verbindungen der Formel (I) zusammen mit mindestens einem weiteren Insektizid und/oder mindestens einem Fungizid eingesetzt.

In einer weiteren Ausführungsform liegen die Verbindungen der Formel (I) als ein anwendungsfertiges (ready-to-use) Schädlingsbekämpfungsmittel vor, d. h., sie können ohne weitere Änderungen auf das entsprechende Material aufgebracht werden. Als weitere Insektizide oder Fungizide kommen insbesondere die oben genannten in Frage.

Überraschenderweise wurde auch gefunden, dass die Verbindungen der Formel (I) zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, verwendet werden können. Gleichfalls können die Verbindungen der Formel (I) allein oder in Kombinationen mit anderen Wirkstoffen als Antifouling-Mittel eingesetzt werden.

**Bekämpfung von tierischen Schädlingen auf dem Hygienesektor**

Die Verbindungen der Formel (I) eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren, Zecken und Milben, die in geschlossenen Räumen, wie beispielsweise Wohnungen, Fabrikhallen, Büros, Fahrzeugkabinen, Tierzuchtanlagen vorkommen. Zur Bekämpfung der tierischen Schädlinge werden die Verbindungen der Formel (I) allein oder in Kombination mit anderen Wirk- und/oder Hilfsstoffen verwendet. Bevorzugt werden sie in Haushaltsinsektizid-Produkten verwendet. Die Verbindungen der Formel (I) sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera, Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z. B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

**Herstellungs beispiele:**

**2-[3-(ethylsulfonyl)-6-(1-methyl-1H-indol-3-yl)pyridin-2-yl]-3-methyl-6-(trifluoromethyl)-3H-imidazo[4,5-c]pyridine (Beispiel I-1)**

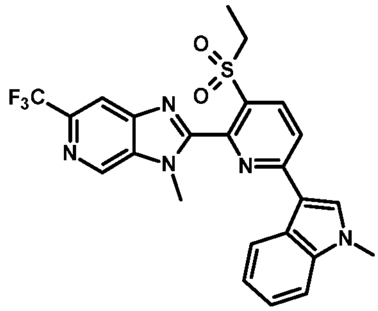

100 mg (0,24 mmol) 2-(6-Chlor-3-ethylsulfonyl-2-pyridyl)-3-methyl-6-(trifluormethyl)imidazo[4,5-c]pyridin, 64 mg (0,24 mmol) 1-Methyl-3-(4,4,5,5-tetramethyl-1,3,2-dioxaborolan-2-yl)-1H-indol und 9 mg (0,01 mmol) Tetrakis(triphenylphosphin)palladium(0) wurden in einem Gemisch aus entgastem Dioxan (2 mL) und entgaster Natriumcarbonatlösung (1M, 1 mL) vorgelegt und 14 h bei 96° C gerührt. Anschließend wurde auf Raumtemperatur abgekühlt, das Reaktionsgemisch wurde im Vakuum eingeengt und der Rückstand wurde in Wasser und Dichlormethan aufgenommen. Die Phasen wurden getrennt, die wässrige Phase wurde dreimal mit Dichlormethan extrahiert und die vereinigten organischen Phasen wurden über Natriumsulfat getrocknet und filtriert. Das Lösungsmittel wurde im Vakuum abdestilliert und der Rückstand durch säulenchromatographische Aufreinigung mit einem Wasser / Acetonitril Gradienten als Laufmittel gereinigt.
logP (neutral): 3,45; $MH^+$: 500; $^1H$-NMR(400 MHz, $D_6$-DMSO) $\delta$ ppm: 1,23 (t, 3H), 3,74 (q, 2H), 3,92 (s, 3H), 3,97 (s, 3H), 7,16-7,19 (m, 1H), 7,26-7,29 (m, 1H), 7,57 (d, 1H), 8,25-8,32 (m, 3H), 8,38 (d, 1H), 8,51 (s, 1H), 9,33 (s, 1H).

**2-[6-(6-Chlorindazol-1-yl)-3-ethylsulfonyl-2-pyridyl]-3-methyl-6-(trifluormethyl)imidazo[4,5-c]pyridin (Beispiel I-2)**

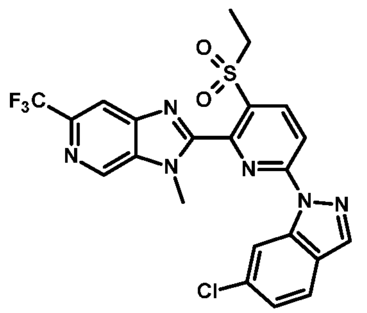

100 mg (0,24 mmol) 2-(6-Chlor-3-ethylsulfonyl-2-pyridyl)-3-methyl-6-(trifluormethyl)imidazo[4,5-c]pyridin wurden in 8 ml Acetonitril gelöst, 120,7 mg Cäsiumcarbonat (0,37 mmol), 20,5 mg Kaliumiodid (0,12 mmol) und 75,4 mg (0,49 mmol) 6-Chlor-1H-indazol zugegeben. Der Ansatz wurde 1 h bei Raumtemperatur und 17 h bei 65-70°C gerührt. Anschließend wurde das Reaktionsgemisch abfiltriert und das Lösungsmittel im Vakuum abdestilliert. Der Rückstand wurde durch säulenchromatographische Aufreinigung über präparative HPLC mit einem Wasser / Acetonitril Gradienten als Laufmittel gereinigt.
logP (neutral): 3,92; $MH^+$: 521; $^1H$-NMR(600 MHz, $D_7$-DMF) $\delta$ ppm: 1,33 (t, 3H), 3,88 (q, 2H), 4,22 (s, 3H), 7,47 (d, 1H), 8,05 (d, 1H), 8,35 (s, 1H), 8,57 (d, 1H), 8,66 (s, 1H), 8,72 (s, 1H), 8,76 (d, 1H), 9,46 (s, 1H).

**2-(6-Chlor-3-ethylsulfonyl-2-pyridyl)-3-methyl-6-(trifluormethyl)imidazo[4,5-c]pyridin**

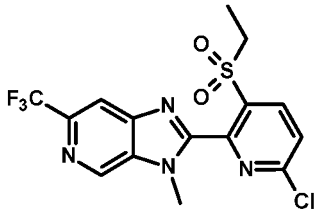

900 mg (2,41 mmol) 2-(6-Chloro-3-ethylsulfanyl-2-pyridyl)-3-methyl-6-(trifluormethyl)imidazo[4,5-c]pyridin wurden in 50 ml Dichlormethan gelöst, bei Raumtemperatur 555,6 mg (12,0 mmol) Ameisensäure und 1,64 g (16,8 mmol) 35%iges Wasserstoffperoxid zugegeben und anschließend 5 h bei Raumtemperatur gerührt. Der Ansatz wurde mit Wasser verdünnt und mit Natriumbisulfit-Lösung versetzt, 1 h gerührt, und anschließend mit gesättigter Natriumhydrogencarbonat Lösung versetzt. Die organische Phase wurde abgetrennt, die wässrige Phase zweimal mit Dichlormethan extrahiert und die vereinigten organischen Phasen anschließend unter Vakuum vom Lösungsmittel befreit. Der Rückstand wurde durch säulenchromatographische Aufreinigung über präparative HPLC mit einem Wasser / Acetonitril Gradienten als Laufmittel gereinigt.
(logP (neutral): 2,54; $MH^+$: 405; $^1$H-NMR(400 MHz, $D_6$-DMSO) δ ppm: 1,20 (t, 3H), 3,77 (q, 2H), 3,91 (s, 3H), 8,13 (d, 1H), 8,32 (s, 1H), 8,56 (d, 1H), 9,30 (s, 1H).

**2-(6-Chlor-3-ethylsulfanyl-2-pyridyl)-3-methyl-6-(trifluormethyl)imidazo[4,5-c]-pyridin**

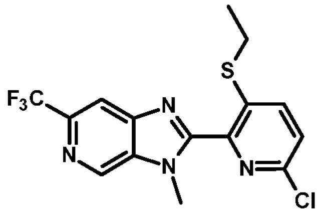

4,00 g (10,7 mmol) 2-(3,6-Dichlor-2-pyridyl)-3-methyl-6-(trifluormethyl)imidazo[4,5-c]pyridin wurden in 60 ml Tetrahydrofuran gelöst, mit 446 mg (11,1 mmol) Natriumhydrid bei -5°C versetzt und 30 Minuten bei 0°C gerührt. Anschließend wurden 733 mg (11,8 mmol) Ethanthiol über 30 Minuten bei - 5°C zugetropft, das Kältebad entfernt und 2 h bei Raumtemperatur nachgerührt. Das Reaktionsgemisch wurde mit Wasser hydrolisiert, die organische Phase wurde abgetrennt und die wässrige Phase zweimal mit Essigester extrahiert. Die organischen Phasen wurden vereinigt, mit Natriumchlorid Lösung gewaschen, über Natriumsulfat getrocknet und anschließend das Lösungsmittel im Vakuum abdestilliert. Der Rückstand wurde durch Verrühren in Methyl-tert.-butylketon / Dichlormethan 25:1 gereinigt.
(logP (neutral): 3,06; $MH^+$: 373

**2-(3,6-Dichlor-2-pyridyl)-3-methyl-6-(trifluormethyl)imidazo[4,5-c]pyridin**

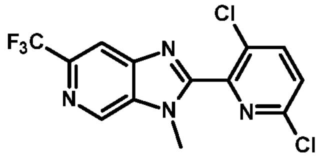

20 g (104,6 mmol) $N^3$-Methyl-6-(trifluormethyl)pyridin-3,4-diamin, 25,11 g (130,8 mmol) 3,6-Dichlorpyridin-2-carbonsäure und 20,06 g (104,6 mmol) 1-(3-Dimethylaminopropyl)-3-ethylcarbodiimid hydrochlorid (EDCI) wurden in 200 ml Pyridin 8 h bei 120 °C gerührt. Das Reaktionsgemisch wurde unter Vakuum vom Lösungsmittel befreit, mit Wasser versetzt und dreimal mit Essigester extrahiert. Die organischen Phasen wurden vereinigt, über Natriumsulfat getrocknet und anschließend das Lösungsmittel im Vakuum abdestilliert. Der Rückstand wurde durch säulenchromatographische Aufreinigung mit einem Cyclohexan / Essigester Gradienten als Laufmittel gereinigt.
(logP (neutral): 2,81; $MH^+$: 347; $^1$H-NMR(400 MHz, $D_6$-DMSO) δ ppm: 3,99 (s, 3H), 7,89 (d, 1H), 8,32 (s, 1H), 8,35 (d,

1H), 9,28 (s, 1H).

In Analogie zu den Beispielen und gemäß den oben beschriebenen Herstellverfahren lassen sich folgende Verbindungen der Formel (I) erhalten:

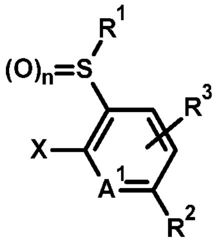

(I)

wobei die Substituenten $R^1$, $R^2$, $R^3$, $A^1$, X und n die in der folgenden Tabelle angegebenen Bedeutungen haben und wobei die Bindung von X bzw. $R^2$ zum Restmolekül mit einer gepunkteten Linie bzw. einer Wellenlinie gekennzeichnet ist:

| Bsp. Nr | $R^1$ | n | $A^1$ | $R^3$ | X | $R^2$ |
|---|---|---|---|---|---|---|
| I-1 | $-CH_2CH_3$ | 2 | N | -H | 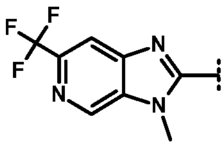 | 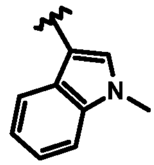 |
| I-2 | $-CH_2CH_3$ | 2 | N | -H | 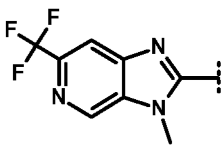 | 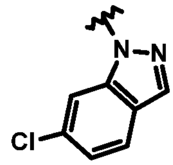 |
| I-3 | $-CH_2CH_3$ | 2 | N | -H | 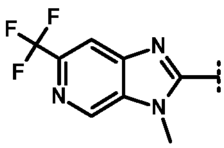 | 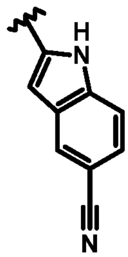 |
| I-4 | $-CH_2CH_3$ | 2 | N | -H | 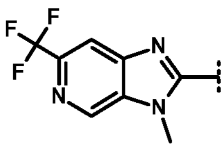 | 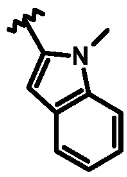 |
| I-5 | $-CH_2CH_3$ | 2 | N | -H | 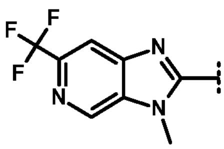 | 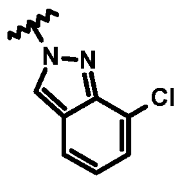 |

(fortgesetzt)

| Bsp. Nr | $R^1$ | n | $A^1$ | $R^3$ | X | $R^2$ |
|---|---|---|---|---|---|---|
| I-6 | $-CH_2CH_3$ | 2 | N | -H | 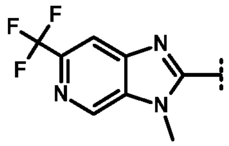 | 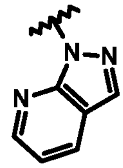 |
| I-7 | $-CH_2CH_3$ | 2 | N | -H | 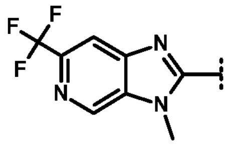 | 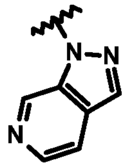 |
| I-8 | $-CH_2CH_3$ | 2 | N | -H | 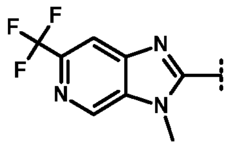 | 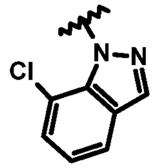 |
| I-9 | $-CH_2CH_3$ | 2 | N | -H | 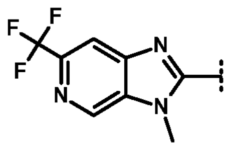 | 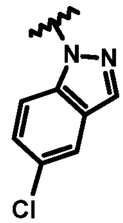 |
| I-10 | $-CH_2CH_3$ | 2 | N | -H | 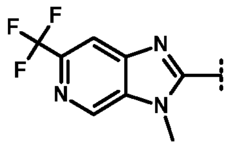 | 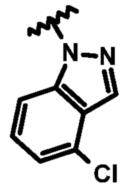 |
| I-11 | $-CH_2CH_3$ | 2 | N | -H | 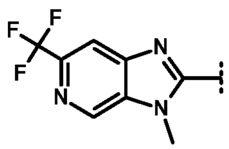 | 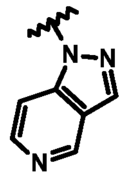 |
| I-12 | $-CH_2CH_3$ | 2 | N | -H | 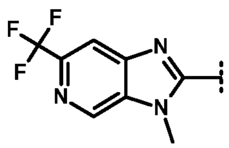 | 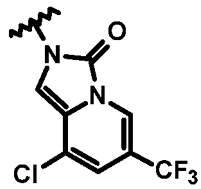 |
| I-13 | $-CH_2CH_3$ | 1 | N | -H | 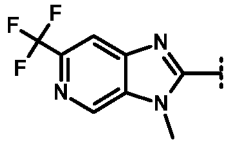 | 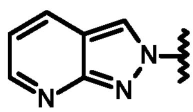 |

(fortgesetzt)

| Bsp. Nr | $R^1$ | n | $A^1$ | $R^3$ | X | $R^2$ |
|---|---|---|---|---|---|---|
| I-14 | $-CH_2CH_3$ | 2 | N | -H | 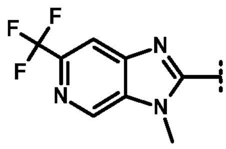 | 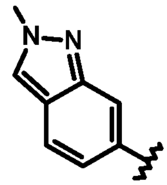 |
| I-15 | $-CH_2CH_3$ | 2 | N | -H | 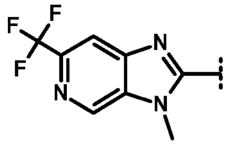 | 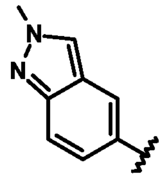 |
| I-16 | $-CH_2CH_3$ | 2 | N | -H | 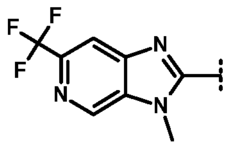 | 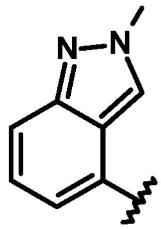 |
| I-17 | $-CH_2CH_3$ | 2 | N | -H | 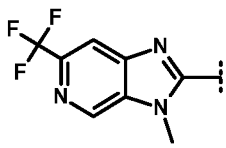 | 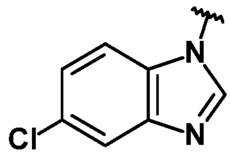 |
| I-18 | $-CH_2CH_3$ | 2 | N | -H | 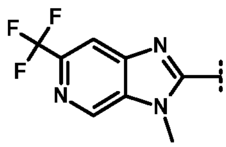 | 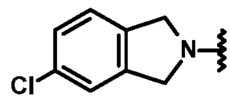 |
| I-19 | $-CH_2CH_3$ | 2 | N | -H | 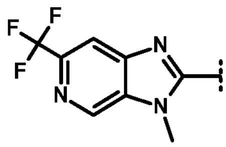 | 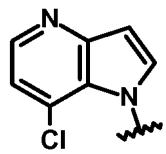 |
| I-20 | $-CH_2CH_3$ | 2 | N | -H | 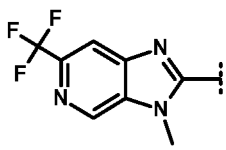 | 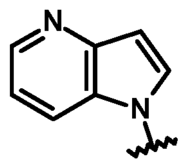 |
| I-21 | $-CH_2CH_3$ | 2 | N | -H | 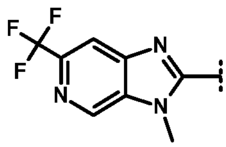 | 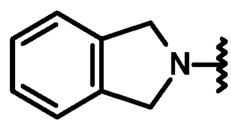 |
| I-22 | $-CH_2CH_3$ | 2 | N | -H | 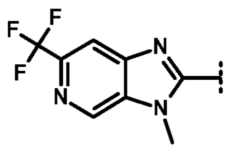 | 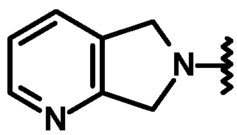 |

(fortgesetzt)

| Bsp. Nr | $R^1$ | n | $A^1$ | $R^3$ | X | $R^2$ |
|---|---|---|---|---|---|---|
| I-23 | $-CH_2CH_3$ | 2 | N | -H | 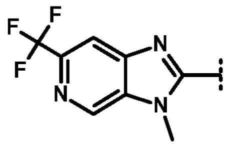 | 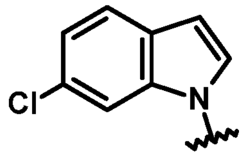 |
| I-24 | $-CH_2CH_3$ | 2 | N | -H | 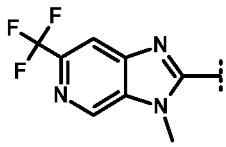 | 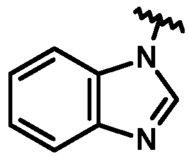 |
| I-25 | $-CH_2CH_3$ | 2 | N | -H | 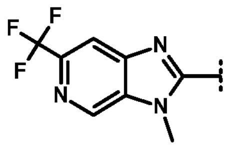 | 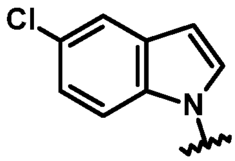 |
| I-26 | $-CH_2CH_3$ | 2 | N | -H | 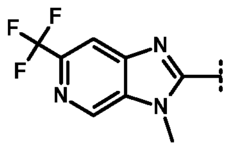 | 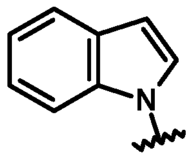 |
| I-27 | $-CH_2CH_3$ | 2 | N | -H | 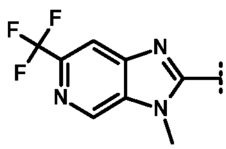 | 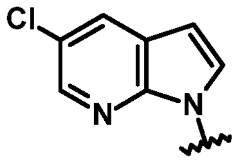 |
| I-28 | $-CH_2CH_3$ | 2 | N | -H | 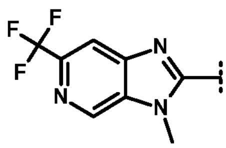 | 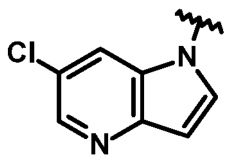 |
| I-29 | $-CH_2CH_3$ | 2 | N | -H | 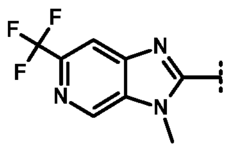 | 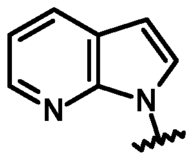 |
| I-30 | $-CH_2CH_3$ | 2 | N | -H | 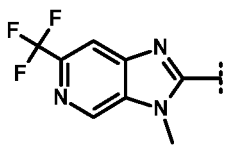 | 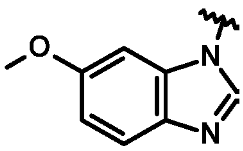 |
| I-31 | $-CH_2CH_3$ | 2 | N | -H | 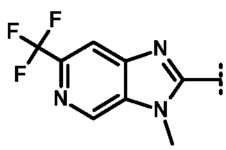 | 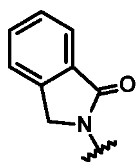 |

(fortgesetzt)

| Bsp. Nr | $R^1$ | n | $A^1$ | $R^3$ | X | $R^2$ |
|---|---|---|---|---|---|---|
| I-32 | $-CH_2CH_3$ | 2 | N | -H | 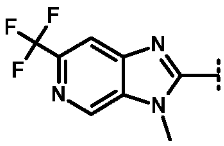 | 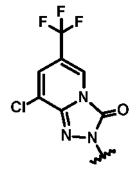 |
| I-33 | $-CH_2CH_3$ | 2 | N | -H | 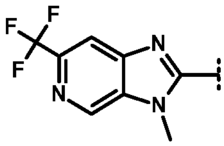 | 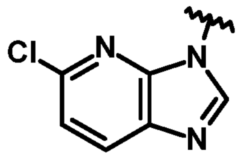 |
| I-34 | $-CH_2CH_3$ | 2 | N | -H | 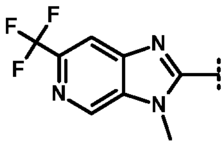 | 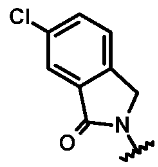 |
| I-35 | $-CH_2CH_3$ | 2 | N | -H | 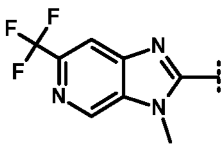 | 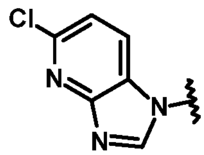 |
| I-36 | $-CH_2CH_3$ | 2 | N | -H | 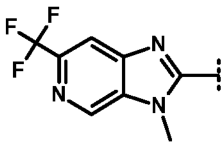 | 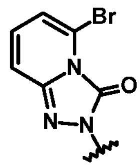 |
| I-37 | $-CH_2CH_3$ | 2 | N | -H | 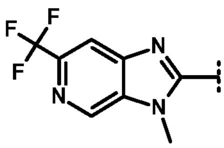 | 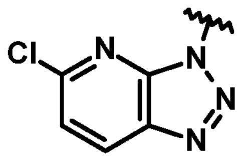 |
| I-38 | $-CH_2CH_3$ | 2 | N | -H | 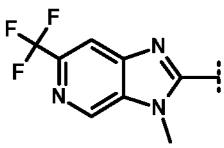 | 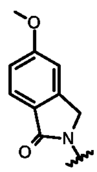 |
| I-39 | $-CH_2CH_3$ | 2 | N | -H | 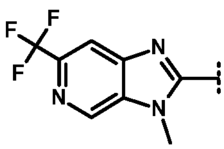 | 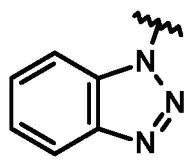 |
| I-40 | $-CH_2CH_3$ | 2 | N | -H | 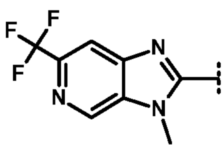 | 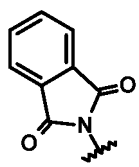 |

(fortgesetzt)

| Bsp. Nr | $R^1$ | n | $A^1$ | $R^3$ | X | $R^2$ |
|---|---|---|---|---|---|---|
| I-41 | $-CH_2CH_3$ | 2 | N | -H | 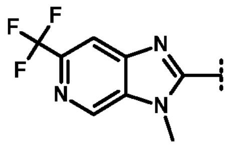 | 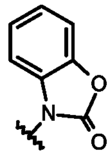 |
| I-42 | $-CH_2CH_3$ | 2 | N | -H | 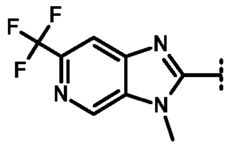 | 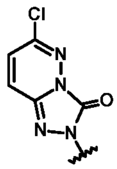 |
| I-43 | $-CH_2CH_3$ | 2 | N | -H | 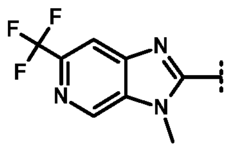 | 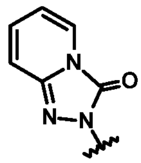 |
| I-44 | $-CH_2CH_3$ | 2 | N | -H | 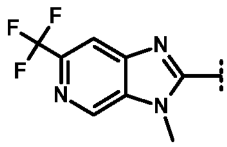 | 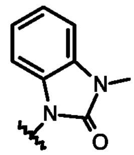 |
| I-45 | $-CH_2CH_3$ | 2 | N | -H | 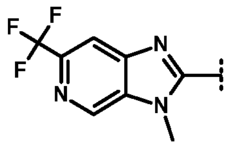 | 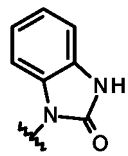 |
| I-46 | $-CH_2CH_3$ | 2 | N | -H | 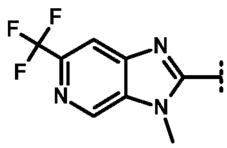 | 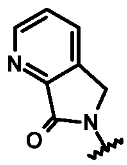 |
| I-47 | $-CH_2CH_3$ | 2 | N | -H | 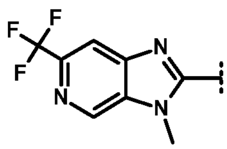 | 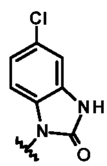 |
| I-48 | $-CH_2CH_3$ | 2 | N | -H | 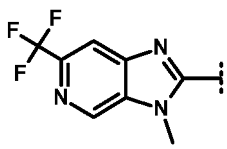 | 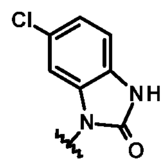 |
| I-49 | $-CH_2CH_3$ | 2 | N | -H | 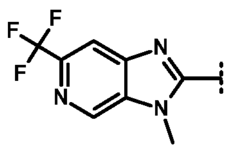 | 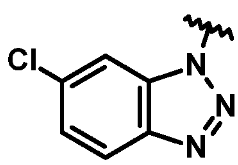 |

(fortgesetzt)

| Bsp. Nr | $R^1$ | n | $A^1$ | $R^3$ | X | $R^2$ |
|---|---|---|---|---|---|---|
| I-50 | $-CH_2CH_3$ | 2 | N | -H | 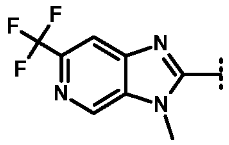 | 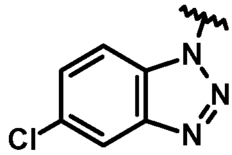 |
| I-51 | $-CH_2CH_3$ | 2 | N | -H | 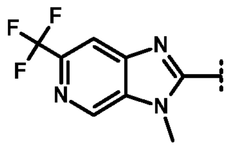 | 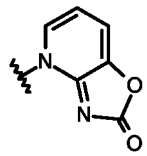 |

| LOGP_ NEUTRAL | LOGP_ HCOOH | NMR |
|---|---|---|
| 3,45 | 3,55 | Beispiel I-1: $^1$H-NMR(400,0 MHz, $d_6$-DMSO): δ= 9,332(4,1);8,511(5,7);8,389(2,7);8,368 (4,0);8,318(4,4);8,306 (1,8);8,286(2,0);8,275(3,7);8,253(2,6);7,581(1,9);7,561(2,2); 7,294(0,9);7,276(1,7);7,258(1,0);7,256(1,1);7,193 (1,2);7,175(1,8);7,155(0,8);5,754 (14,4);3,970(16,0);3,917(14,6);3,769(0,9);3,751(3,3);3,732(3,3);3,714(1,0);3, 319(57,8); 2,676(0,3);2,671(0,5);2,667(0,4);2,524(1,0);2,519(1,7);2,511(27,7);2,507(58,8);2,502 (79,5);2,497(5 7,3);2,493(27,3);2,333(0,3);2,329(0,5);2,324(0,3);2,086(0,3);1,246(3,5); 1,228(8,1);1,209(3,5);0,146(0,5);0,00 8(3,9);0,000(115,3);-0,008(4,2);-0,150(0,5) |
| 3,92 | 4,01 | Beispiel I-2: $^1$H-NMR(400,0 MHz, $d_6$-DMSO): δ= 9,381(4,0);8,700(4,7);8,681(3,2);8,659 (4,0);8,491(3,0);8,489 (3,0);8,484(4,2);8,461(3,3);8,363(4,2);8,313(1,2);8,014(2,7); 7,992(2,9);7,459(1,8);7,454(1,8);7,438(1,7);7,433 (1,7);4,351(1,0);4,073(0,4);4,056 (0,3);4,038(0,9);4,026(16,0);3,988(0,5);3,807(0,9);3,789(3,2);3,771(3,2);3,75 2(1,0); 3,318(348,2);2,675(2,0);2,671(2,7);2,666(2,0);2,524(7,1);2,510(169,4);2,506(353,3); 2,502(472,8);2,49 7(337,5);2,493(159,8);2,333(2,0);2,328(2,7);2,324(2,0);2,073(2,1); 1,988(2,5);1,329(0,5);1,253(3,7);1,235(8,4) ;1,216(3,6);1,193(0,7);1,175(1,4);1,157 (0,7);0,146(1,0);0,008(8,0);0,000(242,5);-0,008(9,1);-0,150(1,1) |
| 3,08 | 3,18 | Beispiel I-3: $^1$H-NMR(400,0 MHz, $d_6$-DMSO): δ= 20,001(0,3);12,365(2,3);9,375(5,0); 9,340(0,4);8,577(12,5);8, 338(5,0);8,313(3,0);8,254(3,7);7,672(3,0);7,630(2,0);7,609 (3,1);7,555(2,8);7,552(2,6);7,531(1,5);5,753(0,4);4, 093(0,6);3,980(16,0);3,963(0,8); 3,897(0,4);3,809(1,3);3,790(3,5);3,772(3,6);3,754(1,1);3,455(0,4);3,315(599, 2);2,756 (0,4);2,670(6,4);2,666(4,8);2,620(0,7);2,505(743,7);2,501(1022,3);2,497(795,1);2,328 (6,0);2,324(4,6) ;2,086(1,8);1,250(3,9);1,232(8,4);1,213(3,7);0,146(1,6);0,008(11,2); 0,000(339,4);-0,008(15,3);-0,150(1,6) |
| 3,83 | 3,90 | Beispiel I-4: $^1$H-NMR(400,0 MHz, $d_6$-DMSO): δ= 9,314(4,0);8,546(2,4);8,525(4,0);8,462 (3,9);8,441(2,5);8,317 (4,3);7,701(1,8);7,681(2,0);7,577(1,7);7,556(2,0);7,420(4,6); 7,320(1,0);7,302(1,6);7,284(1,0);7,282(1,0);7,151 (1,3);7,132(1,9);7,113(1,0);5,754 (6,1);4,048(16,0);3,978(14,8);3,815(0,9);3,796(3,2);3,778(3,2);3,759(1,0);3,3 19(78,6); 2,675(0,4);2,671(0,5);2,666(0,4);2,524(1,3);2,511(28,5);2,506(60,2);2,502(84,5);2,497 (64,0);2,493(3 1,5);2,333(0,4);2,329(0,5);2,324(0,4);1,260(3,3);1,242(7,4);1,223(3,2); 0,000(7,7) |

(fortgesetzt)

| LOGP_ NEUTRAL | LOGP_ HCOOH | NMR |
|---|---|---|
| 3,92 | 3,94 | Beispiel I-5: $^1$H-NMR(400,0 MHz, $d_6$-DMSO): δ= 9,569(1,4);9,506(7,0);9,430(1,6);9,371 (4,0);9,351(0,5);8,821 (3,1);8,799(4,9);8,727(4,5);8,715(1,0);8,705(3,1);8,693(0,7); 8,529(0,8);8,513(0,7);8,381(0,9);8,365(0,6);8,355 (4,2);8,312(0,5);7,873(0,5);7,852 (0,5);7,791(0,4);7,783(2,3);7,771(0,4);7,763(2,5);7,553(2,5);7,535(2,6);7,509 (0,4); 7,184(0,4);7,159(1,9);7,150(0,4);7,141(1,9);7,138(2,0);7,129(0,4);7,120(1,7);4,488 (3,4);4,141(2,1);4,052 (16,0);3,911(0,9);3,893(3,3);3,874(3,4);3,856(1,0);3,362(0,3); 3,322(167,8);3,320(187,8);2,676(0,7);2,671(1,0) ;2,666(0,8);2,662(0,4);2,524(2,5); 2,520(3,9);2,511(59,2);2,507(123,3);2,502(165,7);2,497(123,1);2,493(61,5); 2,333(0,7); 2,329(1,0);2,324(0,8);2,073(3,2);1,358(0,8);1,340(1,8);1,321(0,8);1,281(3,6);1,262 (8,3);1,244(3,6); 0,008(1,3);0,000(42,4);-0,008(1,7) |
| 2,63 | 2,67 | Beispiel I-6: $^1$H-NMR(400,0 MHz, $d_6$-DMSO): δ= 9,335(4,2);9,277(0,5);8,957(3,2);8,935 (4,2);8,790(1,9);8,786 (2,1);8,778(6,0);8,775(2,4);8,756(3,4);8,666(7,8);8,485(1,8); 8,481(1,9);8,465(2,0);8,461(2,0);8,358(0,4);8,341 (4,5);8,314(0,6);7,900(0,4);7,878 (0,3);7,529(1,9);7,518(1,8);7,509(1,8);7,498(1,8);4,070(16,0);3,988(1,9);3,89 1(1,0); 3,873(3,2);3,855(3,2);3,836(1,0);3,342(134,3);3,336(178,0);3,329(143,1);3,326(150,9); 2,676(0,9);2,67 2(1,2);2,667(0,9);2,525(2,6);2,520(4,0);2,511(75,2);2,507(161,7);2,502 (220,3);2,498(159,3);2,494(76,6);2,33 3(0,9);2,329(1,3);2,325(0,9);2,074(0,6);1,280 (3,7);1,262(8,5);1,243(3,6);0,146(0,4);0,008(2,9);0,000(100,5);-0,008(3,5);-0,150(0,4) |
| 2,59 | 2,40 | Beispiel I-7: $^1$H-NMR(400,0 MHz, $d_6$-DMSO): δ= 9,826(3,7);9,347(4,1);9,048(0,8);8,808 (5,5);8,711(3,2);8,689 (4,0);8,518(3,2);8,506(5,2);8,484(3,4);8,349(4,3);8,347(4,3); 8,224(0,7);8,216(0,9);8,210(0,9);7,986(2,2);7,983 (2,3);7,972(2,1);7,969(2,1);7,764 (0,5);7,750(0,4);4,317(0,8);4,020(16,0);3,823(0,9);3,805(3,2);3,786(3,2);3,76 8(1,0); 3,320(72,5);2,677(0,4);2,672(0,5);2,668(0,4);2,525(1,3);2,521(2,1);2,512(32,8);2,508 (69,7);2,503(94,5) ;2,499(69,3);2,494(34,4);2,334(0,4);2,330(0,6);2,325(0,4);1,324 (0,4);1,261(3,6);1,243(8,1);1,224(3,5);0,146(0 ,5);0,008(4,0);0,000(114,8);-0,008(5,1);-0,149(0,5) |
| 3,34 | 3,44 | Beispiel I-8: $^1$H-NMR(601,6 MHz, $d_6$-DMSO): δ= 9,268(3,1);8,780(3,6);8,765(3,9);8,696 (7,0);8,384(3,9);8,370 (3,7);8,316(3,5);8,315(3,4);7,963(2,1);7,962(2,0);7,950(2,2); 7,948(2,0);7,621(2,0);7,620(1,9);7,609(2,3);7,608 (2,1);7,379(2,1);7,366(3,1);7,353 (1,9);3,865(16,0);3,748(1,0);3,736(3,3);3,724(3,3);3,711(0,9);3,315(117,3);2, 616(0,6); 2,613(0,8);2,610(0,5);2,523(1,3);2,520(1,6);2,516(1,4);2,508(39,4);2,505(88,1);2,502 (123,1);2,499(8 7,9);2,496(40,3);2,389(0,6);2,386(0,8);2,383(0,6);1,238(3,6);1,226(8,2); 1,214(3,6);0,005(1,1);0,000(46,2);-0,006(1,7) |
| 4,03 | 4,03 | Beispiel I-9: $^1$H-NMR(400,0 MHz, $d_6$-DMSO): δ= 9,350(4,1);8,670(3,2);8,647(8,3);8,523 (2,3);8,500(2,4);8,483 (4,1);8,460(3,4);8,354(4,4);8,353(4,4);8,312(0,7);8,065(3,1); 8,060(3,3);7,569(2,0);7,564(2,0);7,546(1,9);7,541 (1,9);3,999(16,0);3,815(0,9);3,797 (3,1);3,778(3,2);3,760(1,0);3,339(95,5);3,332(117,9);3,327(119,6);2,676(0, 6);2,671 (0,8);2,667(0,6);2,525(2,1);2,511(51,6);2,507(109,4);2,502(147,9);2,498(106,6);2,493 (51,0);2,334(0, 6);2,329(0,8);2,325(0,6);2,073(0,8);1,989(1,0);1,255(3,5);1,236(8,0); 1,218(3,5);1,175(0,6);0,008(1,1);0,000(3 3,1);-0,008(1,3) |
| 4,24 | 4,27 | Beispiel I-10: $^1$H-NMR(400,0 MHz, $d_6$-DMSO): δ= 9,345(4,0);8,753(4,4);8,751(4,8);8,696 (0,5);8,690(3,5);8,66 8(4,3);8,512(4,3);8,502(2,3);8,490(3,6);8,481(2,4);8,350(4,2); 7,565(1,4);7,546(2,5);7,525(2,4);7,489(3,3);7,47 2(1,7);7,470(1,7);4,301(0,8);4,060 (0,4);4,053(0,7);4,006(16,0);3,823(0,9);3,805(3,1);3,786(3,2);3,768(0,9);3,3 23(64,4); 3,321(60,7);2,676(0,3);2,672(0,5);2,667(0,3);2,525(1,2);2,512(25,5);2,508(53,1);2,503 (71,7);2,498(5 3,9);2,494(27,3);2,330(0,5);2,325(0,3);2,074(10,0);1,321(0,5);1,259(3,7); 1,241(8,3);1,222(3,5);0,008(0,6);0,0 00(21,3);-0,009(0,9) |

(fortgesetzt)

| LOGP_ NEUTRAL | LOGP_ HCOOH | NMR |
|---|---|---|
| 2,47 | 1,75 | Beispiel I-11: $^1$H-NMR(400,0 MHz, $d_6$-DMSO): δ= 9,354(2,9);9,277(2,9);9,274(3,1);8,859 (3,3);8,857(3,6);8,71 3(2,5);8,691(3,2);8,551(2,3);8,536(2,9);8,511(3,2);8,489(2,7); 8,384(1,7);8,370(1,5);8,367(1,0);8,355(3,0);8,35 3(3,1);4,019(11,8);3,837(0,7);3,818 (2,3);3,800(2,4);3,781(0,7);3,322(42,7);2,526(0,7);2,521(1,1);2,512(13,8); 2,508(28,6); 2,503(38,4);2,499(28,5);2,494(14,3);2,075(16,0);1,262(2,6);1,244(6,0);1,225(2,6);0,008 (0,3);0,00 0(10,0);-0,009(0,5) |
| 4,18 | 4,27 | Beispiel I-12: $^1$H-NMR(400,0 MHz, $d_6$-DMSO): δ= 9,322(4,3);8,918(3,1);8,895(4,3);8,767 (3,9);8,745(3,1);8,32 6(4,6);8,313(0,7);8,134(2,5);7,701(4,9);7,099(3,7);3,963(16,0); 3,824(0,9);3,818(1,1);3,800(3,3);3,781(3,4);3,7 63(1,1);3,317(250,3);2,675(1,4);2,670 (2,0);2,666(1,5);2,524(4,5);2,510(119,0);2,506(251,6);2,501(340,8);2,4 97(247,8);2,493 (120,6);2,333(1,4);2,328(2,0);2,324(1,5);2,073(2,7);1,243(3,7);1,224(8,1);1,206(3,5); 1,132(0, 4);0,000(3,4) |
| 2,45 | 2,46 | Beispiel I-13: $^1$H-NMR(400,0 MHz, $d_6$-DMSO): δ= 9,507(7,0);9,434(3,8);8,818(2,6);8,796 (6,1);8,792(2,1);8,78 6(2,0);8,781(1,9);8,735(4,4);8,714(2,8);8,385(4,1);8,378(1,8); 8,373(1,8);8,356(1,9);8,352(1,7);8,314(1,5);7,26 3(1,8);7,253(1,7);7,242(1,7);7,231 (1,8);4,492(16,0);4,339(0,7);3,624(1,0);3,605(1,0);3,591(1,2);3,572(1,0);3,5 52(0,3); 3,318(544,2);3,153(1,2);3,135(1,3);3,120(1,0);3,102(1,0);3,083(0,4);2,680(1,5);2,675 (3,1);2,671(4,2); 2,666(3,0);2,524(9,1);2,519(14,2);2,511(249,2);2,506(531,5);2,502 (716,9);2,497(513,2);2,493(241,8);2,333(3, 0);2,328(4,2);2,324(3,1);1,360(3,7);1,342 (8,2);1,323(3,7);1,232(0,5);0,146(4,2);0,020(0,8);0,008(32,3);0,000( 985,7);-0,009 (33,6);-0,054(0,4);-0,150(4,2) |
| 2,65 | 2,71 | Beispiel I-14: $^1$H-NMR(400,0 MHz, $d_6$-DMSO): δ= 9,321(4,2);8,637(2,2);8,616(3,9);8,566 (4,3);8,545(4,9);8,42 9(4,4);8,322(4,4);8,314(0,3);7,913(0,9);7,910(0,9);7,891(2,8); 7,887(3,0);7,871(3,5);7,849(1,1);5,754(3,6);4,22 4(14,5);3,985(16,0);3,842(1,0);3,823 (3,3);3,805(3,4);3,786(1,0);3,319(29,7);2,675(0,4);2,671(0,5);2,667(0,4); 2,524(1,5); 2,511(29,2);2,507(59,8);2,502(82,5);2,498(62,7);2,493(31,1);2,334(0,4);2,329(0,5); 2,324(0,4);1,26 0(3,6);1,242(8,2);1,223(3,6);0,008(1,7);0,000(43,4);-0,009(1,8) |
| 2,58 | 2,62 | Beispiel I-15: $^1$H-NMR(400,0 MHz, $d_6$-DMSO): δ= 9,324(4,6);8,709(3,7);8,561(0,3);8,539 (13,0);8,516(0,3);8,3 82(0,6);8,320(4,9);8,110(1,9);8,106(1,8);8,087(2,2);8,083(2,0); 7,950(0,6);7,744(2,6);7,721(2,3);7,696(0,5);7,6 78(0,8);7,660(0,5);7,570(0,4);7,566 (0,3);7,477(0,3);7,458(0,6);7,438(0,3);5,754(14,2);4,209(14,3);4,186(3,0); 3,972(16,0); 3,816(1,1);3,798(3,5);3,779(3,5);3,761(1,1);3,317(20,5);2,671(0,8);2,506(105,6);2,502 (132,4);2,4 97(101,1);2,328(0,8);1,252(3,7);1,233(8,2);1,215(3,6);0,000(29,9) |
| 2,67 | 2,73 | Beispiel I-16: $^1$H-NMR(400,0 MHz, $d_6$-DMSO): δ= 10,196(0,5);9,340(4,5);8,801(0,4); 8,699(4,7);8,656(2,1);8,6 35(4,0);8,589(4,2);8,568(2,4);8,342(4,9);8,097(0,4);8,039 (2,4);8,021(2,7);7,866(2,2);7,844(2,5);7,479(1,8);7,4 61(1,9);7,458(1,8);7,440(1,5); 5,754(11,0);4,197(1,4);4,185(16,0);4,132(1,1);3,973(16,0);3,923(1,1);3,848(1,0) ;3,829 (3,4);3,811(3,5);3,792(1,1);3,317(43,2);2,675(0,6);2,671(0,8);2,667(0,6);2,524(2,2); 2,506(92,9);2,502(1 27,0);2,497(95,3);2,333(0,6);2,329(0,8);2,324(0,6);1,264(3,7); 1,245(8,2);1,227(3,6);0,008(1,2);0,000(33,7);-0,009(1,3) |
| 3,12 | 3,19 | Beispiel I-17: $^1$H-NMR(400,0 MHz, $d_6$-DMSO): δ= 9,362(0,4);9,339(6,4);9,265(0,4);8,736 (1,8);8,724(0,4);8,71 4(2,3);8,555(2,1);8,532(1,7);8,351(3,2);8,283(1,8);8,261(1,9); 7,901(1,9);7,897(2,2);7,411(1,1);7,407(1,1);7,38 9(1,0);7,385(1,1);4,011(1,2);3,989 (10,2);3,841(0,7);3,822(2,3);3,804(2,4);3,785(0,7);3,321(18,4);2,671(0,4);2, 502(64,3); 2,329(0,4);2,075(16,0);1,267(2,6);1,248(5,7);1,230(2,6);0,000(15,0) |

(fortgesetzt)

| LOGP_ NEUTRAL | LOGP_ HCOOH | NMR |
|---|---|---|
| 3,99 | 4,03 | Beispiel I-18: $^1$H-NMR(400,0 MHz, $d_6$-DMSO): δ= 9,286(4,6);8,273(5,0);8,160(3,2);8,137 (3,4);7,536(0,6);7,49 2(0,8);7,460(0,6);7,408(2,1);7,389(0,8);6,994(1,9);6,971(1,8); 4,883(4,2);3,914(16,0);3,623(0,8);3,604(2,7);3,5 86(2,8);3,568(0,9);3,318(67,9);2,675 (1,0);2,670(1,4);2,666(1,1);2,506(177,4);2,501(235,3);2,497(179,6);2,33 2(1,0);2,328 (1,4);2,323(1,1);1,194(3,8);1,175(8,5);1,157(3,7);0,146(0,7);0,008(6,8);0,000(148,3);- 0,150(0,7) |
| 2,69 | 2,51 | Beispiel I-19: $^1$H-NMR(400,0 MHz, $d_6$-DMSO): δ= 9,284(2,5);8,755(2,4);8,734(2,7);8,455 (2,7);8,442(2,8);8,31 7(2,6);8,315(2,7);8,232(2,8);8,223(2,3);8,214(2,5);8,211(2,9); 7,422(2,4);7,409(2,4);7,042(2,8);7,033(2,8);3,93 1(10,9);3,821(0,7);3,803(2,2);3,784 (2,2);3,766(0,7);3,317(40,9);2,679(0,4);2,675(0,8);2,670(1,1);2,666(0,8);2, 661(0,4); 2,524(2,5);2,519(3,5);2,510(62,9);2,506(133,9);2,501(179,8);2,497(128,6);2,492(61,4); 2,337(0,4);2, 332(0,8);2,328(1,1);2,323(0,8);2,319(0,4);2,074(16,0);1,260(2,4);1,242 (5,6);1,223(2,3);0,146(0,7);0,008(5,2); 0,000(176,1);-0,009(6,2);-0,150(0,7) |
| 2,47 | 1,75 | Beispiel I-20: $^1$H-NMR(400,0 MHz, $d_6$-DMSO): δ= 9,334(4,0);8,699(1,7);8,678(1,7);8,646 (3,0);8,636(3,1);8,62 6(3,0);8,604(3,7);8,489(1,8);8,485(2,0);8,477(2,0);8,474(2,0); 8,402(3,3);8,379(2,8);8,345(4,4);7,274(1,6);7,26 3(1,6);7,253(1,6);7,242(1,6);7,049 (2,5);7,040(2,5);3,984(16,0);3,801(1,0);3,782(3,3);3,764(3,3);3,745(1,0);3,3 20(17,3); 2,676(0,3);2,671(0,5);2,667(0,4);2,525(1,2);2,520(1,8);2,511(26,4);2,507(55,6);2,502 (75,0);2,498(54 ,9);2,493(27,1);2,329(0,5);2,324(0,4);1,256(3,6);1,238(8,1);1,219(3,5); 0,000(6,7) |
| 3,63 | 3,59 | Beispiel I-21: $^1$H-NMR(400,0 MHz, $d_6$-DMSO): δ= 9,285(4,3);8,272(4,5);8,150(3,2);8,128 (3,5);7,435(0,8);7,39 4(0,8);7,348(2,0);7,337(1,6);7,010(2,9);6,987(2,8);4,904(5,8); 3,926(16,0);3,631(0,9);3,613(3,2);3,594(3,2);3,5 76(1,0);3,320(13,2);2,675(0,4);2,671 (0,6);2,666(0,4);2,524(1,4);2,511(33,0);2,506(69,3);2,502(96,5);2,497(71 ,9);2,493 (34,5);2,333(0,4);2,329(0,6);2,324(0,4);2,074(0,4);1,201(3,4);1,182(7,8);1,164(3,3); 0,008(0,7);0,000( 20,9);-0,008(0,7) |
| 2,46 | 2,44 | Beispiel I-22: $^1$H-NMR(400,0 MHz, $d_6$-DMSO): δ= 9,325(1,1);9,288(6,7);8,901(1,0);8,880 (1,2);8,574(1,2);8,55 4(1,1);8,519(2,4);8,508(2,4);8,348(1,3);8,278(7,5);8,167(1,5); 8,145(1,5);7,837(0,7);7,377(1,3);7,364(1,5);7,34 7(1,2);7,069(0,8);4,940(3,1);4,900 (2,2);4,873(1,1);3,951(5,3);3,932(16,0);3,915(1,4);3,896(1,1);3,878(0,3);3,6 45(1,0); 3,626(2,6);3,605(3,2);3,587(1,9);3,569(0,4);3,317(84,8);2,675(1,5);2,670(2,1);2,666 (1,6);2,524(5,2);2, 519(8,1);2,510(122,9);2,506(257,9);2,501(344,6);2,497(250,0);2,492 (121,9);2,333(1,5);2,328(2,1);2,323(1,5); 2,074(1,1);1,260(1,2);1,249(1,4);1,241(2,7); 1,231(3,2);1,223(1,3);1,212(1,6);1,202(5,5);1,184(12,5);1,165(5,3 );0,008(0,9);0,000 (31,0);-0,009(1,2) |
| 4,04 | 4,04 | Beispiel I-23: $^1$H-NMR(400,0 MHz, $d_6$-DMSO): δ= 9,366(4,1);8,619(3,2);8,597(3,8);8,413 (2,8);8,409(2,8);8,35 4(7,0);8,331(3,1);8,324(3,3);8,315(3,8);7,715(2,9);7,694(3,2); 7,294(2,1);7,289(2,1);7,273(1,9);7,268(2,0);6,95 6(2,5);6,948(2,5);5,755(2,4);4,013 (16,0);3,796(1,0);3,778(3,3);3,759(3,3);3,741(1,0);3,320(51,3);2,676(0,8);2, 671(1,1); 2,667(0,9);2,524(3,6);2,511(64,2);2,507(134,6);2,502(188,5);2,498(144,2);2,493(73,3); 2,333(0,8);2, 329(1,1);2,324(0,8);1,258(3,5);1,239(8,2);1,221(3,5);0,146(0,3);0,008 (2,9);0,000(82,0);-0,009(4,0);-0,150(0,3) |
| 2,64 | 2,66 | Beispiel I-24: $^1$H-NMR(400,0 MHz, $d_6$-DMSO): δ= 9,339(4,2);9,251(6,6);8,718(3,1);8,696 (4,0);8,545(3,7);8,52 2(2,9);8,351(4,3);8,349(4,4);8,315(0,4);8,284(1,3);8,278(1,0); 8,270(1,3);8,261(1,3);7,820(1,3);7,811(1,2);7,80 3(1,0);7,798(1,5);7,397(0,4);7,393 (0,3);7,385(3,0);7,378(1,8);7,375(1,9);7,371(1,8);7,367(1,6);7,361(2,8);7,34 9(0,4); 4,001(16,0);3,838(0,9);3,820(3,3);3,801(3,3);3,783(1,0);3,320(49,6);2,676(0,6);2,671 (0,8);2,667(0,6);2, 525(2,1);2,511(46,0);2,507(97,4);2,502(136,2);2,498(102,0);2,493 (49,1);2,333(0,6);2,329(0,8);2,324(0,6);1,2 69(3,5);1,251(7,9);1,232(3,4);0,008(1,9); 0,000(57,6);-0,008(2,1) |

(fortgesetzt)

| LOGP_ NEUTRAL | LOGP_ HCOOH | NMR |
|---|---|---|
| 4,09 | 4,10 | Beispiel I-25: $^1$H-NMR(400,0 MHz, $d_6$-DMSO): δ= 9,333(4,4);8,599(3,0);8,576(3,7);8,419 (2,5);8,396(2,7);8,38 2(2,9);8,373(2,9);8,346(6,0);8,325(2,8);8,315(0,8);7,750(3,3); 7,744(3,3);7,286(1,7);7,280(1,7);7,263(1,7);7,25 8(1,7);6,922(2,7);6,913(2,7);3,981 (16,0);3,798(1,0);3,780(3,3);3,761(3,4);3,743(1,0);3,320(132,9);2,675(1,3); 2,671(1,8); 2,667(1,4);2,524(5,2);2,511(108,2);2,506(223,6);2,502(309,6);2,497(232,6);2,493 (113,7);2,333(1,3 );2,329(1,8);2,324(1,3);1,254(4,0);1,236(9,1);1,217(3,6);0,146(0,6); 0,008(4,7);0,000(130,6);-0,008(4,8);-0,150(0,6) |
| 3,65 | 3,72 | Beispiel I-26: $^1$H-NMR(400,0 MHz, $d_6$-DMSO): δ= 9,334(4,4);8,580(3,0);8,558(3,7);8,414 (1,5);8,395(1,6);8,34 3(4,7);8,334(3,5);8,312(2,9);8,290(3,0);8,281(3,1);7,684(1,4); 7,677(1,0);7,667(1,7);7,662(1,5);7,275(0,6);7,26 1(1,4);7,257(1,4);7,247(1,6);7,242 (2,8);7,237(1,6);7,229(1,6);7,226(1,6);7,211(0,6);6,931(2,6);6,922(2,6);3,99 0(16,0); 3,790(1,0);3,772(3,3);3,753(3,4);3,735(1,0);3,319(40,3);2,675(0,7);2,671(0,9);2,667 (0,7);2,524(2,4);2, 511(52,8);2,506(110,4);2,502(154,2);2,497(118,0);2,333(0,6);2,329 (0,9);2,324(0,7);1,256(3,5);1,237(7,9);1,2 19(3,5);0,146(0,5);0,008(3,7);0,000(102,7);- 0,008(4,4);-0,150(0,4) |
| 4,39 | 4,41 | Beispiel I-27: $^1$H-NMR(400,0 MHz, $d_6$-DMSO): δ= 9,359(3,5);9,336(7,6);8,772(3,7);8,749 (3,5);8,540(3,4);8,53 4(3,6);8,519(3,3);8,509(3,3);8,340(4,1);8,333(5,3);8,331(5,4); 8,315(0,4);6,870(3,7);6,860(3,7);4,002(16,0);3,8 33(1,0);3,814(3,3);3,796(3,4);3,777 (1,0);3,320(60,7);2,676(0,8);2,671(1,1);2,667(0,8);2,524(3,0);2,511(67,2); 2,507(136,5); 2,502(186,8);2,498(140,9);2,333(0,8);2,329(1,1);2,325(0,8);2,074(1,2);1,260(3,6);1,242 (8,1);1,2 23(3,5);0,146(0,4);0,008(3,5);0,000(92,5);-0,008(3,6);-0,150(0,4) |
| 3,05 | 3,13 | Beispiel I-28: $^1$H-NMR(400,0 MHz, $d_6$-DMSO): δ= 9,358(4,0);8,687(2,8);8,681(4,6);8,671 (3,4);8,663(3,2);8,64 1(3,8);8,532(3,9);8,526(3,6);8,427(3,3);8,405(2,8);8,355(4,3); 7,088(2,5);7,078(2,5);3,998(16,0);3,802(0,9);3,7 84(3,3);3,765(3,3);3,747(1,0);3,318 (57,2);2,675(1,0);2,670(1,4);2,666(1,1);2,524(3,5);2,510(88,3);2,506(179, 0);2,501 (234,7);2,497(168,2);2,493(81,1);2,333(1,0);2,328(1,4);2,324(1,0);2,074(2,1);1,255 (3,6);1,236(8,3);1, 218(3,6);0,146(0,8);0,008(6,2);0,000(176,0);-0,009(6,7);-0,150(0,8) |
| 3,74 | 3,76 | Beispiel I-29: $^1$H-NMR(400,0 MHz, $d_6$-DMSO): δ= 9,513(3,2);9,491(3,4);9,488(2,0);9,334 (5,0);8,753(3,3);8,73 1(3,2);8,520(2,4);8,508(2,6);8,446(3,2);8,436(3,4);8,329(5,2); 8,194(2,4);8,174(2,5);7,410(1,7);7,398(1,8);7,39 1(1,8);7,379(1,7);6,886(3,3);6,883 (2,2);6,876(3,4);4,005(16,0);3,828(1,2);3,810(3,7);3,791(3,7);3,773(1,2);3,3 20(53,6); 2,671(1,3);2,502(212,3);2,498(195,6);2,329(1,2);2,325(1,1);1,262(3,8);1,244(8,2);1,226 (3,9);0,000(3 7,4);-0,004(22,4) |
| 2,67 | 2,69 | Beispiel I-30: $^1$H-NMR(400,0 MHz, $d_6$-DMSO): δ= 9,348(3,1);9,336(3,3);9,216(4,7);9,080 (4,9);8,712(2,3);8,69 0(3,0);8,685(2,7);8,663(3,2);8,511(3,8);8,489(3,0);8,348(6,5); 8,161(2,4);8,138(2,6);7,745(2,2);7,739(2,2);7,71 3(2,2);7,691(2,3);7,339(2,4);7,332 (2,4);7,024(1,4);7,017(1,2);7,002(1,3);6,995(1,2);6,983(1,4);6,977(1,3);6,96 1(1,2); 6,955(1,2);4,038(0,6);4,013(11,9);3,988(13,3);3,832(0,8);3,814(3,3);3,808(16,0);3,796 (3,1);3,784(2,9); 3,766(0,9);3,702(14,0);3,318(186,2);2,679(1,2);2,675(2,2);2,670(3,2); 2,666(2,4);2,556(0,5);2,524(7,0);2,510( 191,2);2,506(398,5);2,501(531,3);2,497(385,7); 2,492(188,5);2,332(2,3);2,328(3, 1);2,323(2,3); 1,988(2,6);1,26 8(2,9);1,260(3,2);1,249 (6,5);1,242(7,2);1,231(3,0);1,223(3,0);1,192(0,7);1,174(1,3);1,157(0,7);0,146(1,6);0,00 8(13,1);0,000(375,2);-0,009(13,7);-0,150(1,6) |
| 3,28 | 3,32 | Beispiel I-31: $^1$H-NMR(400,0 MHz, $d_6$-DMSO): δ= 9,337(4,2);8,980(3,4);8,958(4,0);8,604 (3,7);8,582(3,4);8,32 0(4,4);7,916(2,0);7,897(2,2);7,775(0,7);7,757(1,8);7,740(1,6); 7,738(1,5);7,709(2,4);7,690(1,2);7,613(1,2);7,59 4(1,8);7,576(0,8);5,170(6,3);3,988 (16,0);3,808(1,1);3,789(3,4);3,771(3,4);3,752(1,1);3,319(37,6);2,675(0,5);2, 671(0,7); 2,666(0,5);2,524(2,0);2,510(44,8);2,506(90,1);2,501(118,3);2,497(86,3);2,493(43,1); 2,333(0,5);2,32 8(0,7);2,324(0,6);2,074(0,6);1,243(3,7);1,225(8,3);1,206(3,6);0,008 (0,4);0,000(11,0);-0,008(0,5) |

(fortgesetzt)

| LOGP_ NEUTRAL | LOGP_ HCOOH | NMR |
|---|---|---|
| 3,39 | 3,37 | Beispiel I-32: $^1$H-NMR(400,0 MHz, $d_6$-DMSO): $\delta$= 9,329(4,3);8,778(3,1);8,756(4,0);8,593 (2,1);8,589(3,0);8,58 6(2,2);8,580(4,3);8,558(3,4);8,342(4,5);7,999(3,5);7,996(3,5); 3,996(16,0);3,861(1,0);3,843(3,3);3,825(3,4);3,8 06(1,0);3,318(31,8);2,675(1,0);2,670 (1,4);2,666(1,0);2,523(3,4);2,510(87,0);2,506(180,9);2,501(241,3);2,497( 174,4);2,492 (85,3);2,333(1,0);2,328(1,5);2,324(1,1);1,254(3,7);1,235(8,3);1,217(3,6);0,008(0,6); 0,000(18,1);-0,008(0,8) |
| 3,20 | 3,25 | Beispiel I-33: $^1$H-NMR(400,0 MHz, $d_6$-DMSO): $\delta$= 9,354(4,8);9,350(8,1);9,022(2,8);9,000 (4,3);8,916(4,0);8,89 4(2,8);8,389(3,4);8,368(3,6);8,346(4,6);7,634(3,8);7,613(3,5); 4,041(16,0);3,977(0,7);3,893(1,0);3,875(3,4);3,8 56(3,5);3,838(1,1);3,318(109,4);2,675 (1,8);2,670(2,5);2,666(2,0);2,506(306,8);2,501(413,1);2,497(327,4);2,3 32(1,8);2,328 (2,4);2,324(2,0);1,278(3,7);1,260(8,3);1,241(3,9);0,146(1,0);0,008(9,8);0,000(222,4);-0,150(1,0) |
| 3,79 | 3,83 | Beispiel I-34: $^1$H-NMR(400,0 MHz, $d_6$-DMSO): $\delta$= 9,338(4,3);8,954(3,1);8,931(3,7);8,620 (3,5);8,598(3,2);8,32 2(4,8);7,929(2,9);7,924(3,1);7,825(1,4);7,820(1,3);7,805(2,3); 7,800(2,2);7,748(2,8);7,728(1,7);5,158(6,5);3,97 9(16,0);3,806(1,0);3,788(3,3);3,769 (3,3);3,751(1,1);3,622(0,4);3,319(82,3);3,106(0,3);2,675(1,2);2,670(1,6);2, 666(1,2); 2,524(4,1);2,506(212,2);2,501(271,8);2,497(193,3);2,332(1,2);2,328(1,6);2,324(1,1); 2,074(1,6);1,24 0(3,7);1,221(8,2);1,203(3,7);0,008(0,7);0,000(21,4) |
| 2,53 | 2,55 | Beispiel I-35: $^1$H-NMR(400,0 MHz, $d_6$-DMSO): $\delta$= 9,615(5,2);9,343(3,8);8,787(2,4);8,765 (3,1);8,653(2,8);8,63 2(3,0);8,608(2,9);8,585(2,3);8,350(4,0);7,464(2,9);7,443(2,9); 7,317(0,5);7,296(0,5);4,045(0,5);4,039(0,4);4,02 1(0,4);3,981(13,1);3,851(0,8);3,832 (2,8);3,814(2,8);3,795(0,8);3,323(22,7);2,525(0,7);2,512(15,9);2,507(32,7) ;2,503 (45,3);2,499(34,3);2,075(16,0);1,989(1,5);1,268(3,0);1,249(6,7);1,231(3,0);1,194(0,4); 1,176(0,8);1,158( 0,4);0,007(0,7);0,000(20,1);-0,008(0,8) |
| 2,72 | 2,70 | Beispiel I-36: $^1$H-NMR(400,0 MHz, $d_6$-DMSO): $\delta$= 9,320(4,5);8,720(2,9);8,698(4,0);8,571 (3,9);8,549(3,1);8,33 0(5,0);7,280(2,3);7,256(3,2);7,140(1,9);7,122(2,1);7,116(1,4); 7,099(1,6);6,876(2,9);6,859(2,6);3,972(16,0);3,8 43(1,1);3,825(3,4);3,806(3,5);3,788 (1,1);3,329(32,6);2,675(1,0);2,670(1,3);2,523(3,3);2,506(173,0);2,501(223 ,6);2,497 (160,3);2,328(1,3);2,324(1,0);2,074(7,2);1,249(3,8);1,230(8,3);1,212(3,8);0,146(0,5); 0,008(4,1);0,00 0(102,4);-0,150(0,5) |
| 3,00 | 2,96 | Beispiel I-37: $^1$H-NMR(400,0 MHz, $d_6$-DMSO): $\delta$= 9,339(4,9);8,907(1,3);8,886(5,2);8,872 (5,1);8,850(1,4);8,76 3(3,2);8,740(3,4);8,364(5,5);7,769(3,4);7,747(3,3);4,144(0,4); 4,014(16,0);3,908(1,2);3,891(3,7);3,872(3,7);3,8 53(1,2);3,319(19,1);2,671(1,0);2,502 (174,9);2,328(1,0);2,075(1,0);1,280(4,0);1,262(8,5);1,243(4,0);0,000(59, 8) |
| 3,32 | 3,29 | Beispiel I-38: $^1$H-NMR(400,0 MHz, $d_6$-DMSO): $\delta$= 9,336(3,9);8,942(3,2);8,920(3,8);8,569 (3,5);8,546(3,3);8,31 7(4,4);7,830(2,6);7,808(2,8);7,236(2,1);7,232(2,4);7,145(1,5); 7,140(1,3);7,124(1,4);7,119(1,3);5,101(5,5);3,98 4(15,0);3,871(16,0);3,800(0,9);3,781 (3,0);3,763(3,1);3,745(0,9);3,319(91,4);2,675(1,8);2,670(2,5);2,666(1,8); 2,524(5,5); 2,519(8,8);2,510(155,2);2,506(324,0);2,501(430,0);2,497(306,5);2,492(146,1);2,333 (1,9);2,328(2,5 );2,323(1,9);2,074(1,8);1,239(3,5);1,220(7,7);1,202(3,3);0,146(0,6); 0,008(4,8);0,000(154,4);-0,009(5,6);-0,150(0,7) |
| 3,21 | 3,17 | Beispiel I-39: $^1$H-NMR(400,0 MHz, $d_6$-DMSO): $\delta$= 9,354(4,3);8,855(0,3);8,821(0,6);8,799 (7,6);8,796(6,9);8,77 3(0,5);8,442(2,1);8,421(2,3);8,369(4,5);8,290(2,1);8,269(2,2); 7,716(1,0);7,696(1,8);7,678(1,3);7,615(1,4);7,59 5(1,9);7,577(0,9);4,028(16,0);4,008 (0,4);3,888(1,0);3,869(3,3);3,851(3,4);3,832(1,0);3,318(111,9);2,674(2,8); 2,670(3,8); 2,666(2,9);2,523(9,2);2,505(502,9);2,501(657,9);2,497(471,0);2,332(2,8);2,328(3,9); 2,324(2,8);2,0 74(0,5);1,281(3,7);1,262(8,4);1,244(3,7);0,146(0,5);0,008(3,1);0,000 (111,2);-0,008(4,4);-0,150(0,6) |

(fortgesetzt)

| LOGP_ NEUTRAL | LOGP_ HCOOH | NMR |
|---|---|---|
| 2,96 | 2,92 | Beispiel I-40: $^1$H-NMR(400,0 MHz, $d_6$-DMSO): δ= 9,322(4,4);8,826(3,5);8,805(3,8);8,338 (4,8);8,316(0,6);8,27 4(0,4);8,140(3,8);8,119(3,8);8,080(0,4);8,071(2,8);8,063(3,2); 8,057(3,0);8,049(4,4);8,039(0,7);7,996(0,6);7,98 6(4,1);7,979(2,9);7,973(2,9);7,965 (2,7);4,363(1,1);4,022(16,0);3,953(2,0);3,935(3,5);3,917(3,6);3,899(1,5);3,3 19(52,7); 2,671(1,5);2,666(1,1);2,524(4,0);2,506(202,1);2,502(260,0);2,497(187,7);2,333(1,2); 2,329(1,5);1,29 2(3,8);1,273(8,5);1,255(3,8);1,237(0,6);1,205(0,4);1,188(0,5);1,170 (0,6);0,147(1,5);0,008(15,8);0,000(351,5);-0,008(18,0);-0,149(1,5) |
| 3,26 | 3,25 | Beispiel I-41: $^1$H-NMR(400,0 MHz, $d_6$-DMSO): δ= 9,330(4,2);8,786(2,5);8,763(4,7);8,715 (4,6);8,693(2,7);8,35 4(4,4);8,018(1,8);8,000(1,9);7,511(1,7);7,509(1,8);7,489(2,1); 7,317(0,8);7,314(0,8);7,298(1,9);7,295(1,9);7,27 8(1,3);7,275(1,2);7,253(1,3);7,250 (1,3);7,234(1,8);7,231(1,8);7,214(0,8);3,963(16,0);3,813(1,0);3,795(3,4);3,7 76(3,5); 3,758(1,1);3,721(0,5);3,319(43,8);2,890(0,3);2,675(0,9);2,670(1,3);2,666(0,9);2,510 (85,6);2,506(171, 8);2,501(226,5);2,497(165,7);2,493(83,3);2,333(1,0);2,328(1,3);2,324 (1,0);2,074(0,6);1,245(3,7);1,227(8,3);1, 208(3,7);0,146(1,3);0,008(11,6);0,000(277,7);-0,008(14,3);-0,150(1,2) |
| 2,38 | 2,42 | Beispiel I-42: $^1$H-NMR(400,0 MHz, $d_6$-DMSO): δ= 9,330(1,4);9,323(4,3);8,787(0,3);8,759 (2,9);8,745(1,0);8,73 7(4,3);8,695(0,9);8,673(0,6);8,643(4,1);8,621(3,1);8,332(4,5); 8,316(0,4);8,168(0,5);8,142(0,9);8,117(0,8);8,09 2(0,8);8,081(1,0);8,074(3,6);8,049 (4,0);7,695(0,4);7,670(0,4);7,478(0,9);7,453(1,0);7,448(4,1);7,423(3,8);3,97 9(4,1); 3,964(16,0);3,838(1,2);3,820(3,7);3,802(3,7);3,783(1,3);3,622(0,4);3,604(0,4);3,318 (135,3);2,675(2,7); 2,670(3,6);2,666(2,7);2,523(9,4);2,510(232,4);2,506(471,9);2,501 (623,0);2,497(449,8);2,493(220,9);2,332(2,6 );2,328(3,5);2,323(2,7);1,337(0,5);1,301 (0,7);1,283(1,0);1,247(4,3);1,235(5,1);1,228(9,2);1,210(3,8);1,166(0,4 );0,853(0,4); 0,146(3,7);0,008(32,8);0,000(837,7);-0,008(38,6);-0,061(0,4);-0,150(3,8) |
| 2,31 | 2,26 | Beispiel I-43: $^1$H-NMR(400,0 MHz, $d_6$-DMSO): δ= 9,322(4,1);8,726(2,8);8,704(4,3);8,619 (4,2);8,596(3,0);8,33 3(4,4);7,984(2,0);7,967(2,1);7,389(0,6);7,387(0,6);7,374(0,6); 7,371(0,6);7,365(1,3);7,363(1,4);7,350(1,5);7,34 8(1,5);7,324(2,8);7,300(1,2);6,700 (1,0);6,697(1,1);6,682(1,9);6,667(1,0);6,664(1,0);3,981(16,0);3,843(0,9);3,8 25(3,3); 3,806(3,4);3,788(1,0);3,319(25,5);2,675(0,6);2,670(0,8);2,666(0,6);2,524(1,7);2,510 (47,1);2,506(99,5) ;2,501(134,0);2,497(96,8);2,493(47,1);2,333(0,6);2,328(0,8);2,324 (0,6);1,249(3,6);1,231(8,1);1,212(3,6);0,00 8(1,7);0,000(57,7);-0,008(2,2) |
| 3,06 | 3,10 | Beispiel I-44: $^1$H-NMR(400,0 MHz, $d_6$-DMSO): δ= 9,320(4,6);8,846(2,9);8,823(4,1);8,711 (3,9);8,688(2,9);8,34 3(4,9);8,033(2,2);8,014(2,3);7,322(1,6);7,303(2,6);7,260(1,4); 7,241(2,2);7,222(1,0);7,099(1,2);7,081(2,0);7,06 2(1,0);3,956(16,0);3,782(1,1);3,763 (3,6);3,745(3,6);3,726(1,1);3,452(16,2);3,323(49,1);3,270(0,8);2,671(0,5); 2,507(73,8); 2,502(95,4);2,498(72,0);2,329(0,5);1,243(3,8);1,225(8,3);1,206(3,7);0,000(37,8) |
| 2,62 | 2,59 | Beispiel I-45: $^1$H-NMR(400,0 MHz, $d_6$-DMSO): δ= 11,621(2,6);9,553(1,2);9,321(5,1); 9,212(1,3);9,148(1,2);8,8 37(3,1);8,814(4,0);8,683(3,5);8,661(3,0);8,342(5,9);8,316 (1,4);8,279(1,3);7,991(3,0);7,970(2,8);7,171(1,4);7,1 54(2,9);7,136(2,5);7,114(3,4); 7,097(1,7);7,032(1,7);7,016(2,8);6,998(1,5);3,975(1,2);3,955(16,0);3,770(3,5);3, 751 (3,8);3,733(3,8);3,718(1,6);3,585(2,3);3,399(1,1);3,322(962,1);2,671(8,3);2,502 (1476,0);2,328(8,4);2,073( 3,3);1,237(4,6);1,220(8,9);1,201(4,0);1,161(2,0);1,143(2,0); 0,146(5,7);0,000(1153,4);-0,150(6,0) |
| 2,30 | 2,26 | Beispiel I-46: $^1$H-NMR(400,0 MHz, $d_6$-DMSO): δ= 9,343(4,2);9,016(3,2);8,994(3,7);8,840 (1,6);8,831(1,7);8,64 0(3,6);8,617(3,3);8,328(4,4);8,316(0,4);8,179(1,6);8,161(1,8); 7,733(1,7);7,721(1,7);7,713(1,6);7,701(1,6);5,17 0(6,4);3,989(16,0);3,951(0,3);3,914 (1,3);3,818(1,0);3,800(3,3);3,782(3,3);3,763(1,0);3,318(66,2);2,675(1,7);2, 670(2,4); 2,666(1,7);2,523(5,8);2,510(151,5);2,506(305,5);2,501(398,2);2,497(282,7);2,493 (135,2);2,332(1,7); 2,328(2,3);2,323(1,7);2,074(2,4);1,247(3,9);1,228(8,6);1,210(3,7); 1,203(1,0);1,184(0,4);0,008(2,1);0,000(71,4 );-0,009(2,7);-0,150(0,3) |

(fortgesetzt)

| LOGP_ NEUTRAL | LOGP_ HCOOH | NMR |
|---|---|---|
| | 3,05 | Beispiel I-47: $^1$H-NMR(600,1 MHz, DMF): δ= 9,379(3,8);8,969(3,0);8,954(3,5);8,765(3,4); 8,750(3,0);8,328(4,0 );8,245(2,8);8,230(2,9);8,025(8,7);7,246(3,1);7,242(3,1);7,093 (1,8);7,089(1,7);7,078(1,7);7,074(1,6);4,183(0,7 );4,146(16,0);3,890(1,1);3,877(3,5); 3,865(3,6);3,853(1,1);3,482(1,7);2,952(0,7);2,922(4,6);2,919(8,8);2,916(1 1,8);2,913 (8,4);2,910(4,1);2,784(0,6);2,751(4,8);2,748(9,1);2,745(12,5);2,742(8,8);2,738(4,4); 1,333(3,9);1,32 1(8,2);1,309(3,8);1,280(0,5);0,000(6,6) |
| | 2,97 | Beispiel I-48: $^1$H-NMR(601,6 MHz, DMF): δ= 9,446(3,4);8,909(2,9);8,894(3,9);8,786(3,7); 8,771(3,1);8,337(3,7 );8,336(3,5);8,140(2,7);8,137(2,9);8,024(4,9);7,259(0,9);7,255 (0,8);7,245(2,7);7,242(2,9);7,232(4,1);7,219(1,4 );4,180(16,0);3,871(1,0);3,859(3,5); 3,847(3,7);3,834(1,3);3,461(4,7);2,951(0,4);2,921(2,5);2,918(5,2);2,915(7, 5);2,912 (5,8);2,909(3,3);2,784(0,4);2,751(2,8);2,748(5,6);2,745(8,1);2,741(6,1);2,738(3,6); 1,333(3,6);1,320(8, 2);1,308(4,0);1,281(0,5);1,269(0,4);1,257(0,3);0,000(5,5);-0,006 (0,5) |
| 3,74 | 3,74 | Beispiel I-49: $^1$H-NMR(400,0 MHz, $d_6$-DMSO): δ= 9,359(4,6);9,334(0,7);8,859(0,8);8,842 (0,9);8,830(1,4);8,81 9(0,5);8,808(5,6);8,796(5,5);8,774(1,4);8,469(3,3);8,464(3,4); 8,437(2,8);8,415(3,0);8,371(4,9);8,359(0,9);8,31 3(0,5);8,193(0,4);8,170(0,4);7,712 (1,9);7,707(1,9);7,690(1,9);7,685(1,9);7,634(0,3);4,012(16,0);3,998(2,9);3,8 88(1,1); 3,870(3,6);3,852(3,8);3,833(1,3);3,321(138,4);2,671(1,8);2,506(229,8);2,502(300,0); 2,498(230,5);2,3 33(1,3);2,328(1,7);2,075(0,4);1,277(4,0);1,259(8,7);1,240(4,3);0,146 (0,4);0,000(61,3) |
| 3,77 | 3,72 | Beispiel I-50: $^1$H-NMR(400,0 MHz, $d_6$-DMSO): δ= 9,359(4,0);9,335(0,8);8,860(1,0);8,842 (1,0);8,830(1,2);8,82 0(0,4);8,808(5,1);8,796(5,0);8,774(1,2);8,468(2,9);8,464(3,0); 8,437(2,5);8,414(2,7);8,370(4,4);8,358(0,9);8,30 8(0,6);8,193(0,5);8,170(0,5);7,712 (1,7);7,707(1,8);7,689(1,7);7,685(1,7);7,638(0,4);7,634(0,4);7,615(0,3);7,61 1(0,4); 4,012(14,5);3,998(3,2);3,888(1,0);3,870(3,3);3,852(3,3);3,833(1,1);3,318(25,9);2,671 (1,3);2,666(1,0);2, 506(163,2);2,502(216,6);2,497(163,0);2,332(0,9);2,328(1,2);2,324 (1,0);2,074(16,0);1,277(3,5);1,259(7,8);1,2 40(3,6);0,146(0,7);0,008(7,1);0,000(169,2);- 0,150(0,8) |
| 2,08 | 2,09 | Beispiel I-51: $^1$H-NMR(400,0 MHz, $d_6$-DMSO): δ= 9,337(4,2);8,985(1,5);8,963(5,7);8,950 (5,5);8,928(1,5);8,39 5(2,2);8,378(2,3);8,353(4,4);7,607(2,3);7,591(2,4);7,056(1,8); 7,037(3,4);7,019(1,7);4,012(16,0);3,907(1,0);3,8 89(3,3);3,871(3,4);3,852(1,0);3,323 (148,3);2,671(1,1);2,666(0,8);2,510(74,0);2,506(150,5);2,502(200,7);2,49 7(145,6); 2,493(71,1);2,333(0,8);2,328(1,1);2,323(0,8);1,278(3,6);1,260(8,1);1,241(3,6);0,008 (1,1);0,000(28,5) ;-0,008(1,3) |

Die Messung der logP Werte erfolgt gemäß EEC Directive 79/831 Annex V.A8 durch HPLC (High Performance Liquid Chromatography) an einer Phasenumkehrsäule (C 18). Temperatur: 55°C.

Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril. In der Tabelle logP (HCOOH) genannt.

Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril. In der Tabelle logP (neutral) genannt.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die NMR-Daten ausgewählter Beispiele werden entweder in klassischer Form (δ-Werte, Multiplettaufspaltung, Anzahl der H-Atome) oder als NMR-Peak-Listen aufgeführt.

Das Lösungsmittel, in welchem das NMR-Spektrum aufgenommen wurde ist jeweils angegeben.

NMR-Peak-Listenverfahren

Die $^1$H-NMR-Daten ausgewählter Beispiele werden in Form von $^1$H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:

$$\delta_1 \text{ (Intensität}_1\text{); } \delta_2 \text{ (Intensität}_2\text{);\ldots\ldots..; } \delta_i \text{ (Intensität}_i\text{);\ldots\ldots; } \delta_n \text{ (Intensität}_n\text{)}$$

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von $^1$H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der $^1$H-NMR-Peaks sind ähnlich den klassischen $^1$H-NMR-Ausdrucken und enthalten somit gewöhnlich alle Peaks, die bei einer klassischen NMR-Interpretation aufgeführt werden.

Darüber hinaus können sie wie klassische $^1$H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von $^1$H-NMR-Peaks die gewöhnlichen Lösungsmittelpeaks, zum Beispiel Peaks von DMSO in DMSO-$D_6$ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen $^1$H-NMR-Interpretation.

Weitere Details zu $^1$H-NMR-Peaklisten können der Research Disclosure Database Number 564025 entnommen werden.

## Anwendungsbeispiele

### Ctenocephalides felis - in-vitro Kontakttests mit adulten Katzenflöhen

Für die Beschichtung der Teströhrchen werden zunächst 9 mg Wirkstoff in 1 ml Aceton p.a. gelöst und anschließend mit Aceton p.a. auf die gewünschte Konzentration verdünnt. 250 μl der Lösung werden durch Drehen und Kippen auf einem Rotationsschüttler (2 h Schaukelrotation bei 30 rpm) homogen auf den Innenwänden und dem Boden eines 25ml Glasröhrchens verteilt. Bei 900 ppm Wirkstofflösung und 44,7 $cm^2$ Innenoberfläche wird bei homogener Verteilung eine Flächendosis von 5 $\mu g/cm^2$ erreicht.

Nach Abdampfen des Lösungsmittels werden die Gläschen mit 5-10 adulten Katzenflöhen (*Ctenocephalides felis*) besetzt, mit einem gelochten Kunststoffdeckel verschlossen und liegend bei Raumtemperatur und Umgebungsfeuchte inkubiert. Nach 48 h wird die Wirksamkeit bestimmt. Hierzu werden die Gläschen aufrecht gestellt und die Flöhe auf den Boden des Gläschens geklopft. Flöhe, die unbeweglich auf dem Boden verbleiben oder sich unkoordiniert bewegen, gelten als tot bzw. angeschlagen.

Eine Substanz zeigt gute Wirkung gegen *Ctenocephalides felis*, wenn in diesem Test bei einer Aufwandmenge von 5 $\mu g/cm^2$ mindestens 80% Wirkung erzielt wurde. Dabei bedeutet 100% Wirkung, dass alle Flöhe angeschlagen oder tot waren. 0% Wirkung bedeutet, dass keine Flöhe geschädigt wurden.

Bei diesem Test zeigen z.B. die folgenden Verbindungen der Herstellungsbeispiele eine Wirkung von 100% bei einer Aufwandmenge von 5 $\mu g/cm^2$: I-6, I-18, I-22, I-24, I-31, I-34, I-35

Bei diesem Test zeigen z.B. die folgenden Verbindungen der Herstellungsbeispiele eine Wirkung von 80% bei

einer Aufwandmenge von 5 μg/$cm^2$: I-21

**Boophilus microplus -Injektionstest**

Lösungsmittel: Dimethylsulfoxid

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration.
1μl der Wirkstofflösung wird in das Abdomen von 5 vollgesogenen, adulten, weiblichen Rinderzecken *(Boophilus microplus)* injiziert. Die Tiere werden in Schalen überführt und in einem klimatisierten Raum aufbewahrt.
Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt.
Eine Wirkung von 100 % bedeutet, dass keine der Zecken fertile Eier gelegt hat, 0% bedeutet, dass alle Eier fertil sind.
Bei diesem Test zeigen z.B. die folgenden Verbindungen der Herstellungsbeispiele eine Wirkung von 90% bei einer Aufwandmenge von 20μg/Tier: I-6, I-11
Bei diesem Test zeigen z.B. die folgenden Verbindungen der Herstellungsbeispiele eine Wirkung von 80% bei einer Aufwandmenge von 20μg/Tier: I-5, I-10

**Ctenocephalides felis - Oraltest**

Lösungsmittel: Dimethylsulfoxid

Zwecks Herstellung einer zweckmäßigen Wirkstoffzubereitung vermischt man 10 mg Wirkstoff mit 0,5 ml Dimethylsulfoxid. Durch Verdünnen mit citriertem Rinderblut erhält man die gewünschte Konzentration.
Ca. 20 nüchterne adulte Katzenflöhe (*Ctenocephalides felis*) werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann.
Nach 2 Tagen wird die Abtötung in % bestimmt. Dabei bedeutet 100 %, dass alle Flöhe abgetötet wurden; 0 % bedeutet, dass keiner der Flöhe abgetötet wurde.
Bei diesem Test zeigen z.B. die folgenden Verbindungen der Herstellungsbeispiele eine Wirkung von 100% bei einer Aufwandmenge von 100ppm: I-17, I-18, I-22, I-32
Bei diesem Test zeigen z.B. die folgenden Verbindungen der Herstellungsbeispiele eine Wirkung von 95% bei einer Aufwandmenge von 100ppm: I-3, I-24, I-34, I-35
Bei diesem Test zeigen z.B. die folgenden Verbindungen der Herstellungsbeispiele eine Wirkung von 90% bei einer Aufwandmenge von 100ppm: I-6, I-21
Bei diesem Test zeigen z.B. die folgenden Verbindungen der Herstellungsbeispiele eine Wirkung von 80% bei einer Aufwandmenge von 100ppm: I-28

**Lucilia cuprina - Test**

Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.
Ca. 20 L1-Larven der Australischen Schafgoldfliege (*Lucilia cuprina*) werden in ein Testgefäß überführt, welches gehacktes Pferdefleisch und die Wirkstoffzubereitung der gewünschten Konzentration enthält.
Nach 2 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 100% bei einer Aufwandmenge von 100ppm: I-3, I-5, I-6, I-10, I-15, I-18, I-21, I-22, I-28, I-29, I-31, I-32, I-34

Bei diesem Test zeigen z.B. die folgenden Verbindungen der Herstellungsbeispiele eine Wirkung von 90% bei einer Aufwandmenge von 100ppm: I-4

**Musca domestica-Test**

Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Gefäße, die einen Schwamm enthalten, der mit Zuckerlösung und der Wirkstoffzubereitung der gewünschten Konzentration behandelt wurde, werden mit 10 adulten Stubenfliegen *(Musca domestica)* besetzt.

Nach 2 Tagen wird die Abtötung in % bestimmt. Dabei bedeutet 100 %, dass alle Fliegen abgetötet wurden; 0 % bedeutet, dass keine der Fliegen abgetötet wurde.

Bei diesem Test zeigen z.B. die folgenden Verbindungen der Herstellungsbeispiele eine Wirkung von 100% bei einer Aufwandmenge von 100ppm: I-6, I-10, I-32

Bei diesem Test zeigen z.B. die folgenden Verbindungen der Herstellungsbeispiele eine Wirkung von 80% bei einer Aufwandmenge von 100ppm: I-18

**Meloidogyne incognita- Test**

Lösungsmittel: 125,0 Gewichtsteile Aceton

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, einer Ei-Larven-Suspension des südlichen Wurzelgallenälchens (*Meloidogyne incognita)* und Salatsamen gefüllt. Die Salatsamen keimen und die Pflänzchen entwickeln sich. An den Wurzeln entwickeln sich die Gallen.

Nach 14 Tagen wird die nematizide Wirkung anhand der Gallenbildung in % bestimmt. Dabei bedeutet 100 %, dass keine Gallen gefunden wurden; 0 % bedeutet, dass die Zahl der Gallen an den behandelten Pflanzen der unbehandelten Kontrolle entspricht.

Bei diesem Test zeigen z. B. die folgenden Verbindungen der Herstellungsbeispiele Wirkung von 100% bei einer Aufwandmenge von 20ppm: I-27, I-36

Bei diesem Test zeigen z. B. die folgenden Verbindungen der Herstellungsbeispiele Wirkung von 90% bei einer Aufwandmenge von 20ppm: I-28

**Myzus persicae - Sprühtest**

| | | |
|---|---|---|
| Lösungsmittel: | 78 | Gewichtsteile Aceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 5 Tagen wird die Wirkung in % bestimmt. Dabei bedeutet 100 %, dass alle Blattläuse abgetötet wurden

und 0 %, dass keine Blattläuse abgetötet wurden.

Bei diesem Test zeigen z. B. die folgenden Verbindungen der Herstellungsbeispiele Wirkung von 100% bei einer Aufwandmenge von 500g/ha: I-6

Bei diesem Test zeigen z. B. die folgenden Verbindungen der Herstellungsbeispiele Wirkung von 90% bei einer Aufwandmenge von 500g/ha: I-5, I-12, I-23, I-24, I-33

Bei diesem Test zeigen z. B. die folgenden Verbindungen der Herstellungsbeispiele Wirkung von 70% bei einer Aufwandmenge von 500g/ha: I-14, I-19

Bei diesem Test zeigen z. B. die folgenden Verbindungen der Herstellungsbeispiele Wirkung von 100% bei einer Aufwandmenge von 100g/ha: I-6, I-24

Bei diesem Test zeigen z. B. die folgenden Verbindungen der Herstellungsbeispiele Wirkung von 90% bei einer Aufwandmenge von 100g/ha: I-12, I-19, I-37, I-43

Bei diesem Test zeigen z. B. die folgenden Verbindungen der Herstellungsbeispiele Wirkung von 70% bei einer Aufwandmenge von 100g/ha: I-5

## Phaedon cochleariae - Sprühtest

| | | |
|---|---|---|
| Lösungsmittel: | 78,0 | Gewichtsteile Aceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden und 0 %, dass keine Käferlarven abgetötet wurden.

Bei diesem Test zeigen z. B. die folgenden Verbindungen der Herstellungsbeispiele Wirkung von 100% bei einer Aufwandmenge von 500g/ha: I-3, I-5, I-6, I-7, I-10, I-12, I-15, I-17, I-18, I-19, I-22, I-27, I-28, I-31, I-32, I-33, I-34, I-35, I-40

Bei diesem Test zeigen z. B. die folgenden Verbindungen der Herstellungsbeispiele Wirkung von 83% bei einer Aufwandmenge von 500g/ha: I-11, I-16, I-21, I-24

Bei diesem Test zeigen z. B. die folgenden Verbindungen der Herstellungsbeispiele Wirkung von 100% bei einer Aufwandmenge von 100g/ha: I-3, I-6, I-7, I-10, I-12, I-15, I-17, I-18, I-19, I-22, I-27, I-28, I-31, I-32, I-33, I-34, I-40, I-42, I-49, I-50

Bei diesem Test zeigen z. B. die folgenden Verbindungen der Herstellungsbeispiele Wirkung von 83% bei einer Aufwandmenge von 100g/ha: I-35

## Spodoptera frugiperda - Sprühtest

| | | |
|---|---|---|
| Lösungsmittel: | 78,0 | Gewichtsteile Aceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden

und 0 % , dass keine Raupen abgetötet wurden.

Bei diesem Test zeigen z. B. die folgenden Verbindungen der Herstellungsbeispiele Wirkung von 100% bei einer Aufwandmenge von 500g/ha: I-5, I-6, I-10, I-12, I-14, I-15, I-17, I-18, I-21, I-22, I-24, I-27, I-28, I-29, I-31, I-32, I-33, I-34

Bei diesem Test zeigen z. B. die folgenden Verbindungen der Herstellungsbeispiele Wirkung von 83% bei einer Aufwandmenge von 500g/ha: I-3

Bei diesem Test zeigen z. B. die folgenden Verbindungen der Herstellungsbeispiele Wirkung von 100% bei einer Aufwandmenge von 100g/ha: I-3, I-6, I-5, I-10, I-12, I-14, I-17, I-18, I-21, I-22, I-27, I-29, I-31, I-33, I-34, I-38, I-49, I-50

Bei diesem Test zeigen z. B. die folgenden Verbindungen der Herstellungsbeispiele Wirkung von 83% bei einer Aufwandmenge von 100g/ha: I-24, I-32

**Tetranychus urticae - Sprühtest, OP-resistent**

| | | |
|---|---|---|
| Lösungsmittel: | 78,0 | GewichtsteileAceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Bohnenblattscheiben *(Phaseolus vulgaris),* die von allen Stadien der Gemeinen Spinnmilbe *(Tetranychus urticae)* befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 90% bei einer Aufwandmenge von 500g/ha: I-15, I-31

Bei diesem Test zeigen z. B. die folgenden Verbindungen der Herstellungsbeispiele eine Wirkung von 70% bei einer Aufwandmenge von 500g/ha: I-2

Bei diesem Test zeigen z. B. die folgenden Verbindungen der Herstellungsbeispiele eine Wirkung von 90% bei einer Aufwandmenge von 100g/ha: I-43

Bei diesem Test zeigen z. B. die folgenden Verbindungen der Herstellungsbeispiele eine Wirkung von 70% bei einer Aufwandmenge von 100g/ha: I-39

**Myzus persicae - Sprühtest**

| | |
|---|---|
| Lösungsmittel: | 14 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt. Bei erforderlicher Zugabe von Ammoniumsalzen oder/und Penetrationsförderern werden diese jeweils in einer Konzentration von 1000 ppm der Präparatelösung zugefügt.

Paprikapflanzen (*Capsicum annuum*), die stark von der Grünen Pfirsichblattlaus (*Myzus persicae*) befallen sind, werden durch Sprühen mit der Wirkstoffzubereitung in der gewünschten Konzentration behandelt.

Nach 6 Tagen wird die Abtötung in % bestimmt. Dabei bedeutet 100 %, dass alle Läuse abgetötet wurden; 0 % bedeutet, dass keine Läuse abgetötet wurden.

Bei diesem Test zeigen z. B. die folgenden Verbindungen der Herstellungsbeispiele Wirkung von 100% bei einer Aufwandmenge von 20ppm: I-21, I-34

Bei diesem Test zeigen z. B. die folgenden Verbindungen der Herstellungsbeispiele Wirkung von 99% bei einer Aufwandmenge von 20ppm: I-22

Bei diesem Test zeigen z. B. die folgenden Verbindungen der Herstellungsbeispiele Wirkung von 98% bei einer Aufwandmenge von 20ppm: I-18

Bei diesem Test zeigen z. B. die folgenden Verbindungen der Herstellungsbeispiele Wirkung von 90% bei einer Aufwandmenge von 20ppm: I-17

## Patentansprüche

**1.** Verbindungen der Formel (I)

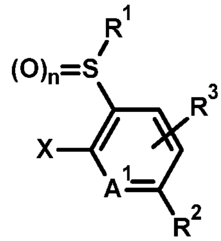

(I)

in welcher

$A^1$ für Stickstoff, $=N^+(O^-)$- oder $=C(R^4)$- steht,

$R^1$ für $(C_1-C_6)$Alkyl, $(C_1-C_6)$Halogenalkyl, $(C_1-C_6)$Cyanoalkyl, $(C_1-C_6)$Hydroxyalkyl, $(C_1-C_6)$Alkoxy-$(C_1-C_6)$alkyl, $(C_1-C_6)$Halogenalkoxy-$(C_1-C_6)$alkyl, $(C_2-C_6)$Alkenyl, $(C_2-C_6)$Alkenyloxy-$(C_1-C_6)$alkyl, $(C_2-C_6)$Halogenalkenyloxy-$(C_1-C_6)$alkyl, $(C_2-C_6)$Halogenalkenyl, $(C_2-C_6)$Cyanoalkenyl, $(C_2-C_6)$Alkinyl, $(C_2-C_6)$Alkinyloxy-$(C_1-C_6)$alkyl, $(C_2-C_6)$Halogenalkinyloxy-$(C_1-C_6)$alkyl, $(C_2-C_6)$Halogenalkinyl, $(C_2-C_6)$Cyanoalkinyl, $(C_3-C_8)$Cycloalkyl, $(C_3-C_8)$Cycloalkyl-$(C_3-C_8)$Cycloalkyl, $(C_1-C_6)$Alkyl-$(C_3-C_8)$Cycloalkyl, Halogen$(C_3-C_8)$cycloalkyl, Amino, $(C_1-C_6)$Alkylamino, Di-$(C_1-C_6)$alkyl-amino, $(C_3-C_8)$Cycloalkylamino, $(C_1-C_6)$Alkylcarbonyl-amino, $(C_1-C_6)$Alkylthio-$(C_1-C_6)$alkyl, $(C_1-C_6)$Halogenalkylthio-$(C_1-C_6)$alkyl, $(C_1-C_6)$Alkylsulfinyl-$(C_1-C_6)$alkyl, $(C_1-C_6)$Halogenalkylsulfinyl-$(C_1-C_6)$alkyl, $(C_1-C_6)$Alkylsulfonyl-$(C_1-C_6)$alkyl, $(C_1-C_6)$Halogenalkylsulfonyl-$(C_1-C_6)$alkyl, $(C_1-C_6)$Alkoxy-$(C_1-C_6)$alkylthio-$(C_1-C_6)$alkyl, $(C_1-C_6)$Alkoxy-$(C_1-C_6)$alkylsulfinyl-$(C_1-C_6)$alkyl, $(C_1-C_6)$Alkoxy-$(C_1-C_6)$alkylsulfonyl-$(C_1-C_6)$alkyl, $(C_1-C_6)$Alkylcarbonyl-$(C_1-C_6)$alkyl, $(C_1-C_6)$Halogenalkylcarbonyl-$(C_1-C_6)$alkyl, $(C_1-C_6)$Alkoxycarbonyl-$(C_1-C_6)$alkyl, $(C_1-C_6)$Halogenalkoxycarbonyl-$(C_1-C_6)$alkyl, $(C_1-C_6)$Alkylsulfonylamino, Aminosulfonyl-$(C_1-C_6)$alkyl, $(C_1-C_6)$Alkylaminosulfonyl-$(C_1-C_6)$alkyl, Di-$(C_1-C_6)$alkyl-aminosulfonyl-$(C_1-C_6)$alkyl,

oder für jeweils gegebenenfalls einfach oder mehrfach, gleich oder verschieden durch Aryl, Hetaryl oder Heterocyclyl substituiertes $(C_1-C_6)$Alkyl, $(C_1-C_6)$Alkoxy, $(C_2-C_6)$Alkenyl, $(C_2-C_6)$Alkinyl, $(C_3-C_8)$Cycloalkyl steht, wobei Aryl, Hetaryl oder Heterocyclyl jeweils gegebenenfalls einfach oder mehrfach, gleich oder verschieden durch Halogen, Cyano, Nitro, Hydroxy, Amino, Carboxy, Carbamoyl, Aminosulfonyl, $(C_1-C_6)$Alkyl, $(C_3-C_6)$Cycloalkyl, $(C_1-C_6)$Alkoxy, $(C_1-C_6)$Halogenalkyl, $(C_1-C_6)$Halogenalkoxy, $(C_1-C_6)$Alkylthio, $(C_1-C_6)$Alkylsulfinyl, $(C_1-C_6)$Alkylsulfonyl, $(C_1-C_6)$Alkylsulfimino, $(C_1-C_6)$Alkylsulfimino-$(C_1-C_6)$alkyl, $(C_1-C_6)$Alkylsulfimino-$(C_1-C_6)$alkylcarbonyl, $(C_1-C_6)$Alkylsulfoximino, $(C_1-C_6)$Alkylsulfoximino-$(C_1-C_6)$alkyl, $(C_1-C_6)$Alkylsulfoximino-$(C_2-C_6)$alkylcarbonyl, $(C_1-C_6)$Alkoxycarbonyl, $(C_1-C_6)$Alkylcarbonyl, $(C_3-C_6)$Trialkylsilyl oder Benzyl substituiert sein können, oder

$R^1$ für jeweils gegebenenfalls einfach oder mehrfach, gleich oder verschieden durch Halogen, Cyano, Nitro, Hydroxy, Amino, Carboxy, Carbamoyl, $(C_1-C_6)$Alkyl, $(C_3-C_8)$Cycloalkyl, $(C_1-C_6)$-Alkoxy, $(C_1-C_6)$Halogenalkyl, $(C_1-C_6)$Halogenalkoxy, $(C_1-C_6)$Alkylthio, $(C_1-C_6)$Alkylsulfinyl, $(C_1-C_6)$Alkylsulfonyl, $(C_1-C_6)$Alkylsulfimino, $(C_1-C_6)$Alkylsulfiniino-$(C_1-C_6)$alkyl, $(C_1-C_6)$Alkylsulfimino-$(C_2-C_6)$alkylcarbonyl, $(C_1-C_6)$Alkylsulfoximino, $(C_1-C_6)$Alkylsulfoximino-$(C_1-C_6)$alkyl, $(C_1-C_6)$Alkylsulfoximino-$(C_2-C_6)$alkylcarbonyl, $(C_1-C_6)$Alkoxycarbonyl, $(C_1-C_6)$Alkylcarbonyl, $(C_3-C_6)$Trialkyl-silyl, (=O) (nur im Fall von Heterocyclyl) oder $(=O)_2$ (nur im Fall von Heterocyclyl) substituiertes Aryl, Hetaryl oder Heterocyclyl steht,

$R^2$ für ein teilweise gesättigtes oder gesättigtes heterozyklisches oder heteroaromatisches 8-, 9-, 10-, 11- oder 12-gliedriges annelliertes bicyclisches Ringsystem steht, wobei gegebenenfalls mindestens eine Carbonylgruppe enthalten sein kann und/oder wobei das Ringsystem gegebenenfalls einfach oder mehrfach, gleich oder verschieden substituiert sein kann, und wobei die Substituenten unabhängig voneinander ausgewählt sein können aus Cyano, Carboxyl, Halogen, Nitro, Acetyl, Hydroxy, Amino, SCN, Tri-$(C_1-C_6)$alkylsilyl, $(C_1-C_6)$Alkyl, $(C_1-C_6)$Halogenalkyl, $(C_1-C_6)$Cyanoalkyl, $(C_1-C_6)$Hydroxyalkyl, Hydroxycarbonyl-$(C_1-C_6)$-alkoxy, $(C_1-C_6)$Alkoxycarbonyl-$(C_1-C_6)$alkyl, $(C_1-C_6)$Alkoxy-$(C_1-C_6)$alkyl, $(C_2-C_6)$Alkenyl, $(C_2-C_6)$Halogenalkenyl, $(C_2-C_6)$Cyanoal-

kenyl, $(C_2-C_6)$Alkinyl, $(C_2-C_6)$Halogenalkinyl, $(C_2-C_6)$Cyanoalkinyl, $(C_3-C_6)$Cycloalkyl, $(C_3-C_6)$Cycloalkyl-$(C_3-C_6)$cycloalkyl, $(C_1-C_4)$Alkyl-$(C_3-C_6)$cycloalkyl $(C_1-C_6)$Alkoxy, $(C_1-C_6)$Halogenalkoxy, $(C_1-C_6)$Cyanoalkoxy, $(C_1-C_6)$Alkoxycarbonyl-$(C_1-C_6)$alkoxy, $(C_1-C_6)$Alkoxy-$(C_1-C_6)$alkoxy, $(C_1-C_6)$Alkoxyimino, -N=C(H)-O$(C_1-C_6)$Alkyl, -C(H)=N-O$(C_1-C_6)$Alkyl, $(C_1-C_6)$Halogenalkyl-$(C_1-C_6)$alkoxyimino, $(C_1-C_6)$Alkylthio, $(C_1-C_6)$Halogenalkylthio, $(C_1-C_6)$Alkoxy-$(C_1-C_6)$alkylthio, $(C_1-C_6)$Alkylthio-$(C_1-C_6)$alkyl, $(C_1-C_6)$Alkylsulfinyl, $(C_1-C_6)$Halogenalkylsulfinyl, $(C_1-C_6)$Alkoxy-$(C_1-C_6)$alkylsulfinyl, $(C_1-C_6)$Alkylsulfinyl-$(C_1-C_6)$alkyl, $(C_1-C_6)$Alkylsulfonyl, $(C_1-C_6)$Halogenalkylsulfonyl, $(C_1-C_6)$Alkoxy-$(C_1-C_6)$alkylsulfonyl, $(C_1-C_6)$Alkylsulfonyl-$(C_1-C_6)$alkyl, $(C_1-C_6)$Alkylsulfonyloxy, $(C_1-C_6)$Alkylcarbonyl, $(C_1-C_6)$Halogenalkylcarbonyl, $(C_1-C_6)$Alkylcarbonyloxy, $(C_1-C_6)$Alkoxycarbonyl, $(C_1-C_6)$Halogenalkoxycarbonyl, Aminocarbonyl, $(C_1-C_6)$Alkylaminocarbonyl, Di-$(C_1-C_6)$alkyl-aminocarbonyl, $(C_2-C_6)$Alkenylaminocarbonyl, Di-$(C_2-C_6)$-alkenylaminocarbonyl, $(C_3-C_8)$Cycloalkylaminocarbonyl, $(C_1-C_6)$Alkylsulfonylamino, $(C_1-C_6)$Alkylamino, Di-$(C_1-C_6)$Alkylamino, Aminosulfonyl, $(C_1-C_6)$Alkylaminosulfonyl, Di-$(C_1-C_6)$alkylaminosulfonyl, $(C_1-C_6)$Alkylsulfoximino, Aminothiocarbonyl, $(C_1-C_6)$Alkylaminothiocarbonyl, Di-$(C_1-C_6)$alkylaminothiocarbonyl, $(C_3-C_8)$Cycloalkylamino, $(C_1-C_6)$Alkylcarbonylamino,

$R^3$ für Wasserstoff, Cyano, Halogen, Nitro, Acetyl, Hydroxy, Amino, SCN, Tri-$(C_1-C_6)$alkylsilyl, $(C_3-C_8)$Cycloalkyl, $(C_3-C_8)$Cycloalkyl-$(C_3-C_8)$Cycloalkyl, $(C_1-C_6)$Alkyl-$(C_3-C_8)$cycloalkyl, Halogen$(C_3-C_8)$cycloalkyl, $(C_1-C_6)$Alkyl, $(C_1-C_6)$Halogenalkyl, $(C_1-C_6)$Cyanoalkyl, $(C_1-C_6)$Hydroxyalkyl, Hydroxycarbonyl-$(C_1-C_6)$-alkoxy, $(C_1-C_6)$Alkoxycarbonyl-$(C_1-C_6)$alkyl, $(C_1-C_6)$Alkoxy-$(C_1-C_6)$alkyl, $(C_2-C_6)$Alkenyl, $(C_2-C_6)$Halogenalkenyl, $(C_2-C_6)$Cyanoalkenyl, $(C_2-C_6)$Alkinyl, $(C_2-C_6)$Halogenalkinyl, $(C_2-C_6)$Cyanoalkinyl, $(C_1-C_6)$Alkoxy, $(C_1-C_6)$Halogenalkoxy, $(C_1-C_6)$Cyanoalkoxy, $(C_1-C_6)$Alkoxycarbonyl-$(C_1-C_6)$alkoxy, $(C_1-C_6)$Alkoxy-$(C_1-C_6)$alkoxy, $(C_1-C_6)$Alkylhydroxyimino, $(C_1-C_6)$Alkoxyimino, $(C_1-C_6)$Alkyl-$(C_1-C_6)$alkoxyimino, $(C_1-C_6)$Halogenalkyl-$(C_1-C_6)$alkoxyimino, $(C_1-C_6)$Alkylthio, $(C_1-C_6)$Halogenalkylthio, $(C_1-C_6)$Alkoxy-$(C_1-C_6)$alkylthio, $(C_1-C_6)$Alkylthio-$(C_1-C_6)$alkyl, $(C_1-C_6)$Alkylsulfinyl, $(C_1-C_6)$Halogenalkylsulfinyl, $(C_1-C_6)$Alkoxy-$(C_1-C_6)$alkylsulfinyl, $(C_1-C_6)$Alkylsulfinyl-$(C_1-C_6)$alkyl, $(C_1-C_6)$Alkylsulfonyl, $(C_1-C_6)$Halogenalkylsulfonyl, $(C_1-C_6)$Alkoxy-$(C_1-C_6)$alkylsulfonyl, $(C_1-C_6)$Alkylsulfonyl-$(C_1-C_6)$alkyl, $(C_1-C_6)$Alkylsulfonyloxy, $(C_1-C_6)$Alkylcarbonyl, $(C_1-C_6)$Alkylthiocarbonyl, $(C_1-C_6)$Halogenalkylcarbonyl, $(C_1-C_6)$Alkylcarbonyloxy, $(C_1-C_6)$Alkoxycarbonyl, $(C_1-C_6)$Halogenalkoxycarbonyl, Aminocarbonyl, $(C_1-C_6)$Alkylaminocarbonyl, $(C_1-C_6)$Alkylaminothiocarbonyl, Di-$(C_1-C_6)$alkyl-aminocarbonyl, Di-$(C_1-C_6)$alkyl-aminothiocarbonyl, $(C_2-C_6)$Alkenylaminocarbonyl, Di-$(C_2-C_6)$-alkenylaminocarbonyl, $(C_3-C_8)$Cycloalkylaminocarbonyl, $(C_1-C_6)$Alkylsulfonylamino, $(C_1-C_6)$Alkylamino, Di-$(C_1-C_6)$Alkylamino, Aminosulfonyl, $(C_1-C_6)$Alkylaminosulfonyl, Di-$(C_1-C_6)$alkyl-aminosulfonyl, $(C_1-C_6)$Alkylsulfoximino, Aminothiocarbonyl, $(C_1-C_6)$Alkylaminothiocarbonyl, Di-$(C_1-C_6)$alkyl-aminothiocarbonyl, $(C_3-C_8)$Cycloalkylamino, NH-CO-$(C_1-C_6)$alkyl (($C_1-C_6$)Alkylcarbonylamino),

für jeweils gegebenenfalls einfach oder mehrfach, gleich oder verschieden substituiertes Aryl oder Hetaryl steht, wobei (im Fall von Hetaryl) gegebenenfalls mindestens eine Carbonylgruppe enthalten sein kann und/oder wobei als Substituenten jeweils in Frage kommen: Cyano, Carboxyl, Halogen, Nitro, Acetyl, Hydroxy, Amino, SCN, Tri-$(C_1-C_6)$alkylsilyl, $(C_3-C_8)$Cycloalkyl, $(C_3-C_8)$Cycloalkyl-$(C_3-C_8)$cycloalkyl, $(C_1-C_6)$Alkyl-$(C_3-C_8)$cycloalkyl, Halogen$(C_3-C_8)$cycloalkyl, $(C_1-C_6)$Alkyl, $(C_1-C_6)$Halogenalkyl, $(C_1-C_6)$Cyanoalkyl, $(C_1-C_6)$Hydroxyalkyl, Hydroxycarbonyl-$(C_1-C_6)$-alkoxy, $(C_1-C_6)$Alkoxycarbonyl-$(C_1-C_6)$alkyl, $(C_1-C_6)$Alkoxy-$(C_1-C_6)$alkyl, $(C_2-C_6)$Alkenyl, $(C_2-C_6)$Halogenalkenyl, $(C_2-C_6)$Cyanoalkenyl, $(C_2-C_6)$Alkinyl, $(C_2-C_6)$Halogenalkinyl, $(C_2-C_6)$Cyanoalkinyl, $(C_1-C_6)$Alkoxy, $(C_1-C_6)$Halogenalkoxy, $(C_1-C_6)$Cyanoalkoxy, $(C_1-C_6)$Alkoxycarbonyl-$(C_1-C_6)$alkoxy, $(C_1-C_6)$Alkoxy-$(C_1-C_6)$alkoxy, $(C_1-C_6)$Alkylhydroxyimino, $(C_1-C_6)$Alkoxyimino, $(C_1-C_6)$Alkyl-$(C_1-C_6)$alkoxyimino, $(C_1-C_6)$Halogenalkyl-$(C_1-C_6)$alkoxyimino, $(C_1-C_6)$Alkylthio, $(C_1-C_6)$Halogenalkylthio, $(C_1-C_6)$Alkoxy-$(C_1-C_6)$alkylthio, $(C_1-C_6)$Alkylthio-$(C_1-C_6)$alkyl, $(C_1-C_6)$Alkylsulfinyl, $(C_1-C_6)$Halogenalkylsulfinyl, $(C_1-C_6)$Alkoxy-$(C_1-C_6)$alkylsulfinyl, $(C_1-C_6)$Alkylsulfinyl-$(C_1-C_6)$alkyl, $(C_1-C_6)$Alkylsulfonyl, $(C_1-C_6)$Halogenalkylsulfonyl, $(C_1-C_6)$Alkoxy-$(C_1-C_6)$alkylsulfonyl, $(C_1-C_6)$Alkylsulfonyl-$(C_1-C_6)$alkyl, $(C_1-C_6)$Alkylsulfonyloxy, $(C_1-C_6)$Alkylcarbonyl, $(C_1-C_6)$Halogenalkylcarbonyl, $(C_1-C_6)$Alkylcarbonyloxy, $(C_1-C_6)$Alkoxycarbonyl, $(C_1-C_6)$Halogenalkoxycarbonyl, Aminocarbonyl, $(C_1-C_6)$Alkylaminocarbonyl, Di-$(C_1-C_6)$alkyl-aminocarbonyl, $(C_2-C_6)$Alkenylaminocarbonyl, Di-$(C_2-C_6)$-alkenylaminocarbonyl, $(C_3-C_8)$Cycloalkylaminocarbonyl, $(C_1-C_6)$Alkylsulfonylamino, $(C_1-C_6)$Alkylamino, Di-$(C_1-C_6)$Alkylamino, Aminosulfonyl, $(C_1-C_6)$Alkylaminosulfonyl, Di-$(C_1-C_6)$alkylaminosulfonyl, $(C_1-C_6)$Alkylsulfoximino, Aminothiocarbonyl, $(C_1-C_6)$Alkylaminothiocarbonyl, Di-$(C_1-C_6)$alkylaminothiocarbonyl, $(C_3-C_8)$Cycloalkylamino, $(C_1-C_6)$Alkylcarbonylamino,

$R^4$ für Wasserstoff, Cyano, Halogen, Nitro, Acetyl, Hydroxy, Amino, SCN, Tri-$(C_1-C_6)$alkylsilyl, $(C_3-C_8)$Cycloalkyl, $(C_3-C_8)$Cycloalkyl-$(C_3-C_8)$Cycloalkyl, $(C_1-C_6)$Alkyl-$(C_3-C_8)$cycloalkyl, Halogen$(C_3-C_8)$cycloalkyl, $(C_1-C_6)$Alkyl, $(C_1-C_6)$Halogenalkyl, $(C_1-C_6)$Cyanoalkyl, $(C_1-C_6)$Hydroxyalkyl, Hydroxycarbonyl-$(C_1-C_6)$-alkoxy, $(C_1-C_6)$Alkoxycarbonyl-$(C_1-C_6)$alkyl, $(C_1-C_6)$Alkoxy-$(C_1-C_6)$alkyl, $(C_2-C_6)$Alkenyl, $(C_2-C_6)$Halogenalkenyl, $(C_2-C_6)$Cyanoalkenyl, $(C_2-C_6)$Alkinyl, $(C_2-C_6)$Halogenalkinyl, $(C_2-C_6)$Cyanoalkinyl, $(C_1-C_6)$Alkoxy, $(C_1-C_6)$Halogenalkoxy, $(C_1-C_6)$Cyanoalkoxy, $(C_1-C_6)$Alkoxycarbonyl-$(C_1-C_6)$alkoxy, $(C_1-C_6)$Alkoxy-$(C_1-C_6)$alkoxy, $(C_1-C_6)$Alkylhydroxyimino, $(C_1-C_6)$Alkoxyimino, $(C_1-C_6)$Alkyl-$(C_1-C_6)$alkoxyimino, $(C_1-C_6)$Halogenalkyl-$(C_1-C_6)$alkoxyimino,

$(C_1-C_6)$Alkylthio, $(C_1-C_6)$Halogenalkylthio, $(C_1-C_6)$Alkoxy-$(C_1-C_6)$alkylthio, $(C_1-C_6)$Alkylthio-$(C_1-C_6)$alkyl, $(C_1-C_6)$Alkylsulfinyl, $(C_1-C_6)$Halogenalkylsulfinyl, $(C_1-C_6)$Alkoxy-$(C_1-C_6)$alkylsulfinyl, $(C_1-C_6)$Alkylsulfinyl-$(C_1-C_6)$alkyl, $(C_1-C_6)$Alkylsulfonyl, $(C_1-C_6)$Halogenalkylsulfonyl, $(C_1-C_6)$Alkoxy-$(C_1-C_6)$alkylsulfonyl, $(C_1-C_6)$Alkylsulfonyl-$(C_1-C_6)$alkyl, $(C_1-C_6)$Alkylsulfonyloxy, $(C_1-C_6)$Alkylcarbonyl, $(C_1-C_6)$Alkylthiocarbonyl, $(C_1-C_6)$Halogenalkylcarbonyl, $(C_1-C_6)$Alkylcarbonyloxy, $(C_1-C_6)$Alkoxycarbonyl, $(C_1-C_6)$Halogenalkoxycarbonyl, Aminocarbonyl, $(C_1-C_6)$Alkylaminocarbonyl, $(C_1-C_6)$Alkylaminothiocarbonyl, Di-$(C_1-C_6)$alkyl-aminocarbonyl, Di-$(C_1-C_6)$alkyl-aminothiocarbonyl, $(C_2-C_6)$Alkenylaminocarbonyl, Di-$(C_2-C_6)$-alkenylaminocarbonyl, $(C_3-C_8)$Cycloalkylaminocarbonyl, $(C_1-C_6)$Alkylsulfonylamino, $(C_1-C_6)$Alkylamino, Di-$(C_1-C_6)$Alkylamino, Aminosulfonyl, $(C_1-C_6)$Alkylaminosulfonyl, Di-$(C_1-C_6)$alkyl-aminosulfonyl, $(C_1-C_6)$Alkylsulfoximino, Aminothiocarbonyl, $(C_1-C_6)$Alkylaminothiocarbonyl, Di-$(C_1-C_6)$alkyl-aminothiocarbonyl, $(C_3-C_8)$Cycloalkylamino oder -NH-CO-$(C_1-C_6)$alkyl ($(C_1-C_6)$Alkylcarbonylamino) steht,

X für ein teilweise gesättigtes oder gesättigtes heterozyklisches oder heteroaromatisches 8-, 9-, 10-, 11- oder 12-gliedriges annelliertes bicyclisches oder tricyclisches Ringsystem steht, wobei gegebenenfalls mindestens eine Carbonylgruppe enthalten sein kann und/oder wobei das Ringsystem gegebenenfalls einfach oder mehrfach, gleich oder verschieden substituiert ist, und wobei die Substituenten unabhängig voneinander ausgewählt sein können aus Cyano, Halogen, Nitro, Acetyl, Hydroxy, Amino, SCN, Tri-$(C_1-C_6)$alkylsilyl, $(C_3-C_8)$Cycloalkyl, $(C_3-C_8)$Cycloalkyl-$(C_3-C_8)$Cycloalkyl, $(C_1-C_6)$Alkyl-$(C_3-C_8)$cycloalkyl, Halogen$(C_3-C_8)$cycloalkyl, $(C_1-C_6)$Alkyl, $(C_1-C_6)$Halogenalkyl, $(C_1-C_6)$Cyanoalkyl, $(C_1-C_6)$Hydroxyalkyl, Hydroxycarbonyl-$(C_1-C_6)$-alkoxy, $(C_1-C_6)$Alkoxycarbonyl-$(C_1-C_6)$alkyl, $(C_1-C_6)$Alkoxy-$(C_1-C_6)$alkyl, $(C_2-C_6)$Alkenyl, $(C_2-C_6)$Halogenalkenyl, $(C_2-C_6)$Cyanoalkenyl, $(C_2-C_6)$Alkinyl, $(C_2-C_6)$Alkinyloxy-$(C_1-C_4)$alkyl, $(C_2-C_6)$Halogenalkinyl, $(C_2-C_6)$Cyanoalkinyl, $(C_1-C_6)$Alkoxy, $(C_1-C_6)$Halogenalkoxy, $(C_1-C_6)$Halogenalkoxy-$(C_1-C_6)$alkyl, $(C_2-C_6)$Alkenyloxy-$(C_1-C_6)$alkyl, $(C_2-C_6)$Halogenalkenyloxy-$(C_1-C_6)$alkyl, $(C_1-C_6)$Cyanoalkoxy, $(C_1-C_6)$Alkoxycarbonyl-$(C_1-C_6)$alkoxy, $(C_1-C_6)$Alkoxy-$(C_1-C_6)$alkoxy, $(C_1-C_6)$Alkylhydroxyimino, $(C_1-C_6)$Alkoxyimino, $(C_1-C_6)$Alkyl-$(C_1-C_6)$alkoxyimino, $(C_1-C_6)$Halogenalkyl-$(C_1-C_6)$alkoxyimino, $(C_1-C_6)$Alkylthio, $(C_1-C_6)$Halogenalkylthio, $(C_1-C_6)$Alkoxy-$(C_1-C_6)$alkylthio, $(C_1-C_6)$Alkylthio-$(C_1-C_6)$alkyl, $(C_1-C_6)$Alkylsulfinyl, $(C_1-C_6)$Halogenalkylsulfinyl, $(C_1-C_6)$Alkoxy-$(C_1-C_6)$alkylsulfinyl, $(C_1-C_6)$Alkylsulfinyl-$(C_1-C_6)$alkyl, $(C_1-C_6)$Alkylsulfonyl, $(C_1-C_6)$Halogenalkylsulfonyl, $(C_1-C_6)$Alkoxy-$(C_1-C_6)$alkylsulfonyl, $(C_1-C_6)$Alkylsulfonyl-$(C_1-C_6)$alkyl, $(C_1-C_6)$Alkylsulfonyloxy, $(C_1-C_6)$Alkylcarbonyl, $(C_1-C_6)$Alkylcarbonyl-$(C_1-C_6)$alkyl, $(C_1-C_6)$Alkylthiocarbonyl, $(C_1-C_6)$Halogenalkylcarbonyl, $(C_1-C_6)$Alkylcarbonyloxy, $(C_1-C_6)$Alkoxycarbonyl, $(C_1-C_6)$Halogenalkoxycarbonyl, Aminocarbonyl, $(C_1-C_6)$Alkylaminocarbonyl, $(C_1-C_6)$Alkylaminothiocarbonyl, Di-$(C_1-C_6)$alkyl-aminocarbonyl, Di-$(C_1-C_6)$alkyl-aminothiocarbonyl, $(C_2-C_6)$Alkenylaminocarbonyl, Di-$(C_2-C_6)$-alkenylaminocarbonyl, $(C_3-C_8)$Cycloalkylaminocarbonyl, $(C_1-C_6)$Alkylsulfonylamino, $(C_1-C_6)$Alkylamino, Di-$(C_1-C_6)$Alkylamino, Aminosulfonyl, $(C_1-C_6)$Alkylaminosulfonyl, Di-$(C_1-C_6)$alkyl-aminosulfonyl, $(C_1-C_6)$Alkylsulfoximino, Aminothiocarbonyl, $(C_1-C_6)$Alkylaminothiocarbonyl, Di-$(C_1-C_6)$alkyl-aminothiocarbonyl, $(C_3-C_8)$Cycloalkylamino, NHCO-$(C_1-C_6)$alkyl ($(C_1-C_6)$Alkylcarbonylamino),

oder wobei die Substituenten unabhängig voneinander ausgewählt sein können aus Phenyl oder einem 5- oder 6-gliedrigen heteroaromatischen Ring, wobei Phenyl oder der Ring gegebenenfalls einfach oder mehrfach, gleich oder verschieden durch $C_1$-$C_6$-Alkyl, $C_2$-$C_6$-Alkenyl, $C_2$-$C_6$-Alkinyl, $C_3$-$C_6$-Cycloalkyl, $C_1$-$C_6$-Haloalkyl, $C_2$-$C_6$-Haloalkenyl, $C_2$-$C_6$-Haloalkinyl, $C_3$-$C_6$-Halocycloalkyl, Halogen, CN, $NO_2$, $C_1$-$C_4$-Alkoxy, $C_1$-$C_4$-Haloalkoxy substituiert sein können,

n für 0, 1 oder 2 steht, ausgenommen

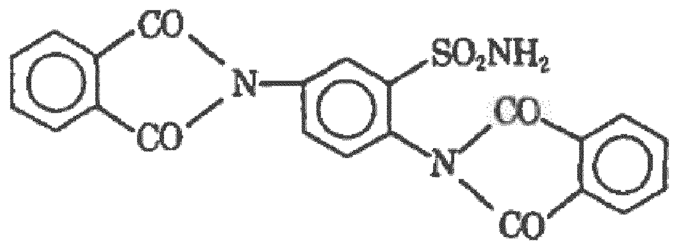

.

**2.** Verbindungen der Formel (I) gemäß Anspruch 1, in welcher

$A^1$ für Stickstoff, $=N^+(O^-)$- oder $=C(R^4)$- steht,

$R^1$ für $(C_1-C_4)$Alkyl, $(C_1-C_4)$Hydroxyalkyl, $(C_1-C_4)$Halogenalkyl, $(C_1-C_4)$Cyanoalkyl, $(C_1-C_4)$Alkoxy-$(C_1-C_4)$alkyl, $(C_1-C_4)$Halogenalkoxy-$(C_1-C_4)$alkyl, $(C_2-C_4)$Alkenyl, $(C_2-C_4)$Alkenyloxy-$(C_1-C_4)$alkyl, $(C_2-C_4)$Halogenalkenyloxy-$(C_1-C_4)$alkyl, $(C_2-C_4)$Halogenalkenyl, $(C_2-C_4)$Cyanoalkenyl, $(C_2-C_4)$Alkinyl, $(C_2-C_4)$Alkinyloxy-$(C_1-C_4)$alkyl,

$(C_2-C_4)$Halogenalkinyloxy-$(C_1-C_4)$alkyl, $(C_2-C_4)$Halogenalkinyl, $(C_2-C_4)$Cyanoalkinyl, $(C_3-C_6)$Cycloalkyl, $(C_3-C_6)$Cycloalkyl$(C_3-C_6)$cycloalkyl, $(C_1-C_4)$Alkyl-$(C_3-C_6)$cycloalkyl, Halogen$(C_3-C_6)$cycloalkyl, $(C_1-C_4)$Alkylamino, Di-$(C_1-C_4)$alkyl-amino, $(C_3-C_6)$Cycloalkylamino, $(C_1-C_4)$Alkylcarbonyl-amino, $(C_1-C_4)$Alkylthio-$(C_1-C_4)$alkyl, $(C_1-C_4)$Halogenalkylthio-$(C_1-C_4)$alkyl, $(C_1-C_4)$Alkylsulfinyl-$(C_1-C_4)$alkyl, $(C_1-C_4)$Halogenalkylsulfinyl-$(C_1-C_4)$alkyl, $(C_1-C_4)$Alkylsulfonyl-$(C_1-C_4)$alkyl, $(C_1-C_4)$Alkylcarbonyl-$(C_1-C_4)$alkyl, $(C_1-C_4)$Halogenalkylcarbonyl-$(C_1-C_4)$alkyl, $(C_1-C_4)$Alkylsulfonylamino,

oder für jeweils gegebenenfalls einfach oder zweifach, gleich oder verschieden durch Aryl, Hetaryl oder Heterocyclyl substituiertes $(C_1-C_4)$Alkyl, $(C_1-C_4)$Alkoxy, $(C_2-C_4)$Alkenyl, $(C_2-C_4)$Alkinyl, $(C_3-C_6)$Cycloalkyl steht, wobei Aryl, Hetaryl oder Heterocyclyl jeweils gegebenenfalls einfach oder zweifach gleich oder verschieden durch Halogen, Cyano, Carbamoyl, Aminosulfonyl, $(C_1-C_4)$Alkyl, $(C_3-C_4)$Cycloalkyl, $(C_1-C_4)$Alkoxy, $(C_1-C_4)$Halogenalkyl, $(C_1-C_4)$Halogenalkoxy, $(C_1-C_4)$Alkylthio, $(C_1-C_4)$Alkylsulfinyl, $(C_1-C_4)$Alkylsulfonyl, $(C_1-C_4)$Alkylsulfimino substituiert sein können, oder

$R^1$ für jeweils gegebenenfalls einfach oder zweifach, gleich oder verschieden durch Halogen, Cyano, Carbamoyl, $(C_1-C_4)$Alkyl, $(C_3-C_6)$Cycloalkyl, $(C_1-C_4)$-Alkoxy, $(C_1-C_4)$Halogenalkyl, $(C_1-C_4)$Halogenalkoxy, $(C_1-C_4)$Alkylthio, $(C_1-C_4)$Alkylsulfinyl, $(C_1-C_4)$Alkylsulfonyl, $(C_1-C_4)$Alkylsulfimino, $(C_1-C_4)$Alkylsulfoximino, $(C_1-C_4)$Alkylcarbonyl, $(C_3-C_4)$Trialkylsilyl, (=O) (nur im Fall von Heterocyclyl) oder $(=O)_2$ (nur im Fall von Heterocyclyl) substituiertes Aryl, Hetaryl oder Heterocyclyl steht,

$R^2$ für ein teilweise gesättigtes oder gesättigtes heterozyklisches oder heteroaromatisches 8-, 9- oder 10-gliedriges annelliertes bicyclisches Ringsystem steht, wobei gegebenenfalls mindestens eine Carbonylgruppe enthalten sein kann, und wobei das Ringsystem gegebenenfalls einfach oder mehrfach, gleich oder verschieden substituiert sein kann wobei die Substituenten unabhängig voneinander ausgewählt sein können aus Cyano, Halogen, Nitro, Acetyl, Amino, $(C_1-C_4)$Alkyl, $(C_1-C_4)$Halogenalkyl, $(C_1-C_4)$Cyanoalkyl, $(C_1-C_4)$Hydroxyalkyl, $(C_1-C_4)$Alkoxy-$(C_1-C_4)$alkyl, $(C_2-C_4)$Alkenyl, $(C_2-C_4)$Halogenalkenyl, $(C_2-C_4)$Cyanoalkenyl, $(C_2-C_4)$Alkinyl, $(C_2-C_4)$Halogenalkinyl, $(C_2-C_4)$Cyanoalkinyl, $(C_3-C_6)$Cycloalkyl, $(C_3-C_6)$Cycloalkyl-$(C_3-C_6)$cycloalkyl, $(C_1-C_4)$Alkyl-$(C_3-C_6)$cycloalkyl, $(C_1-C_4)$Alkoxy, $(C_1-C_4)$Halogenalkoxy, $(C_1-C_4)$Cyanoalkoxy, $(C_1-C_4)$Alkoxy-$(C_1-C_4)$alkoxy, $(C_1-C_4)$Alkoxyimino, -N=C(H)-O$(C_1-C_4)$Alkyl, -C(H)=N-O$(C_1-C_4)$Alkyl, $(C_1-C_4)$Halogenalkyl-$(C_1-C_4)$alkoxyimino, $(C_1-C_4)$Alkylthio, $(C_1-C_4)$Halogenalkylthio, $(C_1-C_4)$Alkylthio-$(C_1-C_4)$alkyl, $(C_1-C_4)$Alkylsulfinyl, $(C_1-C_4)$Halogenalkylsulfinyl, $(C_1-C_4)$Alkylsulfinyl-$(C_1-C_4)$alkyl, $(C_1-C_4)$Alkylsulfonyl, $(C_1-C_4)$Halogenalkylsulfonyl, $(C_1-C_4)$Alkylsulfonyl-$(C_1-C_4)$alkyl, $(C_1-C_4)$Alkylsulfonyloxy, $(C_1-C_4)$Alkylcarbonyl, $(C_1-C_4)$Halogenalkylcarbonyl, Aminocarbonyl, $(C_1-C_4)$Alkylaminocarbonyl, Di-$(C_1-C_4)$alkyl-aminocarbonyl, $(C_1-C_4)$Alkylsulfonylamino, $(C_1-C_4)$Alkylamino, Di-$(C_1-C_4)$Alkylamino, Aminosulfonyl, $(C_1-C_4)$Alkylaminosulfonyl, Di-$(C_1-C_4)$alkylaminosulfonyl, $(C_1-C_4)$Alkylcarbonylamino

$R^3$ für Wasserstoff, Cyano, Halogen, Nitro, Acetyl, Hydroxy, Amino, SCN, Tri-$(C_1-C_4)$alkylsilyl, $(C_3-C_6)$Cycloalkyl, $(C_3-C_6)$Cycloalkyl-$(C_3-C_6)$cycloalkyl, $(C_1-C_4)$Alkyl-$(C_3-C_6)$cycloalkyl, Halogen$(C_3-C_6)$cycloalkyl, $(C_1-C_4)$Alkyl, $(C_1-C_4)$Halogenalkyl, $(C_1-C_4)$Cyanoalkyl, $(C_1-C_4)$Hydroxyalkyl, $(C_1-C_4)$Alkoxy-$(C_1-C_4)$alkyl, $(C_2-C_4)$Alkenyl, $(C_2-C_4)$Halogenalkenyl, $(C_2-C_4)$Cyanoalkenyl, $(C_2-C_4)$Alkinyl, $(C_2-C_4)$Halogenalkinyl, $(C_2-C_4)$Cyanoalkinyl, $(C_1-C_4)$Alkoxy, $(C_1-C_4)$Halogenalkoxy, $(C_1-C_4)$Cyanoalkoxy, $(C_1-C_4)$Alkoxy-$(C_1-C_4)$alkoxy, $(C_1-C_4)$Alkylhydroxyimino, $(C_1-C_4)$Alkoxyimino, $(C_1-C_4)$Alkyl-$(C_1-C_4)$alkoxyimino, $(C_1-C_4)$Halogenalkyl-$(C_1-C_4)$alkoxyimino, $(C_1-C_4)$Alkylthio, $(C_1-C_4)$Halogenalkylthio, $(C_1-C_4)$Alkylthio-$(C_1-C_4)$alkyl, $(C_1-C_4)$Alkylsulfinyl, $(C_1-C_4)$Halogenalkylsulfinyl, $(C_1-C_4)$Alkylsulfinyl-$(C_1-C_4)$alkyl, $(C_1-C_4)$Alkylsulfonyl, $(C_1-C_4)$Halogenalkylsulfonyl, $(C_1-C_4)$Alkylsulfonyl-$(C_1-C_4)$alkyl, $(C_1-C_4)$Alkylsulfonyloxy, $(C_1-C_4)$Alkylcarbonyl, $(C_1-C_4)$Halogenalkylcarbonyl, Aminocarbonyl, Aminothiocarbonyl, $(C_1-C_4)$Alkylaminocarbonyl, Di-$(C_1-C_4)$alkyl-aminocarbonyl, $(C_1-C_4)$Alkylsulfonylamino, $(C_1-C_4)$Alkylamino, Di-$(C_1-C_4)$Alkylamino, Aminosulfonyl, $(C_1-C_4)$Alkylaminosulfonyl, Di-$(C_1-C_4)$alkyl-aminosulfonyl, Aminothiocarbonyl, NHCO-$(C_1-C_4)$alkyl (($C_1-C_4$)Alkylcarbonylamino), weiterhin für jeweils gegebenenfalls einfach oder zweifach, gleich oder verschieden substituiertes Phenyl oder Hetaryl steht, wobei (im Fall von Hetaryl) gegebenenfalls mindestens eine Carbonylgruppe enthalten sein kann und/oder wobei als Substituenten jeweils in Frage kommen: Cyano, Halogen, Nitro, Acetyl, Amino, $(C_3-C_6)$Cycloalkyl, $(C_3-C_6)$Cycloalkyl-$(C_3-C_6)$cycloalkyl, $(C_1-C_4)$Alkyl-$(C_3-C_6)$cycloalkyl, Halogen$(C_3-C_6)$cycloalkyl, $(C_1-C_4)$Alkyl, $(C_1-C_4)$Halogenalkyl, $(C_1-C_4)$Cyanoalkyl, $(C_1-C_4)$Hydroxyalkyl, $(C_1-C_4)$Alkoxy-$(C_1-C_4)$alkyl, $(C_2-C_4)$Alkenyl, $(C_2-C_4)$Halogenalkenyl, $(C_2-C_4)$Cyanoalkenyl, $(C_2-C_4)$Alkinyl, $(C_2-C_4)$Halogenalkinyl, $(C_2-C_4)$Cyanoalkinyl, $(C_1-C_4)$Alkoxy, $(C_1-C_4)$Halogenalkoxy, $(C_1-C_4)$Cyanoalkoxy, $(C_1-C_4)$Alkoxy-$(C_1-C_4)$alkoxy, $(C_1-C_4)$Alkylhydroxyimino, $(C_1-C_4)$Alkoxyimino, $(C_1-C_4)$Alkyl-$(C_1-C_4)$alkoxyimino, $(C_1-C_4)$Halogenalkyl-$(C_1-C_4)$alkoxyimino, $(C_1-C_4)$Alkylthio, $(C_1-C_4)$Halogenalkylthio, $(C_1-C_4)$Alkylthio-$(C_1-C_4)$alkyl, $(C_1-C_4)$Alkylsulfinyl, $(C_1-C_4)$Halogenalkylsulfinyl, $(C_1-C_4)$Alkylsulfinyl-$(C_1-C_4)$alkyl, $(C_1-C_4)$Alkylsulfonyl, $(C_1-C_4)$Halogenalkylsulfonyl, $(C_1-C_4)$Alkylsulfonyl-$(C_1-C_4)$alkyl, $(C_1-C_4)$Alkylsulfonyloxy, $(C_1-C_4)$Alkylcarbonyl, $(C_1-C_4)$Halogenalkylcarbonyl, Aminocarbonyl, $(C_1-C_4)$Alkylaminocarbonyl, Di-$(C_1-C_4)$alkyl-aminocarbonyl, $(C_1-C_4)$Alkylsulfonylamino, $(C_1-C_4)$Alkylamino, Di-$(C_1-C_4)$Alkylamino, Aminosulfonyl, $(C_1-C_4)$Alkylaminosulfonyl, Di-$(C_1-C_4)$alkylaminosulfonyl, NHCO-$(C_1-C_4)$alkyl (($C_1-C_4$)Alkylcarbonylamino),

$R^4$ für Wasserstoff, Cyano, Halogen, Nitro, Acetyl, Hydroxy, Amino, SCN, Tri-($C_1$-$C_4$)alkylsilyl, ($C_3$-$C_6$)Cycloalkyl, ($C_3$-$C_6$)Cycloalkyl-($C_3$-$C_6$)cycloalkyl, ($C_1$-$C_4$)Alkyl-($C_3$-$C_6$)cycloalkyl, Halogen($C_3$-$C_6$)cycloalkyl, ($C_1$-$C_4$)Alkyl, ($C_1$-$C_4$)Halogenalkyl, ($C_1$-$C_4$)Cyanoalkyl, ($C_1$-$C_4$)Hydroxyalkyl, ($C_1$-$C_4$)Alkoxy-($C_1$-$C_4$)alkyl, ($C_2$-$C_4$)Alkenyl, ($C_2$-$C_4$)Halogenalkenyl, ($C_2$-$C_4$)Cyanoalkenyl, ($C_2$-$C_4$)Alkinyl, ($C_2$-$C_4$)Halogenalkinyl, ($C_2$-$C_4$)Cyanoalkinyl, ($C_1$-$C_4$)Alkoxy, ($C_1$-$C_4$)Halogenalkoxy, ($C_1$-$C_4$)Cyanoalkoxy, ($C_1$-$C_4$)Alkoxy-($C_1$-$C_4$)alkoxy, ($C_1$-$C_4$)Alkylhydroxyimino, ($C_1$-$C_4$)Alkoxyimino, ($C_1$-$C_4$)Alkyl-($C_1$-$C_4$)alkoxyimino, ($C_1$-$C_4$)Halogenalkyl-($C_1$-$C_4$)alkoxyimino, ($C_1$-$C_4$)Alkylthio, ($C_1$-$C_4$)Halogenalkylthio, ($C_1$-$C_4$)Alkylthio-($C_1$-$C_4$)alkyl, ($C_1$-$C_4$)Alkylsulfinyl, ($C_1$-$C_4$)Halogenalkylsulfinyl, ($C_1$-$C_4$)Alkylsulfinyl-($C_1$-$C_4$)alkyl, ($C_1$-$C_4$)Alkylsulfonyl, ($C_1$-$C_4$)Halogenalkylsulfonyl, ($C_1$-$C_4$)Alkylsulfonyl-($C_1$-$C_4$)alkyl, ($C_1$-$C_4$)Alkylsulfonyloxy, ($C_1$-$C_4$)Alkylcarbonyl, ($C_1$-$C_4$)Halogenalkylcarbonyl, Aminocarbonyl, Aminothiocarbonyl, ($C_1$-$C_4$)Alkylaminocarbonyl, Di-($C_1$-$C_4$)alkyl-aminocarbonyl, ($C_1$-$C_4$)Alkylsulfonylamino, ($C_1$-$C_4$)Alkylamino, Di-($C_1$-$C_4$)Alkylamino, Aminosulfonyl, ($C_1$-$C_4$)Alkylaminosulfonyl, Di-($C_1$-$C_4$)alkyl-aminosulfonyl, Aminothiocarbonyl oder NHCO-($C_1$-$C_4$)alkyl (($C_1$-$C_4$)Alkylcarbonylamino) steht,
X für ein heteroaromatisches 8-, 9-, 10-, 11- oder 12-gliedriges annelliertes bicyclisches oder tricyclisches Ringsystem steht, wobei das Ringsystem gegebenenfalls einfach oder mehrfach, gleich oder verschieden substituiert ist, und wobei die Substituenten unabhängig voneinander ausgewählt sein können aus Cyano, Halogen, Nitro, Acetyl, Hydroxy, Amino, SCN, Tri-($C_1$-$C_6$)alkylsilyl, ($C_3$-$C_8$)Cycloalkyl, ($C_3$-$C_8$)Cycloalkyl-($C_3$-$C_8$)Cycloalkyl, ($C_1$-$C_6$)Alkyl-($C_3$-$C_8$)cycloalkyl, Halogen($C_3$-$C_8$)cycloalkyl, ($C_1$-$C_6$)Alkyl, ($C_1$-$C_6$)Halogenalkyl, ($C_1$-$C_6$)Cyanoalkyl, ($C_1$-$C_6$)Hydroxyalkyl, ($C_1$-$C_6$)Alkoxy-($C_1$-$C_6$)alkyl, ($C_2$-$C_6$)Alkenyl, ($C_2$-$C_6$)Halogenalkenyl, ($C_2$-$C_6$)Cyanoalkenyl, ($C_2$-$C_6$)Alkinyl, ($C_2$-$C_6$)Alkinyloxy-($C_1$-$C_4$)alkyl, ($C_2$-$C_6$)Halogenalkinyl, ($C_1$-$C_6$)Alkoxy, ($C_1$-$C_6$)Halogenalkoxy, ($C_1$-$C_6$)Halogenalkoxy-($C_1$-$C_6$)alkyl, ($C_2$-$C_6$)Alkenyloxy-($C_1$-$C_6$)alkyl, ($C_2$-$C_6$)Halogenalkenyloxy-($C_1$-$C_6$)alkyl, ($C_1$-$C_6$)Cyanoalkoxy, ($C_1$-$C_6$)Alkoxy-($C_1$-$C_6$)alkoxy, ($C_1$-$C_6$)Alkylhydroxyimino, ($C_1$-$C_6$)Alkoxyimino, ($C_1$-$C_6$)Alkyl-($C_1$-$C_6$)alkoxyimino, ($C_1$-$C_6$)Alkylthio, ($C_1$-$C_6$)Halogenalkylthio, ($C_1$-$C_6$)Alkoxy-($C_1$-$C_6$)alkylthio, ($C_1$-$C_6$)Alkylthio-($C_1$-$C_6$)alkyl, ($C_1$-$C_6$)Alkylsulfinyl, ($C_1$-$C_6$)Halogenalkylsulfinyl, ($C_1$-$C_6$)Alkoxy-($C_1$-$C_6$)alkylsulfinyl, ($C_1$-$C_6$)Alkylsulfinyl-($C_1$-$C_6$)alkyl, ($C_1$-$C_6$)Alkylsulfonyl, ($C_1$-$C_6$)Halogenalkylsulfonyl, ($C_1$-$C_6$)Alkoxy-($C_1$-$C_6$)alkylsulfonyl, ($C_1$-$C_6$)Alkylsulfonyl-($C_1$-$C_6$)alkyl, ($C_1$-$C_6$)Alkylsulfonyloxy, ($C_1$-$C_6$)Alkylcarbonyl, ($C_1$-$C_6$)Alkylcarbonyl-($C_1$-$C_6$)alkyl, ($C_1$-$C_6$)Alkylthiocarbonyl, ($C_1$-$C_6$)Halogenalkylcarbonyl, ($C_1$-$C_6$)Alkylcarbonyloxy, ($C_1$-$C_6$)Alkoxycarbonyl, ($C_1$-$C_6$)Halogenalkoxycarbonyl, Aminocarbonyl, ($C_1$-$C_6$)Alkylaminocarbonyl, ($C_1$-$C_6$)Alkylaminothiocarbonyl, Di-($C_1$-$C_6$)alkyl-aminocarbonyl, Di-($C_1$-$C_6$)alkyl-aminothiocarbonyl, ($C_3$-$C_8$)Cycloalkylaminocarbonyl, ($C_1$-$C_6$)Alkylsulfonylamino, ($C_1$-$C_6$)Alkylamino, Di-($C_1$-$C_6$)Alkylamino, Aminosulfonyl, ($C_1$-$C_6$)Alkylaminosulfonyl, Di-($C_1$-$C_6$)alkyl-aminosulfonyl, ($C_1$-$C_6$)Alkylsulfoximino, Aminothiocarbonyl, ($C_1$-$C_6$)Alkylaminothiocarbonyl, Di-($C_1$-$C_6$)alkyl-aminothiocarbonyl, ($C_3$-$C_8$)Cycloalkylamino, NHCO-($C_1$-$C_6$)alkyl (($C_1$-$C_6$)Alkylcarbonylamino),
oder wobei die Substituenten unabhängig voneinander ausgewählt sein können aus Phenyl oder einem 5- oder 6-gliedrigen heteroaromatischen Ring, wobei Phenyl oder der Ring gegebenenfalls einfach oder mehrfach, gleich oder verschieden durch $C_1$-$C_6$-Alkyl, $C_2$-$C_6$-Alkenyl, $C_2$-$C_6$-Alkinyl, $C_3$-$C_6$-Cycloalkyl, $C_1$-$C_6$-Haloalkyl, $C_2$-$C_6$-Haloalkenyl, $C_2$-$C_6$-Haloalkinyl, $C_3$-$C_6$-Halocycloalkyl, Halogen, CN, $C_1$-$C_4$-Alkoxy, $C_1$-$C_4$-Haloalkoxy substituiert sein können,
n für 0, 1 oder 2 steht.

**3.** Verbindungen der Formel (I) gemäß Anspruch 1, in welcher

$A^1$ für Stickstoff oder =C($R^4$)- steht,
$R^1$ für ($C_1$-$C_4$)Alkyl, ($C_1$-$C_4$)Hydroxyalkyl, ($C_1$-$C_4$)Halogenalkyl, ($C_2$-$C_4$)Alkenyl, ($C_2$-$C_4$)Halogenalkenyl, ($C_2$-$C_4$)Alkinyl, ($C_2$-$C_4$)Halogenalkinyl, ($C_3$-$C_6$)Cycloalkyl, ($C_1$-$C_4$)Alkylthio-($C_1$-$C_4$)alkyl, ($C_1$-$C_4$)Alkylsulfinyl-($C_1$-$C_4$)alkyl oder ($C_1$-$C_4$)Alkylsulfonyl-($C_1$-$C_4$)alkyl steht,
$R^2$ für ein teilweise gesättigtes oder heteroaromatisches annelliertes bicyclisches Ringsystem aus der Reihe Q1 bis Q152 steht, wobei das Ringsystem gegebenenfalls einfach oder mehrfach, gleich oder verschieden substituiert sein kann, wobei die Substituenten unabhängig voneinander ausgewählt sein können aus: Nitro, Hydroxy, Amino, Cyano, Halogen, ($C_1$-$C_4$)Alkyl, ($C_1$-$C_4$)Cycloalkyl, ($C_1$-$C_4$)Halogenalkyl, ($C_1$-$C_4$)Alkoxy, ($C_1$-$C_4$)Halogenalkoxy oder ($C_1$-$C_4$)Halogenalkylthio

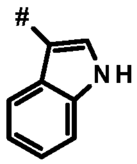
Q1
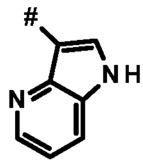
Q2
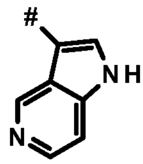
Q3
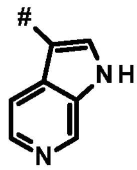
Q4
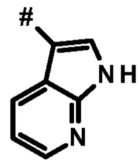
Q5

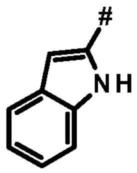
Q6
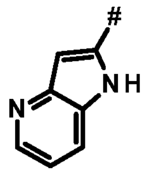
Q7
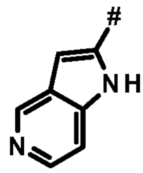
Q8
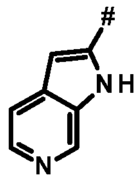
Q9
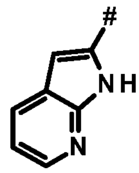
Q10

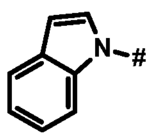
Q11
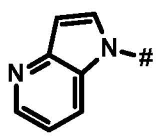
Q12
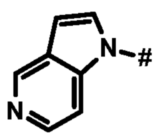
Q13
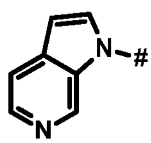
Q14
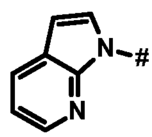
Q15

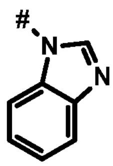
Q16
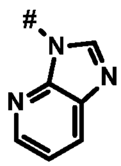
Q17
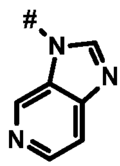
Q18
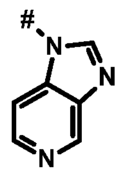
Q19
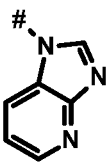
Q20

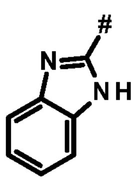
Q21
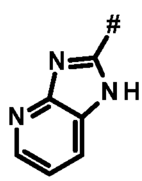
Q22
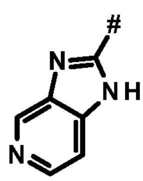
Q23
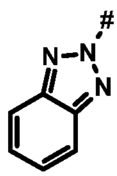
Q24
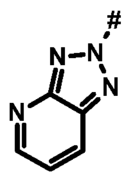
Q25
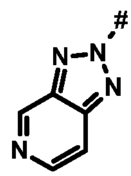
Q26

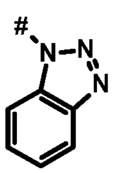
Q27
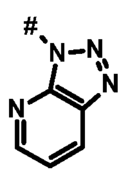
Q28
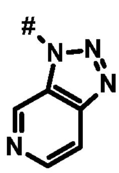
Q29
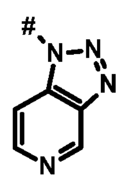
Q30
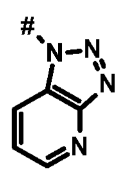
Q31

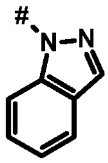

Q32

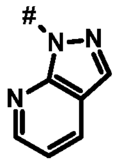

Q33

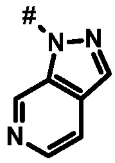

Q34

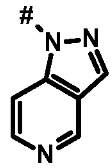

Q35

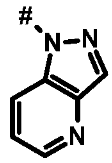

Q36

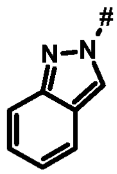

Q37

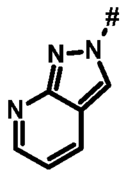

Q38

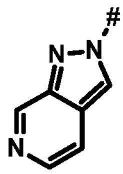

Q39

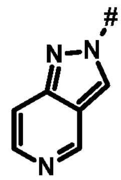

Q40

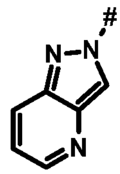

Q41

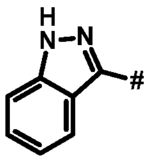

Q42

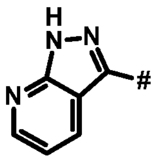

Q43

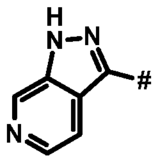

Q44

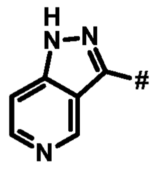

Q45

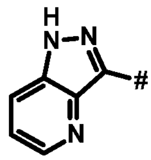

Q46

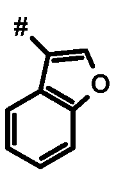

Q47

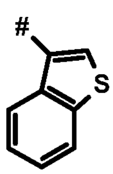

Q48

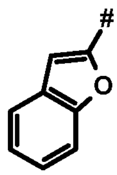

Q49

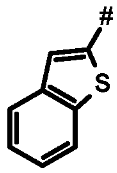

Q50

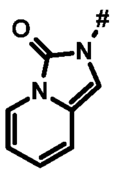

Q51

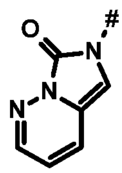

Q52

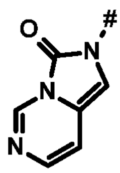

Q53

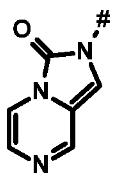

Q54

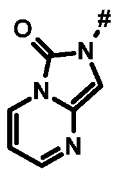

Q55

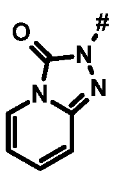

Q56

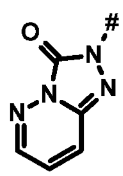

Q57

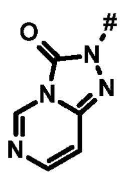

Q58

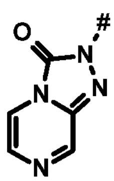

Q59

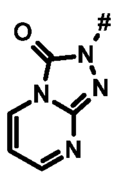

Q60

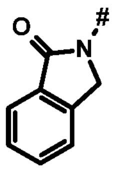
Q61

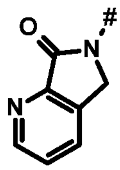
Q62

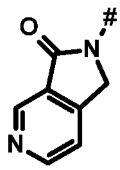
Q63

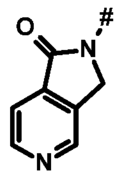
Q64

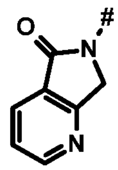
Q65

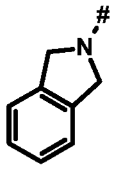
Q66

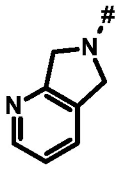
Q67

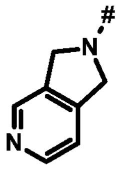
Q68

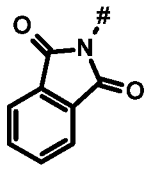
Q69

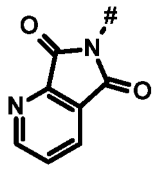
Q70

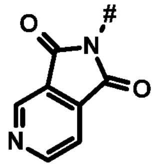
Q71

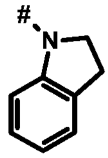
Q72

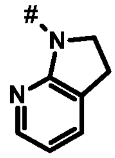
Q73

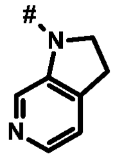
Q74

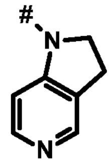
Q75

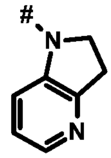
Q76

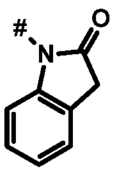
Q77

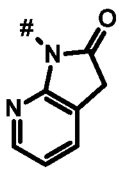
Q78

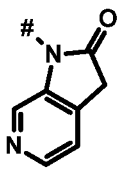
Q79

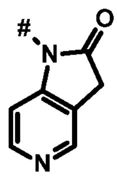
Q80

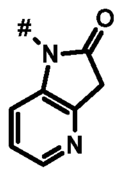
Q81

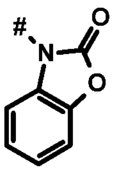
Q82

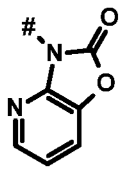
Q83

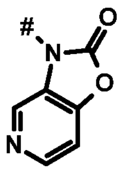
Q84

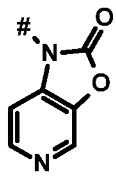
Q85

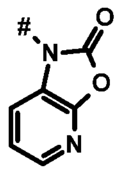
Q86

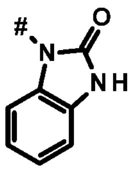
Q87

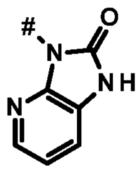
Q88

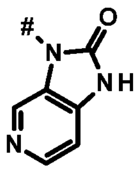
Q89

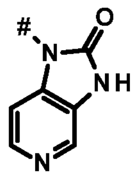
Q90

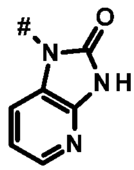
Q91

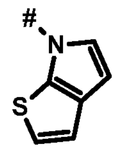

Q92

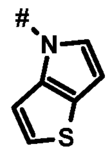

Q93

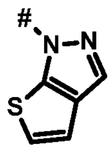

Q94

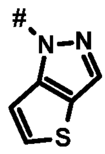

Q95

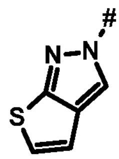

Q96

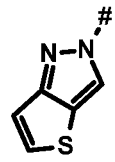

Q97

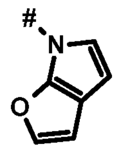

Q98

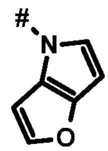

Q99

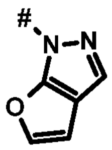

Q100

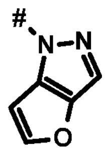

Q101

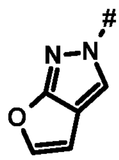

Q102

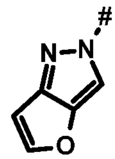

Q103

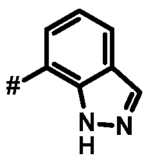

Q104

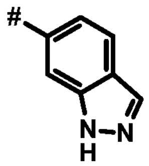

Q105

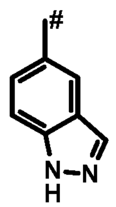

Q106

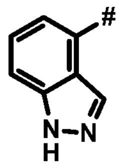

Q107

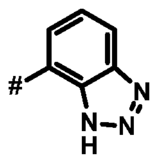

Q108

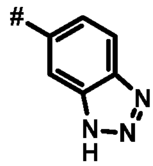

Q109

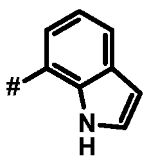

Q110

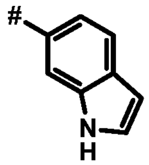

Q111

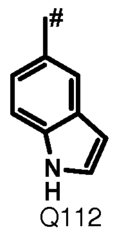

Q112

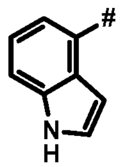

Q113

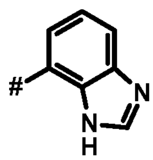

Q114

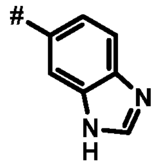

Q115

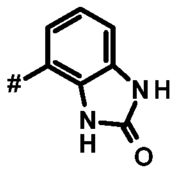

Q116

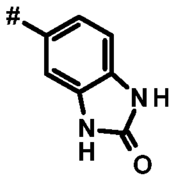

Q117

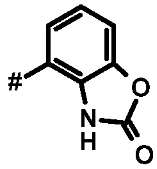

Q118

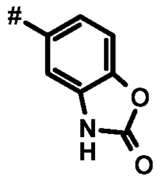

Q119

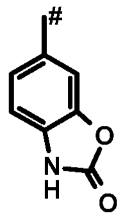

Q120

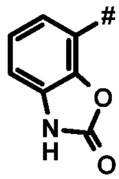

Q121

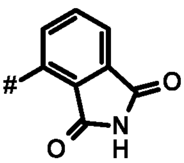

Q122

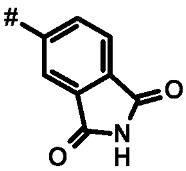

Q123

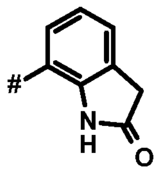

Q124

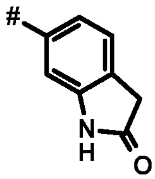

Q125

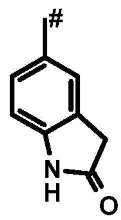

Q126

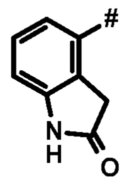

Q127

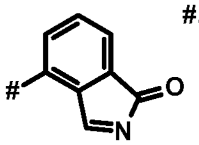
Q128

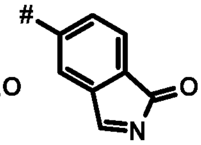
Q129

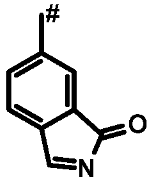
Q130

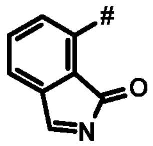
Q131

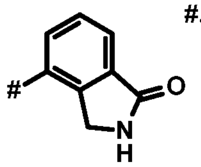
Q132

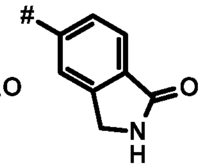
Q133

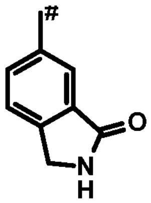
Q134

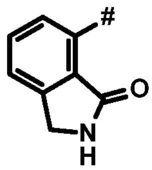
Q135

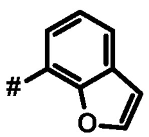
Q136

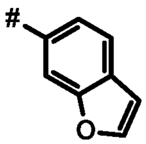
Q137

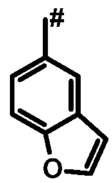
Q138

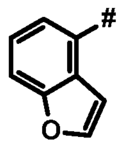
Q139

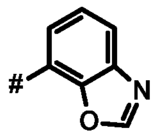
Q140

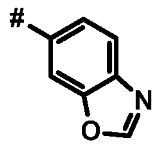
Q141

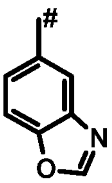
Q142

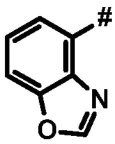
Q143

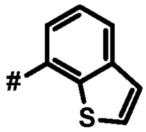
Q144

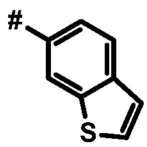
Q145

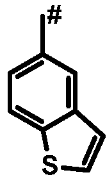
Q146

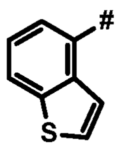
Q147

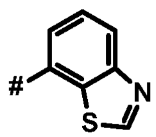
Q148

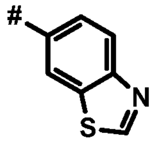
Q149

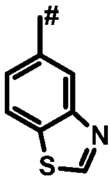
Q150

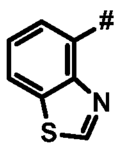
Q151

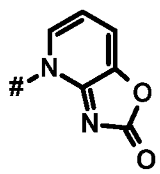
Q152

wobei die Bindung zum Restmolekül mit einem Sternchen # gekennzeichnet ist,

$R^3$ für Wasserstoff, Cyano, Halogen, Nitro, Hydroxy, Amino, SCN, Tri-($C_1$-$C_4$)alkylsilyl, ($C_3$-$C_6$)Cycloalkyl, ($C_3$-$C_6$)Cycloalkyl-($C_3$-$C_6$)cycloalkyl, ($C_1$-$C_4$)Alkyl-($C_3$-$C_6$)cycloalkyl, Halogen($C_3$-$C_6$)cycloalkyl, ($C_1$-$C_4$)Alkyl, ($C_1$-$C_4$)Halogenalkyl, ($C_1$-$C_4$)Cyanoalkyl, ($C_1$-$C_4$)Alkoxy-($C_1$-$C_4$)alkyl, ($C_2$-$C_4$)Alkenyl, ($C_2$-$C_4$)Halogenalkenyl, ($C_2$-$C_4$)Cyanoalkenyl, ($C_2$-$C_4$)Alkinyl, ($C_2$-$C_4$)Halogenalkinyl, ($C_2$-$C_4$)Cyanoalkinyl, ($C_1$-$C_4$)Alkoxy, ($C_1$-$C_4$)Halogenalkoxy, ($C_1$-$C_4$)Cyanoalkoxy, ($C_1$-$C_4$)Alkylhydroxyimino, ($C_1$-$C_4$)Alkoxyimino, ($C_1$-$C_4$)Alkyl-($C_1$-$C_4$)alkoxyimino, ($C_1$-$C_4$)Alkylthio, ($C_1$-$C_4$)Halogenalkylthio, ($C_1$-$C_4$)Alkylsulfinyl, ($C_1$-$C_4$)Halogenalkylsulfinyl, ($C_1$-$C_4$)Alkylsulfonyl, ($C_1$-$C_4$)Halogenalkylsulfonyl, ($C_1$-$C_4$)Alkylsulfonyloxy, ($C_1$-$C_4$)Alkylcarbonyl, ($C_1$-$C_4$)Halogenalkylcarbonyl, Aminocarbonyl, ($C_1$-$C_4$)Alkylaminocarbonyl, Di-($C_1$-$C_4$)alkyl-aminocarbonyl, ($C_1$-$C_4$)Alkylsulfonylamino, ($C_1$-$C_4$)Alkylamino, Di-($C_1$-$C_4$)Alkylamino, Aminosulfonyl, ($C_1$-$C_4$)Alkylaminosulfonyl, Di-($C_1$-$C_4$)alkyl-aminosulfonyl oder NHCO-($C_1$-$C_4$)alkyl (($C_1$-$C_4$)Alkylcarbonylamino) steht,
$R^4$ für Wasserstoff, Halogen, Cyano oder ($C_1$-$C_4$)Alkyl steht,
X für ein heteroaromatisches 9-gliedriges oder 12-gliedriges annelliertes bicyclisches oder tricyclisches Ringsystem aus der Reihe H1 bis H20 steht,

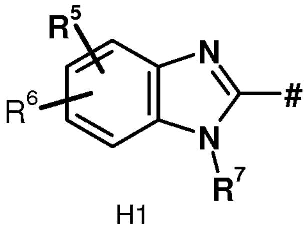

H1

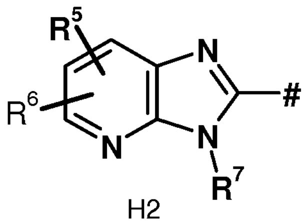

H2

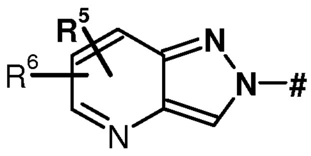

H3

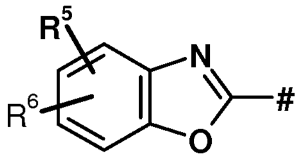

H4

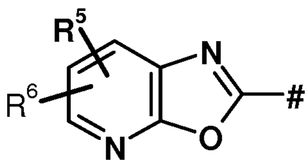

H5

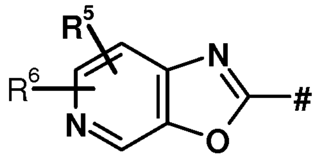

H6

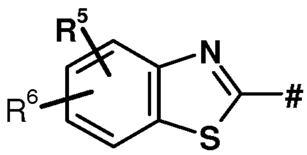

H7

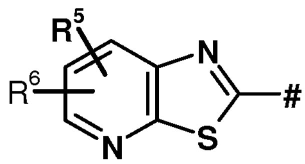

H8

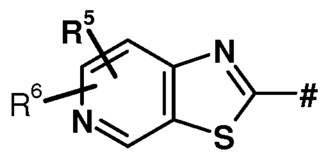

H9

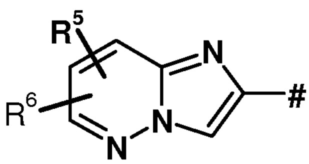

H10

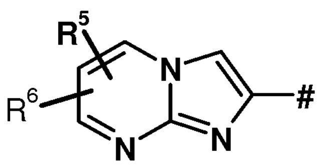

H11

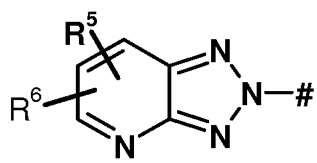

H12

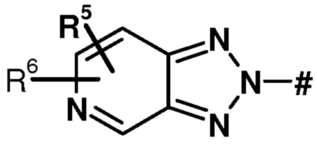

H13

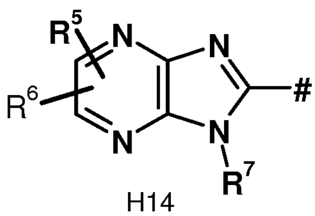

H14

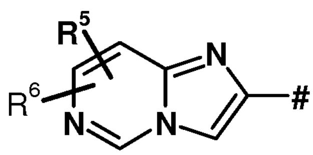

H15

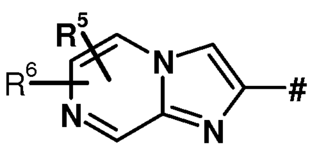

H16

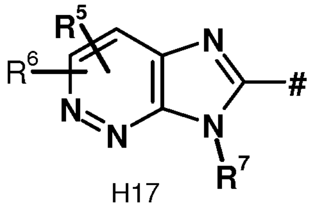

H17

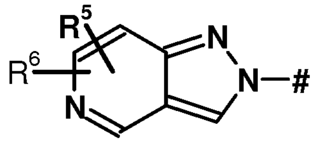

H18

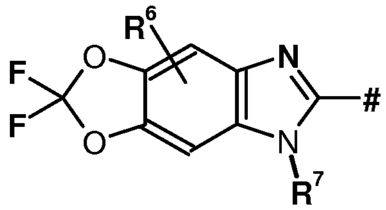

H19

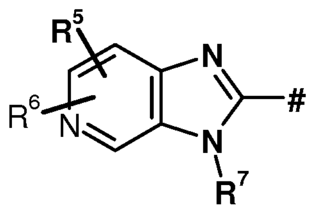

H20

$R^5$, $R^6$ unabhängig voneinander für Wasserstoff, Cyano, Halogen, $(C_1-C_4)$Alkyl, $(C_1-C_4)$Halogenalkyl, $(C_2-C_4)$Alkenyl, $(C_2-C_4)$Halogenalkenyl, $(C_2-C_4)$Alkinyl, $(C_2-C_4)$Halogenalkinyl, $(C_3-C_6)$Cycloalkyl, $(C_3-C_6)$Cycloalkyl-$(C_3-C_6)$cycloalkyl, $(C_1-C_4)$Alkyl-$(C_3-C_6)$cycloalkyl, $(C_1-C_4)$Alkoxy, $(C_1-C_4)$Halogenalkoxy, $(C_1-C_4)$Alkoxyimino, $(C_1-C_4)$Alkylthio, $(C_1-C_4)$Halogenalkylthio, $(C_1-C_4)$Alkylsulfinyl, $(C_1-C_4)$Halogenalkylsulfinyl, $(C_1-C_4)$Alkylsulfonyl, $(C_1-C_4)$Halogenalkylsulfonyl, $(C_1-C_4)$Alkylsulfonyloxy, $(C_1-C_4)$Alkylcarbonyl, $(C_1-C_4)$Halogenalkylcarbonyl, Aminocarbonyl, $(C_1-C_4)$Alkylaminocarbonyl, Di-$(C_1-C_4)$alkyl-aminocarbonyl, $(C_1-C_4)$Alkylsulfonylamino, $(C_1-C_4)$Alkylamino, Di-$(C_1-C_4)$Alkylamino, Aminosulfonyl, $(C_1-C_4)$Alkylaminosulfonyl oder Di-$(C_1-C_4)$alkylaminosulfonyl stehen,

$R^7$ für Wasserstoff, $(C_1-C_4)$Alkyl, $(C_1-C_4)$Halogenalkyl, $(C_1-C_4)$Cyanoalkyl, $(C_1-C_4)$Hydroxyalkyl, $(C_1-C_4)$Alkoxy-$(C_1-C_4)$alkyl, $(C_1-C_4)$Halogenalkoxy-$(C_1-C_4)$alkyl, $(C_2-C_4)$Alkenyl, $(C_2-C_4)$Alkenyloxy-$(C_1-C_4)$alkyl, $(C_2-C_4)$Halogenalkenyloxy-$(C_1-C_4)$alkyl, $(C_2-C_4)$Halogenalkenyl, $(C_2-C_4)$Cyanoalkenyl, $(C_2-C_4)$Alkinyl, $(C_2-C_4)$Alkinyloxy-$(C_1-C_4)$alkyl, $(C_2-C_4)$Halogenalkinyl, $(C_3-C_6)$Cycloalkyl, $(C_3-C_6)$Cycloalkyl-$(C_3-C_6)$cycloalkyl, $(C_1-C_4)$Alkyl-$(C_3-C_6)$cycloalkyl, Halogen$(C_3-C_6)$cycloalkyl, $(C_1-C_4)$Alkylthio-$(C_1-C_4)$alkyl, $(C_1-C_4)$Alkylsulfinyl-$(C_1-C_4)$alkyl, $(C_1-C_4)$Alkylsulfonyl-$(C_1-C_4)$alkyl oder $(C_1-C_4)$Alkylcarbonyl-$(C_1-C_4)$alkyl steht,

n für 0, 1 oder 2 steht.

**4.** Verbindungen der Formel (I) gemäß Anspruch 1, in welcher

$A^1$ für Stickstoff steht,

$R^1$ für Methyl, Ethyl, n-Propyl, i-Propyl, cyclo-Propyl, n-Butyl, i-Butyl, tert.-Butyl, cyclo-Butyl, Fluormethyl, Difluormethyl, Trifluormethyl, Fluorethyl, Difluorethyl, Trifluorethyl, Tetrafluorethyl oder Pentafluorethyl steht,

$R^2$ für ein gegebenenfalls einfach oder zweifach, gleich oder verschieden substituiertes, annelliertes bicyclisches Ringsystem aus der Reihe Q1, Q2, Q3, Q4, Q5, Q6, Q7, Q8, Q9, Q10, Q11, Q12, Q13, Q14, Q15, Q16, Q17, Q18, Q19, Q20, Q21, Q22, Q23, Q27, Q28, Q29, Q30, Q31, Q32, Q33, Q34, Q35, Q36, Q37, Q38, Q39, Q40, Q41, Q42, Q43, Q44, Q45, Q46, Q51, Q52, Q53, Q54, Q55, Q56, Q57, Q58, Q59, Q60, Q61, Q62, Q63, Q64, Q65, Q66, Q67, Q68, Q69, Q70, Q71, Q77, Q78, Q79, Q80, Q81, Q82, Q83, Q84, Q85, Q86, Q87, Q88, Q89, Q90, Q91, Q92, Q93, Q94, Q95, Q96, Q97, Q104, Q105, Q106, Q107, Q108, Q109, Q114, Q115, Q116, Q117, Q118, Q119, Q120, Q121, Q124, Q125, Q126, Q127, Q128, Q129, Q130, Q131, Q132, Q133, Q134, Q135, Q136, Q137, Q138, Q139, Q140, Q141, Q142, Q143, Q144, Q145, Q146, Q147, Q148, Q149, Q150, Q151, Q152 steht und wobei als Substituenten jeweils in Frage kommen: Cyano, Fluor, Chlor, Brom, Methyl, Ethyl, n-Propyl, i-Propyl, Cyclopropyl, Trifluormethyl, Pentafluoroethyl, Methoxy, Ethoxy, Trifluormethoxy, Trifluorethoxy, Pentafluorethoxy oder Trifluormethylthio,

$R^3$ für Wasserstoff, Cyano, Halogen, $(C_1-C_4)$Alkyl, $(C_1-C_4)$Halogenalkyl, $(C_1-C_4)$Halogenalkoxy, $(C_1-C_4)$Alkylthio, $(C_1-C_4)$Alkylsulfinyl, $(C_1-C_4)$Alkylsulfonyl, $(C_1-C_4)$Halogenalkylthio, $(C_1-C_4)$Halogenalkylsulfinyl, $(C_1-C_4)$Halogenalkylsulfonyl oder NHCO-$(C_1-C_4)$alkyl ($(C_1-C_4)$Alkylcarbonylamino) steht,

X für ein heteroaromatisches 9-gliedriges oder 12-gliedriges annelliertes bicyclisches oder tricyclisches Ringsystem aus der Reihe H1, H2, H3, H4, H5, H6, H7, H8, H9, H10, H11, H12, H13, H14, H15, H16, H17, H18, H19 oder H20 steht,

$R^5$ für Fluor, Chlor, Fluormethyl, Difluormethyl, Trifluormethyl, Fluorethyl, Difluorethyl, Trifluorethyl, Tetrafluorethyl, Pentafluorethyl, Trifluormethoxy, Difluorchlormethoxy, Dichlorfluormethoxy, Trifluormethylthio, Trifluormethylsulfonyl oder Trifluormethylsulfinyl steht,

$R^6$ für Wasserstoff steht,

$R^7$ für Wasserstoff, Methyl, Ethyl, n-Propyl, i-Propyl, n-Butyl, i-Butyl, tert.-Butyl, Methoxymethyl oder Methoxyethyl steht,

n für 0, 1 oder 2 steht.

**5.** Verbindungen der Formel (I) gemäß Anspruch 1, in welcher

$A^1$ für Stickstoff steht,
$R^1$ für Methyl, Ethyl, n-Propyl, i-Propyl, cyclo-Propyl, n-Butyl, i-Butyl, tert.-Butyl, cyclo-Butyl, Fluormethyl, Difluormethyl, Trifluormethyl, Fluorethyl, Difluorethyl, Trifluorethyl, Tetrafluorethyl oder Pentafluorethyl steht,
$R^2$ für ein gegebenenfalls einfach oder zweifach, gleich oder verschieden substituiertes, annelliertes bicyclisches Ringsystem aus der Reihe Q1, Q6, Q11, Q12, Q15, Q16, Q17, Q20, Q27, Q28, Q32, Q33, Q34, Q35, Q37, Q38, Q51, Q56, Q57, Q61, Q62, Q66, Q67, Q69, Q82, Q87, Q105, Q106, Q107 oder Q152 steht, und wobei als Substituenten jeweils in Frage kommen: Cyano, Fluor, Chlor, Brom, Methyl, Ethyl, n-Propyl, i-Propyl, Cyclopropyl, Trifluormethyl, Pentafluoroethyl, Methoxy, Ethoxy, Trifluormethoxy, Trifluorethoxy, Pentafluorethoxy oder Trifluormethylthio,
$R^3$ für Wasserstoff, Cyano, Halogen, $(C_1\text{-}C_4)$Alkyl, $(C_1\text{-}C_4)$Halogenalkyl, $(C_1\text{-}C_4)$Halogenalkoxy, $(C_1\text{-}C_4)$Alkylthio, $(C_1\text{-}C_4)$Alkylsulfinyl, $(C_1\text{-}C_4)$Alkylsulfonyl, $(C_1\text{-}C_4)$Halogenalkylthio, $(C_1\text{-}C_4)$Halogenalkylsulfinyl, $(C_1\text{-}C_4)$Halogenalkylsulfonyl oder NHCO-$(C_1\text{-}C_4)$alkyl ($(C_1\text{-}C_4)$Alkylcarbonylamino) steht,
X für ein heteroaromatisches 9-gliedriges oder 12-gliedriges annelliertes bicyclisches oder tricyclisches Ringsystem aus der Reihe H1, H2, H3, H4, H5, H6, H7, H8, H9, H10, H11, H12, H13, H14, H15, H16, H17, H18, H19 oder H20 steht,
$R^5$ für Fluor, Chlor, Fluormethyl, Difluormethyl, Trifluormethyl, Fluorethyl, Difluorethyl, Trifluorethyl, Tetrafluorethyl, Pentafluorethyl, Trifluormethoxy, Difluorchlormethoxy, Dichlorfluormethoxy, Trifluormethylthio, Trifluormethylsulfonyl oder Trifluormethylsulfinyl steht,
$R^6$ für Wasserstoff steht,
$R^7$ für Wasserstoff, Methyl, Ethyl, n-Propyl, i-Propyl, n-Butyl, i-Butyl, tert.-Butyl, Methoxymethyl oder Methoxyethyl steht,
n für 0, 1 oder 2 steht.

**6.** Verbindungen der Formel (I) gemäß Anspruch 1, in welcher

$A^1$ für Stickstoff steht,
$R^1$ für Ethyl steht,
$R^2$ für ein einfach oder zweifach, gleich oder verschieden durch Chlor, Brom, Cyano, Methyl, Methoxy oder Trifluormethyl substituiertes, annelliertes bicyclisches Ringsystemg aus der Reihe Q1, Q6, Q11, Q12, Q15, Q16, Q17, Q20, Q27, Q28, Q32, Q33, Q34, Q35, Q37, Q38, Q51, Q56, Q57, Q61, Q62, Q66, Q67, Q69, Q82, Q87, Q105, Q106, Q107 oder Q152 steht,
$R^3$ für Wasserstoff steht,
X für ein heteroaromatisches Ringsystem aus der Reihe H20 steht,
$R^5$ für Trifluormethyl steht,
$R^6$ für Wasserstoff steht,
$R^7$ für Methyl steht,
n für 1 oder 2 steht.

**7.** Agrochemische Formulierung enthaltend Verbindungen der Formel (I) gemäß Anspruch 1, sowie Streckmittel und/oder oberflächenaktive Substanzen.

**8.** Agrochemische Formulierung gemäß Anspruch 7 zusätzlich enthaltend einen weiteren agrochemischen Wirkstoff.

**9.** Verfahren zur Bekämpfung von tierischen Schädlingen **dadurch gekennzeichnet, dass** man eine Verbindung der Formel (I) gemäß Anspruch 1 oder eine agrochemische Formulierung gemäß einem der Ansprüche 7 oder 8 auf die tierischen Schädlinge und/oder ihren Lebensraum einwirken lässt.

**10.** Verwendung von Verbindungen der Formel (I) gemäß Anspruch 1 oder von agrochemischen Formulierungen gemäß einem der Ansprüche 7 oder 8 zur Bekämpfung von tierischen Schädlingen.

## Claims

**1.** Compounds of the formula (I)

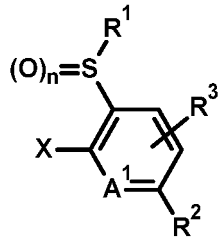

(I)

in which

$A^1$ represents nitrogen, $=N^+(O^-)$- or $=C(R^4)$-,

$R^1$ represents ($C_1$-$C_6$) -alkyl, ($C_1$-$C_6$) -haloalkyl, ($C_1$-$C_6$) -cyanoalkyl, ($C_1$-$C_6$) -hydroxyalkyl, ($C_1$-$C_6$) -alkoxy-($C_1$-$C_6$) -alkyl, ($C_1$-$C_6$) -haloalkoxy- ($C_1$-$C_6$) -alkyl, ($C_2$-$C_6$)-alkenyl, ($C_2$-$C_6$) -alkenyloxy- ($C_1$-$C_6$) -alkyl, ($C_2$-$C_6$)-haloalkenyloxy-($C_1$-$C_6$)-alkyl, ($C_2$-$C_6$)-haloalkenyl, ($C_2$-$C_6$)-cyanoalkenyl, ($C_2$-$C_6$)-alkynyl, ($C_2$-$C_6$)-alkynyloxy-($C_1$-$C_6$)-alkyl, ($C_2$-$C_6$)-haloalkynyloxy-($C_1$-$C_6$)-alkyl, ($C_2$-$C_6$)-haloalkynyl, ($C_2$-$C_6$)-cyanoalkynyl, ($C_3$-$C_8$)-cycloalkyl, ($C_3$-$C_8$)-cycloalkyl-($C_3$-$C_8$)-cycloalkyl, ($C_1$-$C_6$)-alkyl-($C_3$-$C_8$)-cycloalkyl, halo-($C_3$-$C_8$)cycloalkyl, amino, ($C_1$-$C_6$)-alkylamino, di-($C_1$-$C_6$)-alkylamino, ($C_3$-$C_8$)-cycloalkylamino, ($C_1$-$C_6$)-alkylcarbonylamino, ($C_1$-$C_6$)-alkylthio-($C_1$-$C_6$)-alkyl, ($C_1$-$C_6$)-haloalkylthio-($C_1$-$C_6$)-alkyl, ($C_1$-$C_6$)-alkylsulfinyl-($C_1$-$C_6$)-alkyl, ($C_1$-$C_6$)-haloalkylsulfinyl-($C_1$-$C_6$)-alkyl, ($C_1$-$C_6$)-alkylsulfonyl-($C_1$-$C_6$)-alkyl, ($C_1$-$C_6$)-haloalkylsulfonyl-($C_1$-$C_6$)-alkyl, ($C_1$-$C_6$)-alkoxy-($C_1$-$C_6$)-alkylthio-($C_1$-$C_6$)-alkyl, ($C_1$-$C_6$)-alkoxy-($C_1$-$C_6$)-alkylsulfinyl-($C_1$-$C_6$)-alkyl, ($C_1$-$C_6$)-alkoxy-($C_1$-$C_6$)-alkylsulfonyl-($C_1$-$C_6$)-alkyl, ($C_1$-$C_6$)-alkylcarbonyl-($C_1$-$C_6$)-alkyl, ($C_1$-$C_6$)-haloalkylcarbonyl-($C_1$-$C_6$)-alkyl, ($C_1$-$C_6$)-alkoxycarbonyl-($C_1$-$C_6$)-alkyl, ($C_1$-$C_6$)-haloalkoxycarbonyl-($C_1$-$C_6$)-alkyl, ($C_1$-$C_6$)-alkylsulfonylamino, aminosulfonyl- ($C_1$-$C_6$)-alkyl, ($C_1$-$C_6$)-alkylaminosulfonyl-($C_1$-$C_6$)-alkyl, di-($C_1$-$C_6$)-alkylaminosulfonyl-($C_1$-$C_6$)-alkyl,

or represents ($C_1$-$C_6$)-alkyl, ($C_1$-$C_6$)-alkoxy, ($C_2$-$C_6$)-alkenyl, ($C_2$-$C_6$)-alkynyl, ($C_3$-$C_8$)-cycloalkyl, each of which is optionally mono- or polysubstituted by identical or different substituents from the group consisting of aryl, hetaryl and heterocyclyl, where aryl, hetaryl and heterocyclyl may each optionally be mono- or polysubstituted by identical or different substituents from the group consisting of halogen, cyano, nitro, hydroxy, amino, carboxy, carbamoyl, aminosulfonyl, ($C_1$-$C_6$)-alkyl, ($C_3$-$C_6$)-cycloalkyl, ($C_1$-$C_6$)-alkoxy, ($C_1$-$C_6$)-haloalkyl, ($C_1$-$C_6$)-haloalkoxy, ($C_1$-$C_6$)-alkylthio, ($C_1$-$C_6$)-alkylsulfinyl, ($C_1$-$C_6$)-alkylsulfonyl, ($C_1$-$C_6$)-alkylsulfimino, ($C_1$-$C_6$)-alkylsulfimino-($C_1$-$C_6$)-alkyl, ($C_1$-$C_6$)-alkylsulfimino-($C_2$-$C_6$)-alkylcarbonyl, ($C_1$-$C_6$)-alkylsulfoximino, ($C_1$-$C_6$)-alkylsulfoximino-($C_1$-$C_6$)-alkyl, ($C_1$-$C_6$)-alkylsulfoximino-($C_2$-$C_6$)-alkylcarbonyl, ($C_1$-$C_6$)-alkoxycarbonyl, ($C_1$-$C_6$)-alkylcarbonyl, ($C_3$-$C_6$)-trialkylsilyl and benzyl, or

$R^1$ represents aryl, hetaryl or heterocyclyl, each of which is optionally mono- or polysubstituted by identical or different substituents from the group consisting of halogen, cyano, nitro, hydroxy, amino, carboxy, carbamoyl, ($C_1$-$C_6$)-alkyl, ($C_3$-$C_8$)-cycloalkyl, ($C_1$-$C_6$)-alkoxy, ($C_1$-$C_6$)-haloalkyl, ($C_1$-$C_6$)-haloalkoxy, ($C_1$-$C_6$)-alkylthio, ($C_1$-$C_6$)-alkylsulfinyl, ($C_1$-$C_6$)-alkylsulfonyl, ($C_1$-$C_6$)-alkylsulfimino, ($C_1$-$C_6$)-alkylsulfimino-($C_1$-$C_6$)-alkyl, ($C_1$-$C_6$)-alkylsulfimino-($C_2$-$C_6$)-alkylcarbonyl, ($C_1$-$C_6$)-alkylsulfoximino, ($C_1$-$C_6$)-alkylsulfoximino-($C_1$-$C_6$)-alkyl, ($C_1$-$C_6$)-alkylsulfoximino-($C_2$-$C_6$)-alkylcarbonyl, ($C_1$-$C_6$)-alkoxycarbonyl, ($C_1$-$C_6$)-alkylcarbonyl, ($C_3$-$C_6$)-trialkylsilyl, (=O) (only in the case of heterocyclyl) and $(=O)_2$ (only in the case of heterocyclyl),

$R^2$ represents a partially saturated or saturated heterocyclic or heteroaromatic 8-, 9-, 10-, 11- or 12-membered fused bicyclic ring system, where optionally at least one carbonyl group may be present and/or where the ring system may optionally be mono- or polysubstituted by identical or different substituents, and where the substituents independently of one another may be selected from the group consisting of cyano, carboxy, halogen, nitro, acetyl, hydroxy, amino, SCN, tri-($C_1$-$C_6$)-alkylsilyl, ($C_1$-$C_6$)-alkyl, ($C_1$-$C_6$)-haloalkyl, ($C_1$-$C_6$)-cyanoalkyl, ($C_1$-$C_6$)-hydroxyalkyl, hydroxycarbonyl-($C_1$-$C_6$)-alkoxy, ($C_1$-$C_6$)-alkoxycarbonyl-($C_1$-$C_6$)-alkyl, ($C_1$-$C_6$)-alkoxy-($C_1$-$C_6$)-alkyl, ($C_2$-$C_6$) -alkenyl, ($C_2$-$C_6$)-haloalkenyl, ($C_2$-$C_6$)-cyanoalkenyl, ($C_2$-$C_6$)-alkynyl, ($C_2$-$C_6$)-haloalkynyl, ($C_2$-$C_6$)-cyanoalkynyl, ($C_3$-$C_6$)-cycloalkyl, ($C_3$-$C_6$)-cycloalkyl-($C_3$-$C_6$)-cycloalkyl, ($C_1$-$C_4$)-alkyl-($C_3$-$C_6$)-cycloalkyl, ($C_1$-$C_6$)-alkoxy, ($C_1$-$C_6$)-haloalkoxy, ($C_1$-$C_6$)-cyanoalkoxy, ($C_1$-$C_6$)-alkoxycarbonyl-($C_1$-$C_6$)-alkoxy, ($C_1$-$C_6$)-alkoxy-($C_1$-$C_6$)-alkoxy, ($C_1$-$C_6$)-alkoxyimino, -N=C(H)-O($C_1$-$C_6$)-alkyl, -C(H)=N-O($C_1$-$C_6$)-alkyl, ($C_1$-$C_6$)-haloalkyl-($C_1$-$C_6$)-alkoxyimino, ($C_1$-$C_6$)-alkylthio, ($C_1$-$C_6$)-haloalkylthio, ($C_1$-$C_6$)-alkoxy-($C_1$-$C_6$)-alkylthio, ($C_1$-$C_6$)-alkylthio-($C_1$-$C_6$)-alkyl, ($C_1$-$C_6$)-alkylsulfinyl, ($C_1$-$C_6$)-haloalkylsulfinyl, ($C_1$-$C_6$)-alkoxy-($C_1$-$C_6$)-alkylsulfinyl, ($C_1$-$C_6$)-alkylsulfinyl-($C_1$-$C_6$)-alkyl, ($C_1$-$C_6$)-alkylsulfonyl, ($C_1$-$C_6$)-haloalkylsulfonyl, ($C_1$-$C_6$)-alkoxy-($C_1$-$C_6$)-alkylsulfonyl, ($C_1$-$C_6$)-alkylsulfonyl- ($C_1$-$C_6$)-alkyl, ($C_1$-$C_6$)-alkylsulfonyloxy, ($C_1$-$C_6$)-alkylcarbonyl, ($C_1$-$C_6$)-haloalkylcarbonyl, ($C_1$-$C_6$)-alkylcarbonyloxy, ($C_1$-$C_6$)-alkoxycarbonyl, ($C_1$-$C_6$)-haloalkoxycarbonyl, aminocarbonyl, ($C_1$-$C_6$)-alkylaminocarbonyl, di-($C_1$-$C_6$)-alkylaminocarbonyl, ($C_2$-$C_6$)-alkenylaminocarbonyl, di-($C_2$-$C_6$)-alkenylaminocarbonyl, ($C_3$-$C_8$)-cy-

cloalkylaminocarbonyl, ($C_1$-$C_6$)-alkylsulfonylamino, ($C_1$-$C_6$)-alkylamino, di-($C_1$-$C_6$)-alkylamino, aminosulfonyl, ($C_1$-$C_6$)-alkylaminosulfonyl, di-($C_1$-$C_6$)-alkylaminosulfonyl, ($C_1$-$C_6$)-alkylsulfoximino, aminothiocarbonyl, ($C_1$-$C_6$)-alkylaminothiocarbonyl, di-($C_1$-$C_6$)-alkylaminothiocarbonyl, ($C_3$-$C_8$)-cycloalkylamino, ($C_1$-$C_6$)-alkylcarbonylamino,

$R^3$ represents hydrogen, cyano, halogen, nitro, acetyl, hydroxy, amino, SCN, tri-($C_1$-$C_6$)-alkylsilyl, ($C_3$-$C_8$)-cycloalkyl, ($C_3$-$C_8$)-cycloalkyl-($C_3$-$C_8$)-cycloalkyl, ($C_1$-$C_6$)-alkyl-($C_3$-$C_8$)-cycloalkyl, halo-($C_3$-$C_8$)-cycloalkyl, ($C_1$-$C_6$)-alkyl, ($C_1$-$C_6$)-haloalkyl, ($C_1$-$C_6$)-cyanoalkyl, ($C_1$-$C_6$)-hydroxyalkyl, hydroxycarbonyl-($C_1$-$C_6$)-alkoxy, ($C_1$-$C_6$)-alkoxycarbonyl-($C_1$-$C_6$)-alkyl, ($C_1$-$C_6$)-alkoxy-($C_1$-$C_6$)-alkyl, ($C_2$-$C_6$)-alkenyl, ($C_2$-$C_6$)-haloalkenyl, ($C_2$-$C_6$)-cyanoalkenyl, ($C_2$-$C_6$)-alkynyl, ($C_2$-$C_6$)-haloalkynyl, ($C_2$-$C_6$)-cyanoalkynyl, ($C_1$-$C_6$)-alkoxy, ($C_1$-$C_6$)-haloalkoxy, ($C_1$-$C_6$)-cyanoalkoxy, ($C_1$-$C_6$)-alkoxycarbonyl-($C_1$-$C_6$)-alkoxy, ($C_1$-$C_6$)-alkoxy-($C_1$-$C_6$)-alkoxy, ($C_1$-$C_6$)-alkylhydroxyimino, ($C_1$-$C_6$)-alkoxyimino, ($C_1$-$C_6$)-alkyl-($C_1$-$C_6$)-alkoxyimino, ($C_1$-$C_6$)-haloalkyl-($C_1$-$C_6$)-alkoxyimino, ($C_1$-$C_6$)-alkylthio, ($C_1$-$C_6$)-haloalkylthio, ($C_1$-$C_6$)-alkoxy-($C_1$-$C_6$)-alkylthio, ($C_1$-$C_6$)-alkylthio-($C_1$-$C_6$)-alkyl, ($C_1$-$C_6$)-alkylsulfinyl, ($C_1$-$C_6$)-haloalkylsulfinyl, ($C_1$-$C_6$)-alkoxy-($C_1$-$C_6$)-alkylsulfinyl, ($C_1$-$C_6$)-alkylsulfinyl-($C_1$-$C_6$)-alkyl, ($C_1$-$C_6$)-alkylsulfonyl, ($C_1$-$C_6$)-haloalkylsulfonyl, ($C_1$-$C_6$)-alkoxy-($C_1$-$C_6$)-alkylsulfonyl, ($C_1$-$C_6$)-alkylsulfonyl-($C_1$-$C_6$)-alkyl, ($C_1$-$C_6$)-alkylsulfonyloxy, ($C_1$-$C_6$)-alkylcarbonyl, ($C_1$-$C_6$)-alkylthiocarbonyl, ($C_1$-$C_6$)-haloalkylcarbonyl, ($C_1$-$C_6$)-alkylcarbonyloxy, ($C_1$-$C_6$)-alkoxycarbonyl, ($C_1$-$C_6$)-haloalkoxycarbonyl, aminocarbonyl, ($C_1$-$C_6$)-alkylaminocarbonyl, ($C_1$-$C_6$)-alkylaminothiocarbonyl, di-($C_1$-$C_6$)-alkylaminocarbonyl, di-($C_1$-$C_6$)-alkylaminothiocarbonyl, ($C_2$-$C_6$)-alkenylaminocarbonyl, di-($C_2$-$C_6$)-alkenylaminocarbonyl, ($C_3$-$C_8$)-cycloalkylaminocarbonyl, ($C_1$-$C_6$)-alkylsulfonylamino, ($C_1$-$C_6$)-alkylamino, di-($C_1$-$C_6$)-alkylamino, aminosulfonyl, ($C_1$-$C_6$)-alkylaminosulfonyl, di-($C_1$-$C_6$)-alkylaminosulfonyl, ($C_1$-$C_6$)-alkylsulfoximino, aminothiocarbonyl, ($C_1$-$C_6$)-alkylaminothiocarbonyl, di-($C_1$-$C_6$)-alkylaminothiocarbonyl, ($C_3$-$C_8$)-cycloalkylamino, NH-CO-($C_1$-$C_6$)-alkyl (($C_1$-$C_6$)-alkylcarbonylamino),

represents aryl or hetaryl, each of which is optionally mono- or polysubstituted by identical or different substituents, where (in the case of hetaryl) optionally at least one carbonyl group may be present and/or where possible substituents in each case are as follows: cyano, carboxy, halogen, nitro, acetyl, hydroxy, amino, SCN, tri-($C_1$-$C_6$)-alkylsilyl, ($C_3$-$C_8$)-cycloalkyl, ($C_3$-$C_8$)-cycloalkyl-($C_3$-$C_8$)-cycloalkyl, ($C_1$-$C_6$)-alkyl-($C_3$-$C_8$)-cycloalkyl, halo-($C_3$-$C_8$)-cycloalkyl, ($C_1$-$C_6$)-alkyl, ($C_1$-$C_6$)-haloalkyl, ($C_1$-$C_6$)-cyanoalkyl, ($C_1$-$C_6$)-hydroxyalkyl, hydroxycarbonyl-($C_1$-$C_6$)-alkoxy, ($C_1$-$C_6$)-alkoxycarbonyl-($C_1$-$C_6$)-alkyl, ($C_1$-$C_6$)-alkoxy-($C_1$-$C_6$)-alkyl, ($C_2$-$C_6$)-alkenyl, ($C_2$-$C_6$)-haloalkenyl, ($C_2$-$C_6$)-cyanoalkenyl, ($C_2$-$C_6$)-alkynyl, ($C_2$-$C_6$)-haloalkynyl, ($C_2$-$C_6$)-cyanoalkynyl, ($C_1$-$C_6$)-alkoxy, ($C_1$-$C_6$)-haloalkoxy, ($C_1$-$C_6$)-cyanoalkoxy, ($C_1$-$C_6$)-alkoxycarbonyl-($C_1$-$C_6$)-alkoxy, ($C_1$-$C_6$)-alkoxy-($C_1$-$C_6$)-alkoxy, ($C_1$-$C_6$)-alkylhydroxyimino, ($C_1$-$C_6$)-alkoxyimino, ($C_1$-$C_6$)-alkyl-($C_1$-$C_6$)-alkoxyimino, ($C_1$-$C_6$)-haloalkyl-($C_1$-$C_6$)-alkoxyimino, ($C_1$-$C_6$)-alkylthio, ($C_1$-$C_6$)-haloalkylthio, ($C_1$-$C_6$)-alkoxy-($C_1$-$C_6$)-alkylthio, ($C_1$-$C_6$)-alkylthio-($C_1$-$C_6$)-alkyl, ($C_1$-$C_6$)-alkylsulfinyl, ($C_1$-$C_6$)-haloalkylsulfinyl, ($C_1$-$C_6$)-alkoxy-($C_1$-$C_6$)-alkylsulfinyl, ($C_1$-$C_6$)-alkylsulfinyl-($C_1$-$C_6$)-alkyl, ($C_1$-$C_6$)-alkylsulfonyl, ($C_1$-$C_6$)-haloalkylsulfonyl, ($C_1$-$C_6$)-alkoxy-($C_1$-$C_6$)-alkylsulfonyl, ($C_1$-$C_6$)-alkylsulfonyl-($C_1$-$C_6$)-alkyl, ($C_1$-$C_6$)-alkylsulfonyloxy, ($C_1$-$C_6$)-alkylcarbonyl, ($C_1$-$C_6$)-haloalkylcarbonyl, ($C_1$-$C_6$)-alkylcarbonyloxy, ($C_1$-$C_6$)-alkoxycarbonyl, ($C_1$-$C_6$)-haloalkoxycarbonyl, aminocarbonyl, ($C_1$-$C_6$)-alkylaminocarbonyl, di-($C_1$-$C_6$)-alkylaminocarbonyl, ($C_2$-$C_6$)-alkenylaminocarbonyl, di-($C_2$-$C_6$)-alkenylaminocarbonyl, ($C_3$-$C_8$)-cycloalkylaminocarbonyl, ($C_1$-$C_6$)-alkylsulfonylamino, ($C_1$-$C_6$)-alkylamino, di-($C_1$-$C_6$)-alkylamino, aminosulfonyl, ($C_1$-$C_6$)-alkylaminosulfonyl, di-($C_1$-$C_6$)-alkylaminosulfonyl, ($C_1$-$C_6$)-alkylsulfoximino, aminothiocarbonyl, ($C_1$-$C_6$)-alkylaminothiocarbonyl, di-($C_1$-$C_6$)-alkylaminothiocarbonyl, ($C_3$-$C_8$)-cycloalkylamino, ($C_1$-$C_6$)-alkylcarbonylamino,

$R^4$ represents hydrogen, cyano, halogen, nitro, acetyl, hydroxy, amino, SCN, tri-($C_1$-$C_6$)-alkylsilyl, ($C_3$-$C_8$)-cycloalkyl, ($C_3$-$C_8$)-cycloalkyl-($C_3$-$C_8$)-cycloalkyl, ($C_1$-$C_6$)-alkyl-($C_3$-$C_8$)-cycloalkyl, halo-($C_3$-$C_8$)-cycloalkyl, ($C_1$-$C_6$)-alkyl, ($C_1$-$C_6$)-haloalkyl, ($C_1$-$C_6$)-cyanoalkyl, ($C_1$-$C_6$)-hydroxyalkyl, hydroxycarbonyl-($C_1$-$C_6$)-alkoxy, ($C_1$-$C_6$)-alkoxycarbonyl-($C_1$-$C_6$)-alkyl, ($C_1$-$C_6$)-alkoxy-($C_1$-$C_6$)-alkyl, ($C_2$-$C_6$)-alkenyl, ($C_2$-$C_6$)-haloalkenyl, ($C_2$-$C_6$)-cyanoalkenyl, ($C_2$-$C_6$)-alkynyl, ($C_2$-$C_6$)-haloalkynyl, ($C_2$-$C_6$)-cyanoalkynyl, ($C_1$-$C_6$)-alkoxy, ($C_1$-$C_6$)-haloalkoxy, ($C_1$-$C_6$)-cyanoalkoxy, ($C_1$-$C_6$)-alkoxycarbonyl-($C_1$-$C_6$)-alkoxy, ($C_1$-$C_6$)-alkoxy-($C_1$-$C_6$)-alkoxy, ($C_1$-$C_6$)-alkylhydroxyimino, ($C_1$-$C_6$)-alkoxyimino, ($C_1$-$C_6$)-alkyl-($C_1$-$C_6$)-alkoxyimino, ($C_1$-$C_6$)-haloalkyl-($C_1$-$C_6$)-alkoxyimino, ($C_1$-$C_6$) -alkylthio, ($C_1$-$C_6$)-haloalkylthio, ($C_1$-$C_6$)-alkoxy-($C_1$-$C_6$)-alkylthio, ($C_1$-$C_6$)-alkylthio-($C_1$-$C_6$)-alkyl, ($C_1$-$C_6$)-alkylsulfinyl, ($C_1$-$C_6$)-haloalkylsulfinyl, ($C_1$-$C_6$)-alkoxy-($C_1$-$C_6$)-alkylsulfinyl, ($C_1$-$C_6$)-alkylsulfinyl-($C_1$-$C_6$)-alkyl, ($C_1$-$C_6$)-alkylsulfonyl, ($C_1$-$C_6$)-haloalkylsulfonyl, ($C_1$-$C_6$)-alkoxy-($C_1$-$C_6$)-alkylsulfonyl, ($C_1$-$C_6$)-alkylsulfonyl-($C_1$-$C_6$)-alkyl, ($C_1$-$C_6$)-alkylsulfonyloxy, ($C_1$-$C_6$)-alkylcarbonyl, ($C_1$-$C_6$)-alkylthiocarbonyl, ($C_1$-$C_6$)-haloalkylcarbonyl, ($C_1$-$C_6$)-alkylcarbonyloxy, ($C_1$-$C_6$)-alkoxycarbonyl, ($C_1$-$C_6$)-haloalkoxycarbonyl, aminocarbonyl, ($C_1$-$C_6$)-alkylaminocarbonyl, ($C_1$-$C_6$)-alkylaminothiocarbonyl, di-($C_1$-$C_6$)-alkylaminocarbonyl, di-($C_1$-$C_6$)-alkylaminothiocarbonyl, ($C_2$-$C_6$)-alkenylaminocarbonyl, di-($C_2$-$C_6$)-alkenylaminocarbonyl,

$(C_3-C_8)$-cycloalkylaminocarbonyl, $(C_1-C_6)$-alkylsulfonylamino, $(C_1-C_6)$-alkylamino, di-$(C_1-C_6)$-alkylamino, aminosulfonyl, $(C_1-C_6)$-alkylaminosulfonyl, di-$(C_1-C_6)$-alkylaminosulfonyl, $(C_1-C_6)$-alkylsulfoximino, aminothiocarbonyl, $(C_1-C_6)$-alkylaminothiocarbonyl, di-$(C_1-C_6)$-alkylaminothiocarbonyl, $(C_3-C_8)$-cycloalkylamino or -NH-CO-$(C_1-C_6)$-alkyl (($C_1-C_6$)-alkylcarbonylamino),

X represents a partially saturated or saturated heterocyclic or heteroaromatic 8-, 9-, 10-, 11- or 12-membered fused bicyclic or tricyclic ring system, where optionally at least one carbonyl group may be present and/or where the ring system is optionally mono- or polysubstituted by identical or different substituents, and where the substituents independently of one another may be selected from the group consisting of cyano, halogen, nitro, acetyl, hydroxy, amino, SCN, tri-$(C_1-C_6)$-alkylsilyl, $(C_3-C_8)$-cycloalkyl, $(C_3-C_8)$-cycloalkyl-$(C_3-C_8)$-cycloalkyl, $(C_1-C_6)$-alkyl-$(C_3-C_8)$-cycloalkyl, halo-$(C_3-C_8)$-cycloalkyl, $(C_1-C_6)$-alkyl, $(C_1-C_6)$-haloalkyl, $(C_1-C_6)$-cyanoalkyl, $(C_1-C_6)$-hydroxyalkyl, hydroxycarbonyl-$(C_1-C_6)$-alkoxy, $(C_1-C_6)$-alkoxycarbonyl-$(C_1-C_6)$-alkyl, $(C_1-C_6)$-alkoxy-$(C_1-C_6)$-alkyl, $(C_2-C_6)$-alkenyl, $(C_2-C_6)$-haloalkenyl, $(C_2-C_6)$-cyanoalkenyl, $(C_2-C_6)$-alkynyl, $(C_2-C_6)$-alkynyloxy-$(C_1-C_4)$-alkyl, $(C_2-C_6)$-haloalkynyl, $(C_2-C_6)$-cyanoalkynyl, $(C_1-C_6)$-alkoxy, $(C_1-C_6)$-haloalkoxy, $(C_1-C_6)$-haloalkoxy- $(C_1-C_6)$-alkyl, $(C_2-C_6)$-alkenyloxy- $(C_1-C_6)$-alkyl, $(C_2-C_6)$-haloalkenyloxy-$(C_1-C_6)$-alkyl, $(C_1-C_6)$-cyanoalkoxy, $(C_1-C_6)$-alkoxycarbonyl-$(C_1-C_6)$-alkoxy, $(C_1-C_6)$-alkoxy-$(C_1-C_6)$-alkoxy, $(C_1-C_6)$-alkylhydroxyimino, $(C_1-C_6)$-alkoxyimino, $(C_1-C_6)$-alkyl-$(C_1-C_6)$-alkoxyimino, $(C_1-C_6)$-haloalkyl-$(C_1-C_6)$-alkoxyimino, $(C_1-C_6)$-alkylthio, $(C_1-C_6)$-haloalkylthio, $(C_1-C_6)$-alkoxy-$(C_1-C_6)$-alkylthio, $(C_1-C_6)$-alkylthio-$(C_1-C_6)$-alkyl, $(C_1-C_6)$-alkylsulfinyl, $(C_1-C_6)$-haloalkylsulfinyl, $(C_1-C_6)$-alkoxy-$(C_1-C_6)$-alkylsulfinyl, $(C_1-C_6)$-alkylsulfinyl-$(C_1-C_6)$-alkyl, $(C_1-C_6)$-alkylsulfonyl, $(C_1-C_6)$-haloalkylsulfonyl, $(C_1-C_6)$-alkoxy-$(C_1-C_6)$-alkylsulfonyl, $(C_1-C_6)$-alkylsulfonyl-$(C_1-C_6)$-alkyl, $(C_1-C_6)$-alkylsulfonyloxy, $(C_1-C_6)$-alkylcarbonyl, $(C_1-C_6)$-alkylcarbonyl-$(C_1-C_6)$-alkyl, $(C_1-C_6)$-alkylthiocarbonyl, $(C_1-C_6)$-haloalkylcarbonyl, $(C_1-C_6)$-alkylcarbonyloxy, $(C_1-C_6)$-alkoxycarbonyl, $(C_1-C_6)$-haloalkoxycarbonyl, aminocarbonyl, $(C_1-C_6)$-alkylaminocarbonyl, $(C_1-C_6)$-alkylaminothiocarbonyl, di-$(C_1-C_6)$-alkylaminocarbonyl, di-$(C_1-C_6)$-alkylaminothiocarbonyl, $(C_2-C_6)$-alkenylaminocarbonyl, di-$(C_2-C_6)$-alkenylaminocarbonyl, $(C_3-C_8)$-cycloalkylaminocarbonyl, $(C_1-C_6)$-alkylsulfonylamino, $(C_1-C_6)$-alkylamino, di-$(C_1-C_6)$-alkylamino, aminosulfonyl, $(C_1-C_6)$-alkylaminosulfonyl, di-$(C_1-C_6)$-alkylaminosulfonyl, $(C_1-C_6)$-alkylsulfoximino, aminothiocarbonyl, $(C_1-C_6)$-alkylaminothiocarbonyl, di-$(C_1-C_6)$-alkylaminothiocarbonyl, $(C_3-C_8)$-cycloalkylamino, NH-CO-$(C_1-C_6)$-alkyl (($C_1-C_6$)-alkylcarbonylamino),

or where the substituents independently of one another may be selected from phenyl or a 5- or 6-membered heteroaromatic ring, where phenyl or the ring may optionally be mono- or polysubstituted by identical or different substituents from the group consisting of $C_1-C_6$-alkyl, $C_2-C_6$-alkenyl, $C_2-C_6$-alkynyl, $C_3-C_6$-cycloalkyl, $C_1-C_6$-haloalkyl, $C_2-C_6$-haloalkenyl, $C_2-C_6$-haloalkynyl, $C_3-C_6$-halocycloalkyl, halogen, CN, $NO_2$, $C_1-C_4$-alkoxy, $C_1-C_4$-haloalkoxy,

n represents 0, 1 or 2,

with the exception of

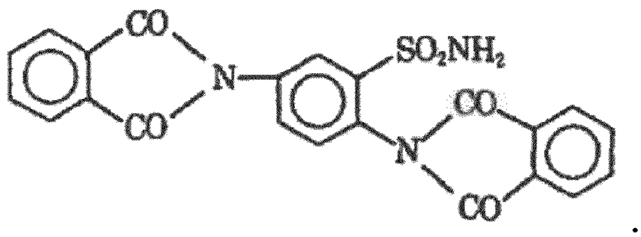

**2.** Compounds of the formula (I) according to Claim 1 in which

$A^1$ represents nitrogen, $=N^+(O^-)-$ or $=C(R^4)-$,

$R^1$ represents $(C_1-C_4)$-alkyl, $(C_1-C_4)$-hydroxyalkyl, $(C_1-C_4)$-haloalkyl, $(C_1-C_4)$-cyanoalkyl, $(C_1-C_4)$-alkoxy-$(C_1-C_4)$-alkyl, $(C_1-C_4)$-haloalkoxy-$(C_1-C_4)$-alkyl, $(C_2-C_4)$-alkenyl, $(C_2-C_4)$-alkenyloxy-$(C_1-C_4)$-alkyl, $(C_2-C_4)$-haloalkenyloxy-$(C_1-C_4)$-alkyl, $(C_2-C_4)$-haloalkenyl, $(C_2-C_4)$-cyanoalkenyl, $(C_2-C_4)$-alkynyl, $(C_2-C_4)$-alkynyloxy-$(C_1-C_4)$-alkyl, $(C_2-C_4)$-haloalkynyloxy-$(C_1-C_4)$-alkyl, $(C_2-C_4)$-haloalkynyl, $(C_2-C_4)$-cyanoalkynyl, $(C_3-C_6)$-cycloalkyl, $(C_3-C_6)$-cycloalkyl-$(C_3-C_6)$-cycloalkyl, $(C_1-C_4)$-alkyl-$(C_3-C_6)$-cycloalkyl, halo-$(C_3-C_6)$-cycloalkyl, $(C_1-C_4)$-alkylamino, di-$(C_1-C_4)$-alkylamino, $(C_3-C_6)$-cycloalkylamino, $(C_1-C_4)$-alkylcarbonylamino, $(C_1-C_4)$-alkylthio-$(C_1-C_4)$-alkyl, $(C_1-C_4)$-haloalkylthio-$(C_1-C_4)$-alkyl, $(C_1-C_4)$-alkylsulfinyl-$(C_1-C_4)$-alkyl, $(C_1-C_4)$-haloalkylsulfinyl-$(C_1-C_4)$-alkyl, $(C_1-C_4)$-alkylsulfonyl-$(C_1-C_4)$-alkyl, $(C_1-C_4)$-alkylcarbonyl-$(C_1-C_4)$-alkyl, $(C_1-C_4)$-haloalkylcarbonyl-$(C_1-C_4)$-alkyl, $(C_1-C_4)$-alkylsulfonylamino,

or represents $(C_1-C_4)$-alkyl, $(C_1-C_4)$-alkoxy, $(C_2-C_4)$-alkenyl, $(C_2-C_4)$-alkynyl, $(C_3-C_6)$-cycloalkyl, each of which

is optionally mono- or disubstituted by identical or different substituents from the group consisting of aryl, hetaryl and heterocyclyl, where aryl, hetaryl or heterocyclyl may each optionally be mono- or disubstituted by identical or different substituents from the group consisting of halogen, cyano, carbamoyl, aminosulfonyl, $(C_1-C_4)$-alkyl, $(C_3-C_4)$-cycloalkyl, $(C_1-C_4)$-alkoxy, $(C_1-C_4)$-haloalkyl, $(C_1-C_4)$-haloalkoxy, $(C_1-C_4)$-alkylthio, $(C_1-C_4)$-alkylsulfinyl, $(C_1-C_4)$-alkylsulfonyl, $(C_1-C_4)$-alkylsulfimino, or

$R^1$ represents aryl, hetaryl or heterocyclyl, each of which is optionally mono- or disubstituted by identical or different substituents from the group consisting of halogen, cyano, carbamoyl, $(C_1-C_4)$-alkyl, $(C_3-C_6)$ -cycloalkyl, $(C_1-C_4)$-alkoxy, $(C_1-C_4)$-haloalkyl, $(C_1-C_4)$-haloalkoxy, $(C_1-C_4)$-alkylthio, $(C_1-C_4)$-alkylsulfinyl, $(C_1-C_4)$-alkylsulfonyl, $(C_1-C_4)$-alkylsulfimino, $(C_1-C_4)$-alkylsulfoximino, $(C_1-C_4)$-alkylcarbonyl, $(C_3-C_4)$-trialkylsilyl, (=O) (only in the case of heterocyclyl) and $(=O)_2$ (only in the case of heterocyclyl),

$R^2$ represents a partially saturated or saturated heterocyclic or heteroaromatic 8-, 9- or 10-membered fused bicyclic ring system, where optionally at least one carbonyl group may be present and where the ring system may optionally be mono- or polysubstituted by identical or different substituents, where the substituents independently of one another may be selected from the group consisting of cyano, halogen, nitro, acetyl, amino, $(C_1-C_4)$-alkyl, $(C_1-C_4)$-haloalkyl, $(C_1-C_4)$-cyanoalkyl, $(C_1-C_4)$-hydroxyalkyl, $(C_1-C_4)$-alkoxy-$(C_1-C_4)$-alkyl, $(C_2-C_4)$-alkenyl, $(C_2-C_4)$-haloalkenyl, $(C_2-C_4)$-cyanoalkenyl, $(C_2-C_4)$-alkynyl, $(C_2-C_4)$-haloalkynyl, $(C_2-C_4)$-cyanoalkynyl, $(C_3-C_6)$-cycloalkyl, $(C_3-C_6)$-cycloalkyl-$(C_3-C_6)$cycloalkyl, $(C_1-C_4)$-alkyl-$(C_3-C_6)$cycloalkyl, $(C_1-C_4)$-alkoxy, $(C_1-C_4)$-haloalkoxy, $(C_1-C_4)$-cyanoalkoxy, $(C_1-C_4)$-alkoxy-$(C_1-C_4)$-alkoxy, $(C_1-C_4)$-alkoxyimino, -N=C(H)-O$(C_1-C_4)$-alkyl,-C(H)=N-O$(C_1-C_4)$-alkyl, $(C_1-C_4)$-haloalkyl-$(C_1-C_4)$-alkoxyimino, $(C_1-C_4)$-alkylthio, $(C_1-C_4)$-haloalkylthio, $(C_1-C_4)$-alkylthio-$(C_1-C_4)$-alkyl, $(C_1-C_4)$-alkylsulfinyl, $(C_1-C_4)$-haloalkylsulfinyl, $(C_1-C_4)$-alkylsulfinyl- $(C_1-C_4)$-alkyl, $(C_1-C_4)$-alkylsulfonyl, $(C_1-C_4)$-haloalkylsulfonyl, $(C_1-C_4)$-alkylsulfonyl-$(C_1-C_4)$-alkyl, $(C_1-C_4)$-alkylsulfonyloxy, $(C_1-C_4)$-alkylcarbonyl, $(C_1-C_4)$-haloalkylcarbonyl, aminocarbonyl, $(C_1-C_4)$-alkylaminocarbonyl, di-$(C_1-C_4)$-alkylaminocarbonyl, $(C_1-C_4)$-alkylsulfonylamino, $(C_1-C_4)$-alkylamino, di-$(C_1-C_4)$-alkylamino, aminosulfonyl, $(C_1-C_4)$-alkylaminosulfonyl, di-$(C_1-C_4)$-alkylaminosulfonyl, $(C_1-C_4)$-alkylcarbonylamino

$R^3$ represents hydrogen, cyano, halogen, nitro, acetyl, hydroxy, amino, SCN, tri-$(C_1-C_4)$-alkylsilyl, $(C_3-C_6)$-cycloalkyl, $(C_3-C_6)$-cycloalkyl-$(C_3-C_6)$cycloalkyl, $(C_1-C_4)$-alkyl-$(C_3-C_6)$-cycloalkyl, halo-$(C_3-C_6)$-cycloalkyl, $(C_1-C_4)$-alkyl, $(C_1-C_4)$-haloalkyl, $(C_1-C_4)$-cyanoalkyl, $(C_1-C_4)$-hydroxyalkyl, $(C_1-C_4)$-alkoxy-$(C_1-C_4)$-alkyl, $(C_2-C_4)$-alkenyl, $(C_2-C_4)$ -haloalkenyl, $(C_2-C_4)$-cyanoalkenyl, $(C_2-C_4)$-alkynyl, $(C_2-C_4)$ -haloalkynyl, $(C_2-C_4)$-cyanoalkynyl, $(C_1-C_4)$-alkoxy, $(C_1-C_4)$-haloalkoxy, $(C_1-C_4)$-cyanoalkoxy, $(C_1-C_4)$-alkoxy-$(C_1-C_4)$-alkoxy, $(C_1-C_4)$-alkylhydroxyimino, $(C_1-C_4)$-alkoxyimino, $(C_1-C_4)$-alkyl-$(C_1-C_4)$-alkoxyimino, $(C_1-C_4)$-haloalkyl-$(C_1-C_4)$-alkoxyimino, $(C_1-C_4)$-alkylthio, $(C_1-C_4)$-haloalkylthio, $(C_1-C_4)$-alkylthio-$(C_1-C_4)$-alkyl, $(C_1-C_4)$-alkylsulfinyl, $(C_1-C_4)$-haloalkylsulfinyl, $(C_1-C_4)$-alkylsulfinyl- $(C_1-C_4)$-alkyl, $(C_1-C_4)$-alkylsulfonyl, $(C_1-C_4)$-haloalkylsulfonyl, $(C_1-C_4)$-alkylsulfonyl-$(C_1-C_4)$-alkyl, $(C_1-C_4)$-alkylsulfonyloxy, $(C_1-C_4)$-alkylcarbonyl, $(C_1-C_4)$-haloalkylcarbonyl, aminocarbonyl, aminothiocarbonyl, $(C_1-C_4)$-alkylaminocarbonyl, di-$(C_1-C_4)$-alkylaminocarbonyl, $(C_1-C_4)$-alkylsulfonylamino, $(C_1-C_4)$-alkylamino, di-$(C_1-C_4)$-alkylamino, aminosulfonyl, $(C_1-C_4)$-alkylaminosulfonyl, di-$(C_1-C_4)$-alkylaminosulfonyl, aminothiocarbonyl, NHCO-$(C_1-C_4)$-alkyl ($(C_1-C_4)$-alkylcarbonylamino),

furthermore represents phenyl or hetaryl, each of which is optionally mono- or disubstituted by identical or different substituents, where (in the case of hetaryl) at least one carbonyl group may optionally be present and/or where possible substituents are in each case as follows: cyano, halogen, nitro, acetyl, amino, $(C_3-C_6)$-cycloalkyl, $(C_3-C_6)$-cycloalkyl-$(C_3-C_6)$-cycloalkyl, $(C_1-C_4)$-alkyl-$(C_3-C_6)$-cycloalkyl, halo-$(C_3-C_6)$-cycloalkyl, $(C_1-C_4)$-alkyl, $(C_1-C_4)$-haloalkyl, $(C_1-C_4)$-cyanoalkyl, $(C_1-C_4)$-hydroxyalkyl, $(C_1-C_4)$-alkoxy-$(C_1-C_4)$-alkyl, $(C_2-C_4)$-alkenyl, $(C_2-C_4)$-haloalkenyl, $(C_2-C_4)$-cyanoalkenyl, $(C_2-C_4)$-alkynyl, $(C_2-C_4)$-haloalkynyl, $(C_2-C_4)$-cyanoalkynyl, $(C_1-C_4)$-alkoxy, $(C_1-C_4)$-haloalkoxy, $(C_1-C_4)$-cyanoalkoxy, $(C_1-C_4)$-alkoxy-$(C_1-C_4)$-alkoxy, $(C_1-C_4)$-alkylhydroxyimino, $(C_1-C_4)$-alkoxyimino, $(C_1-C_4)$-alkyl-$(C_1-C_4)$-alkoxyimino, $(C_1-C_4)$-haloalkyl-$(C_1-C_4)$-alkoxyimino, $(C_1-C_4)$ -alkylthio, $(C_1-C_4)$-haloalkylthio, $(C_1-C_4)$-alkylthio-$(C_1-C_4)$-alkyl, $(C_1-C_4)$-alkylsulfinyl, $(C_1-C_4)$-haloalkylsulfinyl, $(C_1-C_4)$-alkylsulfinyl-$(C_1-C_4)$-alkyl, $(C_1-C_4)$-alkylsulfonyl, $(C_1-C_4)$-haloalkylsulfonyl, $(C_1-C_4)$-alkylsulfonyl-$(C_1-C_4)$-alkyl, $(C_1-C_4)$-alkylsulfonyloxy, $(C_1-C_4)$-alkylcarbonyl, $(C_1-C_4)$-haloalkylcarbonyl, aminocarbonyl, $(C_1-C_4)$-alkylaminocarbonyl, di-$(C_1-C_4)$-alkylaminocarbonyl, $(C_1-C_4)$-alkylsulfonylamino, $(C_1-C_4)$-alkylamino, di-$(C_1-C_4)$-alkylamino, aminosulfonyl, $(C_1-C_4)$-alkylaminosulfonyl, di-$(C_1-C_4)$-alkylaminosulfonyl, NH-CO-$(C_1-C_4)$-alkyl($(C_1-C_4)$-alkylcarbonylamino),

$R^4$ represents hydrogen, cyano, halogen, nitro, acetyl, hydroxy, amino, SCN, tri-$(C_1-C_4)$-alkylsilyl, $(C_3-C_6)$-cycloalkyl, $(C_3-C_6)$-cycloalkyl- $(C_3-C_6)$cycloalkyl, $(C_1-C_4)$-alkyl-$(C_3-C_6)$cycloalkyl, halo-$(C_3-C_6)$cycloalkyl, $(C_1-C_4)$-alkyl, $(C_1-C_4)$-haloalkyl, $(C_1-C_4)$-cyanoalkyl, $(C_1-C_4)$-hydroxyalkyl, $(C_1-C_4)$-alkoxy-$(C_1-C_4)$-alkyl, $(C_2-C_4)$-alkenyl, $(C_2-C_4)$ -haloalkenyl, $(C_2-C_4)$ -cyanoalkenyl, $(C_2-C_4)$-alkynyl, $(C_2-C_4)$-haloalkynyl, $(C_2-C_4)$-cyanoalkynyl, $(C_1-C_4)$-alkoxy, $(C_1-C_4)$-haloalkoxy, $(C_1-C_4)$-cyanoalkoxy, $(C_1-C_4)$-alkoxy-$(C_1-C_4)$-alkoxy, $(C_1-C_4)$-alkylhydroxyimino, $(C_1-C_4)$-alkoxyimino, $(C_1-C_4)$-alkyl-$(C_1-C_4)$-alkoxyimino,

$(C_1-C_4)$-haloalkyl-$(C_1-C_4)$-alkoxyimino, $(C_1-C_4)$-alkylthio, $(C_1-C_4)$-haloalkylthio, $(C_1-C_4)$-alkylthio-$(C_1-C_4)$-alkyl, $(C_1-C_4)$-alkylsulfinyl, $(C_1-C_4)$-haloalkylsulfinyl, $(C_1-C_4)$-alkylsulfinyl-$(C_1-C_4)$-alkyl, $(C_1-C_4)$-alkylsulfonyl, $(C_1-C_4)$-haloalkylsulfonyl, $(C_1-C_4)$-alkylsulfonyl-$(C_1-C_4)$-alkyl, $(C_1-C_4)$-alkylsulfonyloxy, $(C_1-C_4)$-alkylcarbonyl, $(C_1-C_4)$-haloalkylcarbonyl, aminocarbonyl, aminothiocarbonyl, $(C_1-C_4)$-alkylaminocarbonyl, di-$(C_1-C_4)$-alkylaminocarbonyl, $(C_1-C_4)$-alkylsulfonylamino, $(C_1-C_4)$-alkylamino, di-$(C_1-C_4)$-alkylamino, aminosulfonyl, $(C_1-C_4)$-alkylaminosulfonyl, di-$(C_1-C_4)$-alkylaminosulfonyl, aminothiocarbonyl or NHCO-$(C_1-C_4)$-alkyl ($(C_1-C_4)$-alkylcarbonylamino),

X represents a heteroaromatic 8-, 9-, 10-, 11- or 12-membered fused bicyclic or tricyclic ring system, where the ring system is optionally mono- or polysubstituted by identical or different substituents, and where the substituents independently of one another may be selected from the group consisting of cyano, halogen, nitro, acetyl, hydroxy, amino, SCN, tri-$(C_1-C_6)$-alkylsilyl, $(C_3-C_8)$-cycloalkyl, $(C_3-C_8)$-cycloalkyl-$(C_3-C_8)$-cycloalkyl, $(C_1-C_6)$-alkyl-$(C_3-C_8)$-cycloalkyl, halo-$(C_3-C_8)$-cycloalkyl, $(C_1-C_6)$-alkyl, $(C_1-C_6)$-haloalkyl, $(C_1-C_6)$-cyanoalkyl, $(C_1-C_6)$-hydroxyalkyl, $(C_1-C_6)$-alkoxy-$(C_1-C_6)$-alkyl, $(C_2-C_6)$-alkenyl, $(C_2-C_6)$-haloalkenyl, $(C_2-C_6)$-cyanoalkenyl, $(C_2-C_6)$-alkinyl, $(C_2-C_6)$-alkinyloxy-$(C_1-C_4)$-alkyl, $(C_2-C_6)$-haloalkinyl, $(C_1-C_6)$-alkoxy, $(C_1-C_6)$-haloalkoxy, $(C_1-C_6)$-haloalkoxy-$(C_1-C_6)$-alkyl, $(C_2-C_6)$-alkenyloxy-$(C_1-C_6)$-alkyl, $(C_2-C_6)$-haloalkenyloxy-$(C_1-C_6)$-alkyl, $(C_1-C_6)$-cyanoalkoxy, $(C_1-C_6)$-alkoxy-$(C_1-C_6)$-alkoxy, $(C_1-C_6)$-alkylhydroxyimino, $(C_1-C_6)$-alkoxyimino, $(C_1-C_6)$-alkyl- $(C_1-C_6)$-alkoxyimino, $(C_1-C_6)$-alkylthio, $(C_1-C_6)$-haloalkylthio, $(C_1-C_6)$-alkoxy-$(C_1-C_6)$-alkylthio, $(C_1-C_6)$-alkylthio-$(C_1-C_6)$-alkyl, $(C_1-C_6)$-alkylsulfinyl, $(C_1-C_6)$-haloalkylsulfinyl, $(C_1-C_6)$-alkoxy- $(C_1-C_6)$-alkylsulfinyl, $(C_1-C_6)$-alkylsulfinyl-$(C_1-C_6)$-alkyl, $(C_1-C_6)$-alkylsulfonyl, $(C_1-C_6)$-haloalkylsulfonyl, $(C_1-C_6)$-alkoxy-$(C_1-C_6)$-alkylsulfonyl, $(C_1-C_6)$-alkylsulfonyl-$(C_1-C_6)$-alkyl, $(C_1-C_6)$-alkylsulfonyloxy, $(C_1-C_6)$-alkylcarbonyl, $(C_1-C_6)$-alkylcarbonyl-$(C_1-C_6)$-alkyl, $(C_1-C_6)$-alkylthiocarbonyl, $(C_1-C_6)$-haloalkylcarbonyl, $(C_1-C_6)$-alkylcarbonyloxy, $(C_1-C_6)$-alkoxycarbonyl, $(C_1-C_6)$-haloalkoxycarbonyl, aminocarbonyl, $(C_1-C_6)$-alkylaminocarbonyl, $(C_1-C_6)$-alkylaminothiocarbonyl, di-$(C_1-C_6)$-alkylaminocarbonyl, di-$(C_1-C_6)$-alkylaminothiocarbonyl, $(C_3-C_8)$-cycloalkylaminocarbonyl, $(C_1-C_6)$-alkylsulfonylamino, $(C_1-C_6)$-alkylamino, di-$(C_1-C_6)$-alkylamino, aminosulfonyl, $(C_1-C_6)$-alkylaminosulfonyl, di-$(C_1-C_6)$-alkylaminosulfonyl, $(C_1-C_6)$-alkylsulfoximino, aminothiocarbonyl, $(C_1-C_6)$-alkylaminothiocarbonyl, di-$(C_1-C_6)$-alkylaminothiocarbonyl, $(C_3-C_8)$-cycloalkylamino, NHCO-$(C_1-C_6)$-alkyl($(C_1-C_6)$-alkylcarbonylamino),

or where the substituents independently of one another may be selected from phenyl or a 5- or 6-membered heteroaromatic ring, where phenyl or the ring may optionally be mono- or polysubstituted by identical or different substituents from the group consisting of $C_1-C_6$-alkyl, $C_2-C_6$-alkenyl, $C_2-C_6$-alkynyl, $C_3-C_6$-cycloalkyl, $C_1-C_6$-haloalkyl, $C_2-C_6$-haloalkenyl, $C_2-C_6$-haloalkynyl, $C_3-C_6$-halocycloalkyl, halogen, CN, $C_1-C_4$-alkoxy, $C_1-C_4$-haloalkoxy,

n represents 0, 1 or 2.

**3.** Compounds of the formula (I) according to Claim 1 in which

$A^1$ represents nitrogen or =C($R^4$)-,

$R^1$ represents $(C_1-C_4)$-alkyl, $(C_1-C_4)$-hydroxyalkyl, $(C_1-C_4)$-haloalkyl, $(C_2-C_4)$-alkenyl, $(C_2-C_4)$-haloalkenyl, $(C_2-C_4)$-alkynyl, $(C_2-C_4)$-haloalkynyl, $(C_3-C_6)$-cycloalkyl, $(C_1-C_4)$-alkylthio-$(C_1-C_4)$-alkyl, $(C_1-C_4)$-alkylsulfinyl-$(C_1-C_4)$-alkyl or $(C_1-C_4)$-alkylsulfonyl-$(C_1-C_4)$-alkyl,

$R^2$ represents a partially saturated or heteroaromatic fused bicyclic ring system from the series Q1 to Q152, where the ring system may optionally be mono-or polysubstituted by identical or different substituents, where the substituents independently of one another may be selected from the group consisting of: nitro, hydroxy, amino, cyano, halogen, $(C_1-C_4)$-alkyl, $(C_1-C_4)$-cycloalkyl, $(C_1-C_4)$-haloalkyl, $(C_1-C_4)$-alkoxy, $(C_1-C_4)$-haloalkoxy and $(C_1-C_4)$-haloalkylthio,

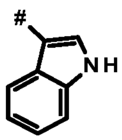
Q1

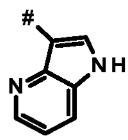
Q2

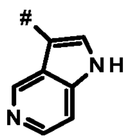
Q3

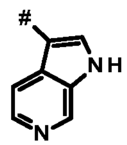
Q4

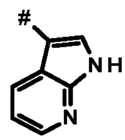
Q5

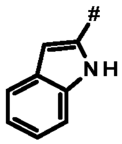
Q6

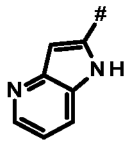
Q7

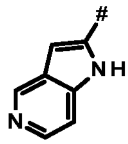
Q8

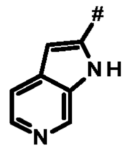
Q9

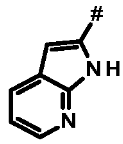
Q10

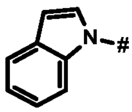
Q11

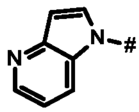
Q12

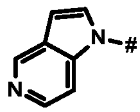
Q13

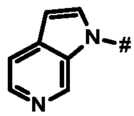
Q14

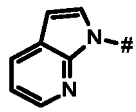
Q15

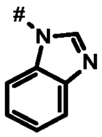
Q16

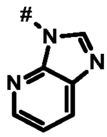
Q17

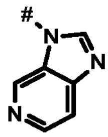
Q18

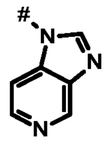
Q19

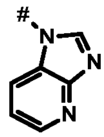
Q20

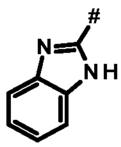
Q21

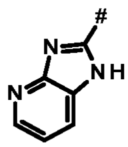
Q22

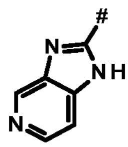
Q23

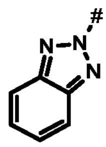
Q24

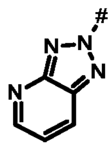
Q25

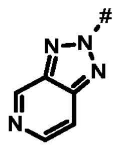
Q26

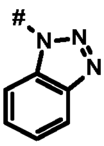
Q27

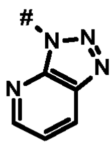
Q28

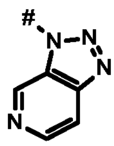
Q29

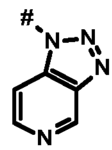
Q30

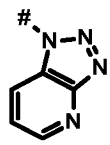
Q31

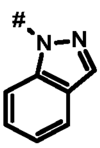
Q32

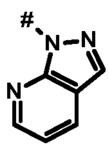
Q33

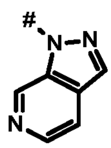
Q34

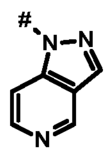
Q35

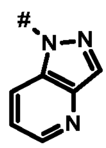
Q36

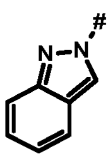
Q37

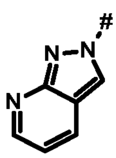
Q38

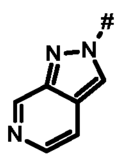
Q39

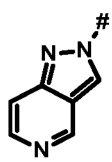
Q40

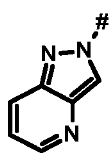
Q41

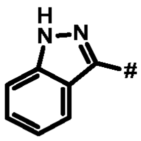
Q42
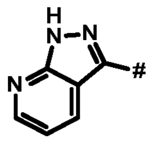
Q43
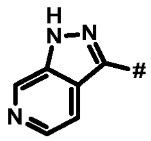
Q44
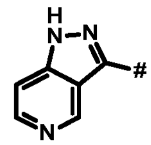
Q45
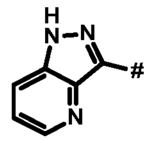
Q46

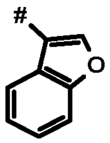
Q47
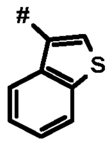
Q48
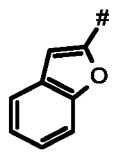
Q49
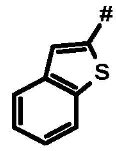
Q50

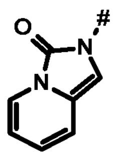
Q51
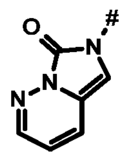
Q52
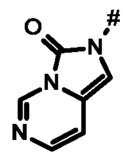
Q53
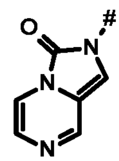
Q54
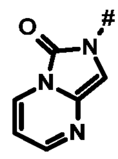
Q55

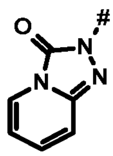
Q56
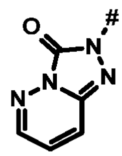
Q57
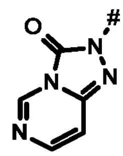
Q58
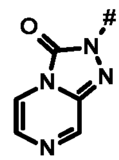
Q59
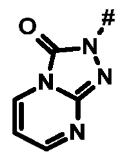
Q60

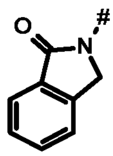
Q61
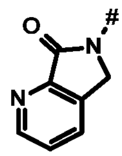
Q62
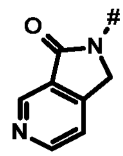
Q63
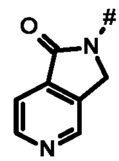
Q64
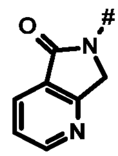
Q65

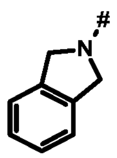
Q66
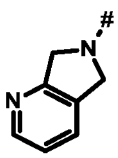
Q67
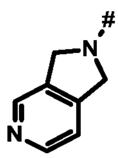
Q68
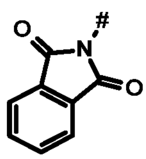
Q69
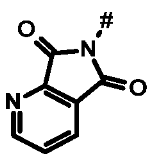
Q70
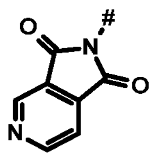
Q71

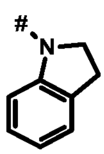
Q72
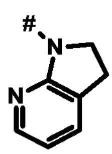
Q73
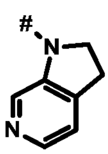
Q74
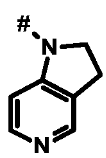
Q75
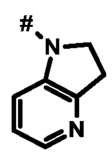
Q76

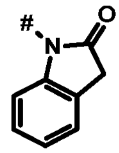
Q77
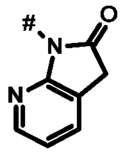
Q78
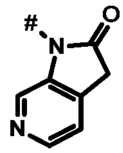
Q79
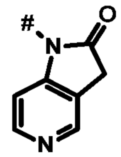
Q80
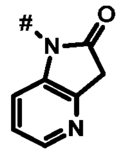
Q81

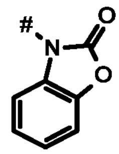
Q82
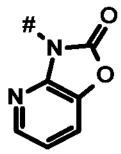
Q83
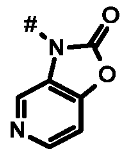
Q84
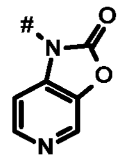
Q85
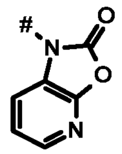
Q86

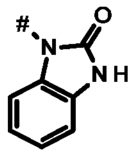
Q87
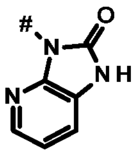
Q88
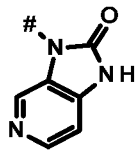
Q89
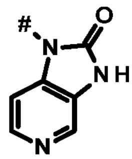
Q90
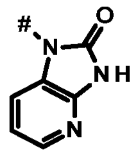
Q91

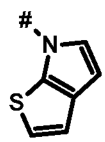
Q92
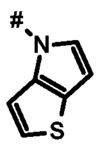
Q93
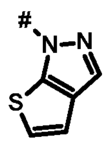
Q94
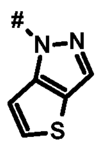
Q95
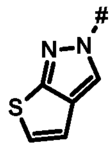
Q96
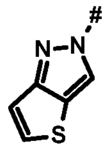
Q97

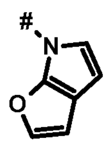
Q98
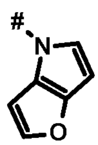
Q99
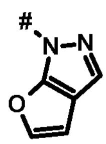
Q100
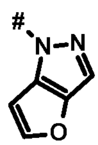
Q101
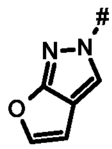
Q102
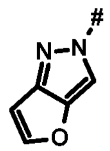
Q103

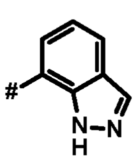
Q104
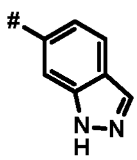
Q105
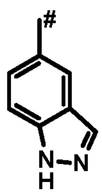
Q106
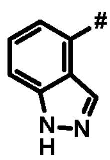
Q107
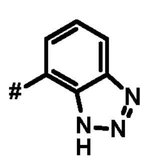
Q108
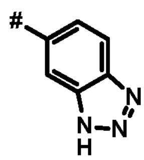
Q109

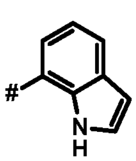
Q110
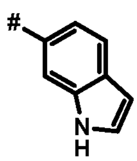
Q111
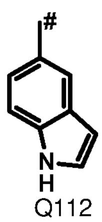
Q112
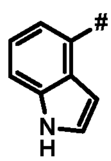
Q113
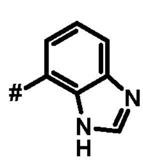
Q114
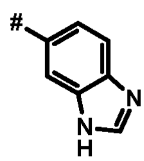
Q115

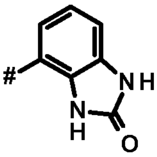 Q116
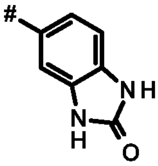 Q117
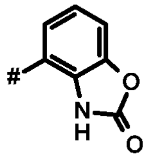 Q118
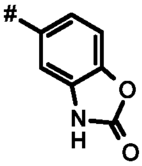 Q119
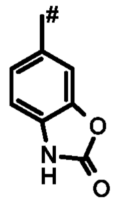 Q120
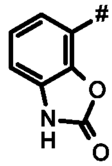 Q121

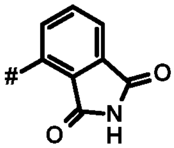 Q122
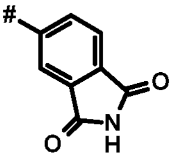 Q123
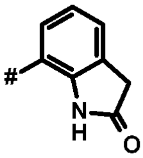 Q124
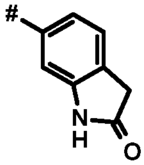 Q125
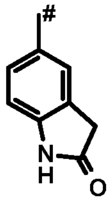 Q126
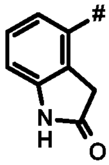 Q127

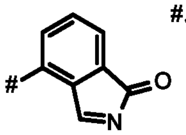 Q128
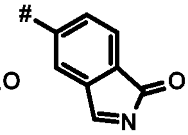 Q129
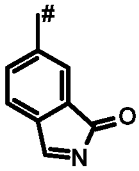 Q130
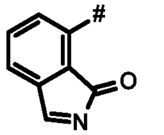 Q131
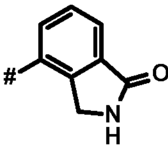 Q132
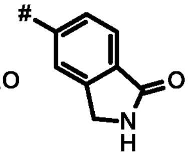 Q133

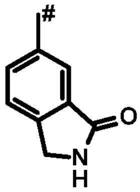 Q134
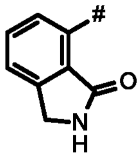 Q135

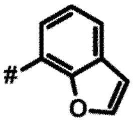 Q136
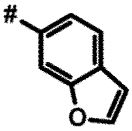 Q137
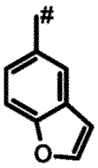 Q138
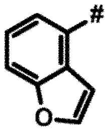 Q139
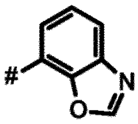 Q140
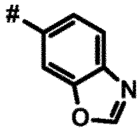 Q141

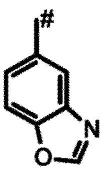 Q142
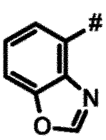 Q143
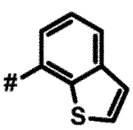 Q144
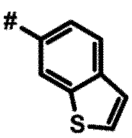 Q145
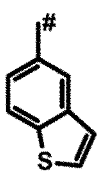 Q146
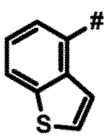 Q147

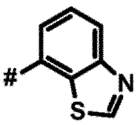 Q148
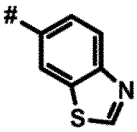 Q149
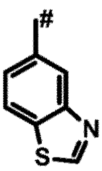 Q150
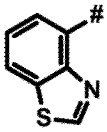 Q151
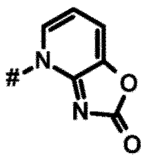 Q152

where the bond to the remainder of the molecule is identified by an asterisk #,

$R^3$ represents hydrogen, cyano, halogen, nitro, hydroxy, amino, SCN, tri-($C_1$-$C_4$)-alkylsilyl, ($C_3$-$C_6$)-cycloalkyl, ($C_3$-$C_6$)-cycloalkyl-($C_3$-$C_6$)-cycloalkyl, ($C_1$-$C_4$)-alkyl-($C_3$-$C_6$)-cycloalkyl, halo-($C_3$-$C_6$)-cycloalkyl, ($C_1$-$C_4$)-alkyl, ($C_1$-$C_4$)-haloalkyl, ($C_1$-$C_4$)-cyanoalkyl, ($C_1$-$C_4$)-alkoxy-($C_1$-$C_4$)-alkyl, ($C_2$-$C_4$)-alkenyl, ($C_2$-$C_4$)-haloalkenyl, ($C_2$-$C_4$)-cyanoalkenyl, ($C_2$-$C_4$)-alkynyl, ($C_2$-$C_4$)-haloalkynyl, ($C_2$-$C_4$)-cyanoalkynyl, ($C_1$-$C_4$)-alkoxy, ($C_1$-$C_4$)-haloalkoxy, ($C_1$-$C_4$)-cyanoalkoxy, ($C_1$-$C_4$)-alkylhydroxyimino, ($C_1$-$C_4$)-alkoxyimino, ($C_1$-$C_4$)-alkyl-($C_1$-$C_4$)-alkoxyimino, ($C_1$-$C_4$)-alkylthio, ($C_1$-$C_4$)-haloalkylthio, ($C_1$-$C_4$)-alkylsulfinyl, ($C_1$-$C_4$)-haloalkylsulfinyl, ($C_1$-$C_4$)-alkylsulfonyl, ($C_1$-$C_4$)-haloalkylsulfonyl, ($C_1$-$C_4$)-alkylsulfonyloxy, ($C_1$-$C_4$)-alkylcarbonyl, ($C_1$-$C_4$)-haloalkylcarbonyl, aminocarbonyl, ($C_1$-$C_4$)-alkylaminocarbonyl, di-($C_1$-$C_4$)-alkylaminocarbonyl, ($C_1$-$C_4$)-alkylsulfonylamino, ($C_1$-$C_4$)-alkylamino, di-($C_1$-$C_4$)-alkylamino, aminosulfonyl, ($C_1$-$C_4$)-alkylaminosulfonyl, di-($C_1$-$C_4$)-alkylaminosulfonyl or NHCO-($C_1$-$C_4$)-alkyl (($C_1$-$C_4$)-alkylcarbonylamino),

$R^4$ represents hydrogen, halogen, cyano or ($C_1$-$C_4$)-alkyl,

X represents a heteroaromatic 9-membered or 12-membered fused bicyclic or tricyclic ring system from the series H1 to H20,

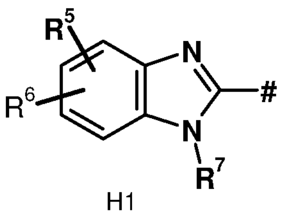
H1

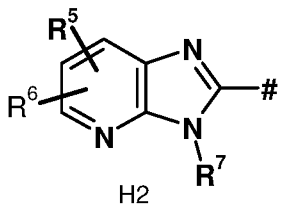
H2

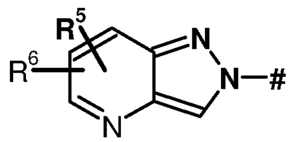
H3

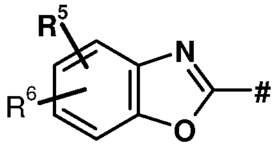
H4

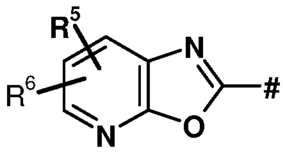
H5

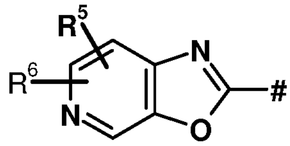
H6

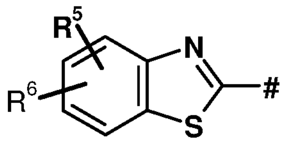
H7

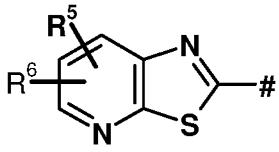
H8

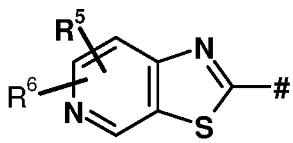
H9

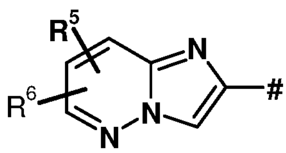
H10

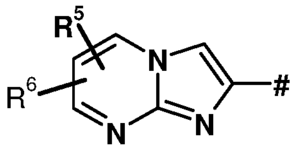
H11

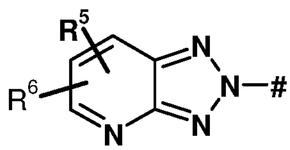
H12

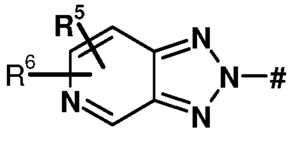
H13

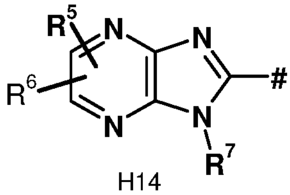
H14

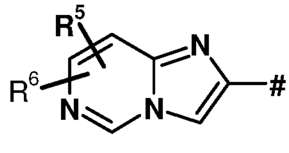
H15

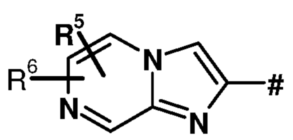
H16

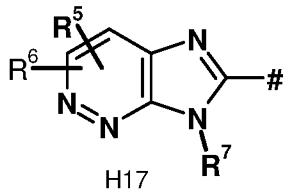
H17

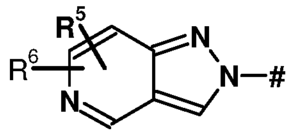
H18

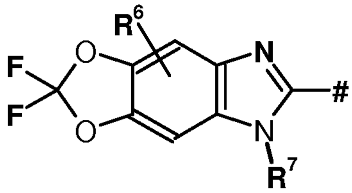
H19

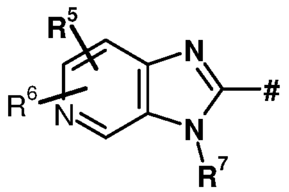
H20

$R^5$, $R^6$ independently of one another represent hydrogen, cyano, halogen, ($C_1$-$C_4$)-alkyl, ($C_1$-$C_4$)-haloalkyl, ($C_2$-$C_4$)-alkenyl, ($C_2$-$C_4$)-haloalkenyl, ($C_2$-$C_4$)-alkynyl, ($C_2$-$C_4$)-haloalkynyl, ($C_3$-$C_6$)-cycloalkyl, ($C_3$-$C_6$)-cycloalkyl-($C_3$-$C_6$)-cycloalkyl, ($C_1$-$C_4$)-alkyl-($C_3$-$C_6$)-cycloalkyl, ($C_1$-$C_4$)-alkoxy, ($C_1$-$C_4$)-haloalkoxy, ($C_1$-$C_4$)-alkoxyimino, ($C_1$-$C_4$)-alkylthio, ($C_1$-$C_4$)-haloalkylthio, ($C_1$-$C_4$)-alkylsulfinyl, ($C_1$-$C_4$)-haloalkylsulfinyl, ($C_1$-$C_4$)-alkylsulfonyl, ($C_1$-$C_4$)-haloalkylsulfonyl, ($C_1$-$C_4$)-alkylsulfonyloxy, ($C_1$-$C_4$)-alkylcarbonyl, ($C_1$-$C_4$)-haloalkylcarbonyl, aminocarbonyl, ($C_1$-$C_4$)-alkylaminocarbonyl, di-($C_1$-$C_4$)-alkylaminocarbonyl, ($C_1$-$C_4$)-alkylsulfonylamino, ($C_1$-$C_4$)-alkylamino, di-($C_1$-$C_4$)-alkylamino, aminosulfonyl, ($C_1$-$C_4$)-alkylaminosulfonyl or di-($C_1$-$C_4$)-alkylaminosulfonyl,

$R^7$ represents hydrogen, ($C_1$-$C_4$)-alkyl, ($C_1$-$C_4$)-haloalkyl, ($C_1$-$C_4$)-cyanoalkyl, ($C_1$-$C_4$)-hydroxyalkyl, ($C_1$-$C_4$)-alkoxy-($C_1$-$C_4$)-alkyl, ($C_1$-$C_4$)-haloalkoxy-($C_1$-$C_4$)-alkyl, ($C_2$-$C_4$)-alkenyl, ($C_2$-$C_4$)-alkenyloxy-($C_1$-$C_4$)-alkyl, ($C_2$-$C_4$)-haloalkenyloxy-($C_1$-$C_4$)-alkyl, ($C_2$-$C_4$)-haloalkenyl, ($C_2$-$C_4$)-cyanoalkenyl, ($C_2$-$C_4$)-alkynyl, ($C_2$-$C_4$)-alkynyloxy-($C_1$-$C_4$)-alkyl, ($C_2$-$C_4$)-haloalkynyl, ($C_3$-$C_6$)-cycloalkyl, ($C_3$-$C_6$)-cycloalkyl-($C_3$-$C_6$)-cycloalkyl, ($C_1$-$C_4$)-alkyl-($C_3$-$C_6$)-cycloalkyl, halo($C_3$-$C_6$)-cycloalkyl, ($C_1$-$C_4$)-alkylthio-($C_1$-$C_4$)-alkyl, ($C_1$-$C_4$)-alkylsulfinyl-($C_1$-$C_4$)-alkyl, ($C_1$-$C_4$)-alkylsulfonyl-($C_1$-$C_4$)-alkyl or ($C_1$-$C_4$)-alkylcarbonyl-($C_1$-$C_4$)-alkyl,

n represents 0, 1 or 2.

**4.** Compounds of the formula (I) according to Claim 1 in which

$A^1$ represents nitrogen,

$R^1$ represents methyl, ethyl, n-propyl, isopropyl, cyclopropyl, n-butyl, isobutyl, tert-butyl, cyclobutyl, fluoromethyl, difluoromethyl, trifluoromethyl, fluoroethyl, difluoroethyl, trifluoroethyl, tetrafluoroethyl or pentafluoroethyl,

$R^2$ represents a fused bicyclic ring system, optionally mono- or disubstituted by identical or different substituents, from the series Q1, Q2, Q3, Q4, Q5, Q6, Q7, Q8, Q9, Q10, Q1, Q12, Q13, Q14, Q15, Q16, Q17, Q18, Q19, Q20, Q21, Q22, Q23, Q27, Q28, Q29, Q30, Q31, Q32, Q33, Q34, Q35, Q36, Q37, Q38, Q39, Q40, Q41, Q42, Q43, Q44, Q45, Q46, Q51, Q52, Q53, Q54, Q55, Q56, Q57, Q58, Q59, Q60, Q61, Q62, Q63, Q64, Q65, Q66, Q67, Q68, Q69, Q70, Q71, Q77, Q78, Q79, Q80, Q81, Q82, Q83, Q84, Q85, Q86, Q87, Q88, Q89, Q90, Q91, Q92, Q93, Q94, Q95, Q96, Q97, Q104, Q105, Q106, Q107, Q108, Q109, Q114, Q115, Q116, Q117, Q118, Q119, Q120, Q121, Q124, Q125, Q126, Q127, Q128, Q129, Q130, Q131, Q132, Q133, Q134, Q135, Q136, Q137, Q138, Q139, Q140, Q141, Q142, Q143, Q144, Q145, Q146, Q147, Q148, Q149, Q150, Q151, Q152, and where possible substituents are in each case as follows: cyano, fluorine, chlorine, bromine, methyl, ethyl, n-propyl, isopropyl, cyclopropyl, trifluoromethyl, pentafluoroethyl, methoxy, ethoxy, trifluoromethoxy, trifluoroethoxy, pentafluoroethoxy or trifluoromethylthio,

$R^3$ represents hydrogen, cyano, halogen, ($C_1$-$C_4$)-alkyl, ($C_1$-$C_4$)-haloalkyl, ($C_1$-$C_4$)-haloalkoxy, ($C_1$-$C_4$)-alkylthio, ($C_1$-$C_4$)-alkylsulfinyl, ($C_1$-$C_4$)-alkylsulfonyl, ($C_1$-$C_4$)-haloalkylthio, ($C_1$-$C_4$)-haloalkylsulfinyl, ($C_1$-$C_4$)-haloalkylsulfonyl or NHCO-($C_1$-$C_4$)-alkyl (($C_1$-$C_4$)-alkylcarbonylamino),

X represents a heteroaromatic 9-membered or 12-membered fused bicyclic or tricyclic ring system from the series H1, H2, H3, H4, H5, H6, H7, H8, H9, H10, H1, H12, H13, H14, H15, H16, H17, H18, H19 and H20,

$R^5$ represents fluorine, chlorine, fluoromethyl, difluoromethyl, trifluoromethyl, fluoroethyl, difluoroethyl, trifluoroethyl, tetrafluoroethyl, pentafluoroethyl, trifluoromethoxy, difluorochloromethoxy, dichlorofluoromethoxy, trifluoromethylthio, trifluoromethylsulfonyl or trifluoromethylsulfinyl,

$R^6$ represents hydrogen,
$R^7$ represents hydrogen, methyl, ethyl, n-propyl, isopropyl, n-butyl, isobutyl, tert-butyl, methoxymethyl or methoxyethyl,
n represents 0, 1 or 2.

**5.** Compounds of the formula (I) according to Claim 1 in which

$A^1$ represents nitrogen,
$R^1$ represents methyl, ethyl, n-propyl, isopropyl, cyclopropyl, n-butyl, isobutyl, tert-butyl, cyclobutyl, fluoromethyl, difluoromethyl, trifluoromethyl, fluoroethyl, difluoroethyl, trifluoroethyl, tetrafluoroethyl or pentafluoroethyl,
$R^2$ represents a fused bicyclic ring system, optionally mono- or disubstituted by identical or different substituents, from the series Q1, Q6, Q1, Q12, Q15, Q16, Q17, Q20, Q27, Q28, Q32, Q33, Q34, Q35, Q37, Q38, Q51, Q56, Q57, Q61, Q62, Q66, Q67, Q69, Q82, Q87, Q105, Q106, Q107 and Q152, and where possible substituents are in each case as follows: cyano, fluorine, chlorine, bromine, methyl, ethyl, n-propyl, isopropyl, cyclopropyl, trifluoromethyl, pentafluoroethyl, methoxy, ethoxy, trifluoromethoxy, trifluoroethoxy, pentafluoroethoxy or trifluoromethylthio,
$R^3$ represents hydrogen, cyano, halogen, $(C_1-C_4)$-alkyl, $(C_1-C_4)$-haloalkyl, $(C_1-C_4)$-haloalkoxy, $(C_1-C_4)$-alkylthio, $(C_1-C_4)$-alkylsulfinyl, $(C_1-C_4)$-alkylsulfonyl, $(C_1-C_4)$-haloalkylthio, $(C_1-C_4)$-haloalkylsulfinyl, $(C_1-C_4)$-haloalkylsulfonyl or NHCO-$(C_1-C_4)$-alkyl (($C_1-C_4$)-alkylcarbonylamino),
X represents a heteroaromatic 9-membered or 12-membered fused bicyclic or tricyclic ring system from the series H1, H2, H3, H4, H5, H6, H7, H8, H9, H10, H1, H12, H13, H14, H15, H16, H17, H18, H19 and H20,
$R^5$ represents fluorine, chlorine, fluoromethyl, difluoromethyl, trifluoromethyl, fluoroethyl, difluoroethyl, trifluoroethyl, tetrafluoroethyl, pentafluoroethyl, trifluoromethoxy, difluorochloromethoxy, dichlorofluoromethoxy, trifluoromethylthio, trifluoromethylsulfonyl or trifluoromethylsulfinyl,
$R^6$ represents hydrogen,
$R^7$ represents hydrogen, methyl, ethyl, n-propyl, isopropyl, n-butyl, isobutyl, tert-butyl, methoxymethyl or methoxyethyl,
n represents 0, 1 or 2.

**6.** Compounds of the formula (I) according to Claim 1 in which

$A^1$ represents nitrogen,
$R^1$ represents ethyl,
$R^2$ represents a fused bicyclic ring system from the series Q1, Q6, Q1, Q12, Q15, Q16, Q17, Q20, Q27, Q28, Q32, Q33, Q34, Q35, Q37, Q38, Q51, Q56, Q57, Q61, Q62, Q66, Q67, Q69, Q82, Q87, Q105, Q106, Q107 and Q152 which is mono- or disubstituted by identical or different substituents from the group consisting of chlorine, bromine, cyano, methyl, methoxy and trifluoromethyl,
$R^3$ represents hydrogen,
X represents a heteroaromatic ring system from the series H20,
$R^5$ represents trifluoromethyl,
$R^6$ represents hydrogen,
$R^7$ represents methyl,
n represents 1 or 2.

**7.** Agrochemical formulation comprising compounds of the formula (I) according to Claim 1 and also extenders and/or surfactants.

**8.** Agrochemical formulation according to Claim 7, additionally comprising a further agrochemically active compound.

**9.** Method for controlling animal pests, **characterized in that** a compound of the formula (I) according to Claim 1 or an agrochemical formulation according to Claim 7 or 8 is allowed to act on the animal pests and/or their habitat.

**10.** Use of compounds of the formula (I) according to Claim 1 or of agrochemical formulations according to either of Claims 7 or 8 for controlling animal pests

## Revendications

**1.** Composés de formule (I)

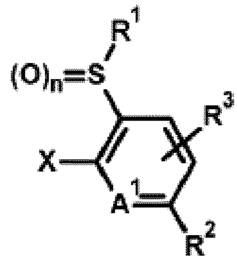

(I)

dans laquelle

$A^1$ représente azote, $=N^+(O^-)$- ou $=C(R^4)$-,

$R^1$ représente $(C_1-C_6)$-alkyle, $(C_1-C_6)$-halogénoalkyle, $(C_1-C_6)$-cyanoalkyle, $(C_1-C_6)$-hydroxyalkyle, $(C_1-C_6)$-alcoxy-$(C_1-C_6)$-alkyle, $(C_1-C_6)$-halogénoalcoxy-$(C_1-C_6)$-alkyle, $(C_2-C_6)$-alcényle, $(C_2-C_6)$-alcényloxy-$(C_1-C_6)$-alkyle, $(C_2-C_6)$-halogénoalcényloxy-$(C_1-C_6)$-alkyle, $(C_2-C_6)$-halogénoalcényle, $(C_2-C_6)$-cyanoalcényle, $(C_2-C_6)$-alcynyle, $(C_2-C_6)$-alcynyloxy-$(C_1-C_6)$-alkyle, $(C_2-C_6)$-halogénoalcynyloxy-$(C_1-C_6)$-alkyle, $(C_2-C_6)$-halogénoalcynyle, $(C_2-C_6)$-cyanoalcynyle, $(C_3-C_8)$-cycloalkyle, $(C_3-C_8)$-cycloalkyl-$(C_3-C_8)$-cycloalkyle, $(C_1-C_6)$-alkyl-$(C_3-C_8)$-cycloalkyle, halogéno-$(C_3-C_8)$-cycloalkyle, amino, $(C_1-C_6)$-alkylamino, di-$(C_1-C_6)$-alkyl-amino, $(C_3-C_8)$-cycloalkylamino, $(C_1-C_6)$-alkylcarbonylamino, $(C_1-C_6)$-alkylthio-$(C_1-C_6)$-alkyle, $(C_1-C_6)$-halogénoalkylthio-$(C_1-C_6)$-alkyle, $(C_1-C_6)$-alkylsulfinyl-$(C_1-C_6)$-alkyle, $(C_1-C_6)$-halogénoalkylsulfinyl-$(C_1-C_6)$-alkyle, $(C_1-C_6)$-alkylsulfonyl-$(C_1-C_6)$-alkyle, $(C_1-C_6)$-halogénoalkylsulfonyl-$(C_1-C_6)$-alkyle, $(C_1-C_6)$-alcoxy-$(C_1-C_6)$-alkylthio-$(C_1-C_6)$-alkyle, $(C_1-C_6)$-alcoxy-$(C_1-C_6)$-alkylsulfinyl-$(C_1-C_6)$-alkyle, $(C_1-C_6)$-alcoxy-$(C_1-C_6)$-alkylsulfonyl-$(C_1-C_6)$-alkyle, $(C_1-C_6)$-alkylcarbonyl-$(C_1-C_6)$-alkyle, $(C_1-C_6)$-halogénoalkylcarbonyl-$(C_1-C_6)$-alkyle, $(C_1-C_6)$-alcoxycarbonyl-$(C_1-C_6)$-alkyle, $(C_1-C_6)$-halogénoalcoxycarbonyl-$(C_1-C_6)$-alkyle, $(C_1-C_6)$-alkylsulfonylamino, aminosulfonyl-$(C_1-C_6)$-alkyle, $(C_1-C_6)$-alkylaminosulfonyl-$(C_1-C_6)$-alkyle, di-$(C_1-C_6)$-alkylaminosulfonyl-$(C_1-C_6)$-alkyle,

ou représente $(C_1-C_6)$-alkyle, $(C_1-C_6)$-alcoxy, $(C_2-C_6)$-alcényle, $(C_2-C_6)$-alcynyle, $(C_3-C_8)$-cycloalkyle, à chaque fois éventuellement substitué une ou plusieurs fois, de manière identique ou différente par aryle, hétaryle ou hétérocyclyle, aryle, hétaryle ou hétérocyclyle pouvant à chaque fois être éventuellement substitué une ou plusieurs fois, de manière identique ou différente par halogène, cyano, nitro, hydroxy, amino, carboxy, carbamoyle, aminosulfonyle, $(C_1-C_6)$-alkyle, $(C_3-C_6)$-cycloalkyle, $(C_1-C_6)$-alcoxy, $(C_1-C_6)$-halogénoalkyle, $(C_1-C_6)$-halogénoalcoxy, $(C_1-C_6)$-alkylthio, $(C_1-C_6)$-alkylsulfinyle, $(C_1-C_6)$-alkylsulfonyle, $(C_1-C_6)$-alkylsulfimino, $(C_1-C_6)$-alkylsulfimino-$(C_1-C_6)$-alkyle, $(C_1-C_6)$-alkylsulfimino-$(C_2-C_6)$-alkylcarbonyle, $(C_1-C_6)$-alkylsulfoximino, $(C_1-C_6)$-alkylsulfoximino-$(C_1-C_6)$-alkyle, $(C_1-C_6)$-alkylsulfoximino-$(C_2-C_6)$-alkylcarbonyle, $(C_1-C_6)$-alcoxycarbonyle, $(C_1-C_6)$-alkylcarbonyle, $(C_3-C_6)$-trialkylsilyle ou benzyle, ou

$R^1$ représente aryle, hétaryle ou hétérocyclyle à chaque fois éventuellement substitué une ou plusieurs fois, de manière identique ou différente par halogène, cyano, nitro, hydroxy, amino, carboxy, carbamoyle, $(C_1-C_6)$-alkyle, $(C_3-C_8)$-cycloalkyle, $(C_1-C_6)$-alcoxy, $(C_1-C_6)$-halogénoalkyle, $(C_1-C_6)$-halogénoalcoxy, $(C_1-C_6)$-alkylthio, $(C_1-C_6)$-alkylsulfinyle, $(C_1-C_6)$-alkylsulfonyle, $(C_1-C_6)$-alkylsulfimino, $(C_1-C_6)$-alkylsulfimino-$(C_1-C_6)$-alkyle, $(C_1-C_6)$-alkylsulfimino-$(C_2-C_6)$-alkylcarbonyle, $(C_1-C_6)$-alkylsulfoximino, $(C_1-C_6)$-alkylsulfoximino-$(C_1-C_6)$-alkyle, $(C_1-C_6)$-alkylsulfoximino-$(C_2-C_6)$-alkylcarbonyle, $(C_1-C_6)$-alcoxycarbonyle, $(C_1-C_6)$-alkylcarbonyle, $(C_3-C_6)$-trialkylsilyle, (=O) (seulement dans le cas d'hétérocyclyle) ou $(=O)_2$ (seulement dans le cas d'hétérocyclyle),

$R^2$ représente un système cyclique bicyclique annelé hétérocyclique ou hétéroaromatique, partiellement saturé ou saturé, à 8, 9, 10, 11 ou 12 chaînons, éventuellement au moins un groupe carbonyle pouvant être contenu et/ou le système cyclique pouvant éventuellement être substitué une ou plusieurs fois, de manière identique ou différente, et les substituants pouvant être choisis indépendamment les uns des autres parmi cyano, carboxyle, halogène, nitro, acétyle, hydroxy, amino, SCN, tri-$(C_1-C_6)$-alkylsilyle, $(C_1-C_6)$-alkyle, $(C_1-C_6)$-halogénoalkyle, $(C_1-C_6)$-cyanoalkyle, $(C_1-C_6)$-hydroxyalkyle, hydroxycarbonyl-$(C_1-C_6)$-alcoxy, $(C_1-C_6)$-alcoxycarbonyl-$(C_1-C_6)$-alkyle, $(C_1-C_6)$-alcoxy-$(C_1-C_6)$-alkyle, $(C_2-C_6)$-alcényle, $(C_2-C_6)$-halogénoalcényle, $(C_2-C_6)$-cyanoalcényle, $(C_2-C_6)$-alcynyle, $(C_2-C_6)$-halogénoalcynyle, $(C_2-C_6)$-cyanoalcynyle, $(C_3-C_6)$-cycloalkyle, $(C_3-C_6)$-cycloalkyl-$(C_3-C_6)$-cycloalkyle, $(C_1-C_4)$-alkyl-$(C_3-C_6)$-cycloalkyle, $(C_1-C_6)$-alcoxy, $(C_1-C_6)$-halogénoalcoxy, $(C_1-C_6)$-cyanoalcoxy, $(C_1-C_6)$-alcoxycarbonyl-$(C_1-C_6)$-alcoxy, $(C_1-C_6)$-alcoxy-$(C_1-C_6)$-alcoxy, $(C_1-C_6)$-al-

coxyimino, -N=C(H)-O($C_1$-$C_6$)-alkyle, -C(H)=N-O($C_1$-$C_6$)-alkyle, ($C_1$-$C_6$)-halogénoalkyl-($C_1$-$C_6$)-alcoxyimino, ($C_1$-$C_6$)-alkylthio, ($C_1$-$C_6$)-halogénoalkylthio, ($C_1$-$C_6$)-alcoxy- ($C_1$-$C_6$)-alkylthio, ($C_1$-$C_6$)-alkylthio- ($C_1$-$C_6$)-alkyle, ($C_1$-$C_6$)-alkylsulfinyle, ($C_1$-$C_6$)-halogénoalkylsulfinyle, ($C_1$-$C_6$)-alcoxy-($C_1$-$C_6$)-alkylsulfinyle, ($C_1$-$C_6$)-alkylsulfinyl-($C_1$-$C_6$)-alkyle, ($C_1$-$C_6$)-alkylsulfonyle, ($C_1$-$C_6$)-halogénoalkylsulfonyle, ($C_1$-$C_6$)-alcoxy-($C_1$-$C_6$)-alkylsulfonyle, ($C_1$-$C_6$)-alkylsulfonyl-($C_1$-$C_6$)-alkyle, ($C_1$-$C_6$)-alkylsulfonyloxy, ($C_1$-$C_6$)-alkylcarbonyle, ($C_1$-$C_6$)-halogénoalkylcarbonyle, ($C_1$-$C_6$)-alkylcarbonyloxy, ($C_1$-$C_6$)-alcoxycarbonyle, ($C_1$-$C_6$)-halogénoalcoxycarbonyle, aminocarbonyle, ($C_1$-$C_6$)-alkylaminocarbonyle, di-($C_1$-$C_6$)-alkylaminocarbonyle, ($C_2$-$C_6$)-alcénylaminocarbonyle, di-($C_2$-$C_6$)-alcénylaminocarbonyle, ($C_3$-$C_8$)-cycloalkylaminocarbonyle, ($C_1$-$C_6$)-alkylsulfonylamino, ($C_1$-$C_6$)-alkylamino, di-($C_1$-$C_6$)-alkylamino, aminosulfonyle, ($C_1$-$C_6$)-alkylaminosulfonyle, di-($C_1$-$C_6$)-alkylaminosulfonyle, ($C_1$-$C_6$)-alkylsulfoximino, aminothiocarbonyle, ($C_1$-$C_6$)-alkylaminothiocarbonyle, di-($C_1$-$C_6$)-alkylaminothiocarbonyle, ($C_3$-$C_8$)-cycloalkylamino, ($C_1$-$C_6$)-alkylcarbonylamino,

$R^3$ représente hydrogène, cyano, halogène, nitro, acétyle, hydroxy, amino, SCN, tri-($C_1$-$C_6$)-alkylsilyle, ($C_3$-$C_8$)-cycloalkyle, ($C_3$-$C_8$)-cycloalkyl-($C_3$-$C_8$)-cycloalkyle, ($C_1$-$C_6$)-alkyl-($C_3$-$C_8$)-cycloalkyle, halogéno-($C_3$-$C_8$)-cycloalkyle, ($C_1$-$C_6$)-alkyle, ($C_1$-$C_6$)-halogénoalkyle, ($C_1$-$C_6$)-cyanoalkyle, ($C_1$-$C_6$)-hydroxyalkyle, hydroxycarbonyl-($C_1$-$C_6$)-alcoxy, ($C_1$-$C_6$)-alcoxycarbonyl-($C_1$-$C_6$)-alkyle, ($C_1$-$C_6$)-alcoxy-($C_1$-$C_6$)-alkyle, ($C_2$-$C_6$)-alcényle, ($C_2$-$C_6$)-halogénoalcényle, ($C_2$-$C_6$)-cyanoalcényle, ($C_2$-$C_6$)-alcynyle, ($C_2$-$C_6$)-halogénoalcynyle, ($C_2$-$C_6$)-cyanoalcynyle, ($C_1$-$C_6$)-alcoxy, ($C_1$-$C_6$)-halogénoalcoxy, ($C_1$-$C_6$)-cyanoalcoxy, ($C_1$-$C_6$)-alcoxycarbonyl-($C_1$-$C_6$)-alcoxy, ($C_1$-$C_6$)-alcoxy-($C_1$-$C_6$)-alcoxy, ($C_1$-$C_6$)-alkylhydroxyimino, ($C_1$-$C_6$)-alcoxyimino, ($C_1$-$C_6$)-alkyl- ($C_1$-$C_6$)-alcoxyimino, ($C_1$-$C_6$)-halogénoalkyl-($C_1$-$C_6$)-alcoxyimino, ($C_1$-$C_6$)-alkylthio, ($C_1$-$C_6$)-halogénoalkylthio, ($C_1$-$C_6$)-alcoxy- ($C_1$-$C_6$)-alkylthio, ($C_1$-$C_6$)-alkylthio- ($C_1$-$C_6$)-alkyle, ($C_1$-$C_6$)-alkylsulfinyle, ($C_1$-$C_6$)-halogénoalkylsulfinyle, ($C_1$-$C_6$)-alcoxy- ($C_1$-$C_6$)-alkylsulfinyle, ($C_1$-$C_6$)-alkylsulfinyl-($C_1$-$C_6$)-alkyle, ($C_1$-$C_6$)-alkylsulfonyle, ($C_1$-$C_6$)-halogénoalkylsulfonyle, ($C_1$-$C_6$)-alcoxy- ($C_1$-$C_6$)-alkylsulfonyle, ($C_1$-$C_6$)-alkylsulfonyl-($C_1$-$C_6$)-alkyle, ($C_1$-$C_6$)-alkylsulfonyloxy, ($C_1$-$C_6$)-alkylcarbonyle, ($C_1$-$C_6$)-alkylthiocarbonyle, ($C_1$-$C_6$)-halogénoalkylcarbonyle, ($C_1$-$C_6$)-alkylcarbonyloxy, ($C_1$-$C_6$)-alcoxycarbonyle, ($C_1$-$C_6$)-halogénoalcoxycarbonyle, aminocarbonyle, ($C_1$-$C_6$)-alkylaminocarbonyle, ($C_1$-$C_6$)-alkylaminothiocarbonyle, di-($C_1$-$C_6$)-alkylaminocarbonyle, di-($C_1$-$C_6$)-alkylaminothiocarbonyle, ($C_2$-$C_6$)-alcénylaminocarbonyle, di-($C_2$-$C_6$)-alcénylaminocarbonyle, ($C_3$-$C_8$)-cycloalkylaminocarbonyle, ($C_1$-$C_6$)-alkylsulfonylamino, ($C_1$-$C_6$)-alkylamino, di-($C_1$-$C_6$)-alkylamino, aminosulfonyle, ($C_1$-$C_6$)-alkylaminosulfonyle, di-($C_1$-$C_6$)-alkylaminosulfonyle, ($C_1$-$C_6$)-alkylsulfoximino, aminothiocarbonyle, ($C_1$-$C_6$)-alkylaminothiocarbonyle, di-($C_1$-$C_6$)-alkylaminothiocarbonyle, ($C_3$-$C_8$)-cycloalkylamino, NHCO-($C_1$-$C_6$)-alkyle (($C_1$-$C_6$)-alkylcarbonylamino), représente aryle ou hétaryle à chaque fois éventuellement substitué une ou plusieurs fois, de manière identique ou différente, (dans le cas d'hétaryle) au moins un groupe carbonyle pouvant éventuellement être contenu et/ou les substituants pouvant être à chaque fois : cyano, carboxyle, halogène, nitro, acétyle, hydroxy, amino, SCN, tri-($C_1$-$C_6$)-alkylsilyle, ($C_3$-$C_8$)-cycloalkyle, ($C_3$-$C_8$)-cycloalkyl- ($C_3$-$C_8$)-cycloalkyle, ($C_1$-$C_6$)-alkyl-($C_3$-$C_8$)-cycloalkyle, halogéno-($C_3$-$C_8$)-cycloalkyle, ($C_1$-$C_6$)-alkyle, ($C_1$-$C_6$)-halogénoalkyle, ($C_1$-$C_6$)-cyanoalkyle, ($C_1$-$C_6$)-hydroxyalkyle, hydroxycarbonyl-($C_1$-$C_6$)-alcoxy, ($C_1$-$C_6$)-alcoxycarbonyl-($C_1$-$C_6$)-alkyle, ($C_1$-$C_6$)-alcoxy- ($C_1$-$C_6$)-alkyle, ($C_2$-$C_6$)-alcényle, ($C_2$-$C_6$)-halogénoalcényle, ($C_2$-$C_6$)-cyanoalcényle, ($C_2$-$C_6$)-alcynyle, ($C_2$-$C_6$)-halogénoalcynyle, ($C_2$-$C_6$)-cyanoalcynyle, ($C_1$-$C_6$)-alcoxy, ($C_1$-$C_6$)-halogénoalcoxy, ($C_1$-$C_6$)-cyanoalcoxy, ($C_1$-$C_6$)-alcoxycarbonyl-($C_1$-$C_6$)-alcoxy, ($C_1$-$C_6$)-alcoxy-($C_1$-$C_6$)-alcoxy, ($C_1$-$C_6$)-alkylhydroxyimino, ($C_1$-$C_6$)-alcoxyimino, ($C_1$-$C_6$)-alkyl-($C_1$-$C_6$)-alcoxyimino, ($C_1$-$C_6$)-halogénoalkyl-($C_1$-$C_6$)-alcoxyimino, ($C_1$-$C_6$)-alkylthio, ($C_1$-$C_6$)-halogénoalkylthio, ($C_1$-$C_6$)-alcoxy- ($C_1$-$C_6$)-alkylthio, ($C_1$-$C_6$)-alkylthio- ($C_1$-$C_6$)-alkyle, ($C_1$-$C_6$)-alkylsulfinyle, ($C_1$-$C_6$)-halogénoalkylsulfinyle, ($C_1$-$C_6$)-alcoxy- ($C_1$-$C_6$)-alkylsulfinyle, ($C_1$-$C_6$)-alkylsulfinyl-($C_1$-$C_6$)-alkyle, ($C_1$-$C_6$)-alkylsulfonyle, ($C_1$-$C_6$)-halogénoalkylsulfonyle, ($C_1$-$C_6$)-alcoxy- ($C_1$-$C_6$)-alkylsulfonyle, ($C_1$-$C_6$)-alkylsulfonyl-($C_1$-$C_6$)-alkyle, ($C_1$-$C_6$)-alkylsulfonyloxy, ($C_1$-$C_6$)-alkylcarbonyle, ($C_1$-$C_6$)-halogénoalkylcarbonyle, ($C_1$-$C_6$)-alkylcarbonyloxy, ($C_1$-$C_6$)-alcoxycarbonyle, ($C_1$-$C_6$)-halogénoalcoxycarbonyle, aminocarbonyle, ($C_1$-$C_6$)-alkylaminocarbonyle, di-($C_1$-$C_6$)-alkylaminocarbonyle, ($C_2$-$C_6$)-alcénylaminocarbonyle, di-($C_2$-$C_6$)-alcénylaminocarbonyle, ($C_3$-$C_8$)-cycloalkylaminocarbonyle, ($C_1$-$C_6$)-alkylsulfonylamino, ($C_1$-$C_6$)-alkylamino, di-($C_1$-$C_6$)-alkylamino, aminosulfonyle, ($C_1$-$C_6$)-alkylaminosulfonyle, di-($C_1$-$C_6$)-alkylaminosulfonyle, ($C_1$-$C_6$)-alkylsulfoximino, aminothiocarbonyle, ($C_1$-$C_6$)-alkylaminothiocarbonyle, di-($C_1$-$C_6$)-alkylaminothiocarbonyle, ($C_3$-$C_8$)-cycloalkylamino, ($C_1$-$C_6$)-alkylcarbonylamino,

$R^4$ représente hydrogène, cyano, halogène, nitro, acétyle, hydroxy, amino, SCN, tri-($C_1$-$C_6$)-alkylsilyle, ($C_3$-$C_8$)-cycloalkyle, ($C_3$-$C_8$)-cycloalkyl- ($C_3$-$C_8$)-cycloalkyle, ($C_1$-$C_6$)-alkyl-($C_3$-$C_8$)-cycloalkyle, halogéno-($C_3$-$C_8$)-cycloalkyle, ($C_1$-$C_6$)-alkyle, ($C_1$-$C_6$)-halogénoalkyle, ($C_1$-$C_6$)-cyanoalkyle, ($C_1$-$C_6$)-hydroxyalkyle, hydroxycarbonyl-($C_1$-$C_6$)-alcoxy, ($C_1$-$C_6$)-alcoxycarbonyl-($C_1$-$C_6$)-alkyle, ($C_1$-$C_6$)-alcoxy- ($C_1$-$C_6$)-alkyle, ($C_2$-$C_6$)-alcényle, ($C_2$-$C_6$)-halogénoalcényle, ($C_2$-$C_6$)-cyanoalcényle, ($C_2$-$C_6$)-alcynyle, ($C_2$-$C_6$)-halogénoalcynyle, ($C_2$-$C_6$)-cyanoalcynyle, ($C_1$-$C_6$)-alcoxy, ($C_1$-$C_6$)-halogénoalcoxy, ($C_1$-$C_6$)-cyanoalcoxy, ($C_1$-$C_6$)-alcoxycarbonyl-($C_1$-$C_6$)-alcoxy, ($C_1$-$C_6$)-alcoxy-($C_1$-$C_6$)-alcoxy, ($C_1$-$C_6$)-alkylhydroxyimino, ($C_1$-$C_6$)-alcoxyimino, ($C_1$-$C_6$)-alkyl- ($C_1$-$C_6$)-alcoxyimino, ($C_1$-$C_6$)-halogénoalkyl-($C_1$-$C_6$)-alcoxyimino, ($C_1$-$C_6$)-alkylthio,

($C_1$-$C_6$)-halogénoalkylthio, ($C_1$-$C_6$)-alcoxy- ($C_1$-$C_6$)-alkylthio, ($C_1$-$C_6$)-alkylthio- ($C_1$-$C_6$)-alkyle, ($C_1$-$C_6$)-alkylsulfinyle, ($C_1$-$C_6$)-halogénoalkylsulfinyle, ($C_1$-$C_6$)-alcoxy- ($C_1$-$C_6$)-alkylsulfinyle, ($C_1$-$C_6$)-alkylsulfinyl-($C_1$-$C_6$)-alkyle, ($C_1$-$C_6$)-alkylsulfonyle, ($C_1$-$C_6$)-halogénoalkylsulfonyle, ($C_1$-$C_6$)-alcoxy- ($C_1$-$C_6$)-alkylsulfonyle, ($C_1$-$C_6$)-alkylsulfonyl-($C_1$-$C_6$)-alkyle, ($C_1$-$C_6$)-alkylsulfonyloxy, ($C_1$-$C_6$)-alkylcarbonyle, ($C_1$-$C_6$)-alkylthiocarbonyle, ($C_1$-$C_6$)-halogénoalkylcarbonyle, ($C_1$-$C_6$)-alkylcarbonyloxy, ($C_1$-$C_6$)-alcoxycarbonyle, ($C_1$-$C_6$)-halogénoalcoxycarbonyle, aminocarbonyle, ($C_1$-$C_6$)-alkylaminocarbonyle, ($C_1$-$C_6$)-alkylaminothiocarbonyle, di-($C_1$-$C_6$)-alkylaminocarbonyle, di-($C_1$-$C_6$)-alkylaminothiocarbonyle, ($C_2$-$C_6$)-alcénylaminocarbonyle, di-($C_2$-$C_6$)-alcénylaminocarbonyle, ($C_3$-$C_8$)-cycloalkylaminocarbonyle, ($C_1$-$C_6$)-alkylsulfonylamino, ($C_1$-$C_6$)-alkylamino, di-($C_1$-$C_6$)-alkylamino, aminosulfonyle, ($C_1$-$C_6$)-alkylaminosulfonyle, di-($C_1$-$C_6$)-alkylaminosulfonyle, ($C_1$-$C_6$)-alkylsulfoximino, aminothiocarbonyle, ($C_1$-$C_6$)-alkylaminothiocarbonyle, di-($C_1$-$C_6$)-alkylaminothiocarbonyle, ($C_3$-$C_8$)-cycloalkylamino ou -NHCO-($C_1$-$C_6$)-alkyle (($C_1$-$C_6$)-alkylcarbonylamino),

X représente un système cyclique annelé, bicyclique ou tricyclique, hétérocyclique ou hétéroaromatique, partiellement saturé ou saturé, à 8, 9, 10, 11 ou 12 chaînons, éventuellement au moins un groupe carbonyle pouvant être contenu et/ou le système cyclique étant éventuellement substitué une ou plusieurs fois, de manière identique ou différente, et les substituants pouvant être choisis indépendamment les uns des autres parmi cyano, halogène, nitro, acétyle, hydroxy, amino, SCN, tri-($C_1$-$C_6$)-alkylsilyle, ($C_3$-$C_8$)-cycloalkyle, ($C_3$-$C_8$)-cycloalkyl- ($C_3$-$C_8$)-cycloalkyle, ($C_1$-$C_6$)-alkyl-($C_3$-$C_8$)-cycloalkyle, halogéno-($C_3$-$C_8$)-cycloalkyle, ($C_1$-$C_6$)-alkyle, ($C_1$-$C_6$)-halogénoalkyle, ($C_1$-$C_6$)-cyanoalkyle, ($C_1$-$C_6$)-hydroxyalkyle, hydroxycarbonyl-($C_1$-$C_6$)-alcoxy, ($C_1$-$C_6$)-alcoxycarbonyl-($C_1$-$C_6$)-alkyle, ($C_1$-$C_6$)-alcoxy- ($C_1$-$C_6$)-alkyle, ($C_2$-$C_6$)-alcényle, ($C_2$-$C_6$)-halogénoalcényle, ($C_2$-$C_6$)-cyanoalcényle, ($C_2$-$C_6$)-alcynyle, ($C_2$-$C_6$)-alcynyloxy-($C_1$-$C_4$)-alkyle, ($C_2$-$C_6$)-halogénoalcynyle, ($C_2$-$C_6$)-cyanoalcynyle, ($C_1$-$C_6$)-alcoxy, ($C_1$-$C_6$)-halogénoalcoxy, ($C_1$-$C_6$)-halogénoalcoxy-($C_1$-$C_6$)-alkyle, ($C_2$-$C_6$)-alcényloxy-($C_1$-$C_6$)-alkyle, ($C_2$-$C_6$)-halogénoalcényloxy-($C_1$-$C_6$)-alkyle, ($C_1$-$C_6$)-cyanoalcoxy, ($C_1$-$C_6$)-alcoxycarbonyl-($C_1$-$C_6$)-alcoxy, ($C_1$-$C_6$)-alcoxy- ($C_1$-$C_6$)-alcoxy, ($C_1$-$C_6$)-alkylhydroxyimino, ($C_1$-$C_6$)-alcoxyimino, ($C_1$-$C_6$)-alkyl-($C_1$-$C_6$)-alcoxyimino, ($C_1$-$C_6$)-halogénoalkyl-($C_1$-$C_6$)-alcoxyimino, ($C_1$-$C_6$)-alkylthio, ($C_1$-$C_6$)-halogénoalkylthio, ($C_1$-$C_6$)-alcoxy- ($C_1$-$C_6$)-alkylthio, ($C_1$-$C_6$)-alkylthio- ($C_1$-$C_6$)-alkyle, ($C_1$-$C_6$)-alkylsulfinyle, ($C_1$-$C_6$)-halogénoalkylsulfinyle, ($C_1$-$C_6$)-alcoxy- ($C_1$-$C_6$)-alkylsulfinyle, ($C_1$-$C_6$)-alkylsulfinyl-($C_1$-$C_6$)-alkyle, ($C_1$-$C_6$)-alkylsulfonyle, ($C_1$-$C_6$)-halogénoalkylsulfonyle, ($C_1$-$C_6$)-alcoxy- ($C_1$-$C_6$)-alkylsulfonyle, ($C_1$-$C_6$)-alkylsulfonyl-($C_1$-$C_6$)-alkyle, ($C_1$-$C_6$)-alkylsulfonyloxy, ($C_1$-$C_6$)-alkylcarbonyle, ($C_1$-$C_6$)-alkylcarbonyl-($C_1$-$C_6$)-alkyle, ($C_1$-$C_6$)-alkylthiocarbonyle, ($C_1$-$C_6$)-halogénoalkylcarbonyle, ($C_1$-$C_6$)-alkylcarbonyloxy, ($C_1$-$C_6$)-alcoxycarbonyle, ($C_1$-$C_6$)-halogénoalcoxycarbonyle, aminocarbonyle, ($C_1$-$C_6$)-alkylaminocarbonyle, ($C_1$-$C_6$)-alkylaminothiocarbonyle, di-($C_1$-$C_6$)-alkylaminocarbonyle, di-($C_1$-$C_6$)-alkylaminothiocarbonyle, ($C_2$-$C_6$)-alcénylaminocarbonyle, di-($C_2$-$C_6$)-alcénylaminocarbonyle, ($C_3$-$C_8$)-cycloalkylaminocarbonyle, ($C_1$-$C_6$)-alkylsulfonylamino, ($C_1$-$C_6$)-alkylamino, di-($C_1$-$C_6$)-alkylamino, aminosulfonyle, ($C_1$-$C_6$)-alkylaminosulfonyle, di-($C_1$-$C_6$)-alkylaminosulfonyle, ($C_1$-$C_6$)-alkylsulfoximino, aminothiocarbonyle, ($C_1$-$C_6$)-alkylaminothiocarbonyle, di-($C_1$-$C_6$)-alkylaminothiocarbonyle, ($C_3$-$C_8$)-cycloalkylamino, NHCO-($C_1$-$C_6$)-alkyle (($C_1$-$C_6$)-alkylcarbonylamino), ou les substituants pouvant être choisis indépendamment les uns des autres parmi phényle et un cycle hétéroaromatique à 5 ou 6 chaînons, le phényle ou le cycle pouvant éventuellement être substitué une ou plusieurs fois, de manière identique ou différente par $C_1$-$C_6$-alkyle, $C_2$-$C_6$-alcényle, $C_2$-$C_6$-alcynyle, $C_3$-$C_6$-cycloalkyle, $C_1$-$C_6$-halogénoalkyle, $C_2$-$C_6$-halogénoalcényle, $C_2$-$C_6$-halogénoalcynyle, $C_3$-$C_6$-halogénocycloalkyle, halogène, CN, $NO_2$, $C_1$-$C_4$-alcoxy, $C_1$-$C_4$-halogénoalcoxy,

n représente 0, 1 ou 2,

à l'exception de

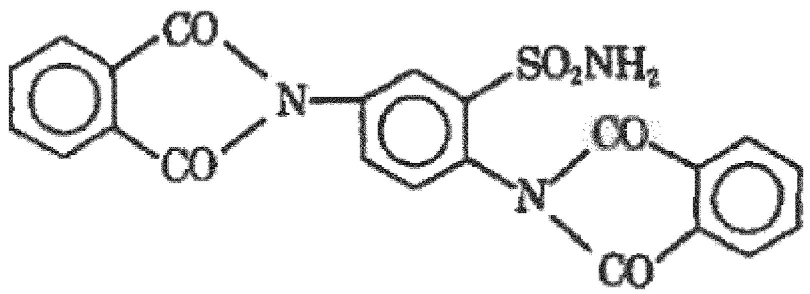

**2.** Composés de formule (I) selon la revendication 1, dans laquelle

$A^1$ représente azote, $=N^+(O^-)$- ou $=C(R^4)$-,

$R^1$ représente ($C_1$-$C_4$)-alkyle, ($C_1$-$C_4$)-hydroxyalkyle, ($C_1$-$C_4$)-halogénoalkyle, ($C_1$-$C_4$)-cyanoalkyle, ($C_1$-$C_4$)-alcoxy-($C_1$-$C_4$)-alkyle, ($C_1$-$C_4$)-halogénoalcoxy-($C_1$-$C_4$)-alkyle, ($C_2$-$C_4$)-alcényle, ($C_2$-$C_4$)-alcényloxy-($C_1$-$C_4$)-alkyle, ($C_2$-$C_4$)-halogénoalcényloxy-($C_1$-$C_4$)-alkyle, ($C_2$-$C_4$)-halogénoalcényle, ($C_2$-$C_4$)-cyanoalcényle, ($C_2$-$C_4$)-alcynyle, ($C_2$-$C_4$)-alcynyloxy-($C_1$-$C_4$)-alkyle, ($C_2$-$C_4$)-halogénoalcynyloxy-($C_1$-$C_4$)-alkyle, ($C_2$-$C_4$)-halogénoalcynyle, ($C_2$-$C_4$)-cyanoalcynyle, ($C_3$-$C_6$)-cycloalkyle, ($C_3$-$C_6$)-cycloalkyl- ($C_3$-$C_6$)-cycloalkyle, ($C_1$-$C_4$)-alkyl-($C_3$-$C_6$)-cycloalkyle, halogéno-($C_3$-$C_6$)-cycloalkyle, ($C_1$-$C_4$)-alkylamino, di-($C_1$-$C_4$)-alkylamino, ($C_3$-$C_6$)-cycloalkylamino, ($C_1$-$C_4$)-alkylcarbonylamino, ($C_1$-$C_4$)-alkylthio-($C_1$-$C_4$)-alkyle, ($C_1$-$C_4$)-halogénoalkylthio-($C_1$-$C_4$)-alkyle, ($C_1$-$C_4$)-alkylsulfinyl-($C_1$-$C_4$)-alkyle, ($C_1$-$C_4$)-halogénoalkylsulfinyl-($C_1$-$C_4$)-alkyle, ($C_1$-$C_4$)-alkylsulfonyl-($C_1$-$C_4$)-alkyle, ($C_1$-$C_4$)-alkylcarbonyl-($C_1$-$C_4$)-alkyle, ($C_1$-$C_4$)-halogénoalkylcarbonyl-($C_1$-$C_4$)-alkyle, ($C_1$-$C_4$)-alkylsulfonylamino,

ou représente ($C_1$-$C_4$)-alkyle, ($C_1$-$C_4$)-alcoxy, ($C_2$-$C_4$)-alcényle, ($C_2$-$C_4$)-alcynyle, ($C_3$-$C_6$)-cycloalkyle, à chaque fois éventuellement substitué une ou deux fois, de manière identique ou différente par aryle, hétaryle ou hétérocyclyle, aryle, hétaryle ou hétérocyclyle pouvant à chaque fois éventuellement être substitué une ou deux fois, de manière identique ou différente par halogène, cyano, carbamoyle, aminosulfonyle, ($C_1$-$C_4$)-alkyle, ($C_3$-$C_4$)-cycloalkyle, ($C_1$-$C_4$)-alcoxy, ($C_1$-$C_4$)-halogénoalkyle, ($C_1$-$C_4$)-halogénoalcoxy, ($C_1$-$C_4$)-alkylthio, ($C_1$-$C_4$)-alkylsulfinyle, ($C_1$-$C_4$)-alkylsulfonyle, ($C_1$-$C_4$)-alkylsulfimino, ou

$R^1$ représente aryle, hétaryle ou hétérocyclyle à chaque fois éventuellement substitué une ou deux fois, de manière identique ou différente par halogène, cyano, carbamoyle, ($C_1$-$C_4$)-alkyle, ($C_3$-$C_6$)-cycloalkyle, ($C_1$-$C_4$)-alcoxy, ($C_1$-$C_4$)-halogénoalkyle, ($C_1$-$C_4$)-halogénoalcoxy, ($C_1$-$C_4$)-alkylthio, ($C_1$-$C_4$)-alkylsulfinyle, ($C_1$-$C_4$)-alkylsulfonyle, ($C_1$-$C_4$)-alkylsulfimino, ($C_1$-$C_4$)-alkylsulfoximino, ($C_1$-$C_4$)-alkylcarbonyle, ($C_3$-$C_4$)-trialkylsilyle, (=O) (seulement dans le cas d'hétérocyclyle) et $(=O)_2$ (seulement dans le cas d'hétérocyclyle),

$R^2$ représente un système cyclique bicyclique annelé hétérocyclique ou hétéroaromatique, partiellement saturé ou saturé, à 8, 9 ou 10 chaînons, éventuellement au moins un groupe carbonyle pouvant être contenu et/ou le système cyclique pouvant éventuellement être substitué une ou plusieurs fois, de manière identique ou différente, et les substituants pouvant être choisis indépendamment les uns des autres parmi cyano, halogène, nitro, acétyle, amino, ($C_1$-$C_4$)-alkyle, ($C_1$-$C_4$)-halogénoalkyle, ($C_1$-$C_4$)-cyanoalkyle, ($C_1$-$C_4$)-hydroxyalkyle, ($C_1$-$C_4$)-alcoxy- ($C_1$-$C_4$)-alkyle, ($C_2$-$C_4$)-alcényle, ($C_2$-$C_4$)-halogénoalcényle, ($C_2$-$C_4$)-cyanoalcényle, ($C_2$-$C_4$)-alcynyle, ($C_2$-$C_4$)-halogénoalcynyle, ($C_2$-$C_4$)-cyanoalcynyle, ($C_3$-$C_6$)-cycloalkyle, ($C_3$-$C_6$)-cycloalkyl-($C_3$-$C_6$)cycloalkyle, ($C_1$-$C_4$)-alkyl-($C_3$-$C_6$)cycloalkyle, ($C_1$-$C_4$)-alcoxy, ($C_1$-$C_4$)-halogénoalcoxy, ($C_1$-$C_4$)-cyanoalcoxy, ($C_1$-$C_4$)-alcoxy- ($C_1$-$C_4$)-alcoxy, ($C_1$-$C_4$)-alcoxyimino,-N=C(H)-O($C_1$-$C_4$)-alkyle, -C(H)=N-O($C_1$-$C_4$)-alkyle, ($C_1$-$C_4$)-halogénoalkyl-($C_1$-$C_4$)-alcoxyimino, ($C_1$-$C_4$)-alkylthio, ($C_1$-$C_4$)-halogénoalkylthio, ($C_1$-$C_4$)-alkylthio- ($C_1$-$C_4$)-alkyle, ($C_1$-$C_4$)-alkylsulfinyle, ($C_1$-$C_4$)-halogénoalkylsulfinyle, ($C_1$-$C_4$)-alkylsulfinyl-($C_1$-$C_4$)-alkyle, ($C_1$-$C_4$)-alkylsulfonyle, ($C_1$-$C_4$)-halogénoalkylsulfonyle, ($C_1$-$C_4$)-alkylsulfonyl-($C_1$-$C_4$)-alkyle, ($C_1$-$C_4$)-alkylsulfonyloxy, ($C_1$-$C_4$)-alkylcarbonyle, ($C_1$-$C_4$)-halogénoalkylcarbonyle, aminocarbonyle, ($C_1$-$C_4$)-alkylaminocarbonyle, di-($C_1$-$C_4$)-alkylaminocarbonyle, ($C_1$-$C_4$)-alkylsulfonylamino, ($C_1$-$C_4$)-alkylamino, di-($C_1$-$C_4$)-alkylamino, aminosulfonyle, ($C_1$-$C_4$)-alkylaminosulfonyle, di-($C_1$-$C_4$)-alkylaminosulfonyle, ($C_1$-$C_4$)-alkylcarbonylamino

$R^3$ représente hydrogène, cyano, halogène, nitro, acétyle, hydroxy, amino, SCN, tri-($C_1$-$C_4$)-alkylsilyle, ($C_3$-$C_6$)-cycloalkyle, ($C_3$-$C_6$)-cycloalkyl-($C_3$-$C_6$)cycloalkyle, ($C_1$-$C_4$)-alkyl-($C_3$-$C_6$)-cycloalkyle, halogéno-($C_3$-$C_6$)-cycloalkyle, ($C_1$-$C_4$)-alkyle, ($C_1$-$C_4$)-halogénoalkyle, ($C_1$-$C_4$)-cyanoalkyle, ($C_1$-$C_4$)-hydroxyalkyle, ($C_1$-$C_4$)-alcoxy- ($C_1$-$C_4$)-alkyle, ($C_2$-$C_4$)-alcényle, ($C_2$-$C_4$)-halogénoalcényle, ($C_2$-$C_4$)-cyanoalcényle, ($C_2$-$C_4$)-alcynyle, ($C_2$-$C_4$)-halogénoalcynyle, ($C_2$-$C_4$)-cyanoalcynyle, ($C_1$-$C_4$)-alcoxy, ($C_1$-$C_4$)-halogénoalcoxy, ($C_1$-$C_4$)-cyanoalcoxy, ($C_1$-$C_4$)-alcoxy- ($C_1$-$C_4$)-alcoxy, ($C_1$-$C_4$)-alkylhydroxyimino, ($C_1$-$C_4$)-alcoxyimino, ($C_1$-$C_4$)-alkyl- ($C_1$-$C_4$)-alcoxyimino, ($C_1$-$C_4$)-halogénoalkyl-($C_1$-$C_4$)-alcoxyimino, ($C_1$-$C_4$)-alkylthio, ($C_1$-$C_4$)-halogénoalkylthio, ($C_1$-$C_4$)-alkylthio- ($C_1$-$C_4$)-alkyle, ($C_1$-$C_4$)-alkylsulfinyle, ($C_1$-$C_4$)-halogénoalkylsulfinyle, ($C_1$-$C_4$)-alkylsulfinyl-($C_1$-$C_4$)-alkyle, ($C_1$-$C_4$)-alkylsulfonyle, ($C_1$-$C_4$)-halogénoalkylsulfonyle, ($C_1$-$C_4$)-alkylsulfonyl-($C_1$-$C_4$)-alkyle, ($C_1$-$C_4$)-alkylsulfonyloxy, ($C_1$-$C_4$)-alkylcarbonyle, ($C_1$-$C_4$)-halogénoalkylcarbonyle, aminocarbonyle, aminothiocarbonyle, ($C_1$-$C_4$)-alkylaminocarbonyle, di-($C_1$-$C_4$)-alkylaminocarbonyle, ($C_1$-$C_4$)-alkylsulfonylamino, ($C_1$-$C_4$)-alkylamino, di-($C_1$-$C_4$)-alkylamino, aminosulfonyle, ($C_1$-$C_4$)-alkylaminosulfonyle, di-($C_1$-$C_4$)-alkylaminosulfonyle, aminothiocarbonyle, NHCO-($C_1$-$C_4$)-alkyle(($C_1$-$C_4$)-alkylcarbonylamino), en outre représente phényle ou hétaryle à chaque fois éventuellement substitué une ou deux fois, de manière identique ou différente, (dans le cas d'hétaryle) éventuellement au moins un groupe carbonyle pouvant être contenu et/ou les substituants pouvant être à chaque fois: cyano, halogène, nitro, acétyle, amino, ($C_3$-$C_6$)-cycloalkyle, ($C_3$-$C_6$)-cycloalkyl- ($C_3$-$C_6$)-cycloalkyle, ($C_1$-$C_4$)-alkyl-($C_3$-$C_6$)-cycloalkyle, halogéno-($C_3$-$C_6$)-cycloalkyle, ($C_1$-$C_4$)-alkyle, ($C_1$-$C_4$)-halogénoalkyle, ($C_1$-$C_4$)-cyanoalkyle, ($C_1$-$C_4$)-hydroxyalkyle, ($C_1$-$C_4$)-alcoxy-($C_1$-$C_4$)-alkyle, ($C_2$-$C_4$)-alcényle, ($C_2$-$C_4$)-halogénoalcényle, ($C_2$-$C_4$)-cyanoalcényle, ($C_2$-$C_4$)-alcynyle, ($C_2$-$C_4$)-halogénoalcynyle, ($C_2$-$C_4$)-cyanoalcynyle, ($C_1$-$C_4$)-alcoxy, ($C_1$-$C_4$)-halogénoalcoxy, ($C_1$-$C_4$)-cyanoal-

coxy, ($C_1$-$C_4$)-alcoxy- ($C_1$-$C_4$)-alcoxy, ($C_1$-$C_4$)-alkylhydroxyimino, ($C_1$-$C_4$)-alcoxyimino, ($C_1$-$C_4$)-alkyl-($C_1$-$C_4$)-alcoxyimino, ($C_1$-$C_4$)-halogénoalkyl-($C_1$-$C_4$)-alcoxyimino, ($C_1$-$C_4$)-alkylthio, ($C_1$-$C_4$)-halogénoalkylthio, ($C_1$-$C_4$)-alkylthio-($C_1$-$C_4$)-alkyle, ($C_1$-$C_4$)-alkylsulfinyle, ($C_1$-$C_4$)-halogénoalkylsulfinyle, ($C_1$-$C_4$)-alkylsulfinyl-($C_1$-$C_4$)-alkyle, ($C_1$-$C_4$)-alkylsulfonyle, ($C_1$-$C_4$)-halogénoalkylsulfonyle, ($C_1$-$C_4$)-alkylsulfonyl-($C_1$-$C_4$)-alkyle, ($C_1$-$C_4$)-alkylsulfonyloxy, ($C_1$-$C_4$)-alkylcarbonyle, ($C_1$-$C_4$)-halogénoalkylcarbonyle, aminocarbonyle, ($C_1$-$C_4$)-alkylaminocarbonyle, di-($C_1$-$C_4$)-alkylaminocarbonyle, ($C_1$-$C_4$)-alkylsulfonylamino, ($C_1$-$C_4$)-alkylamino, di-($C_1$-$C_4$)-alkylamino, aminosulfonyle, ($C_1$-$C_4$)-alkylaminosulfonyle, di-($C_1$-$C_4$)-alkylaminosulfonyle, NHCO-($C_1$-$C_4$)-alkyle (($C_1$-$C_4$)-alkylcarbonylamino),

$R^4$ représente hydrogène, cyano, halogène, nitro, acétyle, hydroxy, amino, SCN, tri-($C_1$-$C_4$)-alkylsilyle, ($C_3$-$C_6$)-cycloalkyle, ($C_3$-$C_6$)-cycloalkyl-($C_3$-$C_6$)cycloalkyle, ($C_1$-$C_4$)-alkyl-($C_3$-$C_6$)cycloalkyle, halogéno-($C_3$-$C_6$)cycloalkyle, ($C_1$-$C_4$)-alkyle, ($C_1$-$C_4$)-halogénoalkyle, ($C_1$-$C_4$)-cyanoalkyle, ($C_1$-$C_4$)-hydroxyalkyle, ($C_1$-$C_4$)-alcoxy- ($C_1$-$C_4$)-alkyle, ($C_2$-$C_4$)-alcényle, ($C_2$-$C_4$)-halogénoalcényle, ($C_2$-$C_4$)-cyanoalcényle, ($C_2$-$C_4$)-alcynyle, ($C_2$-$C_4$)-halogénoalcynyle, ($C_2$-$C_4$)-cyanoalcynyle, ($C_1$-$C_4$)-alcoxy, ($C_1$-$C_4$)-halogénoalcoxy, ($C_1$-$C_4$)-cyanoalcoxy, ($C_1$-$C_4$)-alcoxy- ($C_1$-$C_4$)-alcoxy, ($C_1$-$C_4$)-alkylhydroxyimino, ($C_1$-$C_4$)-alcoxyimino, ($C_1$-$C_4$)-alkyl- ($C_1$-$C_4$)-alcoxyimino, ($C_1$-$C_4$)-halogénoalkyl-($C_1$-$C_4$)-alcoxyimino, ($C_1$-$C_4$)-alkylthio, ($C_1$-$C_4$)-halogénoalkylthio, ($C_1$-$C_4$)-alkylthio- ($C_1$-$C_4$)-alkyle, ($C_1$-$C_4$)-alkylsulfinyle, ($C_1$-$C_4$)-halogénoalkylsulfinyle, ($C_1$-$C_4$)-alkylsulfinyl-($C_1$-$C_4$)-alkyle, ($C_1$-$C_4$)-alkylsulfonyle, ($C_1$-$C_4$)-halogénoalkylsulfonyle, ($C_1$-$C_4$)-alkylsulfonyl-($C_1$-$C_4$)-alkyle, ($C_1$-$C_4$)-alkylsulfonyloxy, ($C_1$-$C_4$)-alkylcarbonyle, ($C_1$-$C_4$)-halogénoalkylcarbonyle, aminocarbonyle, aminothiocarbonyle, ($C_1$-$C_4$)-alkylaminocarbonyle, di-($C_1$-$C_4$)-alkylaminocarbonyle, ($C_1$-$C_4$)-alkylsulfonylamino, ($C_1$-$C_4$)-alkylamino, di-($C_1$-$C_4$)-alkylamino, aminosulfonyle, ($C_1$-$C_4$)-alkylaminosulfonyle, di-($C_1$-$C_4$)-alkylaminosulfonyle, aminothiocarbonyle ou NHCO-($C_1$-$C_4$)-alkyle(($C_1$-$C_4$)-alkylcarbonylamino),

X représente un système cyclique hétéroaromatique annelé bicyclique ou tricyclique à 8, 9, 10, 11 ou 12 chaînons, le système cyclique étant éventuellement substitué une ou plusieurs fois, de manière identique ou différente, et les substituants pouvant être choisis indépendamment les uns des autres parmi cyano, halogène, nitro, acétyle, hydroxy, amino, SCN, tri-($C_1$-$C_6$)-alkylsilyle, ($C_3$-$C_8$)-cycloalkyle, ($C_3$-$C_8$)-cycloalkyl- ($C_3$-$C_8$)-cycloalkyle, ($C_1$-$C_6$)-alkyl-($C_3$-$C_8$)-cycloalkyle, halogéno-($C_3$-$C_8$)-cycloalkyle, ($C_1$-$C_6$)-alkyle, ($C_1$-$C_6$)-halogénoalkyle, ($C_1$-$C_6$)-cyanoalkyle, ($C_1$-$C_6$)-hydroxyalkyle, ($C_1$-$C_6$)-alcoxy- ($C_1$-$C_6$)-alkyle, ($C_2$-$C_6$)-alcényle, ($C_2$-$C_6$)-halogénoalcényle, ($C_2$-$C_6$)-cyanoalcényle, ($C_2$-$C_6$)-alcynyle, ($C_2$-$C_6$)-alcynyloxy-($C_1$-$C_4$)-alkyle, ($C_2$-$C_6$)-halogénoalcynyle, ($C_1$-$C_6$)-alcoxy, ($C_1$-$C_6$)-halogénoalcoxy, ($C_1$-$C_6$)-halogénoalcoxy-($C_1$-$C_6$)-alkyle, ($C_2$-$C_6$)-alcényloxy-($C_1$-$C_6$)-alkyle, ($C_2$-$C_6$)-halogénoalcényloxy-($C_1$-$C_6$)-alkyle, ($C_1$-$C_6$)-cyanoalcoxy, ($C_1$-$C_6$)-alcoxy- ($C_1$-$C_6$)-alcoxy, ($C_1$-$C_6$)-alkylhydroxyimino, ($C_1$-$C_6$)-alcoxyimino, ($C_1$-$C_6$)-alkyl-($C_1$-$C_6$)-alcoxyimino, ($C_1$-$C_6$)-alkylthio, ($C_1$-$C_6$)-halogénoalkylthio, ($C_1$-$C_6$)-alcoxy- ($C_1$-$C_6$)-alkylthio, ($C_1$-$C_6$)-alkylthio-($C_1$-$C_6$)-alkyle, ($C_1$-$C_6$)-alkylsulfinyle, ($C_1$-$C_6$)-halogénoalkylsulfinyle, ($C_1$-$C_6$)-alcoxy- ($C_1$-$C_6$)-alkylsulfinyle, ($C_1$-$C_6$)-alkylsulfinyl-($C_1$-$C_6$)-alkyle, ($C_1$-$C_6$)-alkylsulfonyle, ($C_1$-$C_6$)-halogénoalkylsulfonyle, ($C_1$-$C_6$)-alcoxy-($C_1$-$C_6$)-alkylsulfonyle, ($C_1$-$C_6$)-alkylsulfonyl-($C_1$-$C_6$)-alkyle, ($C_1$-$C_6$)-alkylsulfonyloxy, ($C_1$-$C_6$)-alkylcarbonyle, ($C_1$-$C_6$)-alkylcarbonyl-($C_1$-$C_6$)-alkyle, ($C_1$-$C_6$)-alkylthiocarbonyle, ($C_1$-$C_6$)-halogénoalkylcarbonyle, ($C_1$-$C_6$)-alkylcarbonyloxy, ($C_1$-$C_6$)-alcoxycarbonyle, ($C_1$-$C_6$)-halogénoalcoxycarbonyle, aminocarbonyle, ($C_1$-$C_6$)-alkylaminocarbonyle, ($C_1$-$C_6$)-alkylaminothiocarbonyle, di-($C_1$-$C_6$)-alkylaminocarbonyle, di-($C_1$-$C_6$)-alkylaminothiocarbonyle, ($C_3$-$C_8$)-cycloalkylaminocarbonyle, ($C_1$-$C_6$)-alkylsulfonylamino, ($C_1$-$C_6$)-alkylamino, di-($C_1$-$C_6$)-alkylamino, aminosulfonyle, ($C_1$-$C_6$)-alkylaminosulfonyle, di-($C_1$-$C_6$)-alkylaminosulfonyle, ($C_1$-$C_6$)-alkylsulfoximino, aminothiocarbonyle, ($C_1$-$C_6$)-alkylaminothiocarbonyle, di-($C_1$-$C_6$)-alkylaminothiocarbonyle, ($C_3$-$C_8$)-cycloalkylamino, NHCO-($C_1$-$C_6$)-alkyle (($C_1$-$C_6$)-alkylcarbonylamino),

ou les substituants pouvant être choisis indépendamment les uns des autres parmi phényle ou un cycle hétéroaromatique à 5 ou 6 chaînons, le phényle ou le cycle pouvant être éventuellement substitué une ou plusieurs fois, de manière identique ou différente par $C_1$-$C_6$-alkyle, $C_2$-$C_6$-alcényle, $C_2$-$C_6$-alcynyle, $C_3$-$C_6$-cycloalkyle, $C_1$-$C_6$-halogénoalkyle, $C_2$-$C_6$-halogénoalcényle, $C_2$-$C_6$-halogénoalcynyle, $C_3$-$C_6$-halogénocycloalkyle, halogène, CN, $C_1$-$C_4$-alcoxy, $C_1$-$C_4$-halogénoalcoxy, n représente 0, 1 ou 2.

**3.** Composés de formule (I) selon la revendication 1, dans laquelle

$A^1$ représente azote ou =C($R^4$)-,

$R^1$ représente ($C_1$-$C_4$)-alkyle, ($C_1$-$C_4$)-hydroxyalkyle, ($C_1$-$C_4$) -halogénoalkyle, ($C_2$-$C_4$)-alcényle, ($C_2$-$C_4$)-halogénoalcényle, ($C_2$-$C_4$)-alcynyle, ($C_2$-$C_4$)-halogénoalcynyle, ($C_3$-$C_6$)-cycloalkyle, ($C_1$-$C_4$)-alkylthio-($C_1$-$C_4$)-alkyle, ($C_1$-$C_4$)-alkylsulfinyl-($C_1$-$C_4$)-alkyle ou ($C_1$-$C_4$)-alkylsulfonyl-($C_1$-$C_4$)-alkyle,

$R^2$ représente un système cyclique bicyclique annelé partiellement saturé ou hétéroaromatique de la série Q1 à Q152, le système cyclique pouvant éventuellement être substitué une ou plusieurs fois, de manière identique ou différente, les substituants pouvant être choisis indépendamment les uns des autres parmi : nitro, hydroxy, amino, cyano, halogène, ($C_1$-$C_4$)-alkyle, ($C_1$-$C_4$)-cycloalkyle, ($C_1$-$C_4$)-halogénoalkyle, ($C_1$-$C_4$)-alcoxy,

($C_1$-$C_4$)-halogénoalcoxy et ($C_1$-$C_4$)-halogénoalkylthio,

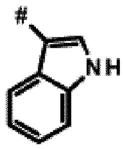
Q1
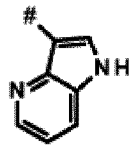
Q2
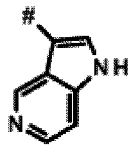
Q3
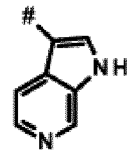
Q4
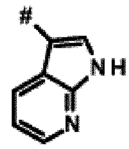
Q5
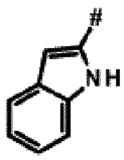
Q6
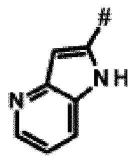
Q7
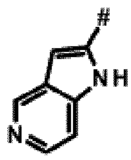
Q8
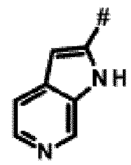
Q9
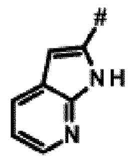
Q10
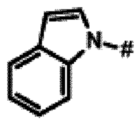
Q11
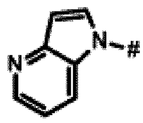
Q12
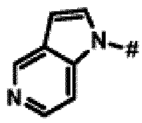
Q13
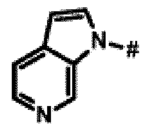
Q14
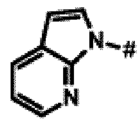
Q15
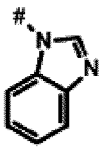
Q16
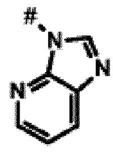
Q17
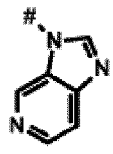
Q18
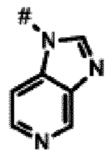
Q19
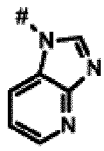
Q20
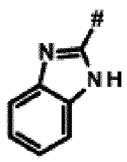
Q21
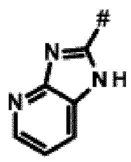
Q22
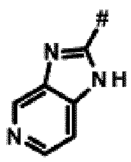
Q23
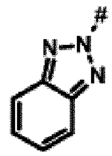
Q24
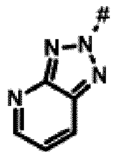
Q25
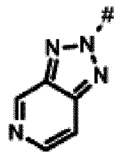
Q26
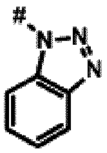
Q27
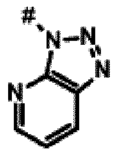
Q28
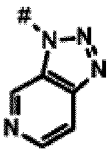
Q29
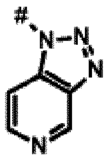
Q30
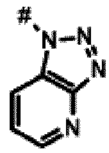
Q31
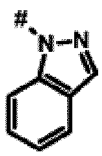
Q32
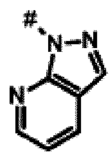
Q33
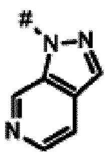
Q34
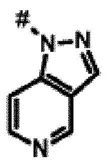
Q35
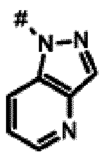
Q36

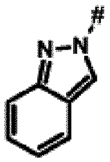
Q37
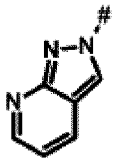
Q38
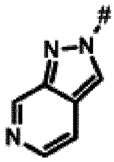
Q39
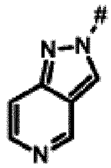
Q40
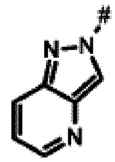
Q41

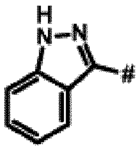
Q42
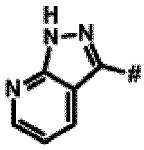
Q43
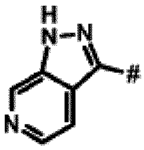
Q44
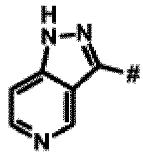
Q45
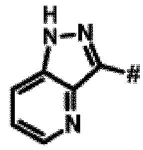
Q46

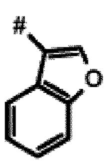
Q47
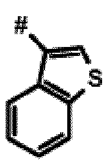
Q48
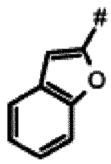
Q49
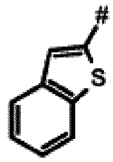
Q50

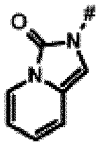
Q51
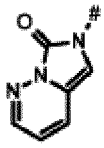
Q52
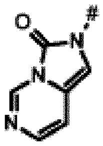
Q53
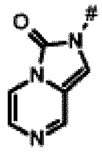
Q54
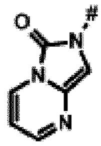
Q55

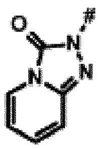
Q56
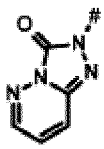
Q57
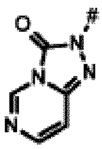
Q58
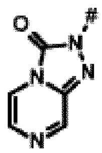
Q59
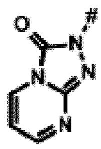
Q60

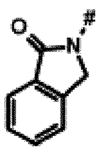
Q61
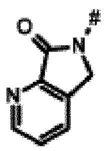
Q62
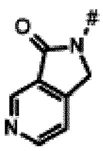
Q63
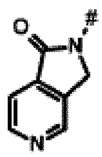
Q64
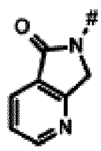
Q65

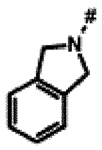
Q66
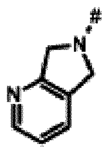
Q67
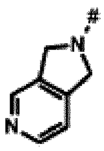
Q68
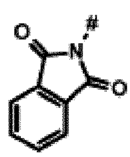
Q69
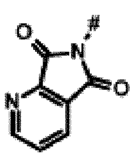
Q70
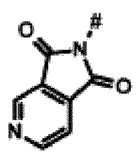
Q71

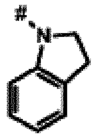
Q72
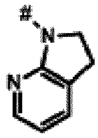
Q73
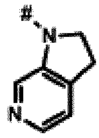
Q74
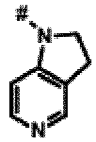
Q75
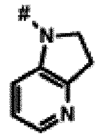
Q76

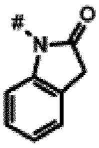
Q77
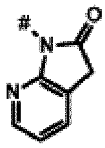
Q78
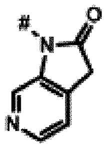
Q79
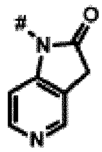
Q80
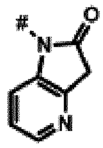
Q81

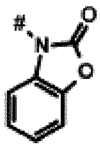
Q82
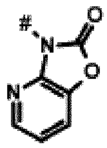
Q83
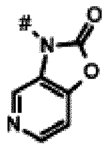
Q84
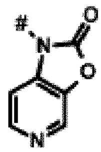
Q85
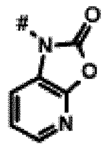
Q86

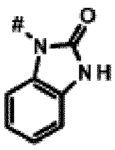
Q87
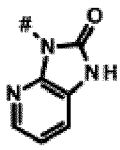
Q88
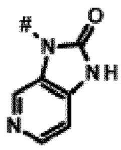
Q89
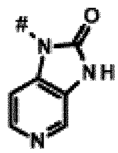
Q90
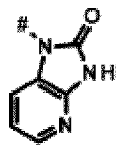
Q91

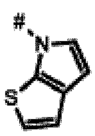
Q92
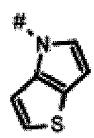
Q93
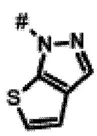
Q94
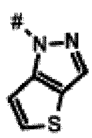
Q95
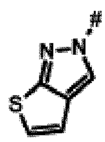
Q96
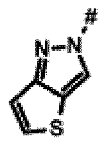
Q97

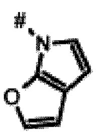
Q98
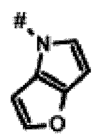
Q99
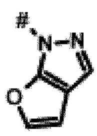
Q100
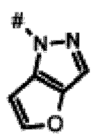
Q101
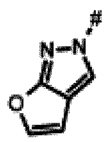
Q102
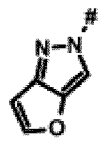
Q103

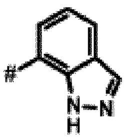
Q104
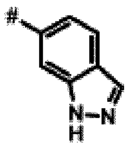
Q105
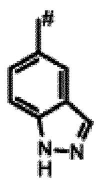
Q106
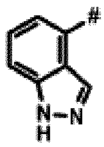
Q107
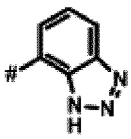
Q108
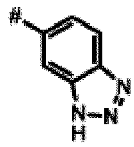
Q109

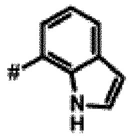
Q110
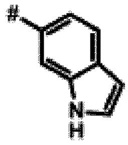
Q111
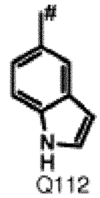
Q112
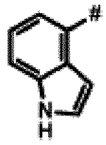
Q113
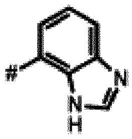
Q114
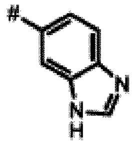
Q115

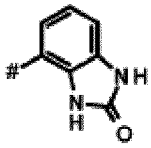
Q116
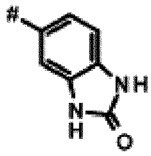
Q117
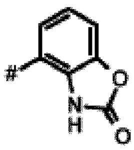
Q118
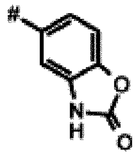
Q119
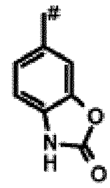
Q120
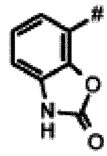
Q121

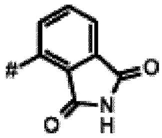
Q122
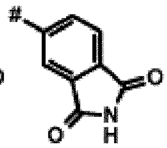
Q123
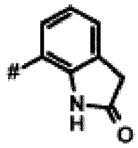
Q124
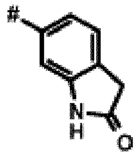
Q125
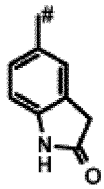
Q126
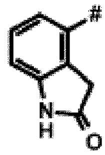
Q127

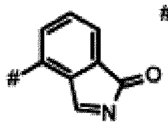
Q128
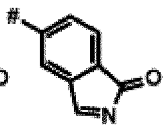
Q129
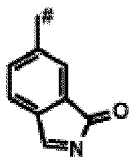
Q130
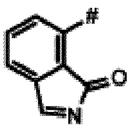
Q131
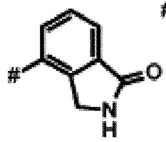
Q132
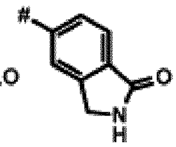
Q133

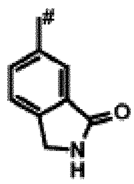
Q134
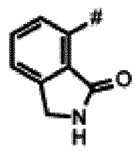
Q135

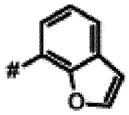
Q136
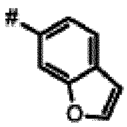
Q137
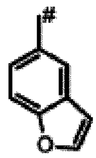
Q138
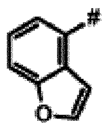
Q139
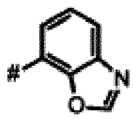
Q140
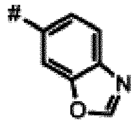
Q141

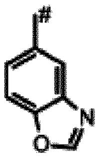
Q142
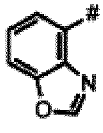
Q143
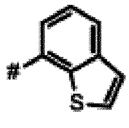
Q144
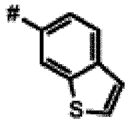
Q145
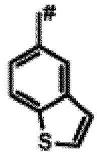
Q146
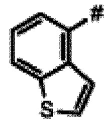
Q147

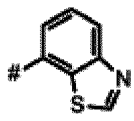
Q148

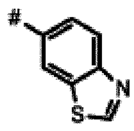
Q149

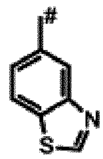
Q150

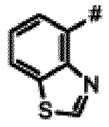
Q151

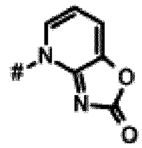
Q152

la liaison au reste de la molécule étant indiquée par un astérisque #,

$R^3$ représente hydrogène, cyano, halogène, nitro, hydroxy, amino, SCN, tri-($C_1$-$C_4$)-alkylsilyle, ($C_3$-$C_6$)-cycloalkyle, ($C_3$-$C_6$)-cycloalkyl- ($C_3$-$C_6$)-cycloalkyle, ($C_1$-$C_4$)-alkyl-($C_3$-$C_6$)-cycloalkyle, halogéno-($C_3$-$C_6$)-cycloalkyle, ($C_1$-$C_4$)-alkyle, ($C_1$-$C_4$)-halogénoalkyle, ($C_1$-$C_4$)-cyanoalkyle, ($C_1$-$C_4$)-alcoxy- ($C_1$-$C_4$)-alkyle, ($C_2$-$C_4$)-alcényle, ($C_2$-$C_4$)-halogénoalcényle, ($C_2$-$C_4$)-cyanoalcényle, ($C_2$-$C_4$)-alcynyle, ($C_2$-$C_4$)-halogénoalcynyle, ($C_2$-$C_4$)-cyanoalcynyle, ($C_1$-$C_4$)-alcoxy, ($C_1$-$C_4$)-halogénoalcoxy, ($C_1$-$C_4$)-cyanoalcoxy, ($C_1$-$C_4$)-alkylhydroxyimino, ($C_1$-$C_4$)-alcoxyimino, ($C_1$-$C_4$)-alkyl-($C_1$-$C_4$)-alcoxyimino, ($C_1$-$C_4$)-alkylthio, ($C_1$-$C_4$)-halogénoalkylthio, ($C_1$-$C_4$)-alkylsulfinyle, ($C_1$-$C_4$)-halogénoalkylsulfinyle, ($C_1$-$C_4$)-alkylsulfonyle, ($C_1$-$C_4$)-halogénoalkylsulfonyle, ($C_1$-$C_4$)-alkylsulfonyloxy, ($C_1$-$C_4$)-alkylcarbonyle, ($C_1$-$C_4$)-halogénoalkylcarbonyle, aminocarbonyle, ($C_1$-$C_4$)-alkylaminocarbonyle, di-($C_1$-$C_4$)-alkylaminocarbonyle, ($C_1$-$C_4$)-alkylsulfonylamino, ($C_1$-$C_4$)-alkylamino, di-($C_1$-$C_4$)-alkylamino, aminosulfonyle, ($C_1$-$C_4$)-alkylaminosulfonyle, di-($C_1$-$C_4$)-alkyl-aminosulfonyle ou NHCO-($C_1$-$C_4$)-alkyle (($C_1$-$C_4$)-alkylcarbonylamino),

$R^4$ représente hydrogène, halogène, cyano ou ($C_1$-$C_4$)-alkyle

X représente un système cyclique annelé hétéroaromatique bicyclique ou tricyclique à 9 ou 12 chaînons parmi la série H1 à H20,

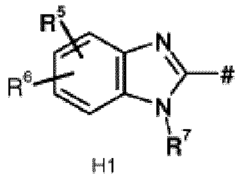
H1

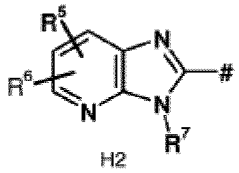
H2

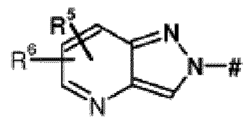
H3

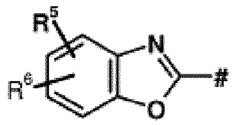
H4

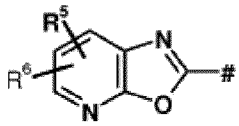
H5

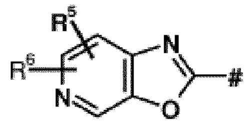
H6

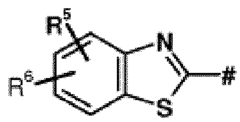
H7

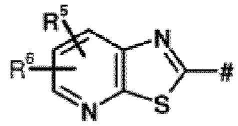
H8

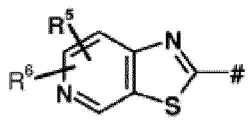
H9

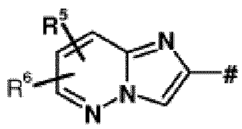
H10

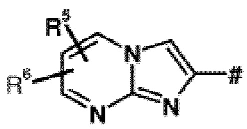
H11

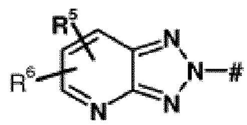
H12

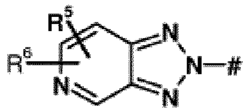

H13

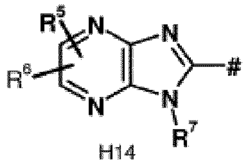

H14

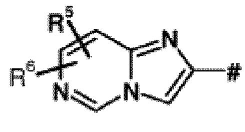

H15

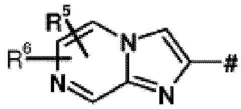

H16

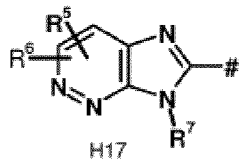

H17

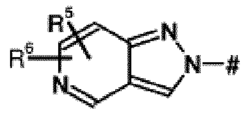

H18

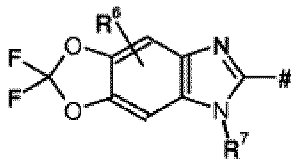

H19

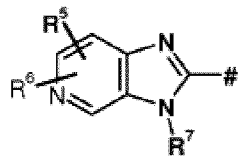

H20

$R^5$, $R^6$ représentent indépendamment l'un de l'autre hydrogène, cyano, halogène, $(C_1-C_4)$-alkyle, $(C_1-C_4)$-halogénoalkyle, $(C_2-C_4)$-alcényle, $(C_2-C_4)$-halogénoalcényle, $(C_2-C_4)$-alcynyle, $(C_2-C_4)$-halogénoalcynyle, $(C_3-C_6)$-cycloalkyle, $(C_3-C_6)$-cycloalkyl- $(C_3-C_6)$-cycloalkyle, $(C_1-C_4)$-alkyl-$(C_3-C_6)$-cycloalkyle, $(C_1-C_4)$-alcoxy, $(C_1-C_4)$-halogénoalcoxy, $(C_1-C_4)$-alcoxyimino, $(C_1-C_4)$-alkylthio, $(C_1-C_4)$-halogénoalkylthio, $(C_1-C_4)$-alkylsulfinyle, $(C_1-C_4)$-halogénoalkylsulfinyle, $(C_1-C_4)$-alkylsulfonyle, $(C_1-C_4)$-halogénoalkylsulfonyle, $(C_1-C_4)$-alkylsulfonyloxy, $(C_1-C_4)$-alkylcarbonyle, $(C_1-C_4)$-halogénoalkylcarbonyle, aminocarbonyle, $(C_1-C_4)$-alkylaminocarbonyle, di-$(C_1-C_4)$-alkylaminocarbonyle, $(C_1-C_4)$-alkylsulfonylamino, $(C_1-C_4)$-alkylamino, di-$(C_1-C_4)$-alkylamino, aminosulfonyle, $(C_1-C_4)$-alkylaminosulfonyle ou di-$(C_1-C_4)$-alkylaminosulfonyle,

$R^7$ représente hydrogène, $(C_1-C_4)$-alkyle, $(C_1-C_4)$-halogénoalkyle, $(C_1-C_4)$-cyanoalkyle, $(C_1-C_4)$-hydroxyalkyle, $(C_1-C_4)$-alcoxy- $(C_1-C_4)$-alkyle, $(C_1-C_4)$-halogénoalcoxy-$(C_1-C_4)$-alkyle, $(C_2-C_4)$-alcényle, $(C_2-C_4)$-alcényloxy-$(C_1-C_4)$-alkyle, $(C_2-C_4)$-halogénoalcényloxy-$(C_1-C_4)$-alkyle, $(C_2-C_4)$-halogénoalcényle, $(C_2-C_4)$-cyanoalcényle, $(C_2-C_4)$-alcynyle, $(C_2-C_4)$-alcynyloxy-$(C_1-C_4)$-alkyle, $(C_2-C_4)$-halogénoalcynyle, $(C_3-C_6)$-cycloalkyle, $(C_3-C_6)$-cycloalkyl- $(C_3-C_6)$-cycloalkyle, $(C_1-C_4)$-alkyl-$(C_3-C_6)$-cycloalkyle, halo $(C_3-C_6)$-cycloalkyle, $(C_1-C_4)$-alkylthio- $(C_1-C_4)$-alkyle, $(C_1-C_4)$-alkylsulfinyl-$(C_1-C_4)$-alkyle, $(C_1-C_4)$-alkylsulfonyl-$(C_1-C_4)$-alkyle ou $(C_1-C_4)$-alkylcarbonyl-$(C_1-C_4)$-alkyle,

n représente 0, 1 ou 2.

**4.** Composés de formule (I) selon la revendication 1, dans laquelle

$A^1$ représente azote,

$R^1$ représente méthyle, éthyle, n-propyle, i-propyle, cyclo-propyle, n-butyle, i-butyle, tert-butyle, cyclo-butyle, fluorométhyle, difluorométhyle, trifluorométhyle, fluoroéthyle, difluoroéthyle, trifluoroéthyle, tétrafluoroéthyle ou pentafluoroéthyle,

$R^2$ représente un système cyclique bicyclique annelé éventuellement substitué une ou deux fois, de manière identique ou différente, parmi la série Q1, Q2, Q3, Q4, Q5, Q6, Q7, Q8, Q9, Q10, Q11, Q12, Q13, Q14, Q15, Q16, Q17, Q18, Q19, Q20, Q21, Q22, Q23, Q27, Q28, Q29, Q30, Q31, Q32, Q33, Q34, Q35, Q36, Q37, Q38, Q39, Q40, Q41, Q42, Q43, Q44, Q45, Q46, Q51, Q52, Q53, Q54, Q55, Q56, Q57, Q58, Q59, Q60, Q61, Q62, Q63, Q64, Q65, Q66, Q67, Q68, Q69, Q70, Q71, Q77, Q78, Q79, Q80, Q81, Q82, Q83, Q84, Q85, Q86, Q87, Q88, Q89, Q90, Q91, Q92, Q93, Q94, Q95, Q96, Q97, Q104, Q105, Q106, Q107, Q108, Q109, Q114, Q115, Q116, Q117, Q118, Q119, Q120, Q121, Q124, Q125, Q126, Q127, Q128, Q129, Q130, Q131, Q132, Q133, Q134, Q135, Q136, Q137, Q138, Q139, Q140, Q141, Q142, Q143, Q144, Q145, Q146, Q147, Q148, Q149, Q150, Q151, Q152 et les substituants pouvant à chaque fois être : cyano, fluor, chlore, brome, méthyle, éthyle, n-propyle, i-propyle, cyclopropyle, trifluorométhyle, pentafluoroéthyle, méthoxy, éthoxy, trifluorométhoxy, trifluoroéthoxy, pentafluoroéthoxy ou trifluorométhylthio,

$R^3$ représente hydrogène, cyano, halogène, $(C_1-C_4)$-alkyle, $(C_1-C_4)$-halogénoalkyle, $(C_1-C_4)$-halogénoalcoxy, $(C_1-C_4)$ -alkylthio, $(C_1-C_4)$-alkylsulfinyle, $(C_1-C_4)$ -alkylsulfonyle, $(C_1-C_4)$-halogénoalkylthio, $(C_1-C_4)$-halogé-

noalkylsulfinyle, $(C_1-C_4)$-halogénoalkylsulfonyle ou NHCO-$(C_1-C_4)$-alkyle ($(C_1-C_4)$-alkylcarbonylamino),
X représente un système cyclique hétéroaromatique annelé bicyclique ou tricyclique à 9 ou 12 chaînons parmi la série H1, H2, H3, H4, H5, H6, H7, H8, H9, H10, H11, H12, H13, H14, H15, H16, H17, H18, H19 et H20,
$R^5$ représente fluor, chlore, fluorométhyle, difluorométhyle, trifluorométhyle, fluoroéthyle, difluoroéthyle, trifluoroéthyle, tétrafluoroéthyle, pentafluoroéthyle, trifluorométhoxy, difluorochlorométhoxy, dichlorofluorométhoxy, trifluorométhylthio, trifluorométhylsulfonyle ou trifluorométhylsulfinyle,
$R^6$ représente hydrogène,
$R^7$ représentant hydrogène, méthyle, éthyle, n-propyle, i-propyle, n-butyle, i-butyle, tert-butyle, méthoxyméthyle ou méthoxyéthyle,
n représente 0, 1 ou 2.

**5.** Composés de formule (I) selon la revendication 1, dans laquelle

$A^1$ représente azote,
$R^1$ représente méthyle, éthyle, n-propyle, i-propyle, cyclo-propyle, n-butyle, i-butyle, tert-butyle, cyclo-butyle, fluorométhyle, difluorométhyle, trifluorométhyle, fluoroéthyle, difluoroéthyle, trifluoroéthyle, tétrafluoroéthyle ou pentafluoroéthyle,
$R^2$ représente un système cyclique bicyclique annelé éventuellement substitué une ou deux fois, de manière identique ou différente, parmi la série Q1, Q6, Q11, Q12, Q15, Q16, Q17, Q20, Q27, Q28, Q32, Q33, Q34, Q35, Q37, Q38, Q51, Q56, Q57, Q61, Q62, Q66, Q67, Q69, Q82, Q87, Q105, Q106, Q107 et Q152, et les substituants pouvant être à chaque fois : cyano, fluor, chlore, brome, méthyle, éthyle, n-propyle, i-propyle, cyclopropyle, trifluorométhyle, pentafluoroéthyle, méthoxy, éthoxy, trifluorométhoxy, trifluoroéthoxy, pentafluoroéthoxy ou trifluorométhylthio,
$R^3$ représente hydrogène, cyano, halogène, $(C_1-C_4)$-alkyle, $(C_1-C_4)$-halogénoalkyle, $(C_1-C_4)$-halogénoalcoxy, $(C_1-C_4)$ -alkylthio, $(C_1-C_4)$-alkylsulfinyle, $(C_1-C_4)$ -alkylsulfonyle, $(C_1-C_4)$-halogénoalkylthio, $(C_1-C_4)$-halogénoalkylsulfinyle, $(C_1-C_4)$-halogénoalkylsulfonyle ou NHCO-$(C_1-C_4)$-alkyle ($(C_1-C_4)$-alkylcarbonylamino),
X représente un système cyclique hétéroaromatique annelé bicyclique ou tricyclique à 9 ou 12 chaînons parmi la série H1, H2, H3, H4, H5, H6, H7, H8, H9, H10, H11, H12, H13, H14, H15, H16, H17, H18, H19 et H20,
$R^5$ représente fluor, chlore, fluorométhyle, difluorométhyle, trifluorométhyle, fluoroéthyle, difluoroéthyle, trifluoroéthyle, tétrafluoroéthyle, pentafluoroéthyle, trifluorométhoxy, difluorochlorométhoxy, dichlorofluorométhoxy, trifluorométhylthio, trifluorométhylsulfonyle ou trifluorométhylsulfinyle,
$R^6$ représente hydrogène,
$R^7$ représente hydrogène, méthyle, éthyle, n-propyle, i-propyle, n-butyle, i-butyle, tert-butyle, méthoxyméthyle ou méthoxyéthyle,
n représente 0, 1 ou 2.

**6.** Composés de formule (I) selon la revendication 1, dans laquelle

$A^1$ représente azote,
$R^1$ représente éthyle,
$R^2$ représente un système cyclique bicyclique annelé substitué une ou deux fois, de manière identique ou différente par chlore, brome, cyano, méthyle, méthoxy ou trifluorométhyle parmi la série Q1, Q6, Q11, Q12, Q15, Q16, Q17, Q20, Q27, Q28, Q32, Q33, Q34, Q35, Q37, Q38, Q51, Q56, Q57, Q61, Q62, Q66, Q67, Q69, Q82, Q87, Q105, Q106, Q107 et Q152,
$R^3$ représente hydrogène,
X représente un système cyclique hétéroaromatique parmi la série H20,
$R^5$ représente trifluorométhyle,
$R^6$ représente hydrogène,
$R^7$ représente méthyle,
n représente 1 ou 2.

**7.** Formulation agrochimique contenant des composés de formule (I) selon la revendication 1, ainsi que des diluants et/ou des substances tensioactives.

**8.** Formulation agrochimique selon la revendication 7 contenant de plus un principe actif agrochimique supplémentaire.

**9.** Procédé pour la lutte contre des parasites animaux **caractérisé en ce qu'**on laisse agir un composé de formule (I) selon la revendication 1 ou une formulation agrochimique selon l'une quelconque des revendications 7 et 8 sur les

parasites animaux et/ou sur leur lieu de vie.

**10.** Utilisation de composés de formule (I) selon la revendication 1 ou de formulations agrochimiques selon l'une quelconque des revendications 7 et 8 pour la lutte contre des parasites animaux.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2010125985 A
- WO 2012074135 A
- WO 2012086848 A
- WO 2013018928 A
- WO 2013191113 A
- WO 2014142292 A
- WO 2014148451 A
- WO 2015000715 A
- WO 2015002211 A
- WO 2015121136 A
- WO 2016124563 A
- WO 2016124557 A
- EP 2016068599 W
- EP 15187145
- WO 2015133603 A
- WO 2015198859 A
- WO 2015071180 A
- WO 2015091945 A
- WO 2016005263 A
- WO 2015198817 A
- WO 2016041819 A
- WO 2016039441 A
- WO 2016026848 A
- WO 2016023954 A
- WO 2016020286 A
- WO 2016046071 A
- WO 2009131237 A
- WO 2011043404 A
- WO 2011040629 A
- US 5576335 A
- US 200369257 B
- WO 200665703 A
- US 2010234604 A
- WO 201261926 A
- WO 201286848 A
- US 200625633 B
- US 2006111591 A
- US 2820062 A
- WO 2010071819 A
- US 2009203705 A
- US 2012258951 A
- WO 20133298 A
- WO 201175643 A
- EP 2671582 A
- US 2012302573 A
- WO 200566177 A
- WO 201288411 A
- WO 2012168733 A
- WO 2014187762 A
- US 5374646 A
- WO 2010091310 A
- WO 201266061 A
- WO 2013099041 A
- WO 2005100353 A
- WO 201343518 A
- EP 2168965 A
- WO 2006043635 A
- WO 2003106457 A
- WO 2006003494 A
- WO 2010052161 A
- EP 2647626 A
- WO 2004099160 A
- JP 2010018586 A
- WO 2012029672 A
- WO 2013144213 A
- WO 2010051926 A
- CN 103232431
- WO 2013050317 A1
- WO 2013162715 A2
- WO 2013162716 A2
- US 20140213448 A1
- CN 101337937 A
- CN 103109816 A
- WO 2012034403 A1
- WO 2011085575 A1
- CN 101337940 A
- CN 101715774 A
- CN 103524422 A
- CN 102391261 A
- US 20140275503 A1
- WO 2007040280 A1
- WO 2007040282 A1
- WO 2015058021 A1
- WO 2015058028 A1


### In der Beschreibung aufgeführte Nicht-Patentliteratur


- *Bioorganic and Medicinal Chemistry Letters,* 2008, vol. 18, 5023-5026
- *Chemical Communications,* 2000, vol. 13, 1163-1164

- *Journal of the American Chemical Society,* 1922, vol. 44, 1329
- *Journal of Organic Chemistry,* 2010, vol. 69, 5578
- *Chem. Rev.,* 1995, vol. 95, 2457-2483
- *Tetrahedron,* 2002, vol. 58, 9633-9695
- Metal-Catalyzed Cross-Coupling Reactions. Wiley-VCH, 2004
- Boronic Acids. Wiley-VCH, 2011
- *J. Med. Chem.,* 1988, vol. 31, 1590-1595
- *Bioorganic and Medicinal Chemistry Letters,* 2003, vol. 13, 1093-1096
- *Angewandte Chemie Int. Ed.,* 2011, vol. 50, 8944-8947
- *European Journal of Medicinal Chemistry,* 2010, vol. 45, 2214-2222
- *Journal of Medicinal Chemistry,* 2003, vol. 46, 1449-1455
- Crop Life International und in Pesticide Specifications, Manual on development and use of FAO and WHO specifications for pesticides, FAO Plant Production and Protection Papers - 173. FAO/WHO Joint Meeting on Pesticide Specifications. 2004
- **BAUR et al.** *Pesticide Science,* 1997, vol. 51, 131-152
- The Pesticide Manual. British Crop Protection Council, 2012
- *CHEMICAL ABSTRACTS,* 885026-50-6
- *CHEMICAL ABSTRACTS,* 637360-23-7
- *CHEMICAL ABSTRACTS,* 872999-66-1
- *CHEMICAL ABSTRACTS,* 1225292-17-0
- *CHEMICAL ABSTRACTS,* 1440516-42-6
- *CHEMICAL ABSTRACTS,* 792914-58-0
- *CHEMICAL ABSTRACTS,* 1204776-60-2
- *CHEMICAL ABSTRACTS,* 1363400-41-2
- *CHEMICAL ABSTRACTS,* 1461743-15-6
- *CHEMICAL ABSTRACTS,* 1226889-14-0
- *CHEMICAL ABSTRACTS,* 1449220-44-3
- *CHEMICAL ABSTRACTS,* 1332628-83-7
- *CHEMICAL ABSTRACTS,* 1477923-37-7
- *CHEMICAL ABSTRACTS,* 1105672-77-2
- *CHEMICAL ABSTRACTS,* 1232543-85-9
- *CHEMICAL ABSTRACTS,* 1268277-22-0
- *CHEMICAL ABSTRACTS,* 1233882-22-8
- *CHEMICAL ABSTRACTS,* 1108184-52-6
- *CHEMICAL ABSTRACTS,* 1542271-46-4
- *CHEMICAL ABSTRACTS,* 1370358-69-2
- *CHEMICAL ABSTRACTS,* 1181213-14-8
- *CHEMICAL ABSTRACTS,* 1253850-56-4
- *CHEMICAL ABSTRACTS,* 933798-27-7
- *CHEMICAL ABSTRACTS,* 934001-66-8
- *CHEMICAL ABSTRACTS,* 1477919-27-9
- Pesticide Manual. British Crop Protection Council
- *CHEMICAL ABSTRACTS,* 129531-12-0
- *CHEMICAL ABSTRACTS,* 71526-07-3
- *CHEMICAL ABSTRACTS,* 52836-31-4
- **R. WEGLER.** Chemie der Pflanzenschutz- und Schädlingsbekämpfungsmittel. Springer Verlag, 1970, vol. 2, 401-412
- Database. 564025